(19) Europäisches Patentamt
European Patent Office
Office européen des brevets

(11) EP 3 781 200 B1

(12) **EUROPEAN PATENT SPECIFICATION**

(45) Date of publication and mention of the grant of the patent:
**29.05.2024 Bulletin 2024/22**

(21) Application number: **19723014.7**

(22) Date of filing: **16.04.2019**

(51) International Patent Classification (IPC):
***A61K 39/395*** *(2006.01)* ***A61K 31/28*** *(2006.01)*
***C07K 16/28*** *(2006.01)* ***C07K 16/30*** *(2006.01)*
***C07K 16/32*** *(2006.01)* ***C07K 16/44*** *(2006.01)*
***A61K 47/68*** *(2017.01)* ***A61P 35/00*** *(2006.01)*

(52) Cooperative Patent Classification (CPC):
(C-Sets available)
**C07K 16/44; A61K 39/39558; A61K 51/0495; A61K 51/109; A61K 51/1096; A61P 35/00; C07K 16/2887; C07K 16/3007; C07K 16/32;**
A61K 2039/505; A61K 2039/545; C07K 2317/24; C07K 2317/31; C07K 2317/34; C07K 2317/52;

(Cont.)

(86) International application number:
**PCT/EP2019/059856**

(87) International publication number:
**WO 2019/201959 (24.10.2019 Gazette 2019/43)**

(54) **ANTIBODIES FOR CHELATED RADIONUCLIDES**
ANTIKÖRPER FÜR CHELATIERTE RADIONUKLIDE
ANTICORPS POUR RADIONUCLÉIDES CHÉLATÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **16.04.2018 US 201862658468 P**

(43) Date of publication of application:
**24.02.2021 Bulletin 2021/08**

(60) Divisional application:
**24169799.4**

(73) Proprietor: **F. Hoffmann-La Roche AG**
**4070 Basel (CH)**

(72) Inventors:
- **KLEIN, Christian**
  **8952 Zuerich (CH)**
- **UMANA, Pablo**
  **8952 Zuerich (CH)**
- **HAAS, Alexander**
  **82377 Penzberg (DE)**
- **WEISER, Barbara**
  **82377 Penzberg (DE)**
- **LIPSMEIER, Florian**
  **4070 Basel (CH)**
- **GEORGES, Guy**
  **82377 Penzberg (DE)**
- **FENN, Sebastian**
  **82377 Penzberg (DE)**
- **MOELLEKEN, Joerg**
  **82377 Penzberg (DE)**
- **BORMANN, Felix**
  **82377 Penzberg (DE)**
- **MATSCHEKO, Daniela**
  **82377 Penzberg (DE)**

(74) Representative: **Mewburn Ellis LLP**
**Aurora Building**
**Counterslip**
**Bristol BS1 6BX (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 781 200 B1

(56) References cited:
**WO-A1-2017/194438 WO-A2-2016/130539**

- **YAZAKI PAUL J ET AL: "A series of anti-CEA/anti-DOTA bispecific antibody formats evaluated for pre-targeting: comparison of tumor uptake and blood clearance.", PROTEIN ENGINEERING, DESIGN & SELECTION : PEDS, vol. 26, no. 3, March 2013 (2013-03), pages 187-193, XP002792685, ISSN: 1741-0134**
- **CHEAL SARAH ET AL: "Comparative efficacy and toxicity of Lu-177-vs Y-90-theranostic anti-HER2/anti-DOTA(metal) pretargeted radioimmunotherapy (anti-HER2 DOT-APRIT) of HER2-expressing breast cancer xenografts with curative intent.", JOURNAL OF NUCLEAR MEDICINE, vol. 58, no. Suppl. 1, 54, 1 May 2017 (2017-05-01), XP002792686, & ANNUAL MEETING OF THE SOCIETY-OF-NUCLEAR-MEDICINE-AND-MOLECULAR- IMAGING (SNMMI); DENVER, CO, USA; JUNE 10 -14, 2017 & Cheal et al.: "Comparative efficacy and toxicity of 177Lu- vs 90Y-theranostic anti-HER2/anti-DOTA(metal) pretargeted radioimmunotherapy (anti-HER2 DOTA-PRIT) of HER2-expressing breast cancer xenografts with curative intent.", J Nucl Med May 1, 2017 vol. 58 no. supplement 1 54 , 1 May 2017 (2017-05-01), Retrieved from the Internet: URL:http://jnm.snmjournals.org/content/58/supplement_1/54 [retrieved on 2019-07-05]**
- **DAMIAN J. GREEN ET AL: "Comparative Analysis of Bispecific Antibody and Streptavidin-Targeted Radioimmunotherapy for B-cell Cancers", CANCER RESEARCH, vol. 76, no. 22, 2 September 2016 (2016-09-02), pages 6669-6679, XP055590462, US ISSN: 0008-5472, DOI: 10.1158/0008-5472.CAN-16-0571**
- **CHRISTIAN KLEIN ET AL: "The use of CrossMAb technology for the generation of bi- and multispecific antibodies", MABS, vol. 8, no. 6, 10 June 2016 (2016-06-10), pages 1010-1020, XP055375293, US ISSN: 1942-0862, DOI: 10.1080/19420862.2016.1197457**
- **P. J. YAZAKI ET AL: "Humanization of the anti-CEA T84.66 antibody based on crystal structure data", PROTEIN ENGINEERING DESIGN AND SELECTION, vol. 17, no. 5, 1 January 2004 (2004-01-01), pages 481-489, XP055003918, ISSN: 1741-0126, DOI: 10.1093/protein/gzh056**
- **LARSON STEVEN M ET AL: "Radioimmunotherapy of human tumours.", HHS Public Access Author Manuscript , vol. 15, no. 6 June 2015 (2015-06), pages 1-35, XP002792687, DOI: 10.1038/nrc3925 Retrieved from the Internet: URL:https://www.ncbi.nlm.nih.gov/pmc/articles/PMC4798425/pdf/nihms767047.pdf [retrieved on 2019-07-05]**

Remarks:
The complete document including Reference Table(s) and the Sequence Listing(s) can be downloaded from the EPO website

(52) Cooperative Patent Classification (CPC): (Cont.)
C07K 2317/64; C07K 2317/92; C07K 2317/94

C-Sets
**A61K 39/39558, A61K 2300/00**

## Description

## FIELD OF THE INVENTION

The present application relates to antibodies which bind specifically to chelated radionuclides, including bispecific antibodies. It further relates to the use of such bispecific antibodies in applications such as radioimmunoimaging and radioimmunotherapy. It additionally relates to clearing agents and compositions useful in such methods.

## BACKGROUND

Monoclonal antibodies have been developed to target drugs to cancer cells. By conjugating a toxic agent to an antibody which binds to a tumour-associated antigen, there is the potential to provide more specific tumour killing with less damage to surrounding tissues.

In pre-targeted radioimmunotherapy (PRIT), use is made of an antibody construct which has affinity for the tumour-associated antigen on the one hand and for a radiolabelled compound on the other. In a first step, the antibody is administered and localizes within the tumour. Subsequently, the radiolabelled compound is administered. Because the radiolabelled compound is small, it can be delivered quickly to the tumour and is fast-clearing, which reduces radiation exposure outside of the tumour (Goldenberg et al Theranostics 2012, 2(5), 523-540). Besides the direct cell-killing effect, PRIT may also act as an inducer of immunogenic cell death and a potential combination partner for cancer immunotherapy and endogenous vaccination approaches. A similar procedure can also be used for imaging. Pre-targeting can make use of a bispecific antibody or systems using avidin-biotin, although the latter has the disadvantage that avidin/streptavidin is immunogenic.

Radionuclides for use in PRIT are commonly in the form of a chelate loaded with the radionuclide of interest.

Su et al (Nucl Med Biol 2005 32:741-747) evaluated an antibody pre-targeting system with mAb-streptavidin and DOTA-biotin. It was found that radiolabelled $^{212}$Pb-DOTA-biotin was not stable, with greater than 30% of free $^{212}$Bi (a decay product of $^{212}$Pb) released from $^{212}$Pb-DOTA.

WO2010/099536 describes a bispecific antibody which is capable of binding DOTA complexes of yttrium, lutetium and gadolinium. However, DOTA does not stably bind all radionuclides, and can exhibit slow complex formation rates (Yong and Brechbiel, Dalton Trans. 2001 June 21; 40(23)6068-6076). Failure of the chelator to stably bind a radionuclide creates the risk of reducing delivery of radiation to the tumour while increasing toxicity.

## SUMMARY OF THE INVENTION

The subject-matter of the invention is defined by the appended claims. Any aspects, embodiments and examples that fall outside the scope of the claims are provided only for illustration.

The present invention provides antibodies that bind to a metal chelate comprising DOTAM and lead (Pb). DOTAM is able to chelate Pb in a stable manner, to form a Pb[DOTAM] complex.

The antibodies of the present invention bind to a chelate comprising DOTAM and Pb, where the Pb may be either a stable (non-radio) isotope or a radioisotope. Radioisotopes of lead are useful in applications such as radioimmunoimaging and radioimmunotherapy.

Preferably, antibodies of the present invention have extremely high affinity to the Pb-DOTAM chelate, in the pM to fM range.

The antibodies additionally bind to bismuth (Bi) chelated by DOTAM. $^{212}$Pb is the parental radionuclide of $^{212}$Bi and can serve as an *in vivo* generator of $^{212}$Bi. The ability of the antibodies to bind chelated Bi as well as chelated Pb increases their utility in applications such as radioimmunotherapy, where a Bi isotope is generated as a decay product from a Pb isotope. In some embodiments, the antibodies may bind to both a Bi-DOTAM chelate and to a Pb-DOTAM chelate with very high affinity, in the pM to fM range.

Furthermore, the present antibodies are optionally or preferably selective for a Bi-DOTAM chelate and a Pb-DOTAM chelate as compared to other chelator-metal complexes, such as a Cu-DOTAM chelate.

In one aspect, the present invention provides an antibody comprising an antigen binding site specific for DOTAM-lead (Pb) chelate, wherein said antigen binding site comprises at least:

a) a heavy chain CDR1;

b) heavy chain CDR2 comprising the amino acid sequence FIGSRGDTYYASWAKG (SEQ ID NO:2), or a variant thereof having up to 1, 2, or 3 substitutions in SEQ ID NO: 2, wherein these substitutions do not include Phe50, Asp56 and Tyr58, and optionally also do not include Gly52 and/or Arg54;

c) heavy chain CDR3 comprising the amino acid sequence ERDPYGGGAYPPHL (SEQ ID NO:3), or a variant thereof having up to 1, 2, or 3 substitutions in SEQ ID NO: 3, wherein these substitutions do not include Glu95, Arg96, Asp97, Pro98, and optionally also do not include Ala100C, Tyr1000, and/or Pro100E and/or optionally also do not include Tyr99;

d) light chain CDR1 comprising the amino acid sequence QSSHSVYSDNDLA (SEQ ID NO:4) or a variant thereof having up to 1, 2, or 3 substitutions in SEQ ID NO: 4, wherein these substitutions do not include Tyr28 and Asp32;

e) a light chain CDR2;

f) light chain CDR3 comprising the amino acid sequence LGGYDDESDTYG (SEQ ID NO:6) or a variant thereof having up to 1, 2, or 3 substitutions in SEQ ID NO: 6,

wherein these substitutions do not include Gly91, Tyr92, Asp93, Thr95c and Tyr96,

wherein numbering is according to Kabat.

In some embodiments, heavy chain CDR1 and light chain CDR2 are optionally:

i) a heavy chain CDR1 comprising the amino acid sequence GFSLSTYSMS (SEQ ID NO:1) or a variant thereof having up to 1, 2, or 3 substitutions in SEQ ID NO: 1;
ii) a light chain CDR2 comprising the amino acid sequence QASKLAS (SEQ ID NO: 5) or a variant thereof having at least 1, 2 or 3 substitutions in SEQ ID NO: 5, optionally not including Gln50.

In some embodiments, the antibody according to the present invention binds to the same epitope, or an overlapping epitope, of a chelated radionuclide as that bound by an antibody disclosed herein.
In some embodiments, the antibody binds to the same epitope, or an overlapping epitope, as the epitope bound by Fab PRIT-0213 or PRIT-0214. For instance, the antibody may bind to the same epitope, or an overlapping epitope, as:

i) an antibody having a heavy chain variable domain comprising an amino acid sequence of SEQ ID NO: 7 and a light chain variable domain comprising an amino acid sequence of SEQ ID NO: 8; or
i) an antibody having a heavy chain variable domain comprising an amino acid sequence of SEQ ID NO: 9 and a light chain variable domain comprising an amino acid sequence of SEQ ID NO: 10.

In one embodiment, the antigen binding site comprises at least one, two, three four, five, or six CDRs selected from:

a) heavy chain CDR1 comprising the amino acid sequence GFSLSTYSMS (SEQ ID NO:1);
b) heavy chain CDR2 comprising the amino acid sequence FIGSRGDTYYASWAKG (SEQ ID NO:2);
c) heavy chain CDR3 comprising the amino acid sequence ERDPYGGGAYPPHL (SEQ ID NO:3);
d) light chain CDR1 comprising the amino acid sequence QSSHSVYSDNDLA (SEQ ID NO:4);
e) light chain CDR2 comprising the amino acid sequence QASKLAS (SEQ ID NO: 5);
f) light chain CDR3 comprising the amino acid sequence LGGYDDESDTYG (SEQ ID NO:6).

Optionally, the antibody in any of the aspects described above is human, chimeric or humanized.
Optionally, the antigen binding site may comprise a heavy chain variable domain comprising an amino acid sequence selected from the group consisting of SEQ ID NO: 7 and SEQ ID NO 9, or a variant thereof comprising an amino acid sequence having at least 90, 91, 92, 93, 94, 95, 96, 97, 98, or 99% identity to SEQ ID NO: 7 or SEQ ID NO: 9.
Optionally, the antigen binding site may comprise a light chain variable domain comprising an amino acid sequence selected from the group consisting of SEQ ID NO: 8 and SEQ ID NO: 10, or a variant thereof comprising an amino acid sequence having at least 90, 91, 92, 93, 94, 95, 96, 97, 98, or 99% identity to SEQ ID NO: 8 or 10.
Optionally, the antigen binding site specific for the Pb-DOTAM chelate may comprise a heavy chain variable domain comprising an amino acid sequence selected from the group consisting of SEQ ID NO: 7 or SEQ ID NO: 9, or a variant thereof as defined above, and a light chain variable domain comprising an amino acid sequence selected from the group consisting of SEQ ID NO: 8 or SEQ ID NO: 10, or a variant thereof as defined above. For example, the antigen binding site specific for the Pb-DOTAM chelate may comprise a heavy chain variable domain comprising the amino acid sequence of SEQ ID NO: 7 or a variant thereof, and a light chain variable domain comprising the amino acid sequence of SEQ ID NO: 8 or a variant thereof.

In another embodiment, it may comprise a heavy chain variable domain comprising the amino acid sequence of SEQ ID NO: 9 or a variant thereof and a light chain variable domain comprising the amino acid sequence of SEQ ID NO: 10 or a variant thereof.

The antibody may be in any format, including whole antibodies and antibody fragments. The antibody can be monospecific. In this form, the antibody finds utility, for instance, in sorting and purification schemes, e.g., to separate successfully radiolabelled moieties.

In some aspects, the antibody that specifically binds to the Pb-DOTAM chelate is coupled to a cell binding agent/targeting moiety to produce a targeted agent. Such an agent is useful for instance, in pre-targeted radioimmunotherapy or pre-targeted radioimmunoimaging.

The coupling may preferably be by expression as a fusion polypeptide or protein. Fusion may be direct or via a linker. The fusion polypeptide or protein may be produced recombinantly, avoiding any need for conjugation chemistry.

In some embodiments, the targeting moiety (comprising the antigen binding site for the target) is an antibody or fragment thereof. That is, in some embodiments, the antibody described above may be in the form of a multispecific (e.g., bispecific) antibody, as discussed further below.

In another aspect, the present invention further relates to a multispecific antibody/antibody complex suitable for targeting a Pb-DOTAM chelate to a target cell.

Accordingly, in another aspect, the present invention relates to a bispecific or multispecific antibody that specifically binds both to the Pb-DOTAM chelate and to a target antigen, e.g., an antigen expressed on the surface of a target cell. The bispecific antibody comprises at least one antigen binding site specific for DOTAM-chelated lead and at least one antigen binding site for the target antigen.

The antigen binding site specific for the Pb-DOTAM chelate is according to any of the embodiments described above.

The target antigen may be any antigen as discussed further herein, e.g., any tumour-specific antigen. In some embodiments it may be a protein or polypeptide expressed by a pathogen such as a prokaryote or a virus.

In some embodiments, the tumour-associated antigen may be CEA (carcinoembryonic antigen). That is, in some embodiments, the bispecific antibody may comprise at least one antigen binding site specific for the Pb-DOTAM chelate and at least one antigen binding site specific for CEA. CEA is advantageous in the context of the present invention because it is relatively slowly internalized, and thus a high percentage of the bispecific antibody will remain available on the surface of the cell after initial treatment, for binding to the radionuclide. Other low internalising targets/tumour associated antigens may also be preferred and are described herein. Other examples of tumour-associated antigen which may be useful in the present invention include CD20 or HER2.

In some embodiments, where the target antigen is CEA, the antigen binding site specific for CEA may comprise a heavy chain comprising at least one, two or three heavy chain CDRs, wherein:

d) heavy chain CDR1 comprises the amino acid sequence of SEQ ID NO: 11;
e) heavy chain CDR2 comprises the amino acid sequence of SEQ ID NO: 12;
f) heavy chain CDR3 comprises the amino acid sequence of SEQ ID NO: 13;
and/or the antigen binding site specific for CEA may comprise a light chain comprising at least one, two or three light chain CDRs, wherein:

  a) light chain CDR1 comprises the amino acid sequence SEQ ID NO: 14;
  b) light chain CDR2 comprises the amino acid sequence SEQ ID NO:15;
  c) light chain CDR3 comprises the amino acid sequence SEQ ID NO: 16.

In some embodiments, the antigen binding site for CEA may comprise at least one, two, three, four, five, or six (i.e., all) of the CDRs selected from:

a) heavy chain CDR1 comprising the amino acid sequence of SEQ ID NO: 11;
b) heavy chain CDR2 comprising the amino acid sequence of SEQ ID NO: 12;
c) heavy chain CDR3 comprising the amino acid sequence of SEQ ID NO: 13;
d) light chain CDR1 comprising the amino acid sequence of SEQ ID NO: 14;
e) light chain CDR2 comprising the amino acid sequence of SEQ ID NO: 15;
f) light chain CDR3 comprising the amino acid sequence of SEQ ID NO: 16.

In some embodiments, the antigen binding site for CEA may comprise a heavy chain variable domain comprising an amino acid sequence of SEQ ID NO: 17, or a variant thereof comprising an amino acid sequence having at least 90, 91, 92, 93, 94, 95, 96, 97, 98, or 99% identity to SEQ ID NO: 17.

Optionally, the antigen binding site may comprise a light chain variable domain comprising an amino acid

sequence of SEQ ID NO: 18, or a variant thereof comprising an amino acid sequence having at least 90, 91, 92, 93, 94, 95, 96, 97, 98, or 99% identity to SEQ ID NO: 18.

Optionally, the antigen binding site specific for CEA may comprise a heavy chain variable domain comprising an amino acid sequence of SEQ ID NO: 17 or a variant thereof, and a light chain variable domain comprising an amino acid sequence of SEQ ID NO: 18 or a variant thereof.

Various possible formats for the bispecific antibodies or multispecific antibodies are known in the art, including those described further herein. The antibodies of the invention may adopt any of these formats. For example, in some embodiments the bispecific antibody may be bivalent, trivalent or tetravalent.

It may be preferred that the present antibodies include an Fc region. The presence of an Fc region has benefits in the context of radioimmunotherapy and radioimaging, e.g. prolonging the protein's circulating half-life and/or resulting in higher tumour uptake than may be observed with smaller fragments.

In some embodiments, where the Fc region is present, it may be preferred that the Fc region is engineered to reduce effector function. This may include substitution of one or more of Fc region residues 234, 235, 238, 265, 269, 270, 297, 327 and/or 329, e.g., one or more of 234, 235 and/or 329. In some embodiments, the Fc region may be engineered to include the substitution of Pro 329 to Gly, Leu 234 to Ala and/or Leu 235 to Ala (numbering according to EU index).

Various formats of multispecific antibodies which include an Fc domain are known.

In one embodiment, the bispecific or multispecific antibody may comprise i) an Fc domain, ii) at least one Fab, cross-Fab, Fv, scFab, or scFv fragment or a single domain antibody (VHH) comprising an antigen binding site specific for the Pb-DOTAM chelate and iii) at least one Fab, cross-Fab, Fv, scFab or scFv fragment or a single domain antibody (VHH) comprising an antigen binding site specific for the target antigen.

In some embodiments, it may be preferred that the bispecific or multispecific antibody is multivalent, for example bivalent, for the target antigen (e.g., the tumour-associated antigen). This has the advantage of increasing avidity.

In some embodiments, it may be preferred that the bispecific or multispecific antibody is monovalent for Pb-DOTAM. This reduces the risk of high molecular weight complex formation when a clearing agent is used (see further discussion below).

Thus, in some embodiments, the antibody may be trivalent: that is, bivalent for the target antigen and monovalent for Pb-DOTAM.

In one exemplary format, the bispecific or multispecific antibody may comprise a full-length antibody (e.g., an IgG) comprising a first and second antibody heavy chain and a first and second antibody light chain, wherein the first heavy chain and the first light chain assemble to form an antigen binding site for the first antigen, and wherein the second heavy chain and second light chain assemble to form an antigen binding site for the second antigen. Optionally, further antigen binding moieties may be fused e.g., via a polypeptide linker to the N- or C- terminus of the first and/or second heavy chain, to increase the valency for one or both antigens. For instance, a further antigen binding moiety for the first antigen may be fused to the N-terminus of one or both of the heavy chain molecules.

In another exemplary format, the bispecific or multispecific antibody may comprise a full length antibody (e.g., an IgG) comprising an antigen binding site for a first antigen (e.g., which may be divalent for the first antigen), and further comprises at least one antigen binding moiety specific for the second antigen. In various embodiments, the antigen binding moiety may be a Fab fragment, a crossover-Fab molecule, a scFab, an Fv molecule, an scFv, or a single domain antibody (VHH) or may be part of a second full-length antibody. For instance, the antibody may comprise a full length antibody comprising an antigen binding site for the first antigen, and further comprise at least a second heavy chain variable domain and a second light chain variable domain which together form an antigen binding site for a second antigen. Either the first or the second antigen is the Pb-DOTAM chelate, and the other antigen is the target antigen.

In some embodiments of the formats herein, the second antigen is the Pb-DOTAM chelate and the first antigen is the target, e.g., a tumour-associated antigen (CEA, CD20 or ERBB2 in some embodiments).

In another exemplary format, the bispecific or multispecific antibody may comprise a full length antibody comprising an antigen binding site for the first antigen (e.g., which may be divalent for the first antigen), wherein the N- or C-terminus of one of the heavy chains is linked via a polypeptide linker to a first polypeptide and wherein the first polypeptide associates with a second polypeptide to form a Fab or a cross-Fab comprising a binding site for the second antigen. For instance, this format may comprise:

i) a first polypeptide consisting of a VH domain and a CH1 domain, which is associated with a second polypeptide consisting of a VL and CL domain; or

ii) a first polypeptide consisting of a VL domain and a CH1 domain, which is associated with a second polypeptide consisting of a VH and CL domain; or

iii) a first polypeptide consisting of a VH domain and a CL domain, which is associated with a second polypeptide consisting of a VL and CH1 domain; such that the first and second polypeptide together form an antigen binding site for a second antigen.

In some embodiments, the fusion may be at the N-terminus of one of the heavy chains of the full length antibody.

In another particular embodiment, the antibody may be a bispecific antibody comprising:

a) a full length antibody specifically binding a first antigen and consisting of two antibody heavy chains and two antibody light chains;

b) a polypeptide consisting of

i) an antibody heavy chain variable domain (VH); or

ii) an antibody heavy chain variable domain (VH) and an antibody heavy constant domain (CH1); or

iii) an antibody heavy chain variable domain (VH) and an antibody light chain constant domain (CL);

wherein said polypeptide is fused with the N-terminus of the VH domain via a peptide linker to the C-terminus of one of the two heavy chains of said full-length antibody;

c) a polypeptide consisting of

i) an antibody light chain variable domain (VL); or

ii) an antibody light chain variable domain (VL) and an antibody light chain constant domain (CL) or

iii) an antibody light chain variable domain (VL) and an antibody heavy chain constant domain (CH1);

wherein said polypeptide is fused with the N-terminus of the VL domain via a peptide linker to the C-terminus of the other of the two heavy chains of said full-length antibody;

and wherein the antibody heavy chain variable domain of the peptide under (b) and the antibody light chain variable domain of the peptide under (c) together form an antigen-binding site to a second antigen.

In this format, either the first or the second antigen may be the Pb-DOTAM chelate. The other will be the target antigen e.g., a tumour-associated antigen.

In some embodiments, the second antigen is the Pb-DOTAM chelate and the first antigen is the target, e.g., a tumour-associated antigen (CEA, CD20 or ERBB2 in some embodiments).

The antibody described above may be trivalent. In another possible embodiment, further antigen binding moieties may be fused to increase the valency for one or both antigens, as discussed further herein.

Optionally said linker (and any linker as discussed herein) may be a peptide of at least 5 amino acids, preferably between 25 and 50 amino acids. The linker may be a rigid linker or a flexible linker. In some embodiments, it is a flexible comprising or consisting of Thr, Ser, Gly and/or Ala residues. For example, it may comprise or consist of Gly and Ser residues. In some embodiments it may have a repeating motif such as $(Gly\text{-}Gly\text{-}Gly\text{-}Gly\text{-}Ser)_n$, where n is for instance 1, 2, 3, 4, 5, 6, 7, 8, 9 or 10. In some embodiments, the linker may be or may comprise the sequence GGGGSGGGGSGGGGSGGGGS (SEQ ID NO: 26). Other linkers may be used and could be identified by the skilled person.

Further details of this antibody format are provided in WO2010/115589 A1 (Roche Glycart AG).

Optionally,

i) in the constant domain CL of the first light chain of the full length antibody under a) the amino acid at position 124 is substituted independently by lysine (K), arginine (R) or histidine (H) (numbering according to Kabat) (in one preferred embodiment independently by lysine (K) or arginine (R)), and in the constant domain CH1 of the first heavy chain under a) the amino acid at position 147 or the amino acid at position 213 is substituted independently by glutamic acid (E) or aspartic acid (D) (numbering according to Kabat EU index); or

ii) in the constant domain CL of the second light chain under b) the amino acid at position 124 is substituted independently by lysine (K), arginine (R) or histidine (H) (numbering according to Kabat) (in one preferred embodiment independently by lysine (K) or arginine (R)), and in the constant domain CH1 of the second heavy chain under b) the amino acid at position 147 or the amino acid at position 213 is substituted independently by glutamic acid (E) or aspartic acid (D) (numbering according to Kabat EU index).

In one embodiment, the bispecific antibody of the present invention may have the trivalent structure as set out above, and may comprise:

a) a full length antibody specifically binding CEA and consisting of two antibody heavy chains and two antibody light chains;

wherein the heavy chains have at least 90, 91, 92, 93, 94, 95, 96, 97, 98, 99 or 100% identity to the heavy chain of amino acids 1- 450 (inclusive, using sequential numbering) of SEQ ID NO: 22 or 23 (i.e., to the portion of the

sequence preceding the linker);
and wherein the light chains have at least 90, 91, 92, 93, 94, 95, 96, 97, 98, 99 or 100% identity to the light chain of SEQ ID NO 21; and/or

b) a polypeptide consisting of

i) an antibody heavy chain variable domain (VH) having at least 90, 91, 92, 93, 94, 95, 96, 97, 98, 99 or 100% identity to the heavy chain variable domain of SEQ ID NO: 7; or
ii) said antibody heavy chain variable domain (VH) having at least 90, 91, 92, 93, 94, 95, 96, 97, 98, 99 or 100% identity to the heavy chain variable of SEQ ID NO: 7 and an antibody heavy chain constant domain (CH1); or
iii) said antibody heavy chain variable domain (VH) having at least 90, 91, 92, 93, 94, 95, 96, 97, 98, 99 or 100% identity to the heavy chain variable of SEQ ID NO: 7 and an antibody light chain constant domain;

wherein said polypeptide is fused with the N-terminus of the VH domain via a peptide linker to the C-terminus of one of the two heavy chains of said full-length antibody; and/or
c) a polypeptide consisting of

i) an antibody light chain variable domain (VL) having at least 90, 91, 92, 93, 94, 95, 96, 97, 98, 99 or 100% identity to the light chain variable domain of SEQ ID NO: 8; or
ii) said antibody light chain variable domain (VL) having at least 90, 91, 92, 93, 94, 95, 96, 97, 98, 99 or 100% identity to the light chain variable of SEQ ID NO: 8 and an antibody light chain constant domain (CL); or
iii) said antibody light chain variable domain (VL) having at least 90, 91, 92, 93, 94, 95, 96, 97, 98, 99 or 100% identity to the light chain variable of SEQ ID NO: 8 and an antibody heavy chain constant domain (CH1);

wherein said polypeptide is fused with the N-terminus of the VL domain via a peptide linker to the C-terminus of the other of the two heavy chains of said full-length antibody;

wherein the antibody heavy chain variable domain of the peptide under (b) and the antibody light chain variable domain of the peptide under (c) together form an antigen-binding site to the Pb-DOTAM chelate.

In one example, one of the heavy chains of the full length antibody comprises the so-called "knob mutations" (T366W and optionally one of S354C or Y349C, preferably S354C) and the other comprises the so-called "hole mutations" (T366S, L368A and Y407V and optionally Y349C or S354C, preferably Y349C) (see, e.g., Carter, P. et al., Immunotechnol. 2 (1996) 73) according to EU index numbering.

In some embodiments, the two antibody heavy chains under (a) comprise i) a first antibody heavy chain which has at least 90, 91, 92, 93, 94, 95, 96, 97, 98, 99 or 100% identity to the heavy chain of amino acids 1- 450 (inclusive, using sequential numbering) of SEQ ID NO: 23 (i.e., the sequence preceding the linker), and which has C at position 349, S at position 366, A at position 368 and V at position 407 (EU numbering); and ii) a second antibody heavy chain which has at least 90, 91, 92, 93, 94, 95, 96, 97, 98, 99 or 100% identity to the heavy chain of amino acids 1-450 (inclusive, using sequential numbering) of SEQ ID NO: 22 which has C at position 354 and W at position 366 (EU numbering).

The herein mentioned "fixed" residues are "knob-into-hole" mutations in CH3 or are other residues such as disulphide bridge forming residues, which pair with corresponding residues on CH3 of the other heavy chain to favour the formation of the desired molecule. Possible residues which may be present on the other heavy chain can be derived from the sequences of table 2 (e.g., sequences 19 and 20 or 22 and 23). For instance, in one embodiment if the first antibody heavy chain has C at position 349, then the second antibody heavy chain has C at 354.

Optionally, the linker is as described above.

In another embodiment, the bispecific antibody of the present invention may have the trivalent structure as set out above, and may comprise:

a) a full length antibody specifically binding CEA and consisting of two antibody heavy chains and two antibody light chains;

wherein the heavy chains have at least 90, 91, 92, 93, 94, 95, 96, 97, 98, 99 or 100% identity to the heavy chain of amino acids 1 to 450 of SEQ ID NO: 19 or 20 (inclusive and based on sequential numbering: i.e., the sequence preceding the linker)
and wherein the light chains have at least 90, 91, 92, 93, 94, 95, 96, 97, 98, 99 or 100% identity to the light chain of SEQ ID NO: 21;

b) a polypeptide consisting of

i) an antibody heavy chain variable domain (VH) having at least 90, 91, 92, 93, 94, 95, 96, 97, 98, 99 or 100% identity to the heavy chain variable domain of SEQ ID NO: 9; or
ii) said antibody heavy chain variable domain (VH) having at least 90, 91, 92, 93, 94, 95, 96, 97, 98, 99 or 100% identity to the heavy chain variable of SEQ ID NO: 9 and an antibody heavy chain constant domain (CH1); or
iii) said antibody heavy chain variable domain (VH) having at least 90, 91, 92, 93, 94, 95, 96, 97, 98, 99 or 100% identity to the heavy chain variable of SEQ ID NO: 9 and an antibody light chain constant domain;

wherein said polypeptide is fused with the N-terminus of the VH domain via a peptide linker to the C-terminus of one of the two heavy chains of said full-length antibody;
c) a polypeptide consisting of

i) an antibody light chain variable domain (VL) having at least 90, 91, 92, 93, 94, 95, 96, 97, 98, 99 or 100% identity to the light chain variable domain of SEQ ID NO: 10; or
ii) said antibody light chain variable domain (VL) having at least 90, 91, 92, 93, 94, 95, 96, 97, 98, 99 or 100% identity to the light chain variable of SEQ ID NO: 10 and an antibody light chain constant domain (CL), or
iii) said antibody light chain variable domain (VL) having at least 90, 91, 92, 93, 94, 95, 96, 97, 98, 99 or 100% identity to the light chain variable of SEQ ID NO: 10 and an antibody heavy chain constant domain (CH1),

wherein said polypeptide is fused with the N-terminus of the VL domain via a peptide linker to the C-terminus of the other of the two heavy chains of said full-length antibody;
and wherein the antibody heavy chain variable domain of the peptide under (b) and the antibody light chain variable domain of the peptide under (c) together form an antigen-binding site to the Pb-DOTAM chelate.

As described above, in any of the above embodiments, one of the heavy chains of the full length antibody may comprise the so-called "knob mutations" (T366W and optionally one of S354C or Y349C, preferably S354C) and the other comprises the so-called "hole mutations" (T366S, L368A and Y407V and optionally Y349C or S354C, preferably Y349C) (see, e.g., Carter, P. et al., Immunotechnol. 2 (1996) 73) according to EU index numbering.
In one embodiment, the two antibody heavy chains under (a) may comprise i) a first antibody heavy chain which has at least 90, 91, 92, 93, 94, 95, 96, 97, 98, 99 or 100% identity to the heavy chain of amino acids 1- 450 of SEQ ID NO: 22, which has C at position 354 and W at position 366 (EU numbering); and
ii) a second antibody heavy chain which has at least 90, 91, 92, 93, 94, 95, 96, 97, 98, 99 or 100% identity to the heavy chain of amino acids 1-450 of SEQ ID NO: 23 and which has C at position 349, S at position 366, A at position 368 and V at position 407 (EU numbering).
Optionally, the linker is as described above.
In another embodiment, the bispecific antibody comprises:

i) a first heavy chain having an amino acid sequence having at least 90, 91, 92, 93, 94, 95, 96, 97, 98, 99 or 100% identity to the heavy chain of SEQ ID NO: 22,
ii) a second heavy chain having at least 90, 91, 92, 93, 94, 95, 96, 97, 98, 99 or 100% identity to the heavy chain of SEQ ID NO: 23,
iii) two antibody light chains having at least 90, 91, 92, 93, 94, 95, 96, 97, 98, 99 or 100% identity to the light chain of SEQ ID NO: 21.

In yet another embodiment, the bispecific antibody is the molecule referred to herein as PRIT-0213, comprising

i) a first heavy chain having the amino acid sequence of SEQ ID NO: 22;
ii) a second heavy chain having the amino acid sequence of SEQ ID NO: 23; and
iii) two antibody light chains having the amino acid sequence of SEQ ID NO: 21.

In another embodiment, the bispecific antibody comprises:

i) a first heavy chain having an amino acid sequence having at least 90, 91, 92, 93, 94, 95, 96, 97, 98, 99 or 100% identity to the heavy chain of SEQ ID NO: 19,
ii) a second heavy chain having at least 90, 91, 92, 93, 94, 95, 96, 97, 98, 99 or 100% identity to the heavy chain of SEQ ID NO: 20,
iii) two antibody light chains having at least 90, 91, 92, 93, 94, 95, 96, 97, 98, 99 or 100% identity to the light chain

of SEQ ID NO: 21.

In yet another embodiment, the bispecific antibody is the molecule referred to herein as PRIT-0214, comprising

i) a first heavy chain having the amino acid sequence of SEQ ID NO: 19;
ii) a second heavy chain having the amino acid sequence of SEQ ID NO: 20; and
iii) two antibody light chains having the amino acid sequence of SEQ ID NO: 21.

In one embodiment, the bispecific antibody of the present invention may have the trivalent structure as set out above, and may comprise:

a) a full length antibody specifically binding ERBB2 and consisting of two antibody heavy chains and two antibody light chains;

wherein the heavy chains have at least 90, 91, 92, 93, 94, 95, 96, 97, 98, 99 or 100% identity to the heavy chain of amino acids 1- 449 (inclusive, using sequential numbering) of SEQ ID NO: 36 or 37 (i.e., to the portion of the sequence preceding the linker);
and wherein the light chains have at least 90, 91, 92, 93, 94, 95, 96, 97, 98, 99 or 100% identity to the light chain of SEQ ID NO 38; and/or

b) a polypeptide consisting of

i) an antibody heavy chain variable domain (VH) having at least 90, 91, 92, 93, 94, 95, 96, 97, 98, 99 or 100% identity to the heavy chain variable domain of SEQ ID NO: 7 or 9 (in some embodiments, preferably 7); or
ii) said antibody heavy chain variable domain (VH) having at least 90, 91, 92, 93, 94, 95, 96, 97, 98, 99 or 100% identity to the heavy chain variable of SEQ ID NO: 7 or 9 (in some embodiments, preferably 7)and an antibody heavy chain constant domain (CH1); or
iii) said antibody heavy chain variable domain (VH) having at least 90, 91, 92, 93, 94, 95, 96, 97, 98, 99 or 100% identity to the heavy chain variable of SEQ ID NO: 7 or 9 (in some embodiments, preferably 7) and an antibody light chain constant domain (CL),

wherein said polypeptide is fused with the N-terminus of the VH domain via a peptide linker to the C-terminus of one of the two heavy chains of said full-length antibody; and/or
c) a polypeptide consisting of

i) an antibody light chain variable domain (VL) having at least 90, 91, 92, 93, 94, 95, 96, 97, 98, 99 or 100% identity to the light chain variable domain of SEQ ID NO: 8 or 10 (in some embodiments, preferably 8); or
ii) said antibody light chain variable domain (VL) having at least 90, 91, 92, 93, 94, 95, 96, 97, 98, 99 or 100% identity to the light chain variable of SEQ ID NO: 8 or 10 (in some embodiments, preferably 8) and an antibody light chain constant domain (CL); or
iii) said antibody light chain variable domain (VL) having at least 90, 91, 92, 93, 94, 95, 96, 97, 98, 99 or 100% identity to the light chain variable of SEQ ID NO: 8 or 10 (in some embodiments, preferably 8) and an antibody heavy chain constant domain (CH1);

wherein said polypeptide is fused with the N-terminus of the VL domain via a peptide linker to the C-terminus of the other of the two heavy chains of said full-length antibody;
wherein the antibody heavy chain variable domain of the peptide under (b) and the antibody light chain variable domain of the peptide under (c) together form an antigen-binding site to the Pb-DOTAM chelate.

In one example, one of the heavy chains of the full length antibody comprises the so-called "knob mutations" (T366W and optionally one of S354C or Y349C, preferably S354C) and the other comprises the so-called "hole mutations" (T366S, L368A and Y407V and optionally S354C or Y349C, preferably Y349C) (see, e.g., Carter, P. et al., Immunotechnol. 2 (1996) 73) according to EU index numbering.

In some embodiments, the two antibody heavy chains under (a) comprise i) a first antibody heavy chain which has at least 90, 91, 92, 93, 94, 95, 96, 97, 98, 99 or 100% identity to the heavy chain of amino acids 1- 449 (inclusive, using sequential numbering) of SEQ ID NO: 37 (i.e., the sequence preceding the linker), and which has C at position 349, S at position 366, A at position 368 and V at position 407 (EU numbering); and ii) a second antibody heavy chain which has at least 90, 91, 92, 93, 94, 95, 96, 97, 98, 99 or 100% identity to the heavy chain of amino acids 1-449

(inclusive, using sequential numbering) of SEQ ID NO: 36 which has C at position 354 and W at position 366 (EU numbering).

Optionally, the linker is as described above.

In another embodiment, the bispecific antibody comprises:

i) a first heavy chain having an amino acid sequence having at least 90, 91, 92, 93, 94, 95, 96, 97, 98, 99 or 100% identity to the heavy chain of SEQ ID NO: 36,
ii) a second heavy chain having at least 90, 91, 92, 93, 94, 95, 96, 97, 98, 99 or 100% identity to the heavy chain of SEQ ID NO: 37,
iii) two antibody light chains having at least 90, 91, 92, 93, 94, 95, 96, 97, 98, 99 or 100% identity to the light chain of SEQ ID NO: 38.

In yet another embodiment, the bispecific antibody is the molecule referred to herein as P1AD9827, comprising

i) a first heavy chain having the amino acid sequence of SEQ ID NO: 36;
ii) a second heavy chain having the amino acid sequence of SEQ ID NO: 37; and
iii) two antibody light chains having the amino acid sequence of SEQ ID NO: 38.

In another embodiment, the bispecific antibody of the present invention may have the trivalent structure as set out above, and may comprise:

a) a full length antibody specifically binding CD20 and consisting of two antibody heavy chains and two antibody light chains;

wherein the heavy chains have at least 90, 91, 92, 93, 94, 95, 96, 97, 98, 99 or 100% identity to the heavy chain of amino acids 1- 448 (inclusive, using sequential numbering) of SEQ ID NO: 47 or 48 (i.e., to the portion of the sequence preceding the linker);
and wherein the light chains have at least 90, 91, 92, 93, 94, 95, 96, 97, 98, 99 or 100% identity to the light chain of SEQ ID NO 49; and/or

b) a polypeptide consisting of

i) an antibody heavy chain variable domain (VH) having at least 90, 91, 92, 93, 94, 95, 96, 97, 98, 99 or 100% identity to the heavy chain variable domain of SEQ ID NO: 7 or 9 (in some embodiments, preferably 7); or
ii) said antibody heavy chain variable domain (VH) having at least 90, 91, 92, 93, 94, 95, 96, 97, 98, 99 or 100% identity to the heavy chain variable domain of SEQ ID NO: 7 or 9 (in some embodiments, preferably 7) and an antibody heavy chain constant domain (CH1),
iii) said antibody heavy chain variable domain (VH) having at least 90, 91, 92, 93, 94, 95, 96, 97, 98, 99 or 100% identity to the heavy chain variable domain of SEQ ID NO: 7 or 9 (in some embodiments, preferably 7) and an antibody light chain constant domain (CL),

wherein said polypeptide is fused with the N-terminus of the VH domain via a peptide linker to the C-terminus of one of the two heavy chains of said full-length antibody; and/or
c) a polypeptide consisting of

i) an antibody light chain variable domain (VL) having at least 90, 91, 92, 93, 94, 95, 96, 97, 98, 99 or 100% identity to the light chain variable domain of SEQ ID NO: 8 or 10(in some embodiments, preferably 8); or
ii) said antibody light chain variable domain (VL) having at least 90, 91, 92, 93, 94, 95, 96, 97, 98, 99 or 100% identity to the light chain variable domain of SEQ ID NO: 8 or 10 (in some embodiments, preferably 8) and an antibody light chain constant domain; or iii) said antibody light chain variable domain (VL) having at least 90, 91, 92, 93, 94, 95, 96, 97, 98, 99 or 100% identity to the light chain variable domain of SEQ ID NO: 8 or 10 (in some embodiments, preferably 8) and an antibody heavy chain constant domain;,

wherein said polypeptide is fused with the N-terminus of the VL domain via a peptide linker to the C-terminus of the other of the two heavy chains of said full-length antibody;
wherein the antibody heavy chain variable domain of the peptide under (b) and the antibody light chain variable domain of the peptide under (c) together form an antigen-binding site to the Pb-DOTAM chelate.

In one example, one of the heavy chains of the full length antibody comprises the so-called "knob mutations" (T366W and optionally one of S354C or Y349C, preferably S354C) and the other comprises the so-called "hole mutations" (T366S, L368A and Y407V and optionally Y349C or S354C, preferably Y349C) (see, e.g., Carter, P. et al., Immunotechnol. 2 (1996) 73) according to EU index numbering.

In some embodiments, the two antibody heavy chains under (a) comprise i) a first antibody heavy chain which has at least 90, 91, 92, 93, 94, 95, 96, 97, 98, 99 or 100% identity to the heavy chain of amino acids 1- 448 (inclusive, using sequential numbering) of SEQ ID NO: 48 (i.e., the sequence preceding the linker), and which has C at position 349, 5 at position 366, A at position 368 and V at position 407 (EU numbering); and ii) a second antibody heavy chain which has at least 90, 91, 92, 93, 94, 95, 96, 97, 98, 99 or 100% identity to the heavy chain of amino acids 1-448 (inclusive, using sequential numbering) of SEQ ID NO: 47 which has C at position 354 and W at position 366 (EU numbering).

Optionally, the linker is as described above.

In another embodiment, the bispecific antibody comprises:

i) a first heavy chain having an amino acid sequence having at least 90, 91, 92, 93, 94, 95, 96, 97, 98, 99 or 100% identity to the heavy chain of SEQ ID NO: 47,
ii) a second heavy chain having at least 90, 91, 92, 93, 94, 95, 96, 97, 98, 99 or 100% identity to the heavy chain of SEQ ID NO: 48,
iii) two antibody light chains having at least 90, 91, 92, 93, 94, 95, 96, 97, 98, 99 or 100% identity to the light chain of SEQ ID NO: 49.

In yet another embodiment, the bispecific antibody is the molecule referred to herein as P1AD9826, comprising

i) a first heavy chain having the amino acid sequence of SEQ ID NO: 47;

ii) a second heavy chain having the amino acid sequence of SEQ ID NO: 48; and

iii) two antibody light chains having the amino acid sequence of SEQ ID NO: 49.

In a further aspect, the present invention relates to an isolated polynucleotide oran isolated set of polynucleotides encoding any of the antibodies of the invention described herein. In further embodiments, the present invention relates to an expression or set of expression vectors comprising said polynucleotide or polynucleotides,. In further objects the present invention an isolated polynucleotide or an insolated set of polynucleotide according to the invention, or relates to a prokaryotic or eukaryotic host cell comprising a vector of the present invention. In addition a method of producing an antibody comprising expressing the antibody from expressing the antibody from the host cell so that the antibody is produced is provided.

Bispecific or multispecific antibodies as described herein may find use in a variety of applications, including therapeutic and diagnostic applications, such as pre-targeted radioimmunotherapy and pre-targeted radioimmunoimaging.

The application describes a bispecific or multispecific antibody as described herein for use in pre-targeted radioimaging. In such embodiments, the chelated Pb is preferably $^{203}$Pb.

A method of targeting a radioisotope to a tissue or organ for imaging may comprise:

i) administering to the subject a multispecific or bispecific antibody as described herein, wherein the antibody binds to the target antigen and localises to the surface of a cell expressing the target antigen; and
ii) subsequently administering a Pb radionuclide chelated with DOTAM or a functional variant thereof to the individual, wherein the Pb radionuclide chelated with DOTAM or said functional variant thereof binds to the antibody localised to the surface of the target cell.

Optionally, between steps (i) and (ii) a clearing agent is administered, wherein the clearing agent binds to the antigen binding site specific for the Pb-DOTAM chelate. The clearing agent blocks the antigen binding site for Pb-DOTAM, preventing circulating antibody from binding to the chelated Pb radionuclide. Alternatively or additionally, the clearing agent may increase the rate of clearance of antibody from the body. The "clearing agent" may alternatively be referred to as a "blocking agent": these terms can be substituted for each other in the discussion that follows.

The clearing agent may comprise a complex of a metal ion with DOTAM or a functional variant thereof, where said complex is recognised by the antigen binding site for Pb-DOTAM. Preferably, the metal ion is a stable isotope or essentially stable isotope. By "stable isotope" we mean an isotope that does not undergo radioactive decay. By "essentially stable isotope" we mean an isotope that undergoes radioactive decay with a very long half-life, making it safe for use.

Preferably, the metal ion is selected from ions of Pb, Ca and Bi. For example, the clearing agent may comprise a stable isotope of Pb complexed with DOTAM or a functional variant thereof, Ca complexed with DOTAM or a functional variant thereof, or $^{209}Bi$ (an essentially stable isotope with a half-life of 1.9 x $10^{19}$ years) complexed with DOTAM or a functional variant thereof. The Pb may be naturally occurring lead, which is a mixture of the stable (non-radioactive) isotopes $^{204}Pb$, $^{206}Pb$, $^{207}Pb$ and $^{208}Pb$.

The DOTAM or functional variant thereof is conjugated to a clearing moiety. This moiety provides the clearing agent with low uptake into the tumour, e.g., by virtue of its size and/or high hydrodynamic radius. Suitable clearing moieties are discussed further below. In some embodiments, the clearing agent may comprise DOTAM or a functional variant thereof conjugated to dextran or a derivative thereof.

In another embodiment of the imaging methods, the multispecific or bispecific antibody may be bound to the chelated Pb radionuclide at the time of administration.

Optionally, in any embodiment, the method may further comprise:

iii) imaging the tissue or organ where the Pb radionuclide chelated with DOTAM or the functional variant thereof has localized.

In some embodiments, the target antigen may be a tumour-specific antigen and the imaging may be a method of imaging a tumour or tumours.

The application describes an antibody as described herein for use in a method of pre-targeted radioimmunotherapy. In such embodiments, the chelated Pb is preferably $^{212}Pb$.

A method of targeting a radioisotope to a tissue or organ for therapy may comprise:

i) administering to the subject a multispecific or bispecific antibody as described herein, wherein the antibody binds to the target antigen and localizes to the surface of a cell expressing the target antigen; and
ii) subsequently administering a Pb radionuclide chelated with DOTAM or with a functional variant thereof, wherein the Pb radionuclide chelated with DOTAM or a functional variant thereof binds to the antibody localised to the surface of the cell.

Optionally, between steps (i) and (ii) a clearing agent is administered as described above.

In some embodiments, the target antigen is a tumour-associated antigen and the method is a method of treating cancer. In other embodiments the target antigen may be an antigen associated with infection, e.g., a protein expressed by a prokaryote or by a virus-infected cell.

In some embodiments, the antibodies described herein may be administered as part of a combination therapy. For example, they may be administered in combination with one or more radiosensitizers and/or chemotherapeutic agents: the radiosensitizer or chemotherapeutic agent and the antibody may be administered simultaneously or sequentially, in either order.

The methods of radioimaging and radioimmunotherapy described herein may optionally be combined, e.g., by administering the antibody and both $^{203}Pb$-DOTAM and $^{212}Pb$-DOTAM, e.g., as a mixture.

The application describes pharmaceutical compositions comprising an antibody of the present invention and a pharmaceutically acceptable excipient.

The application describes a kit comprising an antibody of the present invention and one, two, three, four or all of:

i) a pharmaceutically acceptable excipient;
ii) a Pb radionuclide chelated by DOTAM or a functional variant thereof;
iii) a clearing agent as described herein;
iv) one or more additional chemotherapeutic agents; and/or
v) one or more radiosensitizers.

As described herein the present inventors have developed a novel clearing agent. Such a clearing agent may be used in any of the methods of diagnosis, imaging or treatment as described herein.

The application describes a clearing agent comprising dextran or a derivative thereof conjugated to a chelator selected from DOTAM and a functional variant of DOTAM, wherein said chelator forms a complex with Pb, Zn, Ca or Bi. The clearing agent typically includes a chelated metal ion, such as a Pb, Zn, Ca or Bi ion.

The clearing agent may comprise aminodextran coupled to DOTAM or a functional variant of DOTAM. For example, the clearing agent may comprise DOTAM coupled to aminodextran with isothiocyanate coupling (for example, a compound obtainable by reacting aminodextran with *p*-SCN-Bn-TCMC).

One potential difficulty with the use of clearing agents is the possibility that they may enter tumours, negatively affecting subsequent binding of radioligands.

The present inventors have further found that good clearance from the blood can be achieved together with low clearing agent penetration into tumours, when a dextran-based clearing agent is used which has i) a high average

molecular weight and ii) has been subject to a molecular weight cut-off, such that fragments below a certain size have been removed.

Thus, a preferred clearing agent may be one in which i) the average molecular weight of the dextran or derivative thereof in the clearing agent is 200-800kDa, optionally greater than 300, 350, 400 or 450 kDa, and optionally less than 700, 650, 600 or 550kDa, optionally about 500kDa, and ii) dextran, dextran derivatives or clearing agents of less than a specified molecular weight cut-off have been removed, wherein the molecular weight cut-off is 50kDa or above, 100kDa or above or 200kDa or above, optionally in the range 50kDa-250kDa or 50kDa-200kDa, optionally 100kDa-200kDa and optionally about 100kDa, 150kDa or 200kDa.

The application describes methods of preparing the clearing agent, and use of the clearing agents in methods of radioimmunotherapy or radioimmunoimaging.

These and other aspects of the invention are discussed further below.

## FIGURES

Figure 1 shows a schematic representation of a possible bispecific antibody format. This format comprises two antigen-binding sites for one target (A) and one antigen-binding site for a second target (B) (2:1 format).

Figure 2 shows the structure of PRIT-0213 in complex with Pb-DOTAM.

Figure 3 shows the view on the interaction site, of PRIT-0213 in complex with Pb-DOTAM, numbered according to Kabat.

Figure 4 shows distribution of $^{212}$Pb 24 h after injection of radiolabeled DOTAM (%ID/g ± SD, n = 3). PRIT-0206, -0207, -0208, and -0165 target T84.66, whereas PRIT-0186, -0187, and -0156 target CH1A1A. PRIT-0175 is a non-CEA-binding control.

Figure 5 shows distribution of $^{203}$Pb expressed as counts per minute (CPM) 96 h after injection of PRIT antibodies pre-bound with radiolabeled DOTAM (CPM ± SD, n = 3). PRIT-0205, -0206, -0207, -0208, and -0209 are fully humanized constructs, whereas PRIT-0165 and -0175 are positive and negative controls, respectively.

Figure 6 shows accumulation/clearance of $^{203}$Pb-DOTAM-bsAb in BxPC3 tumors and blood expressed as CPM ± SD (n = 3) at various time points after injection of PRIT antibodies pre-bound with radiolabeled DOTAM. PRIT-0206 is a fully humanized version of PRIT-0165; PRIT-0175 is a non-CEA-binding control.

Figure 7 shows radioactivity distribution in selected tissues 2 hours after injection of $^{212}$Pb-labeled clearing agents in MKN45 tumor-bearing mice (%ID/g ± SD, n=3).

Figure 8 shows radioactivity distribution in selected tissues and urine, 24 hours after injection of $^{212}$Pb-labeled clearing agents in MKN45 tumor-bearing mice (%ID/g ± SD, n=3).

Figure 9 shows organ-wise radioactivity distribution in selected tissues and urine, 24 hours after injection of $^{212}$Pb-labeled clearing agents in MKN45 tumor-bearing mice (%ID ± SD, n=3).

Figure 10 shows radioactivity distribution in selected tissues 1 week after injection of $^{233}$Pb-labeled clearing agents in tumor-free mice (%ID/g ± SD, n=3) .

Figure 11 shows organ-wise radioactivity distribution in selected tissues 1 week after injection of $^{203}$Pb-labeled clearing agents in tumor-free mice (%ID ± SD, n=3).

Figure 12 shows radioactivity content in blood 4 h after injection of $^{212}$Pb-DOTAM (%ID/g + SD, n = 3). The striped bar represents the no-CA control (without clearing agent), with which all candidate reagents were compared. Asterisks mark the level of statistical significance, from lower (*) to higher (***).

Figure 13 shows average radioactivity content in blood 24 h after injection of $^{212}$Pb-DOTAM (%ID/g + SD, n = 3). The striped bar represents the no-CA control (without clearing agent), with which all candidate reagents were compared.

Figure 14 shows radioactivity content in blood and tumors 24 h after injection of $^{212}$Pb-DOTAM (%ID/g ± SD, n = 3) .

Figure 15 shows distribution of $^{212}$Pb 24 h after injection of radiolabeled DOTAM (%ID/g ± SD, n = 3), using 30 or 100 μg of bispecific antibody and 10-100 μg of clearing agents with 100- or 30-kDa filtration cutoffs, or no clearing agent at all (PBS).

Figure 16 shows the effect on activity concentration of $^{212}$Pb in blood and tumor with increasing amounts of clearing agent (0-100 pg). Tumors were pretargeted using 100 μg of PRIT-0165, followed 4 days later by Dex500 diafiltered with a 100-kDa cutoff, or PBS. $^{212}$Pb-DOTAM was administered 2 hours after the clearing agent. The symbols represent the %ID/g 24 h after the radioactive injection, and the line the linear regression of the tumor data.

Figure 17 shows radioactivity distribution in selected tissues 24 h after injection of $^{212}$Pb-DOTAM (%ID/g ± SD, n = 3). The dark grey and black bars represent no-CA positive controls (without clearing agent), with which the candidate reagents were compared.

Figure 18 shows $^{212}$Pb content in blood and tumors 24 h after injection of $^{212}$Pb-DOTAM (%ID/g ± SD, n = 3), and the corresponding tumor-to-blood ratios. The dark grey and black bars represent no-CA positive controls (without clearing agent), with which the candidate reagents were compared.

Figure 19 shows the tumor-to-blood ratio 24 h after injection of $^{212}$Pb-DOTAM as a function of clearing agent (CA) amount (PJRD08-46) and TCMC saturation (9-, 20-, 39-, or 84-to-1). The dashed lines represent linear regression ($R^2$ = 0.82) and nonlinear curve fit ($R^2$ = 0.74) of the respective data.

Figure 20 shows distribution of $^{212}$Pb 24 h after injection of radiolabeled DOTAM (%ID/g ± SD, n = 3). Bars with white and grey background represent targeting of T84.66 and CH1A1A, respectively; the black bar represents the non-CEA-binding control.

Figure 21 shows radioactivity distribution in selected tissues 24 h after injection of $^{212}$Pb-DOTAM (%ID/g ± SEM, n = 3), for treatment cycles 1 and 2 in the BxPC3 model.

Figure 22 shows average body weights in groups A-G (n = 8) after CEA-PRIT in the BxPC3 model. Curves were truncated at the first death in each group. Dotted vertical lines indicate $^{212}$Pb-DOTAM administration for some or all groups, according to the study design.

Figure 23 shows average weight change in groups A-G (n = 8) after CEA-PRIT in the BxPC3 model, expressed as the percentage of initial body weight. Curves were truncated at the first death in each group. Dotted vertical lines indicate $^{212}$Pb-DOTAM administration for some or all groups, according to the study design.

Figure 24 shows tumor growth averages with standard error for groups A-G in the BxPC3 model (n=8). Curves were truncated at the first death in each group. Dotted vertical lines indicate $^{212}$Pb-DOTAM administration for some or all groups, according to the study design.

Figure 25 shows individual tumor growth curves for groups A-G in the BxPC3 model. The dotted vertical lines indicate administration of $^{212}$Pb-DOTAM.

Figure 26 shows Kaplan-Meier curves showing the survival in groups A-G in the BxPC3 model (n=8). The dotted vertical lines indicate administration of $^{212}$Pb-DOTAM.

Figure 27 shows radioactivity distribution in selected tissues 24 h after injection of $^{212}$Pb-DOTAM (%ID/g ± SD, n = 3), for treatment cycle 1 and 2 in the LS174T model.

Figure 28 shows average body weights in groups A-G (n = 8) after CEA-PRIT in the LS174T model. Curves were truncated at the first death in each group. Dotted vertical lines indicate $^{212}$Pb-DOTAM administration for some or all groups, according to the study design.

Figure 29 shows average weight change in groups A-G (n = 8) after CEA-PRIT in the LS174T model, expressed as the percentage of initial body weight. Curves were truncated at the first death in each group. Dotted vertical lines indicate $^{212}$Pb-DOTAM administration for some or all groups, according to the study design.

Figure 30 shows tumor growth averages with standard error for groups A-G (n=8) in the LS174T model. Curves were truncated at the first death in each group. Dotted vertical lines indicate $^{212}$Pb-DOTAM administration for some or all groups, according to the study design.

Figure 31 shows individual tumor growth curves for groups A-G in the LS174T model. The dotted vertical lines indicate administration of $^{212}$Pb-DOTAM.

Figure 32 shows Kaplan-Meier curves showing the survival in groups A-G in the LS174T model (n=8). The dotted vertical lines indicate administration of $^{212}$Pb-DOTAM.

Figure 33 shows radioactivity distribution in selected tissues 24 h after injection of $^{212}$Pb-DOTAM (%ID/g + SD, n = 3). The grey bars represent the tissue accumulation after injection of various amounts of Dex500-(50%) clearing agent (CA); the black bar represents the no-CA control (without clearing agent.

Figure 34 shows $^{212}$Pb content in blood and tumors 24 h after injection of $^{212}$Pb-DOTAM (%ID/g $\pm$ SD, n = 3), and the corresponding tumor-to-blood ratios. The grey bars represent the tissue accumulation after injection of various amounts of the Dex500-(50%) clearing agent (CA); the black bar represents the no-CA control (without clearing agent).

Figure 35 shows radioactivity content in blood 4 h after injection of $^{212}$Pb-DOTAM (%ID/g $\pm$ SD, n = 3).

Figure 36 shows binding of one antibody (PRIT-0165) to MKN-45 cells, detecting it either using secondary detection (right panel, Alexa 488) or DOTAM FITC (left panel, FITC-A).

Figure 37 shows possible formats for bispecific antibodies, using CEA as an exemplary target antigen. Other target antigens may also be used.

Figure 38 shows binding of P1AD8927 to KPL-4 cells to demonstrate Her2 binding competence: Detection of antibodies using human IgG specific secondary antibodies.

Figure 39 show binding of P1AD8927 to KPL-4 cells to demonstrate DOTAM binding competence: Isotypecorrected detection using Pb-DOTAM-FITC.

Figure 40 shows binding of P1AD8926 to Raji cells to demonstrate CD20 binding competence: Detection of antibodies using human IgG specific secondary antibodies.

Figure 41 shows binding of P1AD8926 to Raji cells to demonstrate DOTAM binding competence: Isotypecorrected detection using Pb-DOTAM-FITC.

Figure 42 shows the study outline of protocol 103, assessing CEA-PRIT of s.c. BxPC3 tumors in SCID mice (h = hours, d = days, w = weeks).

Figure 43: Panel A shows the average accumulation of $^{212}$Pb in collected tissues after both treatment cycles, expressed as the % ID/g + SD (n=3). Panel B shows the individual tumor uptake of $^{212}$Pb for each mouse, along with the corresponding tumor volumes ($mm^3$) at euthanasia.

Figure 44 shows average tumor growth curves with standard error for groups A-G in the BxPC3 model (n=10). Curves were truncated at n<5. Dotted vertical lines indicate $^{212}$Pb-DOTAM administration (30 or 10 $\mu$Ci) for some or all groups, according to the study design.

Figure 45 shows individual tumor growth curves for groups A-G in the BxPC3 model (n=10). Dotted vertical lines indicate administration of $^{212}$Pb-DOTAM (30 or 10 $\mu$Ci).

Figure 46 shows Kaplan-Meier curves showing the survival in groups A-G in the BxPC3 model (n=10). Dotted vertical lines indicate administration of $^{212}$Pb-DOTAM (30 or 10 $\mu$Ci).

Figure 47 shows average body weight loss in groups A-G (n = 10) after CEA PRIT in the BxPC3 model. Curves were truncated at n<5. Dotted vertical lines indicate $^{212}$Pb-DOTAM administration for some or all groups, according

to the study design.

Figure 48 shows distribution of $^{203}$Pb-BsAb (20 μCi, 100 μg) in SCID mice bearing s.c. BxPC3 tumors. Mice were injected with 20 μCi of pre-bound $^{203}$Pb-DOTAM-CEA-DOTAH or $^{203}$Pb-DOTAM-DIG-DOTAM (negative control) followed by organ harvest at day 1, 4, 7, or 10 after injection to assess the accumulated radioactivity in collected tissues (% ID/g ± SD, n = 5).

Figure 49 shows accumulation of $^{203}$Pb-BsAb in s.c. BxPC3 tumors 1-10 days after injection of 20 μCi/100 μg of pre-bound $^{203}$Pb-DOTAM-CEA-DOTAM or $^{203}$Pb-DOTAM-DIG-DOTAM (negative control) (% ID/g ± SD, n = 5).

Figure 50 shows distribution of $^{212}$Pb in SCID mice bearing s.c. BxPC3 tumors. Mice were injected with CEA-DOTAM BsAb and CA before $^{212}$Pb-DOTAM administration, from 5 min to 48 h after the radioactive injection (% ID/g + SD, n = 5). *Cumulative $^{212}$Pb content in urine and feces over time, i.e., each time point including the value of the previous. The estimated % ID/g in urine was based on 1/5 (10 mL) of a pooled urine/wash solution from 5 mice (50 mL).

Figure 51 shows the study outline of protocol 131, assessing the in vivo distribution of $^{212}$Pb after PRIT using CEA-DOTAM BsAb, Pb-DOTAM-dextran-500 CA, and $^{212}$Pb-DOTAM quenched with either of 5 different metals (Zn, Gd, Cu, Ca, or Pb) in SCID mice carrying s.c. BxPC3 tumors (d = days, h = hours).

Figure 52 shows distribution of $^{212}$Pb in tumor-bearing SCID mice 2 hours after injection of CEA-DOTAM-pretargeted $^{212}$Pb-DOTAM (% ID/g + SD, n = 4).

Figure 53 shows distribution of $^{212}$Pb in selected normal tissues of tumor-bearing SCID mice 2 hours after injection of $^{212}$Pb-DOTAM, quenched with different metals (% ID/g).

Figure 54 shows distribution of $^{212}$Pb in tumor-bearing SCID mice 24 hours after injection of $^{212}$Pb-DOTAM, pretargeted by CD20-DOTAM BsAb or the negative control DIG-DOTAM (% ID/g + SD, n = 3).

Figure 55 shows distribution of $^{212}$Pb in tumor-bearing SCID mice 24 hours after injection of $^{212}$Pb-DOTAM, pretargeted by HER2-DOTAM BsAb or the negative control DIG-DOTAM (% ID/g ± SD, n = 3).

Figure 56 shows the study outline of Protocol 154, assessing the biodistribution of $^{212}$Pb-DOTAM after CEA-PRIT using various BsAb constructs in SCID mice carrying s.c. HPAF-II tumors (h = hours, d = days).

Figure 57 shows distribution of $^{212}$Pb in tumor-bearing SCID mice 6 hours after injection of $^{212}$Pb-DOTAM, pretargeted by either the negative control DIG-DOTAM, the standard CEA-DOTAM BsAb, or one of the alternative BsAb constructs (% ID/g + SD, n = 3).

Figure 58 shows blood content and tumor accumulation of $^{212}$Pb 6 hours after injection of $^{212}$Pb-DOTAM, pretargeted by either the negative control DIG-DOTAM, the standard CEA-DOTAM BsAb, or one of the alternative BsAb constructs (% ID/g ± SD, n = 3).

Figure 59 shows the experimental schedule of protocol 162. CD20-PRIT was carried out using CD20-DOTAM BsAb, Ca-DOTAM-dextran-500 CA, and $^{212}$Pb-DOTAM in SCID mice carrying s.c. WSU-DLCL2 tumors; 1-step RIT was carried out using CD20-DOTAM BsAb pre-bound with $^{212}$Pb-DOTAM ($^{212}$Pb-DOTAM-CD20-DOTAM) in SCID mice carrying s.c. WSU-DLCL2 tumors.

Figure 60 shows the distribution of $^{212}$Pb in tumor-bearing SCID mice 24 hours after injection of CD20-DOTAM-pretargeted $^{212}$Pb-DOTAM or pre-bound $^{212}$Pb-DOTAM-CD20-DOTAM. The radioactive content in organs and tissues is expressed as average % ID/g and standard deviation (SD; n = 3).

Figure 61 shows average WSU-DLCL2 s.c. tumor growth for groups A-G, expressed in $mm^3$ + SEM (n = 10).

Figure 62 shows average change in mouse body weight after the various treatments, expressed as % of initial body weight + SEM. The dotted lines indicate $^{212}$Pb or antibody injection, depending on the treatment scheme.

DEFINITIONS

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-Pb-DOTAM antibody", "an antibody that binds to Pb-DOTAM", "an antibody that binds to a Pb-DOTAM chelate", and equivalent terms, refer to an antibody that is capable of binding the Pb-DOTAM chelate with sufficient affinity such that the antibody is useful in a sorting and/or purification scheme for separating Pb-DOTAM labelled moieties, and/or such that the antibody is capable of localizing Pb-DOTAM to the site of the antibody, e.g., for the purpose of targeting Pb-DOTAM to a cell. The terms "anti-target antibody" and "an antibody that binds to a target" refer to an antibody that is capable of binding a target with sufficient affinity such that the antibody is useful in therapeutic and/or diagnostic applications involving localization of the antibody to the target, e.g., as expressed on the surface of a cell. In one embodiment, the extent of binding of the antibody to an unrelated moiety and/or an unrelated target protein is less than about 10% of the binding of the antibody to Pb-DOTAM or the target as measured, e.g., by a radioimmunoassay (RIA). In certain embodiments, an antibody has a dissociation constant (Kd) to Pb-DOTAM and/or the target of $\leq 1\mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-6}$ M to $10^{-13}$ M, e.g., from $10^{-9}$ M to $10^{-13}$ M).

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies; linear antibodies; single-chain antibody molecules (e.g. scFv); and multispecific antibodies formed from antibody fragments.

An "antibody that binds to the same epitope" as a reference antibody may refer to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

The term "cytotoxic agent" as used herein refers to a substance that inhibits or prevents a cellular function and/or causes cell death or destruction. Cytotoxic agents include, but are not limited to, radioactive isotopes (e.g., $^{225}Ac$, $^{211}At$, $^{131}I$, $^{125}I$, $^{90}Y$, $^{186}Re$, $^{188}Re$, $^{153}Sm$, $^{212}Bi$, $^{213}Bi$, $^{32}P$, $^{212}Pb$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary

with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein. A full length antibody may be, for instance, an IgG.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs include:

(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Instead of the above, the sequence of CDR-H1 as described herein may extend from Kabat26 to Kabat35.

In one embodiment, HVR or CDR residues comprise those identified in Table 2 or elsewhere in the specification.

Unless otherwise indicated, HVR/CDR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

Molecules as described herein may be "isolated". An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

The term "nucleic acid molecule" or "polynucleotide" includes any compound and/or substance that comprises a polymer of nucleotides. Each nucleotide is composed of a base, specifically a purine- or pyrimidine base (i.e. cytosine (C), guanine (G), adenine (A), thymine (T) or uracil (U)), a sugar (i.e. deoxyribose or ribose), and a phosphate group. Often, the nucleic acid molecule is described by the sequence of bases, whereby said bases represent the primary structure (linear structure) of a nucleic acid molecule. The sequence of bases is typically represented from 5' to 3'. Herein, the term nucleic acid molecule encompasses deoxyribonucleic acid (DNA) including e.g. complementary DNA (cDNA) and genomic DNA, ribonucleic acid (RNA), in particular messenger RNA (mRNA), synthetic forms of DNA or RNA, and mixed polymers comprising two or more of these molecules. The nucleic acid molecule may be linear or circular. In addition, the term nucleic acid molecule includes both, sense and antisense strands, as well as single stranded and double stranded forms. Moreover, the herein described nucleic acid molecule can contain naturally occurring or non-naturally occurring nucleotides. Examples of non-naturally occurring nucleotides include modified nucleotide bases with derivatized sugars or phosphate backbone linkages or chemically modified residues. Nucleic acid molecules also encompass DNA and RNA molecules which are suitable as a vector for direct expression of an antibody of the invention in vitro and/or in vivo, e.g. in a host or patient. Such DNA (e.g. cDNA) or RNA (e.g. mRNA) vectors, can be unmodified or modified. For example, mRNA can be chemically modified to enhance the stability of the RNA vector and/or expression of the encoded molecule so that mRNA can be injected into a subject to generate the antibody in vivo (see e.g. Stadler et al, Nature Medicine 2017, published online 12 June 2017, doi:10.1038/nm.4356 or EP 2 101 823 B1).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N-to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "package insert" is used to refer to instructions customarily included in commercial packages of

therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either tor prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "Pb" or "lead" as used herein include ions thereof, e.g., Pb(II). Thus, the skilled reader understands

that, for example, the terms lead, Pb, $^{212}Pb$ or $^{203}Pb$ are intended to encompass ionic forms of the element, in particular, Pb(II). In various aspect of the invention, Pb may be a radioisotope (e.g., when used in a method of radioimmunotherapy or radioimmunoimaging) or may be a stable, non-radioisotope (e.g., as may be preferred in the context of a clearing agent).

"DOTAM" has the chemical name:

1,4,7,10-Tetrakis(carbamoylmethyl)-1,4,7,10-tetraazacyclododecane, which is a compound of the following formula:

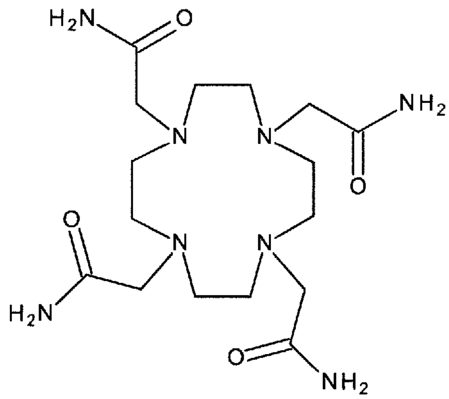

$^{212}Pb$-DOTAM has the following structure:

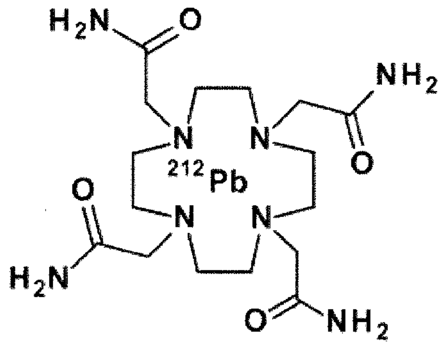

The present invention may in certain aspects and embodiments also make use of functional variants or derivatives of DOTAM incorporating a metal ion. Suitable variants/derivatives of DOTAM have a structure that differs to a certain limited extent from the structure of DOTAM and retain the ability to function (i.e. retains sufficient activity to be used for one or more of the purposes described herein). In such aspects and embodiments, the DOTAM or functional variant/derivative of DOTAM may be one of the active variants disclosed in WO 2010/099536.

Suitable functional variants/derivatives may be a compound of the following formula:

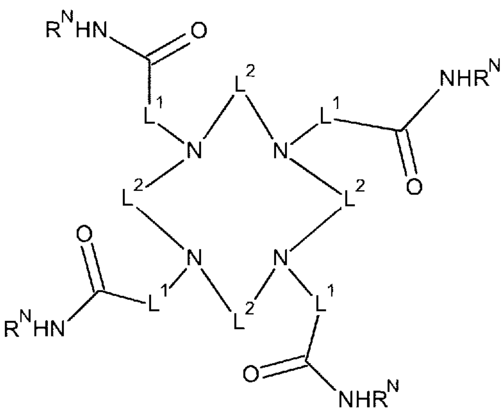

or a pharmaceutically acceptable salt thereof; wherein

$R^N$ is H, $C_{1-6}$ alkyl, $C_{1-6}$ haloalkyl, $C_{2-6}$ alkenyl, $C_{2-6}$ alkynyl, $C_{3-7}$ cycloalkyl, $C_{3-7}$ cycloalkyl-$C_{1-4}$alkyl, $C_{2-7}$ heterocycloalkyl, $C_{2-7}$ heterocycloalkyl-$C_{1-4}$ alkyl, phenyl, phenyl-$C_{1-4}$-alkyl, $C_{1-7}$ heteroaryl, and $C_{1-7}$, heteroaryl-$C_{1-4}$-alkyl; wherein $C_{1-6}$ alkyl, Ci-e haloalkyl, $C_{2-6}$ alkenyl, and $C_{2-6}$ alkynyl are each optionally substituted by 1, 2, 3, or 4 independently selected $R^w$ groups; and wherein said $C_{3-7}$ cycloalkyl, $C_{3-7}$ cycloalkyl-$C_{1-4}$alkyl, $C_{2-7}$ heterocycloalkyl, $C_{2-7}$ heterocycloalkyl-$C_{1-4}$ alkyl, phenyl, phenyl-$C_{1-4}$-alkyl, $C_{1-7}$ heteroaryl, and $C_{1-7}$ heteroaryl-$C_{1-4}$-alkyl are each optionally substituted by 1, 2, 3, or 4 independently selected $R^x$ groups;

$L^1$ is independently $C_{1-6}$ alkylene, $C_{1-6}$ alkenylene, or $C_{1-6}$ alkynylene, each of which is optionally substituted by 1, 2, or 3 groups independently selected $R^1$ groups;

$L^2$ is $C_{2-4}$ straight chain alkylene, which is optionally substituted by an independently selected $R^1$ group; and which is optionally substituted by 1, 2, 3, or 4 groups independently selected from $C_{1-4}$ alkyl and or $C_{1-4}$ haloalkyl;

$R^1$ is independently selected from $D^1$-$D^2$-$D^3$, halogen, cyano, nitro, hydroxyl, $C_{1-6}$ alkoxy, $C_{1-6}$ haloalkoxy, $C_{1-6}$ alkylthio, $C_{1-6}$ alkylsulfinyl, $C_{1-6}$ alkylsulfonyl, amino, $C_{1-6}$ alkylamino, di-$C_{1-6}$ alkylamino, $C_{1-9}$ alkylcarbonyl, carboxy, $C_{1-6}$ alkoxycarbonyl, $C_{1-6}$ alkylcarbonylamino, di-$C_{1-6}$ alkylcarbonylamino, $C_{1-6}$ alkoxycarbonylamino, $C_{1-6}$ alkoxycarbonyl-($C_{1-6}$ alkyl)amino, carbamyl, $C_{1-6}$ alkylcarbamyl, and di-$C_{1-6}$ alkylcarbamyl;

each $D^1$ is independently selected from $C_{6-10}$ aryl-$C_{1-9}$ alkyl, $C_{1-9}$ heteroaryl-$C_{1-4}$ alkyl, $C_{3-10}$ cycloalkyl-$C_{1-4}$ alkyl, $C_{2-9}$ heterocycloalkyl - $C_{1-4}$ alkyl, $C_{1-8}$ alkylene, $C_{1-8}$ alkenylene, and $C_{1-8}$ alkynylene; wherein said $C_{1-6}$ alkylene, $C_{1-8}$ alkenylene, and $C_{1-6}$ alkynylene are optionally substituted by 1, 2, 3, or 4 independently selected $R^4$ groups; and wherein said $C_{6-10}$ aryl-$C_{1-4}$ alkyl, $C_{1-9}$ heteroaryl-$C_{1-4}$ alkyl, $C_{3-10}$ cycloalkyl-$C_{1-4}$ alkyl, $C_{2-9}$ heterocycloalkyl-$C_{1-4}$ alkyl are each optionally substituted by 1, 2, 3, or 4 independently selected $R^5$ groups;

each $D^2$ is independently absent or $C_{1-20}$ straight chain alkylene, wherein from 1 to 6 non-adjacent methylene groups of said $C_{1-20}$ straight chain alkylene are each optionally replaced by an independently selected -$D^4$- moiety, provided that at least one methylene unit in said $C_{1-20}$ straight chain alkylene is not optionally replaced by a -$D^4$- moiety; wherein said $C_{1-20}$ straight chain alkylene is optionally substituted by one or more groups independently selected from halogen, cyano, nitro, hydroxyl, $C_{1-4}$ alkyl, $C_{1-4}$ haloalkyl, $C_{1-4}$ alkoxy, $C_{1-4}$ haloalkoxy, amino, $C_{1-4}$ alkylamino, di-$C_{1-4}$ alkylamino, $C_{1-4}$ alkylcarbonyl, carboxy, $C_{1-4}$ alkoxycarbonyl, $C_{1-4}$ alkylcarbonylamino, di-$C_{1-4}$ alkylcarbonylamino, $C_{1-4}$ alkoxycarbonylamino, $C_{1-4}$ alkoxycarbonyl-($C_{1-4}$ alkyl) amino, carbamyl, $C_{1-4}$ alkylcarbamyl, and di-$C_{1-4}$ alkylcarbamyl;

each $D^3$ is independently selected from H, halogen, cyano, nitro, hydroxyl, $C_{1-6}$ alkyl, $C_{1-6}$ haloalkyl, $C_{2-6}$ alkenyl, $C_{2-6}$ alkynyl, $C_{3-14}$ cycloalkyl, $C_{3-14}$ cycloalkyl-$C_{1-4}$ alkyl, $C_{2-14}$ heterocycloalkyl, $C_{2-14}$ heterocycloalkyl-$C_{1-4}$ alkyl, $C_{6-14}$ aryl, $C_{6-11}$ aryl-$C_{1-4}$ alkyl, $C_{1-13}$ heteroaryl, $C_{1-13}$ heteroaryl-$C_{1-4}$ alkyl; wherein said $C_{1-6}$ alkyl, $C_{1-6}$ haloalkyl, $C_{2-6}$ alkenyl, $C_{2-6}$ alkynyl are each optionally substituted by 1, 2, 3, or 4 independently selected $R^6$ groups; and wherein said $C_{3-14}$ cycloalkyl, $C_{3-14}$ cycloalkyl-$C_{1-9}$ alkyl, $C_{2-14}$ heterocycloalkyl, $C_{2-14}$ heterocycloalkyl-$C_{1-4}$ alkyl, $C_{6-14}$ aryl, $C_{6-14}$ aryl-$C_{1-4}$ alkyl, $C_{1-13}$ heteroaryl, $C_{1-13}$ heteroaryl-$C_{1-4}$ alkyl are each optionally substituted by 1, 2, 3 or 4 independently selected $R^7$ groups;

each $D^4$ is independently selected from -O-, -S-, -$NR^a$C(=O)-, -$NR^a$C(=S)-, -$NR^b$C(=O)$NR^{c-}$, -$NR^b$C(=S)$NR^c$-, -S(=O)-, -S(=O)$_2$-, -S(=O)$NR^a$-, -C(=O)-, -C(=S)-, -C(=O)O-, -OC(=O)$NR^a$-, -OC(=S)$NR^a$-, -$NR^a$-, -$NR^b$S(=O)$NR^c$-, and $NR^b$S (=O) $_2$NR°-;

each $R^4$ and $R^6$ is independently selected from halogen, cyano, nitro, hydroxyl, $C_{1-4}$ alkoxy, $C_{1-4}$ haloalkoxy, $C_{1-4}$ alkylthio, $C_{1-4}$ alkylsulfinyl, $C_{1-4}$ alkylsulfonyl, amino, $C_{1-4}$ alkylamino, di-$C_{1-4}$ alkylamino, $C_{1-4}$ alkylcarbonyl, carboxy, $C_{1-4}$ alkoxycarbonyl, $C_{1-4}$ alkylcarbonylamino, di-$C_{1-4}$ alkylcarbonylamino, $C_{1-4}$ alkoxycarbonylamino, $C_{1-4}$ alkoxycarbonyl- ($C_{1-4}$ alkyl)amino, carbamyl, $C_{1-4}$ alkylcarbamyl, and di-$C_{1-4}$ alkylcarbamyl;

each $R^3$ is independently selected from halogen, cyano, cyanate, isothiocyanate, nitro, hydroxyl, $C_{1-4}$ alkyl, $C_{2-4}$ alkenyl, $C_{2-4}$ alkynyl, $C_{1-4}$ alkoxy, $C_{1-4}$ haloalkoxy, $C_{1-4}$ alkylthio, $C_{1-4}$ alkylsulfinyl, $C_{1-4}$ alkylsulfonyl, amino, $C_{1-4}$ alkylamino, di-$C_{1-4}$ alkylamino, $C_{1-4}$ alkylcarbonyl, carboxy, $C_{1-4}$ alkoxycarbonyl, $C_{1-4}$ alkylcarbonylamino, di-$C_{1-4}$ alkylcarbonylamino, $C_{1-4}$ alkoxycarbonylamino, $C_{1-4}$ alkoxycarbonyl- ($C_{1-4}$ alkyl)amino, carbamyl, $C_{1-4}$ alkylcarbamyl, and di-$C_{1-4}$ alkylcarbamyl;

each $R^7$ is independently selected from halogen, cyano, nitro, hydroxyl, $C_{1-6}$ alkyl, $C_{2-6}$ alkenyl, $C_{2-6}$ alkynyl, $C_{3-7}$ cycloalkyl, $C_{3-7}$ cycloalkyl-$C_{1-4}$ alkyl, $C_{2-7}$ heterocycloalkyl, $C_{2-7}$ heterocycloalkyl-$C_{1-4}$ alkyl, phenyl, phenyl-$C_{1-4}$ alkyl, $C_{1-7}$ heteroaryl, $C_{1-7}$ heteroaryl-$C_{1-4}$ alkyl, -$OR^O$, -$SR^O$, -S(=O)$R^P$, -S(=O)$_2R^P$, -S(=O)$NR^sR^t$, -C(=O)$R^P$, -C(=O)$OR^P$, -C(=O)$NR^sR^t$, -OC(=O)$R^P$, -OC(=O)$NR^sR^t$, -$NR^sR^t$, -$NR^q$C(=O)$R^r$, -$NR^q$C(=O)$OR^r$, -$NR^q$C(=O)$NP^r$, -$NR^q$S (=O) $_2R^r$, and -$NR^P$S(=O)$_2NR^sR^t$; wherein said $C_{1-6}$ alkyl, $C_{2-6}$ alkenyl, $C_{2-6}$ alkynyl are each optionally substituted by 1, 2, 3, or 4 independently selected R' groups; and wherein said $C_{3-7}$ cycloalkyl, $C_{3-7}$ cycloalkyl-$C_{1-4}$ alkyl, $C_{2-7}$ heterocycloalkyl, $C_{2-7}$ heterocycloalkyl-$C_{1-4}$ alkyl, phenyl, phenyl-$C_{1-4}$ alkyl, $C_{1-7}$ heteroaryl, $C_{1-7}$ heteroaryl-$C_{1-4}$ alkyl are each optionally substituted by 1, 2, 3, or 4 independently selected R" groups;

each $R^a$, $R^b$, and $R^c$ is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, $C_{3-7}$ cycloalkyl-$C_{1-4}$ alkyl, $C_{2-7}$ heterocycloalkyl, $C_{2-7}$ heterocycloalkyl-$C_{1-4}$ alkyl, phenyl, phenyl-$C_{1-4}$ alkyl, $C_{1-7}$ heteroaryl, $C_{1-7}$ heteroaryl-$C_{1-4}$ alkyl; wherein said $C_{1-6}$ alkyl, $C_{1-6}$ haloalkyl, $C_{2-6}$ alkenyl, $C_{2-6}$ alkynyl are each optionally substituted by 1, 2, 3, or 4 independently selected $R^w$ groups; and wherein said $C_{3-7}$ cycloalkyl,

$C_{3-7}$ cycloalkyl-$C_{1-4}$ alkyl, $C_{2-7}$ heterocycloalkyl, $C_{2-7}$ heterocycloalkyl-$C_{1-4}$ alkyl, phenyl, phenyl-$C_{1-4}$ alkyl, $C_{1-7}$ heteroaryl, $C_{1-7}$ heteroaryl-$C_{1-4}$ alkyl are each optionally substituted by 1, 2, 3, or 4 independently selected $R^x$ groups;
each $R^o$, $R^p$, $R^q$, $R^r$, $R^s$ and $R^t$ is independently selected from H, $C_{1-6}$ alkyl, $C_{1-6}$ haloalkyl, $C_{2-6}$ alkenyl, $C_{2-6}$ alkynyl, $C_{3-7}$ cycloalkyl, $C_{3-7}$ cycloalkyl-$C_{1-4}$ alkyl, $C_{2-7}$ heterocycloalkyl, $C_{2-7}$ heterocycloalkyl-$C_{1-4}$ alkyl, phenyl, phenyl-$C_{1-4}$ alkyl, $C_{1-7}$ heteroaryl, $C_{1-7}$ heteroaryl-$C_{1-4}$ alkyl; wherein said $C_{1-6}$ alkyl, $C_{1-6}$ haloalkyl, $C_{2-6}$ alkenyl, $C_{2-6}$ alkynyl are each optionally substituted by 1, 2, 3, or 4 independently selected $R^y$ groups; and wherein said $C_{3-7}$ cycloalkyl, $C_{3-7}$ cycloalkyl-$C_{1-4}$ alkyl, $C_{2-7}$ heterocycloalkyl, $C_{2-7}$ heterocycloalkyl-$C_{1-4}$ alkyl, phenyl, phenyl-$C_{1-4}$ alkyl, $C_{2-7}$ heteroaryl, $C_{1-7}$ heteroaryl-$C_{1-4}$ alkyl are each optionally substituted by 1, 2, 3, or 4 independently selected $R^z$ groups;
each R', $R^w$ and $R^y$ is independently selected from hydroxyl, cyano, nitro, $C_{1-4}$ alkoxy, $C_{1-4}$ haloalkoxy, amino, $C_{1-4}$ alkylamino, and di-$C_{1-4}$ alkylamino; and
each R", $R^x$, and $R^z$ is independently selected from hydroxyl, halogen, cyano, nitro, $C_{1-4}$ alkyl, $C_{1-4}$ haloalkyl, $C_{1-4}$ alkoxy, $C_{1-4}$ haloalkoxy, amino, $C_{1-4}$ alkylamino, and di-$C_{1-4}$ alkylamino;
provided that the valency of each atom in the optionally substituted moieties is not exceeded.

Suitably, the functional variants/derivatives of the above formula have an affinity for an antibody of the present invention which is comparable to or greater than that of DOTAM, and have a binding strength for Pb which is comparable to or greater than that of DOTAM ("affinity" being as measured by the dissociation constant, as described above). For example, the dissociation constant of the functional/variant derivative with the antibody of the present invention or/Pb may be 1.1 times or less, 1.2 times or less, 1.3 times or less, 1.4 times or less, 1.5 times or less, or 2 times or less than the dissociation constant of DOTAM with the same antibody/Pb.

Each $R^N$ may be H, $C_{1-6}$ alkyl, or $C_{1-6}$ haloalkyl; preferably H, $C_{1-4}$ alkyl, or $C_{1-4}$ haloalkyl. Most preferably, each $R^N$ is H.

For DOTAM variants, it is preferred that 1, 2, 3 or most preferably each $L^2$ is $C_2$ alkylene. Advantageously, the $C_2$ alkylene variants of DOTAM can have particularly high affinity for Pb. The optional substituents for $L^2$ may be $R^1$, $C_{1-4}$ alkyl, or $C_{1-4}$ haloalkyl. Suitably, the optional substituents for $L^2$ may be $C_{1-4}$ alkyl or $C_{1-4}$ haloalkyl.

Optionally, each $L^2$ may be unsubstituted $C_2$ alkylene $-CH_2CH_2-$.

Each $L^1$ is preferably $C_{1-4}$ alkylene, more preferably $C_1$ alkylene such as $-CH_2-$.

Functional variants/derivatives may also include DOTAM or a compound as described above conjugated to one or more additional moieties, for example, a small molecule, a polypeptide or a carbohydrate. This attachment may occur via one of the carbons in the backbone of the macrocycle ring. A small molecule can be, for example, a dye (such as Alexa 647 or Alexa 488), biotin or a biotin moiety. A polypeptide may be, for example, an oligo peptide, for example, a therapeutic peptide or polypeptide such as an antibody. Exemplary carbohydrates include dextran, linear or branched polymers or co-polymers (e.g. polyalkylene, poly(ethylene-lysine), polymethacrylate, polyamino acids, poly- or oligosaccharides, dendrimers).

The functional variant/derivative of DOTAM may be a compound of the following formula:

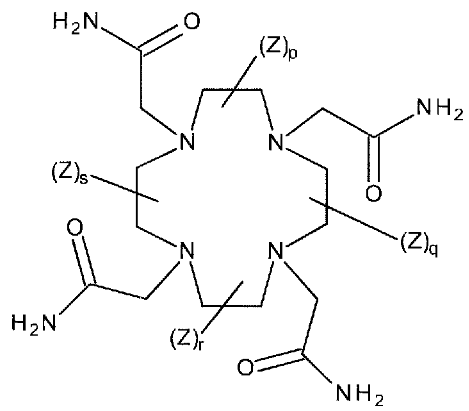

wherein each Z is independently $R^1$ as defined above; p, q, r, and s are 0, 1 or 2; and p+q+r+s is 1 or greater. Preferably, p, q, r, and s are 0 or 1 and/or p+q+r+s is 1. For example, the compound may have p+q+r+s = 1, where Z is p-SCN-benzyl moiety - such a compound is commercially available from Macrocyclics, Inc. (Plano, Texas).

## DETAILED DESCRIPTION

COMPOSITIONS AND METHODS

Antibodies

In one aspect, the invention is based, in part, on the provision of antibodies which bind specifically to Pb-DOTAM (i.e., a chelate comprising DOTAM complexed with Pb, also referred to herein as a "Pb-DOTAM chelate").

In certain embodiments, an antibody that binds specifically to Pb-DOTAM may have one or more of the following properties:

- Binds specifically to Pb-DOTAM and to Bi-DOTAM;
- Is selective for Pb-DOTAM as compared to other chelated metals, such as Cu-DOTAM;
- Binds to Pb-DOTAM with a very high affinity;
- Binds to the same epitope on Pb-DOTAM as antibodies described herein, e.g., PRIT-0213 or PRIT-0214 and/or has the same contact residues as said antibodies.

Radioisotopes of Pb are useful in methods of diagnosis and therapy. Particular radioisotopes of lead which may be of use in the present invention include $^{212}$Pb and $^{203}$Pb. Stable isotopes of lead may also be used in clearing agents, e.g., $^{204}$Pb, $^{206}$Pb, $^{207}$Pb or $^{208}$Pb. The Pb may be naturally occurring lead, which is a mixture of the stable (non-radioactive) isotopes $^{204}$Pb, $^{206}$Pb, $^{207}$Pb and $^{208}$Pb.

Radionuclides which are $\alpha$-particle emitters have the potential for more specific tumour cell killing with less damage to the surrounding tissue than $\beta$-emitters because of the combination of short path length and high linear energy transfer. $^{212}$Bi is an $\alpha$-particle emitter but its short half-life hampers its direct use. $^{212}$Pb is the parental radionuclide of $^{212}$Bi and can serve as an *in vivo* generator of $^{212}$Bi, thereby effectively overcoming the short half-life of $^{212}$Bi (Yong and Brechbiel, Dalton Trans. 2001 June 21; 40(23)6068-6076).

$^{203}$Pb is useful as an imaging isotope. Thus, an antibody bound to $^{203}$Pb-DOTAM may have utility in radioimmunoimaging (RII).

Generally, radiometals are used in chelated form. In aspects of the present invention, DOTAM is used as the chelating agent. DOTAM is a stable chelator of Pb(II) (Yong and Brechbiel, Dalton Trans. 2001 June 21; 40(23)6068-6076; Chappell et al Nuclear Medicine and Biology, Vol. 27, pp. 93-100, 2000). Thus, DOTAM is particularly useful in conjunction with isotopes of lead as discussed above, such as $^{212}$Pb and $^{203}$Pb.

As discussed above, antibodies according to the present invention bind to Pb-DOTAM. In some embodiments, it may be preferred that the antibodies bind Pb-DOTAM with a Kd value of the binding affinity of 100pM, 50pM, 20pM, 10pM, 5pM, 1pM or less, e.g, 0.9pM or less, 0.8pM or less, 0.7pM or less, 0.6pM or less or 0.5pM or less.

The antibodies additionally bind to Bi chelated by DOTAM. In some embodiments, it may be preferred that the antibodies bind Bi-DOTAM (i.e., a chelate comprising DOTAM complexed with bismuth, also termed herein a "Bi-DOTAM chelate") with a Kd value of the binding affinity of 1nM, 500pM, 200pM, 100pM, 50pM, 10pM or less, e.g., 9pM, 8pM, 7pM, 6pM, 5pM or less.

In some embodiments, the antibodies may bind to Bi-DOTAM and to Pb-DOTAM with a similar affinity. For instance, it may be preferred that the ratio of affinity, e.g., the ratio of Kd values, for Bi-DOTAM/Pb-DOTAM is in the range of 0.1-10, for example 1-10.

Sample affinity values for an exemplary antibody according to the invention (PRIT-0213) are provided below:

**Metal-DOTAM Chelate Affinities of CEA-DOTAM BsAb**

| Antigen | KD [pM] | 95% CI [pM] |
|---|---|---|
| Pb-DOTAM | 0.84 | 0.44-1.4 |
| Ca-DOTAM | 0.95 | 0.43-1.7 |
| Bi-DOTAM | 5.7 | 4.6-6.2 |
| Cu-DOTAM | 122000 | 60000 - 206000 |

Affinities were determined by KinExA equilibrium measurements.

Furthermore, the present antibodies are preferably selective for Bi-DOTAM and/or Pb-DOTAM as compared to other chelated metals, such as Cu-DOTAM.

For example, the ratio of affinity, e.g., the ratio of Kd values, for Pb-DOTAM /Cu-DOTAM may be at least 100,000.

In some embodiments, it may be preferred that the antibodies bind to Pb-DOTAM and/or Bi-DOTAM with an affinity (e.g., Kd value of the affinity) equal to or greater than that of a bispecific antibody (herein termed PRIT-0213) having:

i) a first heavy chain having the amino acid sequence of SEQ ID NO: 22;
ii) a second heavy chain having the amino acid sequence of SEQ ID NO: 23; and
iii) two antibody light chains having the amino acid sequence of SEQ ID NO: 21.

In some embodiments, it may be preferred that the antibodies bind to DOTAM-chelated Pb and/or DOTAM-chelated Bi with an affinity (e.g., KD value of the affinity) equal to or greater than that of a bispecific antibody (herein termed PRIT-0214) having:

i) a first heavy chain having the amino acid sequence of SEQ ID NO: 19;
ii) a second heavy chain having the amino acid sequence of SEQ ID NO: 20; and
iii) two antibody light chains having the amino acid sequence of SEQ ID NO: 21.

In some embodiments, the antibody according to the present invention binds to the same epitope, or an overlapping epitope, of a chelated radionuclide as an antibody disclosed herein.

In some embodiments, the antibody binds to the same epitope, or an overlapping epitope, of the Pb-DOTAM chelate (Pb-DOTAM) as Fab PRIT-0213, having

i) a first heavy chain having the amino acid sequence of SEQ ID NO: 22;
ii) a second heavy chain having the amino acid sequence of SEQ ID NO: 23; and
iii) two antibody light chains having the amino acid sequence of SEQ ID NO: 21.

The epitope of a chelated radionuclide (e.g. Pb-DOTAM) bound by a given antibody can be determined, and this can be can be compared to the epitope of the chelated radionuclide which is bound by an antibody disclosed herein (e.g. Fab PRIT-0213).

The present disclosure at example 14 describes characterisation of the binding interaction between Fab PRIT-0213 to Pb-DOTAM, based on determination of the crystal structure of Fab PRIT-0213 in complex with Pb-DOTAM at 1.40 A resolution, and analysis of this structure using the protein interfaces surfaces and assemblies (PISA) program (Krissinel and Henrick, J Mol Biol (2007) 372(3):774-97).

In some embodiments, the antibody according to the present invention may display interaction with one or more of the following sites with respect to Pb-DOTAM, e.g. as determined by PISA analysis of the structure of the antibody in complex with Pb-DOTAM: edge-to-face to the azacyclododecane ring region below the azacyclododecane ring (e.g. the tetracyclododecane ring), N6, N7, N8, N5 and/or C12. In some embodiments, the antibody may display interaction with one or more of the following sites with respect to Pb-DOTAM: N7, N8, edge-to-face to the azacyclododecane ring, the tetracyclododecane ring and/or N6.

In some embodiments, the antibody may display one or more of the following interactions with respect to Pb-DOTAM, e.g. as determined by PISA analysis of the structure of the antibody in complex with Pb-DOTAM: apolar interaction edge-to-face to the azacyclododecane ring, polar interaction with N8, hydrogen bond with N7, hydrogen bond with N8, polar interaction with N5, apolar interaction with C12, polar interaction with N7, polar (hydrogen) bond to N6, and/or apolar interaction with the tetracyclododecane ring.

In some embodiments, the antibody may display one or more of the following interactions with respect to Pb-DOTAM, e.g. as determined by PISA analysis of the structure of the antibody in complex with Pb-DOTAM: hydrogen bond between one or more residues of antibody heavy chain CDR3 and N7, hydrogen bond between one or more residues of antibody heavy chain CDR3 and N8, apolar interaction between one or more residues of antibody heavy chain CDR2 edge-to-face to the azacyclododecane ring, apolar interaction between antibody light chain CDR3 and the tetracyclododecane ring, and/or apolar interaction between antibody light chain CDR1 and N6.

Antibodies may share the same contact residues as the described herein: e.g., these residues may be invariant. These residues may include the following:

a) in heavy chain CDR2: Phe50, Asp56 and/or Tyr58, and optionally also Gly52 and/or Arg 54;
b) in heavy chain CDR3: Glu95, Arg96, Asp97, Pro98, Tyr99, Ala100C and/or Tyr100D and optionally also Pro100E;
c) in light chain CDR1: Tyr28 and/or Asp32;

d) in light chain CDR3: Gly91, Tyr92, Asp93, Thr95c and/or Tyr96;

e) in light chain CDR2: optionally Gln50. Antibodies of the invention comprise invariant residues as set out in the claims.

Certain aspects and embodiments of antibodies according to the present invention are discussed above. Further suitable aspects and embodiments according to the invention are discussed in the following. In all embodiments, antibodies retain the ability to bind Pb-DOTAM, and preferably also Bi-DOTAM, still more preferably with the affinity and/or selectivity as discussed above.

In one embodiment, the invention may provide an anti-Pb-DOTAM antibody comprising at least one, two, three, four, five, or six CDRs selected from (a) CDR-H1 comprising the amino acid sequence of SEQ ID NO:1; (b) CDR-H2 comprising the amino acid sequence of SEQ ID NO:2; (c) CDR-H3 comprising the amino acid sequence of SEQ ID NO:3; (d) CDR-L1 comprising the amino acid sequence of SEQ ID NO:4; (e)CDR-L2 comprising the amino acid sequence of SEQ ID NO:5; and (f) CDR-L3 comprising the amino acid sequence of SEQ ID NO: 6.

In one embodiment, the invention provides an antibody comprising at least one, at least two, or all three VH CDR sequences selected from (a) CDR-H1 comprising the amino acid sequence of SEQ ID NO:1; (b) CDR-H2 comprising the amino acid sequence of SEQ ID NO:2; and (c) CDR-H3 comprising the amino acid sequence of SEQ ID NO:3. In one embodiment, the antibody comprises CDR-H3 comprising the amino acid sequence of SEQ ID NO:3. In another embodiment, the antibody comprises CDR-H3 comprising the amino acid sequence of SEQ ID NO:3 and CDR-L3 comprising the amino acid sequence of SEQ ID NO:6. In a further embodiment, the antibody comprises CDR-H3 comprising the amino acid sequence of SEQ ID NO:3, CDR-L3 comprising the amino acid sequence of SEQ ID NO:6, and CDR-H2 comprising the amino acid sequence of SEQ ID NO:2. In a further embodiment, the antibody comprises CDR-H3 comprising the amino acid sequence of SEQ ID NO:3, CDR-L3 comprising the amino acid sequence of SEQ ID NO:6, CDR-H2 comprising the amino acid sequence of SEQ ID NO:2 and CDR-L1 comprising the amino acid sequence of SEQ ID NO:4. In a further embodiment, the antibody comprises (a) CDR-H1 comprising the amino acid sequence of SEQ ID NO:1; (b) CDR-H2 comprising the amino acid sequence of SEQ ID NO:2; and (c) CDR-H3 comprising the amino acid sequence of SEQ ID NO:3.

In another embodiment the invention provides an antibody comprising at least one, at least two, or all three VL CDR sequences selected from (a) CDR-L1 comprising the amino acid sequence of SEQ ID NO:4; (b) CDR-L2 comprising the amino acid sequence of SEQ ID NO:5; and (c) CDR-L3 comprising the amino acid sequence of SEQ ID NO:6. In one embodiment, the antibody comprises (a) CDR-L1 comprising the amino acid sequence of SEQ ID NO:4; (b) CDR-L2 comprising the amino acid sequence of SEQ ID NO:5; and (c) CDR-L3 comprising the amino acid sequence of SEQ ID NO:6.

In another embodiment, an antibody of the invention comprises (a) a VH domain comprising at least one, at least two, or all three VH CDR sequences selected from (i) CDR-H1 comprising the amino acid sequence of SEQ ID NO:1, (ii) CDR-H2 comprising the amino acid sequence of SEQ ID NO:2, and (iii) CDR-H3 comprising an amino acid sequence selected from SEQ ID NO:3; and (b) a VL domain comprising at least one, at least two, or all three VL CDR sequences selected from (i) CDR-L1 comprising the amino acid sequence of SEQ ID NO:4, (ii) CDR-L2 comprising the amino acid sequence of SEQ ID NO:5, and (c) CDR-L3 comprising the amino acid sequence of SEQ ID NO:6.

In another embodiment, the invention provides an antibody comprising (a) CDR-H1 comprising the amino acid sequence of SEQ ID NO:1; (b) CDR-H2 comprising the amino acid sequence of SEQ ID NO:2; (c) CDR-H3 comprising the amino acid sequence of SEQ ID NO:3; (d) CDR-L1 comprising the amino acid sequence of SEQ ID NO:4; (e) CDR-L2 comprising the amino acid sequence of SEQ ID NO:5; and (f) CDR-L3 comprising an amino acid sequence selected from SEQ ID NO:6.

In some embodiments, the antibodies may comprise one or more of CDR-H1, CDR-H2, CDR-H3, CDR-L1, CDR-L2 and/or CDR-L3 having substitutions as compared to the amino acid sequences of SEQ ID NO:s 1-6, respectively, e.g., 1, 2 or 3 substitutions.

In antibodies of the present invention, CDR-H2 comprises the amino acid sequence FIGSRGDTYYASWAKG (SEQ ID NO:2), or a variant thereof having up to 1, 2, or 3 substitutions in SEQ ID NO: 2, wherein these substitutions do not include Phe50, Asp56 and Tyr58, and optionally also do not include Gly52 and/or Arg 54, all numbered according to Kabat.

In some embodiments, CDR-H2 may be substituted at one or more positions as shown below, excluding the residues mentioned above as fixed. Here and in the substitution tables that follow, substitutions are based on the germline residues (underlined) or by amino acids which theoretically sterically fit and also occur in the crystallized repertoire at the site.

| WolfGuy | Kabat | AA | Substitution |
|---|---|---|---|
| 251 | 50 | F | <u>Y</u>, <u>H</u> |

(continued)

| WolfGuy | Kabat | AA | Substitution |
|---|---|---|---|
| 252 | 51 | I | |
| 253 | 52 | G | |
| 254 | 53 | S | A, G, T, I, N |
| 288 | 54 | R | A, D, G, N, S, T, F, Y |
| 289 | 55 | G | D, S, Y, T, A, N, R, V |
| 290 | 56 | D | |
| 291 | 57 | T | K, I, A, P, S |
| 292 | 58 | Y | F, W, H |
| 293 | 59 | Y | N, F, H, L, S |
| 294 | 60 | A | G, N, S, T |
| 295 | 61 | S | A, G, N, Q, T |
| 296 | 62 | W | K, P, S, A, T, D, N, R, Q |
| 297 | 63 | A | F, L, V, M, I |
| 298 | 64 | K | N, Q, R, E |
| 299 | 65 | G | S, T, D, N, A |

In antibodies of the present invention, CDR-H3 comprises the amino acid sequence ERDPYGGGAYPPHL (SEQ ID NO:3), or a variant thereof having up to 1, 2, or 3 substitutions in SEQ ID NO: 3, wherein these substitutions do not include Glu95, Arg96, Asp97, Pro98, and optionally also do not include Ala100C, Tyr100D, and/or Pro100E and/or optionally also do not include Tyr99. For instance, in some embodiments the substitutions do not include Glu95, Arg96, Asp97, Pro98, Tyr99 Ala100C and Tyr100D.

In certain embodiments, CDR-H3 may be substituted at one or more positions as shown below, excluding the residues mentioned above as fixed.

| WolfGuy | Kabat | AA | Substitution |
|---|---|---|---|
| 351 | 95 | E | |
| 352 | 96 | R | K, E |
| 353 | 97 | D | |
| 354 | 98 | P | |
| 355 | 99 | Y | F, G, S, T, D |
| 356 | 100 | G | |
| 392 | 100A | G | |
| 393 | 100B | G | |
| 394 | 100C | A | S, T |
| 395 | 100D | Y | F |
| 396 | 100E | P | |
| 397 | 100F | P | |
| 398 | 101 | H | A, T, V, D |
| 399 | 102 | L | Y, V, I, H, F |

In antibodies of the present invention, CDR-L1 comprises the amino acid sequence QSSHSVYSDNDLA (SEQ

ID NO:4) or a variant thereof having up to 1, 2, or 3 substitutions in SEQ ID NO: 4, wherein these substitutions do not include Tyr28 and Asp32 (Kabat numbering).

In certain embodiments, CDR-L1 may be substituted at one or more positions as shown below excluding the residues mentioned above as fixed.

| WolfGuy | Kabat | AA | Substitution |
|---|---|---|---|
| 551 | 24 | Q | R, K |
| 552 | 25 | S | A, G |
| 554 | 26 | S | T |
| 555 | 27 | H | Q, S, R, K |
| 556 | 27A | S | Q |
| 557 | 27B | V | I, D, N |
| 561 | 28 | Y | F |
| 562 | 29 | S | T, V |
| 571 | 30 | D | R, S, N, G |
| 572 | 31 | N | K |
| 597 | 32 | D | |
| 598 | 33 | L | I, V, M |
| 599 | 34 | A | S |

In antibodies of the present invention, CDR-L3 comprises the amino acid sequence LGGYDDESDTYG (SEQ ID NO:6) or a variant thereof having up to 1, 2, or 3 substitutions in SEQ ID NO: 6, wherein these substitutions do not include Gly91, Tyr92, Asp93, Thr95c and Tyr96 (Kabat).

In certain embodiments, CDR-L3 may be substituted at the following positions as shown below, excluding the residues mentioned above as fixed. (Since most residues are solvent exposed and without antigen contacts, many substitutions are conceivable).

| WolfGuy | Kabat | AA | Substitution |
|---|---|---|---|
| 751 | 89 | L | A, V, Q |
| 752 | 90 | G | A |
| 753 | 91 | G | |
| 754 | 92 | Y | A, D, E, F, G, H, I, K, L, N, Q, R, S, T, V |
| 755 | 93 | D | A, E, F, G, H, I, K, L, M, N, Q, R, S, T, V, W, Y |
| 756 | 94 | D | A, E, F, G, H, I, K, L, M, N, Q, R, S, T, V, W, Y |
| 794 | 95 | E | A, D, F, G, H, I, K, L, M, N, Q, R, S, T, V, W, Y |
| 795 | 95A | S | A, F, G, H, I, K, L, M, N, Q, R, T, V, W, Y |
| 796 | 95B | D | A, E, F, G, H, I, L, M, N, Q, S, T, V, W, Y |
| 797 | 95C | T | S |
| 798 | 96 | Y | F, H, R |
| 799 | 97 | G | A, E, I, K, L, M, N, Q, S, T, V |

The antibodies of the present invention further comprise CDR-H1 and CDR-L2, optionally having the sequence of SEQ ID NO: 1 or SEQ ID NO: 5 respectively, or a variant thereof having at least 1, 2 or 3 substitutions relative thereto, optionally conservative substitutions.

In any of the above embodiments, the anti-Pb-DOTAM antibody may be humanized. In one embodiment, an

anti-Pb-DOTAM antibody comprises CDRs as in any of the above embodiments, and further comprises an acceptor human framework, e.g. a human immunoglobulin framework or a human consensus framework. In another embodiment, an anti-Pb-DOTAM antibody comprises CDRs as in any of the above embodiments, and further comprises framework regions derived from vk 1 39 and/or vh 2 26. For vk 1 39, in some embodiments there may be no back mutations. For vh 2 26, the germline Ala49 residue may be backmutated to Gly49.

Optionally, the antigen binding site may comprise a heavy chain variable domain (VH) comprising an amino acid sequence selected from the group consisting of SEQ ID NO: 7 or SEQ ID NO 9, or a variant thereof comprising an amino acid sequence having at least 90, 91, 92, 93, 94, 95, 96, 97, 98, or 99% identity to SEQ ID NO: 7 or SEQ ID NO: 9. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an antibody comprising that sequence retains the ability to bind to Pb-DOTAM, preferably with an affinity as described herein. The VH sequence may retain the invariant residues as set out above. In certain embodiments, a total of 1 to 10 amino acids have been substituted, inserted and/or deleted in SEQ ID NO: 7 or SEQ ID NO 9. In certain embodiments, substitutions, insertions, or deletions occur in regions outside the CDRs (i.e., in the FRs). Optionally, the antibody comprises the VH sequence in SEQ ID NO:7 or SEQ ID NO: 9, including post-translational modifications of that sequence. In a particular embodiment, the VH comprises one, two or three CDRs selected from: (a) CDR-H1 comprising the amino acid sequence of SEQ ID NO:1, (b) CDR-H2 comprising the amino acid sequence of SEQ ID NO:2, and (c) CDR-H3 comprising the amino acid sequence of SEQ ID NO:3.

Optionally, the anti-Pb-DOTAM antibody comprises a light chain variable domain (VL) having at least 90%, 91%, 92%, 93%, 94%, 95%, 96%, 97%, 98%, 99%, or 100% sequence identity to the amino acid sequence of SEQ ID NO:8 or SEQ ID NO: 10. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-Pb-DOTAM antibody comprising that sequence retains the ability to bind to Pb-DOTAM, preferably with an affinity as described herein. The VL sequence may retain the invariant residues as set out above. In certain embodiments, a total of 1 to 10 amino acids have been substituted, inserted and/or deleted in SEQ ID NO:8 or SEQ ID NO: 10. In certain embodiments, the substitutions, insertions, or deletions occur in regions outside the CDRs (i.e., in the FRs). Optionally, the anti-Pb-DOTAM antibody comprises the VL sequence in SEQ ID NO:8 or SEQ ID NO: 10, including post-translational modifications of that sequence. In a particular embodiment, the VL comprises one, two or three CDRs selected from (a) CDR-L1 comprising the amino acid sequence of SEQ ID NO:4; (b) CDR-L2 comprising the amino acid sequence of SEQ ID NO:5; and (c) CDR-L3 comprising the amino acid sequence of SEQ ID NO:6.

Optionally, the anti-Pb-DOTAM antibody comprises a VH as in any of the embodiments provided above, and a VL as in any of the embodiments provided above. In one embodiment, the antibody comprises the VH and VL sequences in SEQ ID NO: 7 and SEQ ID NO:8, respectively, including post-translational modifications of those sequences. In another embodiment, the antibody comprises the VH and VL sequences in SEQ ID NO: 9 and SEQ ID NO:10, respectively, including post-translational modifications of those sequences.

In a further aspect of the invention, an antibody according to any of the above embodiments is a monoclonal antibody, including a chimeric, humanized or human antibody. In one embodiment, the antibody is an antibody fragment, e.g., a Fv, Fab, Fab',scFab, scFv, diabody, or $F(ab')_2$ fragment. Antibody fragments include, but are not limited to, Fab, Fab', Fab'-SH, $F(ab')_2$, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and $F(ab')_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain, or all or a portion of the light chain variable domain of an antibody. In certain embodiments, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

In another embodiment, the antibody is a full length antibody, e.g., an intact IgG antibody or other antibody class or isotype as defined herein.

Targeted agents

In some aspects, an antibody that specifically binds to DOTAM-chelated Pb is coupled to a cell binding agent/targeting moiety to produce a targeted agent. Optionally, the antibody that specifically binds to DOTAM-chelated Pb may be an antibody according to any of the embodiments described above.

The coupling may preferably be by expression as a fusion polypeptide or protein. Fusion may be direct or via a linker. The fusion polypeptide or protein may be produced recombinantly, avoiding any need for conjugation chemistry. Optionally said linker may be a peptide of at least 5 amino acids, preferably between 25 and 50 amino acids. The linker may be a rigid linker or a flexible linker. In some embodiments, it is a flexible comprising or consisting of Thr, Ser, Gly and/or Ala residues. For example, it may comprise or consist of Gly and Ser residues. In some embodiments it may have a repeating motif such as (Gly-Gly-Gly-Gly-Ser)n, where n is for instance 1, 2, 3, 4, 5, 6, 7, 8, 9 or 10. In some embodiments, the linker may be or may comprise the sequence GGGGSGGGGSGGGGSGGGGS (SEQ ID NO: 26). Other linkers may be used and could be identified by the skilled person.

Thus, there is provided a multispecific (e.g., bispecific) antibody complex that specifically binds both to a Pb-DOTAM chelate and to another target antigen, e.g., an antigen present on the surface of a target cell.

Insofar as the application describes treatment methods and products for use in methods of treatment, it is applicable to any condition that is treatable by cytotoxic activity targeted to diseased cells of the patient. The treatment is preferably of a tumour or cancer (e.g. pancreatic, breast or prostate cancer). However, the applicability of the invention is not limited to tumours and cancers. For example, the treatment may also be of viral infection. Immunotoxins directed against viral antigens expressed on the surface of infected cells have been investigated for a variety of viral infections such as HIV, rabies and EBV. Cai and Berger 2011 Antiviral Research 90(3):143-50 used an immunotoxin containing PE38 for targeted killing of cells infected with Kaposi's sarcoma-associated herpesvirus. In addition, Resimmune® (A-dmDT390-bisFv(UCHT1)) selectively kills human malignant T cells and transiently depletes normal T cell and is considered to have potential for the treatment of T-cell driven autoimmune diseases such as multiple sclerosis and graft-versus-host disease, as well as T cell blood cancers for which it is undergoing clinical trials.

Thus, suitable target antigens may include cancer cell antigens, particularly human cancer cell antigens, viral antigens or microbial antigens.

The targeted antibodies described herein are designed to bind to diseased cells such as tumour cells via their cell surface antigens. The antigens are usually normal cell surface antigens which are either over-expressed or expressed at abnormal times. Ideally the target antigen is expressed only on diseased cells (such as tumour cells), however this is rarely observed in practice. As a result, target antigens are usually selected on the basis of differential expression between diseased and healthy tissue.

Thus, the targeted antibody may specifically bind to any suitable cell surface marker. The choice of a particular targeting moiety and/or cell surface marker may be chosen depending on the particular cell population to be targeted. Cell surface markers are known in the art (see, e.g., Mufson et al., Front. Biosci., 11:337-43 (2006); Frankel et al., Clin. Cancer Res., 6:326-334 (2000); and Kreitman et al., AAPS Journal, 8(3): E532-E551 (2006)) and may be, for example, a protein or a carbohydrate. In an embodiment of the invention, the targeting moiety (cell-binding agent) is a ligand that specifically binds to a receptor on a cell surface. Exemplary ligands include, but are not limited to, vascular endothelial growth factor (VEGF), Fas, TNF-related apoptosis-inducing ligand (TRAIL), a cytokine (e.g., IL-2, IL-15, IL-4, IL-13), a lymphokine, a hormone, and a growth factor (e.g., transforming growth factor (TGFa), neuronal growth factor, epidermal growth factor).

The cell surface marker can be, for example, a tumour-associated antigen.

The term "tumour-associated antigen" or "tumour specific antigen" as used herein refers to any molecule (e.g., protein, peptide, lipid, carbohydrate, etc.) solely or predominantly expressed or over-expressed by tumour cells and/or cancer cells, such that the antigen is associated with the tumour(s) and/or cancer(s). The tumour-associated antigen can additionally be expressed by normal, non-tumour, or non-cancerous cells. However, in such cases, the expression of the tumour-associated antigen by normal, non-tumour, or non-cancerous cells is not as robust as the expression by tumour or cancer cells. In this regard, the tumour or cancer cells can over-express the antigen or express the antigen at a significantly higher level, as compared to the expression of the antigen by normal, non-tumour, or non-cancerous cells. Also, the tumour-associated antigen can additionally be expressed by cells of a different state of development or maturation. For instance, the tumour-associated antigen can be additionally expressed by cells of the embryonic or fetal stage, which cells are not normally found in an adult host. Alternatively, the tumour-associated antigen can be additionally expressed by stem cells or precursor cells, which cells are not normally found in an adult host.

The tumour-associated antigen can be an antigen expressed by any cell of any cancer or tumour, including the cancers and tumours described herein. The tumour-associated antigen may be a tumour-associated antigen of only one type of cancer or tumour, such that the tumour-associated antigen is associated with or characteristic of only one type of cancer or tumour. Alternatively, the tumour-associated antigen may be a tumour-associated antigen (e.g., may be characteristic) of more than one type of cancer or tumour. For example, the tumour-associated antigen may be

expressed by both breast and prostate cancer cells and not expressed at all by normal, non-tumour, or non-cancer cells.

Exemplary tumour-associated antigens to which the cell-binding agent may specifically bind include, but are not limited to, mucin 1 (MUCl; tumour-associated epithelial mucin), preferentially expressed antigen of melanoma (PRAME), carcinoembryonic antigen (CEA), prostate specific membrane antigen (PSMA), PSCA, EpCAM, Trop2, granulocyte-macrophage colony-stimulating factor receptor (GM-CSFR), CD56, human epidermal growth factor receptor 2 (HER2/neu) (also known as erbB-2), CDS, CD7, tyrosinase related protein (TRP) I, and TRP2. In a preferred embodiment, the cell surface marker, to which the targeting moiety (cell-binding agent) specifically binds, is selected from the group consisting of cluster of differentiation (CD) 19, CD20, CD21, CD22, CD25, CD30, CD33 (sialic acid binding Ig-like lectin 3, myeloid cell surface antigen), CD79b, CD123 (interleukin 3 receptor alpha), transferrin receptor, EGF receptor, mesothelin, cadherin, Lewis Y, Glypican-3, FAP (fibroblast activation protein alpha), PSMA (prostate specific membrane antigen), CA9 = CAIX (carbonic anhydrase IX), L1 CAM (neural cell adhesion molecule L1), endosialin, HER3 (activated conformation of epidermal growth factor receptor family member 3), Alkl/BMP9 complex (anaplastic lymphoma kinase 1/bone morphogenetic protein 9), TPBG = 5T4 (trophoblast glycoprotein), ROR1 (receptor tyrosine kinase-like surface antigen), HER1 (activated conformation of epidermal growth factor receptor), and CLL1 (C-type lectin domain family 12, member A). Mesothelin is expressed in, e.g., ovarian cancer, mesothelioma, non-small cell lung cancer, lung adenocarcinoma, fallopian tube cancer, head and neck cancer, cervical cancer, and pancreatic cancer. CD22 is expressed in, e.g., hairy cell leukemia, chronic lymphocytic leukemia (CLL), prolymphocytic leukemia (PLL), non-Hodgkin's lymphoma, small lymphocytic lymphoma (SLL), and acute lymphatic leukemia (ALL). CD25 is expressed in, e.g., leukemias and lymphomas, including hairy cell leukemia and Hodgkin's lymphoma. Lewis Y antigen is expressed in, e.g., bladder cancer, breast cancer, ovarian cancer, colorectal cancer, esophageal cancer, gastric cancer, lung cancer, and pancreatic cancer. CD33 is expressed in, e.g., acute myeloid leukemia (AML), chronic myelomonocytic leukemia (CML), and myeloproliferative disorders.

In an embodiment of the invention, the targeting moiety is an antibody (including an antibody fragment) that specifically binds to the target e.g., the tumour-associated antigen. In such embodiments, the agent may be referred to as a bispecific or multispecific antibody.

Exemplary antibodies that specifically bind to tumour-associated antigens include, but are not limited to, antibodies against the transferrin receptor (e.g., HB21 and variants thereof), antibodies against CD22 (e.g., RFB4 and variants thereof), antibodies against CD25 (e.g., anti-Tac and variants thereof), antibodies against mesothelin (e.g., SS 1, MORAb-009, SS, HN1, HN2, MN, MB, and variants thereof) and antibodies against Lewis Y antigen (e.g., B3 and variants thereof). In this regard, the targeting moiety (cell-binding agent) may be an antibody selected from the group consisting ofB3, RFB4, SS, SS1, MN, MB, HN1, HN2, HB21, and MORAb-009, and antigen binding portions thereof. Further exemplary targeting moieties suitable for use in the inventive chimeric molecules are disclosed e.g., in U.S. Patents 5,242,824 (anti-transferrin receptor); 5,846,535 (anti-CD25); 5,889,157 (anti-Lewis Y); 5,981,726 (anti-Lewis Y); 5,990,296 (anti-Lewis Y); 7,081,518 (anti-mesothelin); 7,355,012 (anti-CD22 and anti-CD25); 7,368,110 (anti-mesothelin); 7,470,775 (anti-CD30); 7,521,054 (anti-CD25); and 7,541,034 (anti-CD22); U.S. Patent Application Publication 2007/0189962 (anti-CD22); Frankel et al., Clin. Cancer Res., 6: 326-334 (2000), and Kreitman et al., AAPS Journal, 8(3): E532-E551 (2006).

Antibodies have been raised to target specific tumour related antigens including: Cripto, CD30, CD19, CD33, Glycoprotein NMB, CanAg, Her2 (ErbB2/Neu), CD56 (NCAM), CD22 (Siglec2), CD33 (Siglec3), CD79, CD138, PSCA, PSMA (prostate specific membrane antigen), BCMA, CD20, CD70, E-selectin, EphB2, Melanotransferin, Muc16 and TMEFF2.

In some embodiments of the present invention, it may be preferred that the tumour-associated antigen is carcinoembryonic antigen (CEA). CEA may have the amino acid sequence of human CEA, in particular Carcinoembryonic antigen-related cell adhesion molecule 5 (CEACAM5), which is shown in UniProt (www.uniprot.org) accession no. P06731 (version 119), or NCBI (www.ncbi.nlm.nih.gov/) RefSeq NP_004354.2. Antibodies that have been raised against CEA include T84.66 and humanized and chimeric versions thereof, such as T84.66-LCHA as described in WO2016/075278 A1 and/or WO2017/055389, CH1A1a, an anti-CEA antibody as described in WO2011/034660, and CEA hMN-14 as described in table 2 below (see also US 6 676 924 and US 5 874 540).

CEA is advantageous in the context of the present invention because it is relatively slowly internalized, and thus a high percentage of the antibody will remain available on the surface of the cell after initial treatment, for binding to the radionuclide. Other low internalizing targets/tumour associated antigens may also be preferred. For instance, in some embodiments, the tumour-associated antigen may be CD20 or HER2. GenBank Accession Nos.: NP_001005862, NP_004439, XP_005257196, and XP_005257197 disclose Her2 protein sequences, as provided by GenBank on October 4, 2013, and the SwissProt database entry P11836 discloses a CD20 sequence. In still further embodiments, the target may be EGP-1 (epithelial glycoprotein-1, also known as trophoblast-2), colon-specific antigen-p (CSAp) or a pancreatic mucin MUC1. See for instance Goldenberg et al 2012 (Theranostics 2(5)). This reference also describes antibodies such as Mu-9 binding to CSAp (see also Sharkey et al Cancer Res. 2003; 63: 354-63), hPAM4 binding to MUC1 (see also Gold et al Cancer Res. 2008: 68: 4819-26), valtuzumab binding to CD20 (see also Sharkey et al Cancer Res. 2008;

68: 5282-90) and hRS7 which binds to EGP-1 (see also Cubas et al Biochim Biophys Acta 2009; 1796: 309-14). Any of these or antigen-binding portions thereof may be useful in the present invention, i.e., may be incorporated into the antibodies described herein.

Multispecific Antibodies

As discussed above, in certain embodiments, an antibody provided herein is a multispecific antibody, e.g. a bispecific antibody. Multispecific antibodies are monoclonal antibodies that have binding specificities for at least two different sites. Bispecific antibodies can be prepared as full length antibodies or antibody fragments.

A wide variety of recombinant antibody formats have been developed in the recent past, e.g. tetravalent bispecific antibodies by fusion of, e.g., an IgG antibody format and single chain domains (see e.g. Coloma, M.J., et al., Nature Biotech 15 (1997) 159-163; WO 2001/077342; and Morrison, S.L., Nature Biotech 25 (2007) 1233-1234).

Also several other new formats wherein the antibody core structure (IgA, IgD, IgE, IgG or IgM) is no longer retained such as dia-, tria- or tetrabodies, minibodies, several single chain formats (scFv, Bis-scFv), which are capable of binding two or more antigens, have been developed (Holliger, P., et al., Nature Biotech 23 (2005) 1126-1136; Fischer, N., Leger, 0., Pathobiology 74 (2007) 3-14; Shen, J., et al., Journal of Immunological Methods 318 (2007) 65-74; Wu, C., et al., Nature Biotech. 25 (2007) 1290-1297).

All such formats use linkers either to fuse the antibody core (IgA, IgD, IgE, IgG or IgM) to a further binding protein (e.g. scFv) or to fuse e.g. two Fab fragments or scFvs (Fischer, N., Leger, 0., Pathobiology 74 (2007) 3-14). It has to be kept in mind that one may want to retain effector functions, such as e.g. complement dependent cytotoxicity (CDC) or antibody dependent cellular cytotoxicity (ADCC), which are mediated through the Fc receptor binding, by maintaining a high degree of similarity to naturally occurring antibodies.

In WO 2007/024715 are reported dual variable domain immunoglobulins as engineered multivalent and multispecific binding proteins. A process for the preparation of biologically active antibody dimers is reported in US 6,897,044. Multivalent Fv antibody construct having at least four variable domains which are linked with each over via peptide linkers are reported in US 7,129,330. Dimeric and multimeric antigen binding structures are reported in US 2005/0079170. Tri- or tetra-valent monospecific antigen-binding protein comprising three or four Fab fragments bound to each other covalently by a connecting structure, which protein is not a natural immunoglobulin are reported in US 6,511,663. In WO 2006/020258 tetravalent bispecific antibodies are reported that can be efficiently expressed in prokaryotic and eukaryotic cells, and are useful in therapeutic and diagnostic methods. A method of separating or preferentially synthesizing dimers which are linked via at least one interchain disulfide linkage from dimers which are not linked via at least one interchain disulfide linkage from a mixture comprising the two types of polypeptide dimers is reported in US 2005/0163 782. Bispecific tetravalent receptors are reported in US 5,959,083. Engineered antibodies with three or more functional antigen binding sites are reported in WO 2001/077342.

Multispecific and multivalent antigen-binding polypeptides are reported in WO 1997/001580. WO 1992/004053 reports homoconjugates, typically prepared from monoclonal antibodies of the IgG class which bind to the same antigenic determinant are covalently linked by synthetic cross-linking. Oligomeric monoclonal antibodies with high avidity for antigen are reported in WO 1991/06305 whereby the oligomers, typically of the IgG class, are secreted having two or more immunoglobulin monomers associated together to form tetravalent or hexavalent IgG molecules. Sheep-derived antibodies and engineered antibody constructs are reported in US 6,350,860, which can be used to treat diseases wherein interferon gamma activity is pathogenic. In US 2005/0100543 are reported targetable constructs that are multivalent carriers of bispecific antibodies, i.e., each molecule of a targetable construct can serve as a carrier of two or more bispecific antibodies. Genetically engineered bispecific tetravalent antibodies are reported in WO 1995/009917. In WO 2007/109254 stabilized binding molecules that consist of or comprise a stabilized scFv are reported.

Multi-specific antibodies may also be provided in an asymmetric form with a domain crossover in one or more binding arms of the same antigen specificity, i.e. by exchanging the VH/VL domains (see e.g., WO 2009/080252 and WO 2015/150447), the CH1/CL domains (see e.g., WO 2009/080253) or the complete Fab arms (see e.g., WO 2009/080251, WO 2016/016299, also see Schaefer et al, PNAS, 108 (2011) 1187-1191, and Klein at al., MAbs 8 (2016) 1010-20). In one aspect, the multispecific antibody comprises a cross-Fab fragment. The term "cross-Fab fragment" or "xFab fragment" or "crossover Fab fragment" refers to a Fab fragment, wherein either the variable regions or the constant regions of the heavy and light chain are exchanged. A cross-Fab fragment comprises a polypeptide chain composed of the light chain variable region (VL) and the heavy chain constant region 1 (CH1), and a polypeptide chain composed of the heavy chain variable region (VH) and the light chain constant region (CL). Asymmetrical Fab arms can also be engineered by introducing charged or non-charged amino acid mutations into domain interfaces to direct correct Fab pairing. See e.g., WO 2016/172485.

Any of the above formats may be used for multispecific antibodies according to the present invention.

In one exemplary format, the bispecific antibody is a "trimerizer", e.g., as described in WO214/180754. This refers to a trimeric antigen binding molecule comprising three fusion polypeptides, each comprising at least one antigen

binding moiety fused to a trimerization domain derived from human cartilage matrix protein (CMP), wherein said trimerization domain is capable of mediating stable association of the trimeric antigen binding molecule. The antigen binding moieties may be, for instance, a Fab molecule, a crossover-Fab molecule, a scFab, an Fv molecule, an scFv, or a single domain antibody (VHH). In some embodiments, the fusion proteins each comprise two (a first and a second) antigen binding moieties, e.g,. where the first antigen binding moiety is fused to the N-terminal amino acid of said trimerization domain and the second antigen binding moiety is fused to the C-terminal amino acid of said trimerization domain, both optionally through a peptide linker. In this format, either the first or the second antigen binding moiety may bind the Pb-DOTAM chelate. The other will bind the target antigen e.g., a tumour-associated antigen. The three antigen binding molecules fused to the C-terminus may each be specific for the same antigen; the three antigen binding molecules fused to the N-terminus may each be specific for the other.

The CMP trimerization domain useful therein has been derived from human cartilage protein as shown below and in one embodiment comprises a sequence having at least 95% identity and most preferably at least 98% identity to the sequence of the trimerization domain shown below. In one embodiment said trimerization domain comprises the sequence of said trimerization domain.

Sequence of human cartilage protein (496aa)

MRVLSGTSLM LCSLLLLLQA LCSPGLAPQS RGHLCRTRPT DLVFVVDSSR
SVRPVEFEKV KVFLSQVIES LDVGPNATRV GMVNYASTVK QEFSLRAHVS
KAALLQAVRR IQPLSTGTMT GLAIQFAITK AFGDAEGGRS RSPDISKVVI

VVTDGRPQDS VQDVSARARA SGVELFAIGV GSVDKATLRQ IASEPQDEHV
DYVESYSVIE KLSRKFQEAF CVVSDLCATG DHDCEQVCIS SPGSYTCACH
EGFTLNSDGK TCNVCSGGGG SSATDLVFLI DGSKSVRPEN FELVKKFISQ
IVDTLDVSDK LAQVGLVQYS SSVRQEFPLG RFHTKKDIKA AVRNMSYMEK
GTMTGAALKY LIDNSFTVSS GARPGAQKVG IVFTDGRSQD YINDAAKKAK
DLGFKMFAVG VGNAVEDELR EIASEPVAEH YFYTADFKTI NQIGKKLQKK
ICVEEDPCAC ESLVKFQAKV EGLLQALTRK LEAVSKRLAI LENTVV

Exemplary sequence of trimerization domain (39aa)
CACESLVKFQ AKVEGLLQAL TRKLEAVSKR LAILENTVV

Another exemplary format comprises a full-length antibody (e.g., an IgG) comprising a first and second antibody heavy chain and a first and second antibody light chain, wherein the first heavy chain and the first light chain assemble to form an antigen binding site for the first antigen, and wherein the second heavy chain and second light chain assemble to form an antigen binding site for the second antigen.

Correct assembly of the heterodimeric heavy chains can be assisted e.g. by the use of knob into hole mutations and/or other modifications as discussed further below.

Correct assembly of the light chains with their respective heavy chain can be assisted by using cross-mab technology. In this approach, either the first heavy chain and the first light chain, or the second heavy chain and the second light chain, can assemble to form a cross-Fab fragment (while the others assemble to form a conventional Fab). Thus, in one embodiment, the first heavy chain may comprise a VL domain in place of the VH domain (e.g., VL-CH1-hinge-CH2-CH3) and the first light chain may comprise a VH domain exchanged for the VL domain (e.g., VH-CL), or the first heavy chain may comprise a CL domain in place of the HC1 domain (e.g., VH-CL-hinge-CH2-CH3) and the first light chain may comprise a CH1 domain in place of the CL domain (e.g., VL-CH1). In this embodiment, the second heavy chain and the second light chain have the conventional domain structure (e.g., VH-CH1-hinge-CH2-CH3 and VL-CL, respectively). In an alternative embodiment, the second heavy chain may comprise a VL domain in place of the VH domain (e.g., VL-CH1-hinge-CH2-CH3) and the second light chain may comprise a VH domain exchanged for the VL domain (e.g., VH-CL), or the second heavy chain may comprise a CL domain in place of the HC1 domain (e.g., VH-CL-hinge-CH2-CH3) and the second light chain may comprise a CH1 domain in place of the CL domain (e.g., VL-CH1) . In this embodiment, the first heavy chain and the first light chain have the conventional domain structure.

In some embodiments, correct assembly of the light chains with their respective heavy chain can additionally or alternatively be assisted by using charge modification, as discussed further below.

One such antibody is shown in figure 37 as P1AE1768. Here, the second heavy chain comprises a CL domain

in place of the HC1 domain (e.g., VH-CL-hinge-CH2-CH3) and the second light chain comprises a CH1 domain in place of the CL domain (e.g., VL-CH1); the first heavy chain and the first light chain have the conventional domain structure. The Fab with conventional structure comprises charge modification. Thus, in one embodiment, an antibody of the invention comprises a first and second heavy chain of SEQ ID NO: 59 and 58 respectivly, and a first and second light chain of SEQ ID NO: 57 and 60 respectively.

In some embodiments of the above format, the format may be bivalent. In another possible embodiment, further antigen binding moieties may be fused e.g., to the first and/or second heavy chain to increase the valency for one or both antigens. For instance, a further antigen binding moiety for the first antigen may be fused to the N-terminus of one or both of the heavy chain molecules. The antibody may be multivalent, e.g, bivalent, for the first antigen (e.g., the tumour associated antigen) and monovalent for the second antigen (e.g, DOTAM-chelated Pb).

The further antigen binding moiety may for instance be an scFab e.g., comprising an antigen binding site for the first antigen (e.g., the tumour associated antigen). The scFab comprises a VH and CH1 domain, linked via a polypeptide linker to a VL and CL domain, so as to be expressed as a single chain. In other words, the scFab comprises a polypeptide linker between the Fd and the light chain.

In another embodiment, the further antigen binding moiety is a Fab or a cross-Fab. For instance, the N- or C-terminus of one of the heavy chains may be linked via a polypeptide linker to a first polypeptide consisting of a VH domain and a CH1 domain, which associates with a second polypeptide consisting of a VL and CL domain to form a Fab. In another embodiment, the N- or C-terminus of one of the heavy chains may be linked via a polypeptide linker to a first polypeptide consisting of a VL domain and a CH1 domain, which associates with a second polypeptide consisting of a VH and CL domain. In another embodiment, the N- or C-terminus of one of the heavy chains may be linked via a polypeptide linker to a first polypeptide consisting of a VH domain and a CL domain, which associates with a second polypeptide consisting of a VL and CH1 domain.

In this format, it may be preferred that binding arms of the same antigen specificity are formed by association with the same light chain. Thus, the antigen binding moieties/arms for the first antigen may be cross-Fabs, and the antigen binding moiety(s)/arm(s) for the second antigen may be conventional Fabs. Alternatively, the antigen binding moieties/arms for the first antigen may be conventional Fabs, and the antigen binding moiety(s)/arm(s) for the second antigen may be cross-Fabs.

The format may also incoroporate charge modification, as discussed further below.

In one embodiment of this format, there is provided a multivalent antibody comprising

> a full length antibody comprising a first and second antibody heavy chain and a first and second antibody light chain, wherein the first heavy chain and the first light chain assemble to form a Fab comprising an antigen binding site for the first antigen (e.g., a tumour specific antigen, e.g., CEA), and wherein the second heavy chain and second light chain assemble to form a cross-Fab comprising an antigen binding site for the second antigen (e.g., DOTAM-chelated Pb) (e.g., the second heavy chain has a VL domain in place of a VH domain, and the second light chain has a VH domain in place of the VL domain);
>
> and wherein either the first or second antibody heavy chain is fused via a linker to a polypeptide comprising a CH1 and VH domain, and said first polypeptide is assembled with a second polypeptide comprising a CL and VL, such that the first and second polypeptide assemble to form a Fab comprising an antigen binding site for the first antigen.

The fusion may be at the N-terminus of one of the heavy chains of the full length antibody, optionally the second heavy chain.

Optionally, charge modification may also be used. For instance, the Fabs comprising an antigen binding site for the first antigen may comprise charge-modifying substitutions as discussed below.

An example of such a format is P1AE1769 shown in figure 37. Thus, in one embodiment, an antibody of the invention comprises a first and second heavy chain of SEQ ID NO: 64 and 63 respectivly, and a first and second light chain of SEQ ID NO: 62 and 61 respectively.

Another exemplary format comprises a full length antibody such as an IgG comprising an antigen binding site for the first antigen (e.g., which may be divalent for the first antigen), linked to an antigen binding moiety for the second antigen.

For example, the antigen binding moiety for the second antigen may be a scFab comprising an antigen binding site for the second antigen (e.g., the Pb-DOTAM chelate). In some embodiments, the scFab may be fused to the C-terminus of one of the two heavy chains of the full-length antibody, e.g., at the C-terminus of its CH3 domain. Correct assembly of heterodimeric heavy chains may be assisted e.g. by the use of knob into hole mutations and/or other modifications as discussed further below. One such antibody is exemplified in figure 37, as P1AE1770. Thus, in one embodiment, an antibody of the invention comprises heavy chains of SEQ ID NO: 66 and 67, and a light chain of SEQ ID NO: 65.

Another exemplary format comprises a full length antibody comprising an antigen binding site for the first antigen

(e.g., which may be divalent for the first antigen), wherein the N- or C-terminus of one of the heavy chains is linked via a polypeptide linker to a first polypeptide and wherein the first polypeptide associates with a second polypeptide to form a Fab or a cross-Fab comprising a binding site for the second antigen. For instance, this format may comprise:

i) a first polypeptide consisting of a VH domain and a CH1 domain, which is associated with a second polypeptide consisting of a VL and CL domain; or
ii) a first polypeptide consisting of a VL domain and a CH1 domain, which is associated with a second polypeptide consisting of a VH and CL domain; or
iii) a first polypeptide consisting of a VH domain and a CL domain, which is associated with a second polypeptide consisting of a VL and CH1 domain;

such that the first and second polypeptide together form an antigen binding site for a second antigen.

Correct assembly of the heterodimeric heavy chains may be assisted e.g. by the use of knob into hole mutations and/or other modifications as discussed further below, including charge modifications. For instance, the Fab domains of the full-length antibody may include charge modifications.

In one embodiment, the first polypeptide is linked via a polypeptide linker to the C-terminus of one of the heavy chains, e.g., at the C-terminus of its CH3 domain. The first polypeptide may comprise an N-terminal VL domain and a C-terminal CH1 domain. Thus, the heavy chain having the fusion may comprise from N- to C-terminus VH-CH1-hinge-CH2-CH3-linker-VL-CH1. The light chain may comprise VH-CL. The Fabs of the full length antibody may include charge modifying substitutions. One such embodiment is shown in figure 37 as P1AE1767. Thus, in one embodiment, an antibody of the invention comprises heavy chains of SEQ ID NO: 63 and 64, and light chains of SEQ ID NO: 61 and 62.

In another embodiment, the first polypeptide is linked via a polypeptide linker to the N-terminus of the VH domain of the heavy chain. The first polypeptide may comprise an N-terminal VL domain and a C-terminal CH1 domain. Thus, the heavy chain with the fusion may comprise from N- to C-terminus VL-CH1-linker-VH-CH1-hinge-CH2-CH3. The light chain may comprise VH-CL.

In another exemplary format, the antibody may comprise a full-length antibody specifically binding a first antigen and consisting of two antibody heavy chains and two antibody light chains, wherein the C-terminus of each of the heavy chains is fused to an antigen binding moiety specifically binding the second antigen.

In one embodiment, the first antigen is the target, e.g., the tumour specific antigen and the second is the Pb-DOTAM chelate, but these may also be reversed.

In another exemplary format the antibody may be a bispecific antibody comprising:

a) a full length antibody specifically binding a first antigen and consisting of two antibody heavy chains and two antibody light chains;
b) a polypeptide consisting of

  i) an antibody heavy chain variable domain (VH); or
  ii) an antibody heavy chain variable domain (VH) and an antibody heavy chain constant domain (CH1); or
  iii) an antibody heavy chain variable domain (VH) and an antibody light chain constant domain (CL);

wherein said polypeptide is fused with the N-terminus of the VH domain via a peptide linker to the C-terminus of one of the two heavy chains of said full-length antibody;
c) a polypeptide consisting of

  i) an antibody light chain variable domain (VL); or
  ii) an antibody light chain variable domain (VL) and an antibody light chain constant domain (CL); or
  iii) an antibody light chain variable domain (VL) and an antibody heavy chain constant domain (CH1);

  wherein said polypeptide is fused with the N-terminus of the VL domain via a peptide linker to the C-terminus of the other of the two heavy chains of said full-length antibody;
  and wherein the antibody heavy chain variable domain (VL) of the peptide under (b) and the antibody light chain variable domain of the peptide under (c) together form an antigen-binding site to a second antigen.

In this format, if the first polypeptide is as set out in b(i), then the second polypeptide is as set out in c(i); if the first polypeptide is as set out in b(ii), then the second polypeptide is as set out in c(ii); and if the first polypeptide is as set out in b(iii), then the second polypeptide is as set out in c(iii). Charge modifying substitutions may also be used, e.g., in the Fabs of the full length antibody.

In this format, either the first or the second antigen may be DOTAM-chelated Pb. The other may be the target,

e.g., a tumour-associated antigen, e.g. CEA, CD20 or ERBB2. In some embodiments, the second antigen is DOTAM-chelated Pb and the first antigen is the target.

The antibody described above may be trivalent. In another possible embodiment, further antigen binding moieties may be fused to increase the valency for one or both antigens. For instance, a further antigen binding moiety for the first antigen may be fused to the carboxy terminus of either or both of the heavy chain of the full-length antibody (e.g., the tumour associated antigen), e.g., such that the antibody has a valency of 4 for the first antigen (where it is fused to the carboxy terminus of both the heavy chains) and a valency of 1 for the second antigen.

Examples of the format above in which the antibody of (b) consists of a VH domain and the antibody of (c) consists of a VL domain is PRIT-213 and PRIT214. An example of the format above in which (b) consists of a VH domain and a CL domain, and (c) consists of a VL domain and a CH1 domain is P1AE1766, as shown in Figure 37. Thus, in one embodiment, an antibody of the invention comprises a first and second heavy chain of SEQ ID NO: 51 and 52 respectivly, and a light chain of SEQ ID NO: 50.

Optionally, the format used for the multispecific antibodies of the present invention may be the trivalent format as described in WO2010/115589 A1 (Roche Glycart AG).

WO2010/115589 describes optional stabilization of the structure, whereby the antibody heavy chain variable region (VH) of the polypeptide under (b) and the antibody light chain variable domain (VL) of the polypeptide under (c) are linked and stabilized via an interchain disulfide bridge, e.g., by introduction of a disulfide bond between the following positions:

i) heavy chain variable domain positon 44 to light chain variable domain position 100,
ii) heavy chain variable domain position 105 to light chain variable domain position 43, or
iii) heavy chain variable domain position 101 to light chain variable domain positon 100 (numbering always according to EU index of Kabat).

WO2010/115589 also describes that the CH3 domains of said full length antibody according to the invention can be altered by the "knob-into-holes" technology which is described in detail with several examples in e.g. WO 96/027011, Ridgway, J.B., et al., Protein Eng 9 (1996) 617-621; and Merchant, A.M., et al., Nat Biotechnol 16 (1998) 677-681.

Thus in some embodiments said trivalent, bispecific antibody is further characterized in that: the CH3 domain of one heavy chain of the full length antibody and the CH3 domain of the other heavy chain of the full length antibody each meet at an interface which comprises an original interface between the antibody CH3 domains; wherein said interface is altered to promote the formation of the trivalent, bispecific antibody, wherein the alteration is characterized in that:

a) the CH3 domain of one heavy chain is altered,

so that within the original interface the CH3 domain of one heavy chain that meets the original interface of the CH3 domain of the other heavy chain within the trivalent, bispecific antibody,
an amino acid residue is replaced with an amino acid residue having a larger side chain volume, thereby generating a protuberance within the interface of the CH3 domain of one heavy chain which is positionable in a cavity within the interface of the CH3 domain of the other heavy chain
and

b) the CH3 domain of the other heavy chain is altered,
so that within the original interface of the second CH3 domain that meets the original interface of the first CH3 domain within the trivalent, bispecific antibody an amino acid residue is replaced with an amino acid residue having a smaller side chain volume, thereby generating a cavity within the interface of the second CH3 domain within which a protuberance within the interface of the first CH3 domain is positionable.

Said amino acid residue having a larger side chain volume may optionally be selected from the group consisting of arginine (R), phenylalanine (F), tyrosine (Y), tryptophan (W). Said amino acid residue having a smaller side chain volume may optionally be selected from the group consisting of alanine (A), serine (S), threonine (T), valine (V).

Optionally, in some embodiments, both CH3 domains are further altered by the introduction of cysteine (C) as amino acid in the corresponding positions of each CH3 domain such that a disulfide bridge between both CH3 domains can be formed.

These and other details of the bispecific, trivalent antibody format as described in WO2010/115589 A1 may be utilised in the present invention.

As used herein, the term "full length antibody" denotes an antibody consisting of two "full length antibody heavy

chains" and two "full length antibody light chains". A "full length antibody heavy chain" may be a polypeptide consisting in N-terminal to C-terminal direction of an antibody heavy chain variable domain (VH), an antibody constant heavy chain domain 1 (CH1), an antibody hinge region (HR), an antibody heavy chain constant domain 2 (CH2), and an antibody heavy chain constant domain 3 (CH3), abbreviated as VH-CH1-HR-CH2-CH3; and optionally an antibody heavy chain constant domain 4 (CH4) in case of an antibody of the subclass IgE. Preferably the "full length antibody heavy chain" is a polypeptide consisting in N-terminal to C-terminal direction of VH, CH1, HR, CH2 and CH3. The possibility of cross-Mab formation is not intended to be excluded by the reference to "full length" - thus, the heavy chain may have the VH domain swapped for a VL domain, or the CH1 domain swapped for a CL domain. A "full length antibody light chain" may be a polypeptide consisting in N-terminal to C-terminal direction of an antibody light chain variable domain (VL), and an antibody light chain constant domain (CL), abbreviated as VL-CL. Alternatively, in the case of a cross-Mab, the VL domain may be swapped for a VH domain or the CL domain may be swapped for a CH1 domain. The antibody light chain constant domain (CL) can be κ (kappa) or γ (lambda). The two full length antibody chains are linked together via inter-polypeptide disulfide bonds between the CL domain and the CH1 domain and between the hinge regions of the full length antibody heavy chains. Examples of typical full length antibodies are natural antibodies like IgG (e.g. IgG1 and IgG2), IgM, IgA, IgD, and IgE.) The full length antibodies according to the invention can be from a single species e.g. human, or they can be chimerized or humanized antibodies. The full length antibodies as described herein comprise two antigen binding sites each formed by a pair of VH and VL. The C-terminus of the heavy or light chain of said full length antibody denotes the last amino acid at the C-terminus of said heavy or light chain.

The N-terminus of the antibody heavy chain variable domain (VH) of the polypeptide under b) and the antibody light chain variable domain (VL) of the polypeptide under c) denotes the last amino acid at the N- terminus of VH or VL domain.

In any of the formats described above, the first antigen may be a tumour-associated antigen and the second antigen may be Pb-DOTAM, (but these can also be reversed in some embodiments).

In any of the formats described above, the correct assembly of heavy chain heterodimers may be assisted by modifications to the sequence of the heavy chain. In one embodiment, knob-into-hole technology is used. The interaction surfaces of the two CH3 domains may be altered to increase the heterodimerisation of both heavy chains containing these two CH3 domains. Each of the two CH3 domains (of the two heavy chains) can be the "knob", while the other is the "hole". For instance one comprises called "knob mutations" (T366W and optionally one of S354C or Y349C, preferably S354C) and the other comprises the so-called "hole mutations" (T366S, L368A and Y407V and optionally Y349C or S354C, preferably Y349C) (see, e.g., Carter, P. et al., Immunotechnol. 2 (1996) 73) according to EU index numbering.

The introduction of a disulfide bridge may additionally or alternatively be used to stabilize the heterodimers (Merchant, A.M., et al., Nature Biotech 16 (1998) 677-681; Atwell, S., et al., J. Mol. Biol. 270 (1997) 26-35) and increase the yield. Examples include introduction of a disulfide bond between the following positions:

i) heavy chain variable domain positon 44 to light chain variable domain position 100,
ii) heavy chain variable domain position 105 to light chain variable domain position 43, or
iii) heavy chain variable domain position 101 to light chain variable domain positon 100 (numbering always according to EU index of Kabat).

Charge modifications

The multispecific antibodies of the invention may comprise amino acid substitutions in Fab molecules comprised therein which are particularly efficient in reducing mispairing of light chains with non-matching heavy chains (Bence-Jones-type side products), which can occur in the production of Fab-based bi-/multispecific antigen binding molecules with a VH/VL exchange in one (or more, in case of molecules comprising more than two antigen-binding Fab molecules) of their binding arms (see also PCT publication no. WO 2015/150447, particularly the examples therein. The ratio of a desired multispecific antibodies compared to undesired side products, in particular Bence Jones-type side products occurring in one of their binding arms, can be improved by the introduction of charged amino acids with opposite charges at specific amino acid positions in the CH1 and CL domains of a Fab molecule(sometimes referred to herein as "charge modifications").

Therefore, in some embodiments, the antibodies of the present invention comprising Fab molecules, comprises at least one Fab with a heavy chain constant domain CH1 domain comprising charge modifications as described herein, and a light chain constant CL domain comprising charge modifications as described herein.

Charge modifications are made either in the conventional Fab molecule(s) comprised in the antibodies of the present invention (such as shown e.g. in Figure 37: P1AE1766, P1AE1767 P1AE1768, P1AE1769), or in the crossover Fab molecule(s) comprised in the antibodies of the present invention (but not in both). In particular embodiments, the charge modifications are made in the conventional Fab molecule(s) comprised in the antibodies of the present invention (which in particular embodiments specifically bind(s) to the target cell antigen).

Accordingly, in some embodiments the antibodies of the present invention, comprising a) a first antigen binding moiety binding to a first antigen (e.g., a tumour-associated antigen) and b) a second binding moiety binding to a second antigen (e.g. Dotam-Pb) wherein the first and the second antigen binding moiety of the bispecific antigen binding molecule are both Fab molecules, and one of the antigen binding moieties (in some embodiments, particularly the second antigen binding moiety) is a cross-Fab fragment, one of the Fab molecules comprises a CH1 domain comprising charge modifications as described herein, and a CL domain comprising charge modifications as described herein. It may be preferred that the Fab comprising the charge modifications is the conventional (non-cross) Fab, e.g., in some embodiments is the antigen binding moiety binding to the first antigen.

The antibodies according to the invention may further comprise a third Fab molecule which specifically binds to the first antigen. In particular embodiments, said third Fab molecule is identical to the first Fab molecule under a). In these embodiments, the amino acid substitutions according to the following embodiments (charge modifications) may be made in the constant domain CL and the constant domain CH1 of each of the first Fab molecule and the third Fab molecule. Alternatively, the amino acid substitutions according to the following embodiments may be made in the constant domain CL and the constant domain CH1 of the second Fab molecule under b), but not in the constant domain CL and the constant domain CH1 of the first Fab molecule and the third Fab molecule.

In some embodiments, in a Fab molecule comprising a light chain constant domain CL comprising charge modifications and a heavy chain constant domain CH1 comprising charge modifications, charge modifications in the light chain constant domain CL are at position 124 and optionally at position 123 (numbering according to Kabat), and charge modifications in the heavy chain constant domain CH1 are at position 147 and/or 213 (numbering according to Kabat).

In some embodiments, in the light chain constant domain CL the amino acid at position 124 is substituted independently by lysine (K), arginine (R) or histidine (H) (numbering according to Kabat) (in one preferred embodiment independently by lysine (K)), and

in the heavy chain constant domain CH1 the amino acid at position 147 and/or the amino acid at position 213 is substituted independently by glutamic acid (E) or aspartic acid (D) (numbering according to Kabat EU index.

In another embodiment, in the light chain constant domain CL the amino acid at position 124 is substituted independently by lysine (K), arginine (R) or histidine (H) (numbering according to Kabat) (in one preferred embodiment independently by lysine (K) or arginine (R)), and in the heavy chain constant domain CH1 the amino acid at position 147 or the amino acid at position 213 is substituted independently by glutamic acid (E) or aspartic acid (D) (numbering according to Kabat EU index).

In a further embodiment, in the light chain constant domain CL the amino acid at position 124 is substituted independently by lysine (K) or arginine (R) (numbering according to Kabat), (in one preferred embodiment independently by lysine (K) or arginine (R)), and in the heavy chain constant domain CH1 the amino acid at position 213 is substituted independently by glutamic acid (E) or aspartic acid (D) (numbering according to Kabat EU index)

In a further embodiment, in the light chain constant domain CL of the amino acid at position 124 is substituted independently by lysine (K), arginine (R) or histidine (H) (in one preferred embodiment independently by lysine (K) or arginine (R)) (numbering according to Kabat), and in the heavy chain constant domain CH1 the amino acid at position 147 is substituted independently by glutamic acid (E) or aspartic acid (D) (numbering according to Kabat EU index).

In a further embodiment, in the light chain constant domain the amino acid at position 124 is substituted independently by lysine (K), arginine (R) or histidine (H) (numbering according to Kabat) (in one preferred embodiment independently by lysine (K) or arginine (R)) and the amino acid at position 123 is substituted independently by lysine (K), arginine (R) or histidine (H) (numbering according to Kabat) (in one preferred embodiment independently by lysine (K) or arginine (R)),

and in the heavy chain constant domain CH1 the amino acid at position 147 is substituted independently by glutamic acid (E) or aspartic acid (D) (numbering according to Kabat EU index) and the amino acid at position 213 is substituted independently by glutamic acid (E) or aspartic acid (D) (numbering according to Kabat EU index).

In a further embodiment, in the light chain constant domain CL the amino acid at position 124 is substituted by lysine (K) (numbering according to Kabat) and the amino acid at position 123 is substituted by arginine (R) (numbering according to Kabat),

and in the heavy chain constant domain CH1 the amino acid at position 147 is substituted by glutamic acid (E) (numbering according to Kabat EU index) and the amino acid at position 213 is substituted by glutamic acid (E) (numbering according to Kabat EU index).

In a further embodiment, in the light chain constant domain CL the amino acid at position 124 is substituted by lysine (K) (numbering according to Kabat) and the amino acid at position 123 is substituted by lysine (K) (numbering according to Kabat),

and in the heavy chain constant domain CH1 the amino acid at position 147 is substituted by glutamic acid (E) (numbering according to Kabat EU index) and the amino acid at position 213 is substituted by glutamic acid (E) (numbering according to Kabat EU index).

In a further embodiment, in the light chain constant domain CL the amino acid at position 124 is substituted by lysine (K) (numbering according to Kabat) and the amino acid at position 123 is substituted by arginine (R) (numbering according to Kabat),
and in a heavy chain constant domain CH1 the amino acid at position 147 is substituted by glutamic acid (E) (numbering according to Kabat EU index) and the amino acid at position 213 is substituted by aspartic acid (D) (numbering according to Kabat EU index).

In a further embodiment, in the light chain constant domain the amino acid at position 124 is substituted by lysine (K) (numbering according to Kabat) and the amino acid at position 123 is substituted by lysine (K) (numbering according to Kabat),
and the heavy chain constant domain CH1 the amino acid at position 147 is substituted by glutamic acid (E) (numbering according to Kabat EU index) and the amino acid at position 213 is substituted by aspartic acid (D) (numbering according to Kabat EU index).

In one embodiment, the antibody comprises a first heavy chain and a first light chain which specifically binds to a first antigen, and a second heavy chain and a second light which specifically binds to a second antigen, wherein

a) the constant domain CL of the first light chain and the constant domain CH1 of the first heavy chain comprises the charge variant substitutions described herein; and
b) the light chain constant domain CL and the heavy chain constant domain CH1 of the second light chain and the second heavy chain are replaced with each other (thus forming a cross-Fab). An example of such an arrangement is shown for P1AE1768.

In another embodiment, the antibody comprises
a full length antibody specifically binding a first antigen and consisting of two heavy chains and two light chains, wherein the two heavy chain constant domains CH1 and the two light chain constant domains CL of the full length antibody comprise charge modifications as described herein; and
a scFab comprising a VH and CH1 domain linked via a polypeptide linker to a VL and CL domain (VH-CH1-linker-VL-CL), wherein scFab fused to the N-terminus of one of the heavy chains, and wherein the scFab forms an antigen-binding site to a second antigen. An example of such an arrangement is P1AE1770.

In another embodiment, the multispecific antibody comprises a full length antibody comprising an antigen binding site for the first antigen (e.g., which may be divalent for the first antigen), wherein the two heavy chain constant domains CH1 and the two light chain constant domains CL of the full length antibody comprise the charge modifications as described herein,
and wherein the C-terminus of one of the heavy chains (e.g., the C-terminus of its CH3 domain) is linked via a polypeptide linker to a first polypeptide and wherein the first polypeptide associates with a second polypeptide to form a cross-Fab comprising a binding site for the second antigen.

The first polypeptide may comprise an N-terminal VL domain and a C-terminal CH1 domain. Thus, the heavy chain having the fusion may comprise from N-to C-terminus VH-CH1-hinge-CH2-CH3-linker-VL-CH1. The light chain may comprise VH-CL. An example of such an arrangement is P1AE1767.

In a further embodiment, antibody may be a bispecific antibody comprising:

a) a full length antibody specifically binding a first antigen and consisting of two antibody heavy chains and two antibody light chains, wherein the CH1 domain of the heavy chains and the CL domain of the light chains comprise charge modifications as described herein;
b) a polypeptide consisting of

i) an antibody heavy chain variable domain (VH); or
ii) an antibody heavy chain variable domain (VH) and an antibody heavy chain constant domain (CH1) or
iii) an antibody heavy chain variable domain (VH) and an antibody light chain constant domain (CL)

wherein said polypeptide is fused with the N-terminus of the VH domain via a peptide linker to the C-terminus of one of the two heavy chains of said full-length antibody;
c) a polypeptide consisting of

i) an antibody light chain variable domain (VL); or
ii) an antibody light chain variable domain (VL) and an antibody light chain constant domain (CL); or
iii) an antibody light chain variable domain (VL) and an antibody heavy chain constant domain (CH1),

wherein said polypeptide is fused with the N-terminus of the VL domain via a peptide linker to the C-terminus

of the other of the two heavy chains of said full-length antibody;

and wherein the antibody heavy chain variable domain of the peptide under (b) and the antibody light chain variable domain of the peptide under (c) together form an antigen-binding site to a second antigen. Examples of such an arrangement are PRIT-213 and p1AE1766.

In some embodiments, the antibody of the invention comprises a) a first antigen binding moiety binding to a first antigen, b) a second antigen-binding moiety binding to a second antigen, and c) a third antigen-binding moiety binding to the first antigen, wherein the first, the second and the third antigen binding moiety of the antibody are all Fab molecules, and in one of the antigen binding moieties (particularly the second antigen binding moiety) the variable domains VL and VH of the Fab light chain and the Fab heavy chain respectively are replaced by each other, wherein i) the amino acid substitutions according to the above embodiments are made in the constant domain CL and the constant domain CH1 of each of the first Fab molecule and the third Fab molecule, but not in the constant domain CL and the constant domain CH1 of the second Fab molecule under b); or ii) the amino acid substitutions according to the above embodiments are made in the constant domain CL and the constant domain CH1 of the second Fab molecule under b), but not in the constant domain CL and the constant domain CH1 of the first Fab molecule and the third Fab molecule. In some embodiments, the charge modifications are present in the conventional (non-swapped) Fab: thus, for example, where the second antigen binding moiety is a cross-Fab, option (i) is preferred.

In one particular embodiment, a multivalent antibody of the invention comprises

a full length antibody comprising a first and second antibody heavy chain and a first and second antibody light chain, wherein the first heavy chain and the first light chain assemble to form a Fab comprising an antigen binding site for the first antigen (e.g., a tumour specific antigen, e.g., CEA), and wherein the second heavy chain and second light chain assemble to form a cross-Fab comprising an antigen binding site for the second antigen (e.g., DOTAM-chelated Pb) (e.g., the second heavy chain has a VL domain in place of a VH domain, and the second light chain has a VH domain in place of the VL domain);

and wherein either the first or second antibody heavy chain is fused via a linker to a polypeptide comprising a CH1 and VH domain, and said first polypeptide is assembled with a second polypeptide comprising a CL and VL, such that the first and second polypeptide assemble to form a Fab comprising an antigen binding site for the first antigen wherein the CH1 domain of the first heavy chain and the CL domain of the first light chain comprise charge modifications as described herein.

The CH1 domain of the first polypeptide and the CL domain of the second polypeptide may also comprise charge modifications as described herein.

The fusion may be at the N-terminus of one of the heavy chains of the full length antibody, optionally the second heavy chain. An example of such an arrangement is P1AE1769.

<u>Multispecific antibodies binding to Pb-DOTAM and CEA</u>

In some embodiments, it may be preferred that the antibodies of the present invention are multispecific, e.g, bispecific, antibodies that bind to both Pb-DOTAM and CEA. Thus, they comprise an antigen binding site for the Pb-DOTAM chelate and an antigen binding site for CEA. In such embodiments, the antigen-binding site specific for the Pb-DOTAM chelate may be in accordance with any of the embodiments described herein. The format may be any of the formats described herein.

Optionally, the antigen-binding site which binds to CEA may bind with a Kd value of 1nM or less, 500pM or less, 200pM or less, or 100pM or less for monovalent binding.

Optionally, the antigen-binding site which binds to CEA may comprise at least one, two, three, four, five, or six CDRs selected from (a) CDR-H1 comprising the amino acid sequence of SEQ ID NO:11; (b) CDR-H2 comprising the amino acid sequence of SEQ ID NO:12; (c) CDR-H3 comprising the amino acid sequence of SEQ ID NO:13; (d) CDR-L1 comprising the amino acid sequence of SEQ ID NO:14; (e) CDR-L2 comprising the amino acid sequence of SEQ ID NO:15; and (f) CDR-L3 comprising the amino acid sequence of SEQ ID NO:16.

Optionally, the antigen-binding site which binds to CEA may comprise at least one, at least two, or all three VH CDR sequences selected from (a) CDR-H1 comprising the amino acid sequence of SEQ ID NO:11; (b) CDR-H2 comprising the amino acid sequence of SEQ ID NO:12; and (c) CDR-H3 comprising the amino acid sequence of SEQ ID NO:13. In one embodiment, the antibody comprises CDR-H3 comprising the amino acic sequence of SEQ ID NO:13. In another embodiment, the antibody comprises CDR-H3 comprising the amino acid sequence of SEQ ID NO:13 and CDR-L3 comprising the amino acid sequence of SEQ ID NO:16. In a further embodiment, the antibody comprises CDR-H3 comprising the amino acid sequence of SEQ ID NO:13, CDR-L3 comprising the amino acid sequence of SEQ ID NO:16, and CDR-H2 comprising the amino acid sequence of SEQ ID NO:12. In a further embodiment, the antibody

comprises (a) CDR-H1 comprising the amino acid sequence of SEQ ID NO:11; (b) CDR-H2 comprising the amino acid sequence of SEQ ID NO:12; and (c) CDR-H3 comprising the amino acid sequence of SEQ ID NO:13.

Optionally, the antigen-binding site which binds to CEA comprises at least one, at least two, or all three VL CDRs sequences selected from (a) CDR-L1 comprising the amino acid sequence of SEQ ID NO:14; (b) CDR-L2 comprising the amino acid sequence of SEQ ID NO:15; and (c) CDR-L3 comprising the amino acid sequence of SEQ ID NO:16. In one embodiment, the antibody comprises (a) CDR-L1 comprising the amino acid sequence of SEQ ID NO:13, (b) CDR-L2 comprising the amino acid sequence of SEQ ID NO:14; and (c) CDR-L3 comprising the amino acid sequence of SEQ ID NO:15.

Optionally, the antigen-binding site which binds to CEA comprises (a) a VH domain comprising at least one, at least two, or all three VH CDR sequences selected from (i) CDR-H1 comprising the amino acid sequence of SEQ ID NO:11, (ii) CDR-H2 comprising the amino acid sequence of SEQ ID NO:12, and (iii) CDR-H3 comprising an amino acid sequence selected from SEQ ID NO:13; and (b) a VL domain comprising at least one, at least two, or all three VL CDR sequences selected from (i) CDR-L1 comprising the amino acid sequence of SEQ ID NO:14, (ii) CDR-L2 comprising the amino acid sequence of SEQ ID NO:15, and (c) CDR-L3 comprising the amino acid sequence of SEQ ID NO:16.

In another aspect, the antigen-binding site which binds to CEA comprises (a) CDR-H1 comprising the amino acid sequence of SEQ ID NO:11; (b) CDR-H2 comprising the amino acid sequence of SEQ ID NO:12; (c) CDR-H3 comprising the amino acid sequence of SEQ ID NO:13; (d) CDR-L1 comprising the amino acid sequence of SEQ ID NO:14; (e) CDR-L2 comprising the amino acid sequence of SEQ ID NO:15; and (f) CDR-L3 comprising an amino acid sequence selected from SEQ ID NO:16.

In any of the above embodiments, the multispecific antibody may be humanized. In one embodiment, the anti-CEA antigen binding site comprises CDRs as in any of the above embodiments, and further comprises an acceptor human framework, e.g. a human immunoglobulin framework or a human consensus framework.

In another embodiment, the antigen-binding site which binds to CEA comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:17. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but the antigen binding site comprising that sequence retains the ability to bind to CEA, preferably with the affinity as set out above. In certain embodiments, a total of 1 to 10 amino acids have been substituted, inserted and/or deleted in SEQ ID NO:17. In certain embodiments, substitutions, insertions, or deletions occur in regions outside the HVRs (i.e., in the FRs). Optionally, the antigen-binding site which binds to CEA comprises the VH sequence in SEQ ID NO:17, including post-translational modifications of that sequence. In a particular embodiment, the VH comprises one, two or three CDRs selected from: (a) CDR-H1 comprising the amino acid sequence of SEQ ID NO:11, (b) CDR-H2 comprising the amino acid sequence of SEQ ID NO:12, and (c) CDR-H3 comprising the amino acid sequence of SEQ ID NO:13.

In another embodiment, the antigen-binding site which binds to CEA comprises a light chain variable domain (VL) having at least 90%, 91%, 92%, 93%, 94%, 95%, 96%, 97%, 98%, 99%, or 100% sequence identity to the amino acid sequence of SEQ ID NO:18. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but the antigen-binding site comprising that sequence retains the ability to bind to CEA, preferably with the affinity set out above. In certain embodiments, a total of 1 to 10 amino acids have been substituted, inserted and/or deleted in SEQ ID NO:18. In certain embodiments, the substitutions, insertions, or deletions occur in regions outside the HVRs (i.e., in the FRs). Optionally, the antigen-binding site for CEA comprises the VL sequence in SEQ ID NO:18, including post-translational modifications of that sequence. In a particular embodiment, the VL comprises one, two or three CDRs selected from (a) CDR-L1 comprising the amino acid sequence of SEQ ID NO:14; (b) CDR-L2 comprising the amino acid sequence of SEQ ID NO:15; and (c) CDR-L3 comprising the amino acid sequence of SEQ ID NO:16.

In another embodiment, the antigen-binding site which binds to CEA comprises a VH as in any of the embodiments provided above, and a VL as in any of the embodiments provided above. In one embodiment, the antibody comprises the VH and VL sequences in SEQ ID NO:17 and SEQ ID NO:18, respectively, including post-translational modifications of those sequences.

In some embodiments, the multispecific antibody may binds to the same CEA-epitope as a PRIT-0213 or PRIT-0214 antibody provided herein.

Multispecific antibodies binding to Pb-DOTAM and ERBB2

In some embodiments, it may be preferred that the antibodies of the present invention are multispecific, e.g, bispecific, antibodies that bind to both Pb-DOTAM and ERBB2. Thus, they comprise an antigen binding site for the Pb-DOTAM chelate and an antigen binding site for ERBB2. In such embodiments, the antigen-binding site specific for the

Pb-DOTAM chelate may be in accordance with any of the embodiments described herein. The format may be any of the formats described herein.

Optionally, the antigen-binding site which binds to ERBB2 may bind with a Kd value of 1nM or less, 500pM or less, 200pM or less, or 100pM or less for monovalent binding.

Optionally, the antigen-binding site which binds to ERBB2 may comprise at least one, two, three, four, five, or six CDRs selected from (a)

CDR-H1 comprising the amino acid sequence of SEQ ID NO:28; (b) CDR-H2 comprising the amino acid sequence of SEQ ID NO:29; (c) CDR-H3 comprising the amino acid sequence of SEQ ID NO:30; (d) CDR-L1 comprising the amino acid sequence of SEQ ID NO:31; (e) CDR-L2 comprising the amino acid sequence of SEQ ID NO:32; and (f) CDR-L3 comprising the amino acid sequence of SEQ ID NO:33.

Optionally, the antigen-binding site which binds to ERBB2 may comprise at least one, at least two, or all three VH CDR sequences selected from (a) CDR-H1 comprising the amino acid sequence of SEQ ID NO:28; (b) CDR-H2 comprising the amino acid sequence of SEQ ID NO:29; and (c) CDR-H3 comprising the amino acid sequence of SEQ ID NO:30. In one embodiment, the antibody comprises CDR-H3 comprising the amino acid sequence of SEQ ID NO:30. In another embodiment, the antibody comprises CDR-H3 comprising the amino acid sequence of SEQ ID NO:30 and CDR-L3 comprising the amino acid sequence of SEQ ID NO:33. In a further embodiment, the antibody comprises CDR-H3 comprising the amino acid sequence of SEQ ID NO:30, CDR-L3 comprising the amino acid sequence of SEQ ID NO:33, and CDR-H2 comprising the amino acid sequence of SEQ ID NO:29. In a further embodiment, the antibody comprises (a) CDR-H1 comprising the amino acid sequence of SEQ ID NO:28; (b) CDR-H2 comprising the amino acid sequence of SEQ ID NO:29; and (c) CDR-H3 comprising the amino acid sequence of SEQ ID NO:30.

Optionally, the antigen-binding site which binds to ERBB2 comprises at least one, at least two, or all three VL CDRs sequences selected from (a) CDR-L1 comprising the amino acid sequence of SEQ ID NO:31; (b) CDR-L2 comprising the amino acid sequence of SEQ ID NO:32; and (c) CDR-L3 comprising the amino acid sequence of SEQ ID NO:33. In one embodiment, the antibody comprises (a) CDR-L1 comprising the amino acid sequence of SEQ ID NO:31, (b) CDR-L2 comprising the amino acid sequence of SEQ ID NO:32; and (c) CDR-L3 comprising the amino acid sequence of SEQ ID NO:33.

Optionally, the antigen-binding site which binds to ERBB2 comprises (a) a VH domain comprising at least one, at least two, or all three VH CDR sequences selected from (i) CDR-H1 comprising the amino acid sequence of SEQ ID NO:28, (ii) CDR-H2 comprising the amino acid sequence of SEQ ID NO:29, and (iii) CDR-H3 comprising an amino acid sequence selected from SEQ ID NO:30; and (b) a VL domain comprising at least one, at least two, or all three VL CDR sequences selected from (i) CDR-L1 comprising the amino acid sequence of SEQ ID NO:31, (ii) CDR-L2 comprising the amino acid sequence of SEQ ID NO:32, and (c) CDR-L3 comprising the amino acid sequence of SEQ ID NO:33.

In another aspect, the antigen-binding site which binds to ERBB2 comprises (a) CDR-H1 comprising the amino acid sequence of SEQ ID NO:28; (b) CDR-H2 comprising the amino acid sequence of SEQ ID NO:29; (c) CDR-H3 comprising the amino acid sequence of SEQ ID NO:30; (d) CDR-L1 comprising the amino acid sequence of SEQ ID NO:31; (e) CDR-L2 comprising the amino acid sequence of SEQ ID NO:32; and (f) CDR-L3 comprising an amino acid sequence selected from SEQ ID NO:33.

In any of the above embodiments, the multispecific antibody may be humanized. In one embodiment, the anti-ERBB2 antigen binding site comprises CDRs as in any of the above embodiments, and further comprises an acceptor human framework, e.g. a human immunoglobulin framework or a human consensus framework.

In another embodiment, the antigen-binding site which binds to ERBB2 comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:34. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but the antigen binding site comprising that sequence retains the ability to bind to ERBB2, preferably with the affinity as set out above. In certain embodiments, a total of 1 to 10 amino acids have been substituted, inserted and/or deleted in SEQ ID NO:34. In certain embodiments, substitutions, insertions, or deletions occur in regions outside the HVRs (i.e., in the FRs). Optionally, the antigen-binding site which binds to ERBB2 comprises the VH sequence in SEQ ID NO:34, including post-translational modifications of that sequence. In a particular embodiment, the VH comprises one, two or three CDRs selected from: (a) CDR-H1 comprising the amino acid sequence of SEQ ID NO:28, (b) CDR-H2 comprising the amino acid sequence of SEQ ID NO:29, and (c) CDR-H3 comprising the amino acid sequence of SEQ ID NO:30.

In another embodiment, the antigen-binding site which binds to ERBB2 comprises a light chain variable domain (VL) having at least 90%, 91%, 92%, 93%, 94%, 95%, 96%, 97%, 98%, 99%, or 100% sequence identity to the amino acid sequence of SEQ ID NO:35. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but the antigen-binding site comprising that sequence retains the ability to bind to ERBB2, preferably with the affinity set out above. In certain embodiments, a total of 1 to 10 amino acids have been

substituted, inserted and/or deleted in SEQ ID NO:35. In certain embodiments, the substitutions, insertions, or deletions occur in regions outside the HVRs (i.e., in the FRs). Optionally, the antigen-binding site for CEA comprises the VL sequence in SEQ ID NO:35, including post-translational modifications of that sequence. In a particular embodiment, the VL comprises one, two or three CDRs selected from (a) CDR-L1 comprising the amino acid sequence of SEQ ID NO:31; (b) CDR-L2 comprising the amino acid sequence of SEQ ID NO:32; and (c) CDR-L3 comprising the amino acid sequence of SEQ ID NO:33.

In another embodiment, the antigen-binding site which binds to ERBB2 comprises a VH as in any of the embodiments provided above, and a VL as in any of the embodiments provided above. In one embodiment, the antibody comprises the VH and VL sequences in SEQ ID NO:34 and SEQ ID NO:35, respectively, including post-translational modifications of those sequences.

In some embodiments, the multispecific antibody may binds to the same ERBB2-epitope as a P1AD9827 antibody provided herein.

Multispecific antibodies binding to Pb-DOTAM and CD20

In some embodiments, it may be preferred that the antibodies of the present invention are multispecific, e.g, bispecific, antibodies that bind to both Pb-DOTAM and CD20. Thus, they comprise an antigen binding site for the Pb-DOTAM chelate and an antigen binding site for CD20. In such embodiments, the antigen-binding site specific for the Pb-DOTAM chelate may be in accordance with any of the embodiments described herein. The format may be any of the formats described herein.

Optionally, the antigen-binding site which binds to CD20 may bind with a Kd value of 1nM or less, 500pM or less, 200pM or less, or 100pM or less for monovalent binding.

Optionally, the antigen-binding site which binds to CD20 may comprise at least one, two, three, four, five, or six CDRs selected from (a)

CDR-H1 comprising the amino acid sequence of SEQ ID NO:39; (b) CDR-H2 comprising the amino acid sequence of SEQ ID NO:40; (c) CDR-H3 comprising the amino acid sequence of SEQ ID NO:41; (d) CDR-L1 comprising the amino acid sequence of SEQ ID NO:42; (e) CDR-L2 comprising the amino acid sequence of SEQ ID NO:43; and (f) CDR-L3 comprising the amino acid sequence of SEQ ID NO:44.

Optionally, the antigen-binding site which binds to CD20 may comprise at least one, at least two, or all three VH CDR sequences selected from (a) CDR-H1 comprising the amino acid sequence of SEQ ID NO:39; (b) CDR-H2 comprising the amino acid sequence of SEQ ID NO:40; and (c) CDR-H3 comprising the amino acid sequence of SEQ ID NO:41. In one embodiment, the antibody comprises CDR-H3 comprising the amino acid sequence of SEQ ID NO:41. In another embodiment, the antibody comprises CDR-H3 comprising the amino acid sequence of SEQ ID NO:41 and CDR-L3 comprising the amino acid sequence of SEQ ID NO:44. In a further embodiment, the antibody comprises CDR-H3 comprising the amino acid sequence of SEQ ID NO:41, CDR-L3 comprising the amino acid sequence of SEQ ID NO:44, and CDR-H2 comprising the amino acid sequence of SEQ ID NO:40. In a further embodiment, the antibody comprises (a) CDR-H1 comprising the amino acid sequence of SEQ ID NO:39; (b) CDR-H2 comprising the amino acid sequence of SEQ ID NO:40; and (c) CDR-H3 comprising the amino acid sequence of SEQ ID NO:41.

Optionally, the antigen-binding site which binds to CD20 comprises at least one, at least two, or all three VL CDRs sequences selected from (a) CDR-L1 comprising the amino acid sequence of SEQ ID NO:42; (b) CDR-L2 comprising the amino acid sequence of SEQ ID NO:43; and (c) CDR-L3 comprising the amino acid sequence of SEQ ID NO:44. In one embodiment, the antibody comprises (a) CDR-L1 comprising the amino acid sequence of SEQ ID NO:42, (b) CDR-L2 comprising the amino acid sequence of SEQ ID NO:43; and (c) CDR-L3 comprising the amino acid sequence of SEQ ID NO:44.

Optionally, the antigen-binding site which binds to CD20 comprises (a) a VH domain comprising at least one, at least two, or all three VH CDR sequences selected from (i) CDR-H1 comprising the amino acid sequence of SEQ ID NO:39, (ii) CDR-H2 comprising the amino acid sequence of SEQ ID NO:40, and (iii) CDR-H3 comprising an amino acid sequence selected from SEQ ID NO:41; and (b) a VL domain comprising at least one, at least two, or all three VL CDR sequences selected from (i) CDR-L1 comprising the amino acid sequence of SEQ ID NO:42, (ii) CDR-L2 comprising the amino acid sequence of SEQ ID NO:43, and (c) CDR-L3 comprising the amino acid sequence of SEQ ID NO:44.

In another aspect, the antigen-binding site which binds to CD20 comprises (a) CDR-H1 comprising the amino acid sequence of SEQ ID NO:39; (b) CDR-H2 comprising the amino acid sequence of SEQ ID NO:40; (c) CDR-H3 comprising the amino acid sequence of SEQ ID NO:41; (d) CDR-L1 comprising the amino acid sequence of SEQ ID NO:42; (e) CDR-L2 comprising the amino acid sequence of SEQ ID NO:43; and (f) CDR-L3 comprising an amino acid sequence selected from SEQ ID NO:44.

In any of the above embodiments, the multispecific antibody may be humanized. In one embodiment, the anti-CD20 antigen binding site comprises CDRs as in any of the above embodiments, and further comprises an acceptor human framework, e.g. a human immunoglobulin framework or a human consensus framework.

In another embodiment, the antigen-binding site which binds to CD20 comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO:45. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but the antigen binding site comprising that sequence retains the ability to bind to CD20, preferably with the affinity as set out above. In certain embodiments, a total of 1 to 10 amino acids have been substituted, inserted and/or deleted in SEQ ID NO:45. In certain embodiments, substitutions, insertions, or deletions occur in regions outside the HVRs (i.e., in the FRs). Optionally, the antigen-binding site which binds to CD20 comprises the VH sequence in SEQ ID NO:45, including post-translational modifications of that sequence. In a particular embodiment, the VH comprises one, two or three CDRs selected from: (a) CDR-H1 comprising the amino acid sequence of SEQ ID NO:39, (b) CDR-H2 comprising the amino acid sequence of SEQ ID NO:40, and (c) CDR-H3 comprising the amino acid sequence of SEQ ID NO:41.

In another embodiment, the antigen-binding site which binds to CD20 comprises a light chain variable domain (VL) having at least 90%, 91%, 92%, 93%, 94%, 95%, 96%, 97%, 98%, 99%, or 100% sequence identity to the amino acid sequence of SEQ ID NO:46. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but the antigen-binding site comprising that sequence retains the ability to bind to CD20, preferably with the affinity set out above. In certain embodiments, a total of 1 to 10 amino acids have been substituted, inserted and/or deleted in SEQ ID NO:46. In certain embodiments, the substitutions, insertions, or deletions occur in regions outside the HVRs (i.e., in the FRs). Optionally, the antigen-binding site for CD20 comprises the VL sequence in SEQ ID NO:46, including post-translational modifications of that sequence. In a particular embodiment, the VL comprises one, two or three CDRs selected from (a) CDR-L1 comprising the amino acid sequence of SEQ ID NO:42; (b) CDR-L2 comprising the amino acid sequence of SEQ ID NO:43; and (c) CDR-L3 comprising the amino acid sequence of SEQ ID NO:44.

In another embodiment, the antigen-binding site which binds to CD20 comprises a VH as in any of the embodiments provided above, and a VL as in any of the embodiments provided above. In one embodiment, the antibody comprises the VH and VL sequences in SEQ ID NO:45 and SEQ ID NO:46, respectively, including post-translational modifications of those sequences.

In some embodiments, the multispecific antibody may binds to the same CD20-epitope as a P1AD9826 antibody provided herein.

Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

Substitution, Insertion, and Deletion Variants

In certain embodiments, antibody variants having one or more amino acid substitutions are provided. Sites of interest for substitutional mutagenesis include the HVRs (CDRs) and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

TABLE 1

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |

(continued)

| Original Residue | Exemplary Substitutions | Preferred Substitutions |
|---|---|---|
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gin;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.
One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).
Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular

are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUTB,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody

variants may have improved CDC function. Such antibody variants are described, e.g., in WO 1997/30087 (Patel et al.); WO 1998/58964 (Raju, S.); and WO 1999/22764 (Raju, S.).

It may be preferred that the antibody is modified to reduce the extent of glycosylation. In some embodiments the antibody may be aglycosylated or deglycosylated. The antibody may include a substitution at N297, e.g., N297D/A.

Fc region variants

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the invention contemplates an antibody variant with reduced effector function, e.g., reduced or eliminated CDC, ADCC and/or FcγR binding. In certain aspects, the invention contemplates an antibody variant that possesses some but not all effector functions, which make it a desirable candidate for applications in which the half life of the antibody in vivo is important yet certain effector functions (such as complement-dependent cytotoxicity (CDC) and antibody-dependent cell-mediated cytotoxicity (ADCC)) are unnecessary or deleterious.

*In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity). The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of in *vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in *vivo,* e.g., in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half life determinations can also be performed using methods known in the art (see, e.g., Petkova, S.B. et al., Int'l. Immunol. 18(12):1759-1769 (2006); WO 2013/120929 A1).

Antibodies with reduced effector function include those with substitution of one or more of Fc region residues 238, 265, 269, 270, 297, 327 and 329 (U.S. Patent No. 6,737,056), e.g., P329G. Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

In certain aspects, an antibody variant comprises an Fc region with one or more amino acid substitutions which diminish FcγR binding, e.g., substitutions at positions 234 and 235 of the Fc region (EU numbering of residues). In one aspect, the substitutions are L234A and L235A (LALA). In certain aspects, the antibody variant further comprises D265A and/or P329G in an Fc region derived from a human IgG1 Fc region. In one aspect, the substitutions are L234A, L235A and P329G (LALA-PG) in an Fc region derived from a human IgG1 Fc region. (See, e.g., WO 2012/130831). In another aspect, the substitutions are L234A, L235A and D265A (LALA-DA) in an Fc region derived from a human IgG1 Fc region.

In other embodiments, it may be possible to use a ZgG subtype with reduced effector function such as IgG4 or IgG2.

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In some embodiments, alterations are made in the Fc region that result in altered (i.e., either improved or diminished, preferably diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

In some embodiments, FcRn binding may be reduced, e.g, for shorter half-life. In other embodiments, binding of FcRn may be normal. For instance, in some embodiments, normal FcRn binding may be used in methods involving a clearing agent.

In certain aspects, an antibody variant comprises an Fc region with one or more amino acid substitutions, which reduce FcRn binding, e.g., substitutions at positions 253, and/or 310, and/or 435 of the Fc-region (EU numbering of residues). In certain aspects, the antibody variant comprises an Fc region with the amino acid substitutions at positions

253, 310 and 435. In one aspect, the substitutions are I253A, H310A and H435A in an Fc region derived from a human IgG1 Fc-region. See, e.g., Grevys, A., et al., J. Immunol. 194 (2015) 5497-5508.

In certain aspects, an antibody variant comprises an Fc region with one or more amino acid substitutions, which reduce FcRn binding, e.g., substitutions at positions 310, and/or 433, and/or 436 of the Fc region (EU numbering of residues). In certain aspects, the antibody variant comprises an Fc region with the amino acid substitutions at positions 310, 433 and 436. In one aspect, the substitutions are H310A, H433A and Y436A in an Fc region derived from a human IgG1 Fc-region. (See, e.g., WO 2014/177460 Al). For instance, in some embodiments, normal FcRn binding may be used. See also Duncan & Winter, Nature 322:738-40 (1988); U.S. Patent No. 5,648,260; U.S. Patent No. 5,624,821; and WO 94/29351 concerning other examples of Fc region variants.

The C-terminus of the heavy chain of the antibody as reported herein can be a complete C-terminus ending with the amino acid residues PGK. The C-terminus of the heavy chain can be a shortened C-terminus in which one or two of the C terminal amino acid residues have been removed. In one preferred aspect, the C-terminus of the heavy chain is a shortened C-terminus ending PG.

In one aspect of all aspects as reported herein, an antibody comprising a heavy chain including a C-terminal CH3 domain, as specified herein, comprises a C-terminal glycine residue (G446, EU index numbering of amino acid positions). This is still explicitly encompassed with the term "full length antibody" or "full length heavy chain" as used herein

Antibody Derivatives

In certain embodiments, an antibody provided herein may be further modified to contain additional nonproteinaceous moieties that are known in the art and readily available. The moieties suitable for cerivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranchec. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

Recombinant Methods and Compositions

Antibodies may be produced using recombinant methods and compositions, e.g., as described in U.S. Patent No. 4,816,567. In one embodiment, isolated nucleic acid encoding an antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody.

In the case of multispecific antibodies, nucleic acids may be provided encoding each of the heavy and light chain components of the particular antibody format. A vector or set of vectors comprising such nucleic acids are also provided.

In one embodiment, the host cell is eukaryotic, e.g. a Chinese Hamster Ovary (CHO) cell or lymphoid cell (e.g., Y0, NS0, Sp20 cell). In one embodiment, a method of making an antibody according to the invention is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. *coli.)* After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977; baby hamster kidney cells (BHK); mouse sertoli cells (TM4 cells as described, e.g., in Mather, Biol. Reprod. 23:243-251 (1980); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including $DHFR^-$ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

## Assays

Antibodies provided herein may be identified, screened for, or characterized for their physical/chemical properties and/or biological activities by various assays known in the art.

### Binding assays and other assays

In one aspect, an antibody of the invention is tested for its antigen binding activity, e.g., by known methods such as ELISA, Western blot, etc.

In another aspect, competition assays may be used to identify an antibody that competes with e.g., PRIT-0213 or PRIT-0214 for binding to Pb-DOTAM or CEA. In certain embodiments, such a competing antibody binds to the same epitope (e.g., a linear or a conformational epitope) that is bound by PRIT-0213 or PRIT-0214. Detailed exemplary methods for mapping an epitope to which an antibody binds are provided in Morris (1996) "Epitope Mapping Protocols," in Methods in Molecular Biology vol. 66 (Humana Press, Totowa, NJ) .

In an exemplary competition assay, immobilized antigen is incubated in a solution comprising a first labeled antibody that binds to the antigen (e.g., PRIT-0213 and PRIT-0214) and a second unlabeled antibody that is being tested for its ability to compete with the first antibody for binding to the antigen. The second antibody may be present in a hybridoma supernatant. As a control, immobilized antigen is incubated in a solution comprising the first labeled antibody but not the second unlabeled antibody. After incubation under conditions permissive for binding of the first antibody to the antigen, excess unbound antibody is removed, and the amount of label associated with immobilized antigen is measured. If the amount of label associated with immobilized antigen is substantially reduced in the test sample relative to the control sample, then that indicates that the second antibody is competing with the first antibody for binding to the antigen. See Harlow and Lane (1988) Antibodies: A Laboratory Manual ch.14 (Cold Spring Harbor Laboratory, Cold Spring Harbor, NY) .

Antibody affinity

In certain embodiments, an antibody provided herein has a dissociation constant (Kd) of 1 nM or less, 500pM or less, 200pM or less, 100pM or less, 50pM or less, 20pM or less, 10pM or less, 5pM or less or 1pM or less, or as otherwise stated herein.

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 μg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 μl/well of scintillant (MICROSCINT-20 ™; Packard) is added, and the plates are counted on a TOPCOUNT ™ gamma counter (Packard) for ten minutes. Concentrations of each Fab that give less than or equal to 20% of maximal binding are chosen for use in competitive binding assays.

According to another embodiment, Kd is measured using a BIACORE® surface plasmon resonance assay. For example, an assay using a BIACORE®-2000 or a BIACORE ®-3000 (BIAcore, Inc., Piscataway, NJ) is performed at 25°C with immobilized antigen CM5 chips at ~10 response units (RU). In one embodiment, carboxymethylated dextran biosensor chips (CM5, BIACORE, Inc.) are activated with *N*-ethyl-*N*'- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 μg/ml (~0.2 pM) before injection at a flow rate of 5 μl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 μl/min. Association rates ($k_{on}$) and dissociation rates ($k_{off}$) are calculated using a simple one-to-one Langmuir binding model (BIACORE ® Evaluation Software version 3.2) by simultaneously fitting the association and dissociation sensorgrams. The equilibrium dissociation constant (Kd) is calculated as the ratio $k_{off}/k_{on.}$ See, e.g., Chen et al., J. Mol. Biol. 293:865-881 (1999). If the on-rate exceeds $10^6$ $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

In another embodiment, Kd is measured using a SET (solution equilibration titration) assay. According to this assay, test antibodies are typically applied in a constant concentration and mixed with serial dilutions of the test antigen. After incubation to establish an equilibrium, the portion of free antibodies is captured on an antigen coated surface and detected with labelled/tagged anti-species antibody, generally using electochemiluminescence (e.g., as described in Haenel et al Analytical Biochemistry 339 (2005) 182-184).

For example, in one embodiment 384-well streptavidin plates (Nunc, Microcoat #11974998001) are incubated overnight at 4°C with 25 μl/well of an antigen-Biotin-Isomer Mix in PBS-buffer at a concentration of 20 ng/ml. For equilibration of antibody samples with free antigen: 0.01 nM - 1 nM of antibody is titrated with the relevant antigen in 1:3, 1:2 or 1:1.7 dilution steps starting at a concentration of 2500 nM, 500 nM or 100 nM of antigen. The samples are incubated at 4°C overnight in sealed REMP Storage polypropylene microplates (Brooks). After overnight incubation, streptavidin plates are washed 3x with 90 μl PBST per well. 15 μl of each sample from the equilibration plate is transferred to the assay plate and incubated for 15 min at RT, followed by 3x 90 μl washing steps with PBST buffer. Detection is carried out by adding 25 μl of a goat anti-human IgG antibody-POD conjugate (Jackson, 109-036-088, 1:4000 in OSEP), followed by 6x 90 μl washing steps with PBST buffer. 25 μl of TMB substrate (Roche Diagnostics GmbH, Cat. No.: 11835033001) are added to each well. Measurement takes place at 370/492 nm on a Safire2 reader (Tecan).

In another embodiment, Kd is measured using a KinExA (kinetic exclusion) assay. According to this assay, the antigen is typically titrated into a constant concentration of antibody binding sites, the samples are allowed to equilibrate, and then drawn quickly through a flow cell where free antibody binding sites are captured on antigen-coated beads, while the antigen-saturated antibody complex is washed away. The bead-captured antibody is then detected with a

labeled anti-species antibody, e.g., fluorescently labelled (Bee et al PloS One, 2012; 7(4): e36261). For example, in one embodiment, KinExA experiments are performed at room temperature (RT) using PBS pH 7.4 as running buffer. Samples are prepared in running buffer supplemented with 1 mg/ml BSA ("sample buffer"). A flow rate of 0.25 ml/min is used. A constant amount of antibody with 5 pM binding site concentration is titrated with antigen by twofold serial dilution starting at 100 pM (concentration range 0.049 pM - 100 pM). One sample of antibody without antigen serves as 100% signal (i.e. without inhibition). Antigen-antibody complexes are incubated at RT for at least 24 h to allow equilibrium to be reached. Equilibrated mixtures are then drawn through a column of antigen-coupled beads in the KinExA system at a volume of 5 ml permitting unbound antibody to be captured by the beads without perturbing the equilibrium state of the solution. Captured antibody is detected using 250 ng/ml Dylight 650©-conjugated anti-human Fc-fragment specific secondary antibody in sample buffer. Each sample is measured in duplicates for all equilibrium experiments. The KD is obtained from non-linear regression analysis of the data using a one-site homogeneous binding model contained within the KinExA software (Version 4.0.11) using the "standard analysis" method.

Therapeutic Methods and Compositions

As discussed above, multispecific antibodies according to the present invention are suitable for any treatment in which it is desired to deliver a radionuclide to a target. According, the application describes a targeted antibody such as a multispecific or bispecific antibody as described herein for use in a method of treatment. More particularly, there is described a targeted antibody (e.g., multispecific or bispecific antibody) as described herein for use in a method of pre-targeted radioimmunotherapy. In such embodiments, the chelated Pb is preferably $^{212}Pb$.

As noted above, the treatment may be of any condition that is treatable by cytotoxic activity targeted to diseased cells of the patient. The treatment is preferably of a tumour or cancer. However, the applicability of the invention is not limited to tumours and cancers. For example, the treatment may also be of viral infection, or infection by another pathogenic organism, e.g., a prokaryote. Optionally, targeting may also be to T-cells for treatment of T-cell driven autoimmune disease or T-cell blood cancers. Thus, conditions to be treated may include viral infections such as HIV, rabies, EBV and Kaposi's sarcoma-associated herpesvirus, and autoimmune diseases such as multiple sclerosis and graft-versus-host disease drugs.

The term "cancer" as used herein include both solid and haematologic cancers, such as lymphomas, lymphocytic leukemias, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumours, brain stem glioma, glioblastoma multiforme, astrocytomas, schwanomas, ependymomas, medulloblastomas, meningiomas, squamous cell carcinomas, pituitary adenoma and Ewings sarcoma, including refractory versions of any of the above cancers, or a combination of one or more of the above cancers.

A method of targeting a radioisotope to a tissue or organ for therapy may comprise:

i) administering to the subject a multispecific or bispecific antibody as described herein, wherein the antibody binds to the target antigen and localises to the surface of a cell expressing the target antigen; and
ii) subsequently administering a Pb radionuclide chelated with DOTAM or a functional variant thereof to the individual, wherein a Pb radionuclide chelated with DOTAM or a functional variant thereof binds to the antibody localised at the cell surface.

Optionally, between steps (i) and (ii) a clearing/blocking agent is administered. The clearing/blocking agent may bind to the antigen binding site specific for Pb-DOTAM and block subsequent binding by the chelated radionuclide. The clearing agent may comprise DOTAM or a functional variant thereof, chelated with a metal ion and conjugated to a clearing moiety.

Examples of suitable clearing moieties may include moieties which increase the size and/or hydrodynamic radius of the molecule, hindering the ability of the molecule to access the tumour, without interfering with the ability of the molecule to bind to the antibody in the circulation. Exemplary moieties include hydrophilic polymers. The moiety may be a polymer or copolymer e.g., of dextran, dextrin, PEG, polysialic acids (PSAs), hyaluronic acid, hydroxyethyl-starch (HES) or poly(2-ethyl 2-oxazoline) (PEOZ). In other embodiments the moiety may be a non-structured peptide or protein such as XTEN polypeptides (unstructured hydrophilic protein polymers), homo-amino acid polymer (HAP), proline-alanine-serine polymer (PAS), elastin-like peptide (ELP), or gelatin-like protein (GLK). Suitable molecular weights for

the polymers may be in the range e.g., of at least 50 kDa, for example between 50 kDa to 2000 kDa. For example, the molecular weight may be 200-800kDa, optionally greater than 300, 350, 400 or 450 kDa, and optionally less than 700, 650, 600 or 550kDa, optionally about 500kDa.

In some embodiments, the clearing agent may be DOTAM or a functional variant thereof (chelated with a metal ion), conjugated to dextran or a derivative thereof, e.g., as described further below.

In some embodiments, the weight ratio of antibody to clearing agent may be in the range of from 1:1, 2:1, 3:1 or 4:1 up to 20:1, 15:1, 10:1, 8:1, 6:1 or 5:1, e.g., in the range 1:1 to 20:1, 1:1 to 10:1, 2:1 to 8:1 or 2:1 to 6:1.

In some embodiments, the clearing agent may be administered a matter of hours or days after the treatment with the multispecific antibody. In some embodiments it may be preferred that the clearing agent is administered at least 2, 4, 6, 8, 10, 12, 16, 18, 22 or 24 hours after the multispecific antibody, or at least 1, 2, 3, 4, 5, 6 or 7 days. In some embodiments, it may be preferred that the clearing agent is administered not more than 14 days after the antibody, e.g., not more than 10, 9, 8, 7, 6, 5, 4, 3 or 2 days.

Optionally, the clearing agent is administered in the period between 4 and 10 days, 4 and 7 days, 2 and 7 days, or 2 to 4 days after the multispecific antibody.

In some embodiments, the Pb radionuclide is administered a matter of minutes, hours or days after the clearing agent. In some embodiments it may be preferred that the Pb radionuclide is administered at least 30 minutes after the clearing agent, and optionally within 48 hours, 24 hours, 8 hours or 4 hours of administration of the clearing agent. In some embodiments, the Pb radionuclide may be administered the day after admistration of the clearing agent.

In some embodiments, the antibodies described herein may be administered as part of a combination therapy. For example, they may be administered in combination with one or more chemotherapeutic agents: the chemotherapeutic agent and the antibody may be administered simultaneously or sequentially, in either order.

In some embodiments, the antibodies described herein may additionally or alternatively be administered in combination with radiosensitizers. The radiosensitizer and the antibody may be administered simultaneously or sequentially, in either order.

## Pharmaceutical Formulations

Pharmaceutical formulations of an anti-Pb-DOTAM antibody as described herein, e.g., a multispecific or bispecific antibody, are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.* Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide chemotherapeutic agents and/or radiosensitizers as discussed above. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, *e.g.* films, or microcapsules.

The formulations to be used for *in vivo* administration are generally sterile. Sterility may be readily accomplished, e.g., by filtration through sterile filtration membranes.

Methods and Compositions for Diagnosis and Detection

The application describes the target antibody, e.g., multispecific antibody as described herein, for use in a method of diagnosis carried out on a subject. The method of diagnosis may be a method of pre-targeted radioimmunoimaging, e.g., for the purpose of diagnosing a subject suspected of having a proliferative disorder or an infectious disease. In such embodiments, the chelated Pb is preferably $^{203}$Pb.

A method of targeting a radioisotope to a tissue or organ for imaging may comprise:

i) administering to the subject a multispecific or bispecific antibody as described herein, wherein the antibody binds to a target antigen and localises to the surface of a cell expressing the target antigen; and
ii) subsequently administering a Pb radionuclide chelated with DOTAM or a functional variant thereof to the individual, wherein the Pb radionuclide chelated with DOTAM of said functional variant thereof binds to the antibody localised at the surface of the cell.

In another embodiment, the multispecific or bispecific antibody as described herein may be bound with the chelated Pb radionuclide at the time of administration.

Optionally, the method may further comprise:
iii) imaging the tissue or organ where the Pb radionuclide chelated with DOTAM or the functional variant thereof has localized, or is expected to be localized.

In another embodiment, method described herein may comprise imaging a tissue or organ of a subject, wherein the subject has been previously administered with:

i) a multispecific or bispecific antibody as described herein, wherein the antibody binds to a target antigen and localises to the surface of a cell expressing the target antigen; and
ii) a Pb radionuclide chelated with DOTAM or a functional variant thereof to the individual, wherein the Pb radionuclide chelated with DOTAM of said functional variant thereof binds to the antibody localised to the surface of the cell.

Optionally, between steps (i) and (ii) a clearing/blocking agent is administered. The clearing agent, the administration regimen for the clearing agent and the weight ratio of antibody to clearing agent may be as described above.

The target antigen may be any target antigen as discussed herein. In some embodiments, the target antigen may be a tumour-specific antigen as discussed above, and the imaging may be a method of imaging a tumour or tumours. The individual may be known to or suspected of having a tumour.

For example, the method may be a method of imaging tumours in an individual having or suspected of having lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumours, brain stem glioma, glioblastoma multiforme, astrocytomas, schwanomas, ependymomas, medulloblastomas, meningiomas, squamous cell carcinomas, pituitary adenoma and Ewings sarcoma, including refractory versions of any of the above cancers, or a combination of one or more of the above cancers.

Clearing Agents

As described herein, the present inventors have developed a novel clearing agent. Such a clearing agent may be used in any of the methods of diagnosis, imaging or treatment as described herein.

The application describes a dextran-based clearing agent comprising dextran or a derivative thereof, conjugated to M-DOTAM or a functional variant thereof.

In some embodiments, the clearing agent may be a compound of the following formula:

dextran-(linker-(M-DOTAM))$_x$

wherein

dextran is dextran or derivative thereof;
linker is a linking moiety;
M-DOTAM is DOTAM or a functional variant thereof incorporating a metal ion; and

$$x \geq 1.$$

In some embodiments, the linking moiety may be or comprise one or more bivalent functional groups selected from a urea group (-NH-C(O)-NH-), a substituted urea group (-NR$^x$-C(O)-NR$^x$-, where one or both R$^x$ groups are not H), a thiourea group (-NH-C(S)-NH-), a substituted thiourea group (-NR$^x$-C(S)-NR$^x$-, where one or both R$^x$ groups are not H), an amide group (-C(O)-NH-), a substituted amide group (-C(O)-NR$^x$-, where R$^x$ is not H), a thioamide group (-C(S)-NH-), a substituted amide group (-C(S)-NR$^x$-, where R$^x$ is not H), a triazole group, or a substituted triazole. In these embodiments, the linking moiety may optionally comprise one or more additional bivalent functional groups, such as an alkylene group, an arylene group, a heteroarylene group, an aralkylene group and a heteroaralklyene group. Substituent R$^x$ is not particularly limited. In particular embodiments, R$^x$, when present, is selected from the group consisting of C1-C6 alkyl, C5-C12 aryl, C5-C12 heteroaryl and halo groups.

In particular embodiments, the linking moiety may be or comprise one or more bivalent functional groups selected from a urea group, a thiourea group, an amide group, a thioamide group, or a triazole group.

In a preferred embodiment, the linking moiety comprises a bivalent thiourea functional group or a bivalent thioamide functional group.

In some embodiments, the linking moiety comprises a bivalent thiourea functional group and an optionally substituted arylene group. In some embodiments, the linking moiety comprises a bivalent thiourea functional group, an optionally substituted arylene group and an optionally substituted alkylene group. In particular embodiments, the linking moiety comprises a bivalent thiourea functional group covalently bonded through one of its nitrogen atoms to an optionally substituted arylene group. In further embodiments, the linking moiety comprises a bivalent thiourea functional group covalently bonded through one of its nitrogen atoms to an optionally substituted arylene group and the optionally substituted arylene group is covalently bonded to an optionally substituted alkylene group. In preferred embodiments, the arylene group is unsubstituted. In particular embodiments, the arylene group is a phenylene group. In preferred embodiments, the alkylene group is unsubstituted. In particular embodiments, the alkylene group is a C1-C6 alkyene group. In particularly preferred embodiments, the alkylene group is selected from methylene and ethylene. When present in the linking moiety, the arylene group and alkylene group may be unsubstituted. In particular embodiments, the linking moiety consists of a bivalent thiourea functional group covalently bonded through one of its nitrogen atoms to an arylene group and the arylene group is covalently bonded to an alkylene group.

In some embodiments, the linking moiety comprises a bivalent thioamide functional group and an optionally substituted arylene group. In some embodiments, the linking moiety comprises a bivalent thioamide functional group, an optionally substituted arylene group and an optionally substituted alkylene group. In particular embodiments, the linking moiety comprises a bivalent thioamide functional group covalently bonded through one of its nitrogen atom to an optionally substituted arylene group. In further embodiments, the linking moiety comprises a bivalent thioaide functional group covalently bonded through one of its nitrogen atoms to an optionally substituted arylene group and the optionally substituted arylene group is covalently bound to an optionally substituted alkylene group. In preferred embodiments, the arylene group is unsubstituted. In particular embodiments, the arylene group is a phenylene group. In preferred embodiments, the alkylene group is unsubstituted. In particular embodiments, the alkylene group is a C1-C6 alkyene group. In particularly preferred embodiments, the alkylene group is selected from methylene and ethylene. When present in the linking moeity, the arylene group and alkylene group may be unsubstituted. In particular embodiments, the linking moiety consists of a bivalent thioamide functional group covalently bonded through one of its nitrogen atoms to an arylene group and the arylene group is covalently bonded to an alkylene group.

In some embodiments, the linking moiety may be or comprise a group of the following formula:

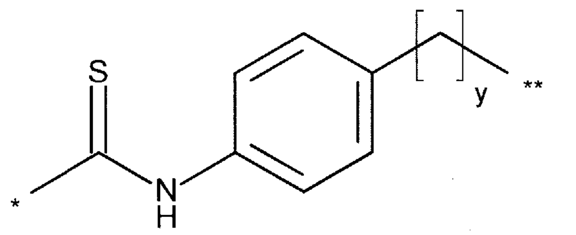

where y is 1 to 6 (preferably 1 or 2), * represents the point of attachment to the dextran or a derivative thereof, and ** represents the point of attachment to a ring atom of DOTAM or a functional variant thereof.

In some embodiments, the linking moiety may be formed from the conjugation of an amine (preferably a primary amine) and an isocyanate or isothiocyanate. Such conjugation forms a bivalent urea functional group and a thiourea functional group, respectively. In such embodiments when an isocyanate is one of the reactants, the linking moiety may be considered to comprise a bivalent urea functional group or a bivalent amide functional group, as appropriate. In such embodiments when an isothiocyanate is one of the reactants, the linking moeity may be considered to comprise a bivalent thiourea functional group or a bivalent thioamide functional group, as appropriate.

Preferably, x is greater than 1, so that each dextran has an average of greater than 1 M-DOTAM or functional variant thereof per molecule. For example, x may be 2 or more, 5 or more, 10 or more, 15 or more, 20 or more, 25 or more, 30 or more, 35 or more, 40 or more, or preferably 50 or more. The present inventors have found that improved clearing can be achieved using dextran labelled with multiple M-DOTAM groups.

The DOTAM or functional variant thereof may incorporate only one linker, so as to prevent cross-linking of dextran.

Derivatives of dextran which may find use in the clearing agent include aminodextrans, in which dextran is substituted with one or more amines. Of particular use are aminodextrans in which one or more hydroxyl groups of the dextran are substituted with an amino-substituted carboxymethyl amide group. Such compounds may be produced by modifying the dextran with a carboxymethyl group (for example, by reacting with chloroacetic acid), and then further reacting with an optionally substituted diamine (preferably an alkyldiamine, such as an $\alpha,\omega$-alkylenediamine, e.g. ethylenediamine).

The amino group provides a point of attachment for the linker. At least 30% of the available amino groups may be substituted with DOTAM or a functional variant thereof, preferably at least 40%, more preferably at least 50%.

Preferably, "dextran" in the formula above is an aminodextran, corresponding to a dextran substituted with one or more carboxymethyl groups which are themselves substituted with ethylenediamine.

The dextran may be substituted with one or more groups of formula - $CH_2C(=O)NH(CH_2)_fNHR^F$, where $R^F$ denotes hydrogen or the linker to DOTAM, and f is 1-6, most preferably 2. For example, the groups may have the following formula:

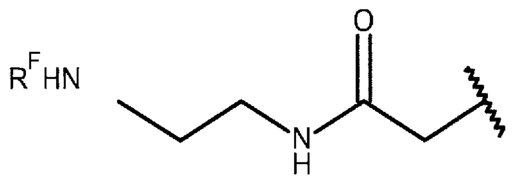

where the wavy line indicates the point of attachment to oxygen on the dextran.

The aminodextran can have a backbone of predominantly $\alpha$(1,6)-linked glucopyranosyl repeat units, optionally with branches of other glucopyranosyl units linked for instance via $\alpha$(1,2), $\alpha$(1,3) or $\alpha$(1,4) glycosidic bonds,. At least some of the hydroxyl groups are substituted with an amino-substituted carboxymethyl amide group as discussed above (in particular, a group of formula $-CH_2C(=O)NHCH_2CH_2NHR^F$). In other words, the aminodextran may comprise units of the following formula:

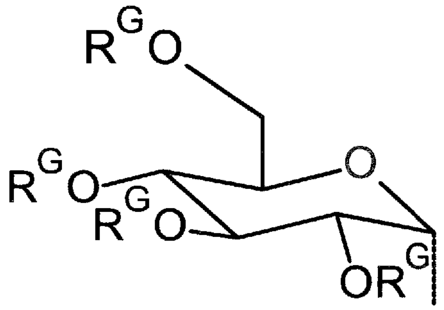

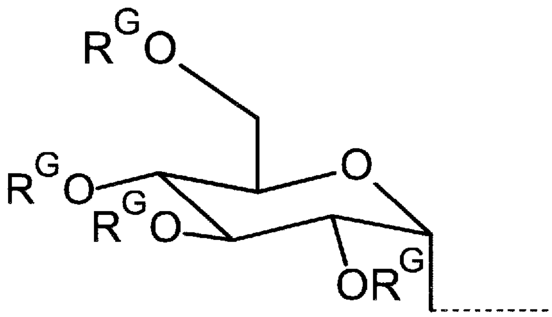

wherein each $R^G$ is H, an amino-substituted carboxymethyl amide group (such as $-CH_2C(=O)NHCH_2CH_2NHR^F$), or a bond to a further glucopyranosyl unit, predominantly via $\alpha(1,6)$-linkage and wherein the dashed line indicates bonding to an adjacent unit.

The clearing agent may include one or more units of the formula below:

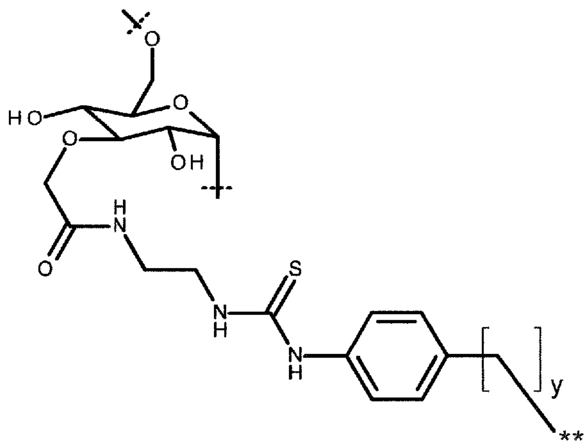

where ** represents the point of attachment to DOTAM or a functional variant thereof, and y is as defined above.

The derivatives of dextran may include dextran or aminodextran modified with one or more groups selected from an amino acid, or a saccharide other than glucose. For example, the dextran may be modified (e.g. capped) with one or more glutamic acid or polyglutamic acid units, including Glu, $(Glu)_2$, $(Glu)_3$, or $(Glu)_4$. Additionally, or alternatively, the dextran may be modified (e.g. capped) with a saccharide other than glucose, such as N-acetylgalactosamine (GalNAc), or a polysaccharide formed from such saccharides such as tri-GalNAc.

In some embodiments, the molecular weight of the dextran component may be at least 50 kDa, for example between 50 kDa to 2000 kDa. For example, the molecular weight may be 200-800kDa, optionally greater than 300, 350, 400 or 450 kDa, and optionally less than 700, 650, 600 or 550kDa, optionally about 500kDa.

It may be preferred that the number of amino groups as a percentage of the number of glucose units of the dextran or dextran derivative thereof (the "saturation" of the glucose units with amino groups) may be at least 0.5%, at least 10, at least 2, at least 5%, at least 5%, at least 10%, at least 20%, at least 30%, at least 40%, at least 50%, at least 60%, at least 70%, at least 80%, at least 90% or is 100%. In some embodiments it may be preferred that the saturation of dextran with amino groups is at least or about 1% or 10%, e.g., 1%-10%

It may be preferred that the number of DOTAM groups as a percentage of the number of amino units of the dextran derivative (the "saturation" of the amino dextran component with DOTAM) may be at least 5%, at least 10%, at least 20%, at least 300, at least 40%, at least 50%, at least 60%, at least 70%, at least 80%, at least 90% or is 100%. In some embodiments it may be preferred that the saturation of the available amino groups of the dextran derivative with DOTAM is at least or about 40% or 50%, e.g., 40%-60%.

One potential difficulty with the use of clearing agents is the possibility that they may enter tumours, and bind to tumour associated antibodies negatively affecting subsequent binding of radioligands.

The present inventors have further found that good clearance from the blood can be achieved together with low clearing agent penetration into tumours, when a dextran-based clearing agent is used which has i) a high average molecular weight and ii) has been subject to a molecular weight cut-off, such that fragments below a certain size have been removed. The cut-off may be applied to the dextran or dextran-derivative prior to the conjugation step; and/or applied to the clearing agent following conjugation; and/or applied to the clearing agent after complexation with the metal.

Thus, a clearing agent useful in the present invention may be a dextran-based clearing agent comprising dextran or a derivative thereof (e.g., as defined above, preferably an aminodextran), conjugated to a metal chelate, wherein i) the average molecular weight of the dextran or derivative thereof is preferably 200-800kDa, optionally greater than 300, 350, 400 or 450 kDa, and optionally less than 700, 650, 600 or 550kDa, optionally about 500kDa, and ii) dextran, dextran derivatives or clearing agents of less than a molecular weight cut-off have been removed, wherein the molecular weight cut-off is 50kDa or above, 100kDa or above or 200kDa or above, optionally in the range 50kDa-250kDa or 50kDa-200kDa, optionally 100kDa-200kDa, optionally around 100kDa or 150kDa or 200kDa. (For the avoidance of doubt, we note that if the cut-off is stated as 50kDa or above, this means that the cut off may be 50kDa or any value over 50kDa, but it is still dextran, dextran derivatives or clearing agents of less than the cut-off which are removed).

The amount of species having a molecular weight below the cut-off may be, for example, 5 wt.% or less, 4 wt.% or less, 3 wt.% or less, 2 wt.% or less, 1 wt.% or less, 0.5 wt.% or less, 0.4 wt.% or less, 0.3 wt.% or less, 0.2 wt.% or less, 0.1 wt.% or less or 0.01 wt.% or less, as a weight percentage of the clearing agent. Preferably, the clearing agent is essentially free of species having a molecular weight below the cut-off.

The molecular weight cut-off can be achieved by filtration, for example by diafiltration, ultrafiltration, tangential flow filtration or crossflow filtration. Preferably, at least 2 filtration steps are carried out, optionally at least 3. By "average molecular weight", we mean weight average molecular weight as determined by SEC-MALS analysis.

It will be appreciated that when incorporated in DOTAM or a functional variant thereof, the metals will be present as metal ions, and that the oxidation states will vary depending on the specific element. Thus, the skilled reader understands that, for example, the terms lead, Pb, or $^{206}Pb$ are intended to encompass ionic forms of the element, in particular, Pb(II).

The metal present in the clearing agent may be a stable (non-radioactive) isotope of lead, or a stable or essentially stable isotope of another metal ion, provided that the metal ion-DOTAM complex is recognised with high affinity by the antibody. For example, other suitable metals may be Zn ($Zn^{2+}$), Ca ($Ca^{2+}$) or $^{209}Bi$ ($Bi^{2+}$), the latter of which is radioactive but is considered to be virtually stable due to its very long half-life.

The application describes a method of preparing a clearing agent, comprising conjugating a dextran or dextran derivative to DOTAM or a functional variant or derivative thereof, wherein the method involves chelating DOTAM with Pb or another metal ion as described above [e.g. Pb(II)] before and/or after conjugation of DOTAM or a functional variant thereof to the dextran.

The application describes a method of preparing a clearing agent, comprising:

forming a conjugate by conjugating DOTAM or a functional variant or derivative thereof to a dextran or dextran derivative;

wherein prior to conjugation the dextran or dextran derivative is subject to a filtration step to remove species below a molecular weight cut-off/threshold e.g., of 50kDa or above, 100kDa or above or 200kDa or above, optionally in the range of 50kDa-250kDa or 50kDa-200kDa, optionally 100kDa-200kDa, e.g., species below 100kDa, 150Kda or 200kDa, or wherein

the method further comprises subjecting the conjugate to a filtration step to remove species below a molecular weight cut-off/threshold e.g., of 50kDa or above, 100kDa or above or 200kDa or above, optionally in the range of 50kDa-250kDa or 50kDa-200kDa, optionally 100kDa-200kDa, e.g., species below 100kDa, 150kDa or 200kDa.

As described above, the present inventors have found it beneficial to apply a molecular weight cut-off, to remove fragments below a certain size. The filtration method may be, for example, diafiltration. The skilled reader will appreciate that the word "remove" in "remove species below a molecular weight cut-off/threshold" is synonymous with "reduce in number", and that some residual low molecular weight species may remain, depending on the particular filtration method employed. The amount of species having a molecular weight below the cut-off may be, for example, 5 wt.% or less, 4 wt.% or less, 3 wt.% or less, 2 wt.% or less, 1 wt.% or less, 0.5 wt.% or less, 0.4 wt.% or less, 0.3 wt.% or less, 0.2 wt.%

or less, 0.1 wt.% or less or 0.01 wt.% or less, as a weight percentage of the clearing agent. Preferably, the clearing agent is essentially free of species having a molecular weight below the cut-off/threshold after filtration.

The DOTAM functional variant or derivative may be as defined above, wherein at least one of the $R^1$ groups serves as the linker moiety. For example, a suitable (linker-(M-DOTAM)) group may be formed by reacting a compound of the following formula with an aminodextran as described above:

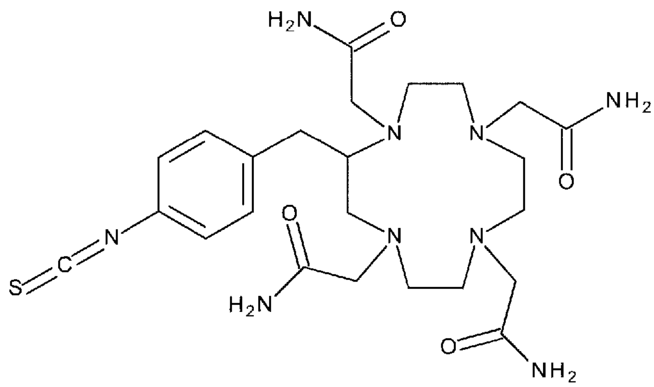

The synthesis of this compound is described in Chappell et al. Nuclear Medicine and Biology, Vol. 27, pp. 93-100, 2000, and the DOTAM derivatives are available commercially from Macrocyclics, Inc. (Plano, Texas).

The DOTAM or functional variant thereof may be added in excess, so that each dextran derivative has an average of greater than 1 DOTAM. The average number of DOTAM or functional variants thereof on each dextran may be greater than 1, for example, 2 or more, 3 or more, 4 or more, 5 or more, 10 or more, 15 or more, 20 or more, 25 or more, 30 or more, 35 or more, 40 or more, 50 or more, 100 or more or preferably 40 or more. The present inventors have found that improved clearing can be achieved using dextran conjugated to multiple M-DOTAM groups.

Preferably, the dextran has an average molecular weight of 200-800kDa, optionally greater than 300, 350, 400 or 450 kDa, and optionally less than 700, 650, 600 or 550kDa, optionally about 500kDa

The method of preparing a clearing agent may also comprise a chelating step, involving chelating the DOTAM or functional variant thereof with a metal ion. The metal ion may be a non-radioactive isotope, for example a non-radioactive isotope of Pb, Ca, Zn, or a virtually stable isotope, such as $^{209}Bi$.

The chelating step is carried out before conjugation of the DOTAM or functional variant thereof to the dextran and/or after conjugation of the DOTAM or functional variant thereof to the dextran but optionally before the filtration step. Chelation of the metal ion by the DOTAM or functional variant thereof may be necessary to ensure proper binding of the bispecific antibody to the clearing agent, for example to ensure that the DOTAM or functional variant thereof adopts the correct conformation for engaging with the antibody.

When the method comprises a chelating step, the method preferably also involves a subsequent step of removing unbound metal. This may be achieved by adding further chelating agent which can be subsequently separated from the dextran-bound DOTAM or functional variant thereof during the filtration step. The further chelating agent preferentially is different from DOTAM or a functional variant thereof. Preferably, the further chelating agent has a lower molecular weight than the dextran-chelating agent conjugate, to facilitate size-based separation. For example, the further chelating agent may be a polyaminocarboxylic acid, such as ethylenediaminotetraacetic acid (EDTA) or a salt thereof.

Preferably, the method of preparing a clearing agent involves:

i) forming a conjugate by conjugating DOTAM or a functional variant or derivative thereof to a dextran or dextran derivative;
ii) optionally, removing low molecular weight species from the product of step (i);
iii) chelating the conjugate with a metal ion, e.g., an ion of Pb, Bi, Zn or Ca;
iv) adding a further chelating agent to chelate unbound metal ion; and
v) carrying out a filtration step to remove species below a molecular weight cut-off/threshold.

DOTAM-Chelated Pb radionuclide

A Pb radionuclide chelated with DOTAM or a functional variant thereof may be used in any of the methods of diagnosis, imaging or treatment as described herein. It will be appreciated that when used in such methods, the Pb radionuclide chelated with DOTAM or a functional variant thereof is comprised in a composition. In one particular embodiment, the composition comprises the Pb radionuclide chelated by DOTAM or a functional variant thereof and DOTAM

or a functional variant thereof which is not chelated with the Pb radionuclide. Thus, in another aspect the present invention relates to such a composition, and/or to such a composition for use in any of the methods of imaging or treatment described herein. A Pb radionuclide chelated with DOTAM as referred to in such methods may be in the form of a composition as described herein.

The DOTAM or a functional variant thereof which is not chelated with the Pb radionuclide may be unchelated DOTAM or a functional variant thereof. When used in vivo, the unchelated DOTAM or a functional variant thereof may form complexes with metal ions from the environment, e.g. with calcium ions. Such calcium ions chelated with DOTAM or a functional variant thereof are pharmacologically inactive and can potentially block the pharmacologically active Pb radionuclide chelated with DOTAM or a variant thereof from targets in the tumor and therefore may reduce the efficacy of the treatment, and/or standardized uptake value of the imaging and diagnosis. The present inventors found that by quenching the unchelated DOTAM or functional variant thereof under defined conditions, the control of the in vivo formulation of the chelated Pb radionuclide can be increased and/or potential competition between the pharmaceutically active chelate and pharmaceutically inactive chelate can be avoided or reduced. Therefore, in some embodiments, the DOTAM or a functional variant thereof which is not chelated with the Pb radionuclide is DOTAM or a functional variant that is chelated with a non-radioactive metal ion.

In some embodiment, the chelated Pb radionuclide is $^{212}Pb$. In some embodiments the chelated Pb radionuclide is $^{203}Pb$.

It will be appreciated that when incorporated in DOTAM or a functional variant thereof, Pb radionuclide will be present as metal ions, and that the oxidation states will vary depending on the specific element. Thus, the skilled reader understands that, for example, the terms lead, Pb, or $^{206}Pb$ are intended to encompass ionic forms of the element, in particular, Pb(II).

The non-radioactive metal present in the composition may be a stable (non-radioactive) isotope of lead, or a stable or essentially stable isotope of another metal ion. For example, other suitable metals may be Gd (Gd2+), Cu (Cu2+), Zn (Zn2+), Ca (Ca2+) or $^{209}Bi$ (Bi2+), the latter of which is radioactive but is considered to be virtually stable due to its very long half-life. In some embodiment, the metal is Ca or Cu, In some embodiment, the metal is Ca.

The DOTAM functional variant or derivative may be as defined above.

The application describes a method of preparing a composition comprising Pb radionuclide chelated with DOTAM or a functional variant thereof, comprising:

i) providing Pb radionuclides,
ii) chelating the Pb radionuclide with DOTAM or a functional variant thereof
iii) chelating the unchelated DOTAM or a functional variant thereof with a non-radioactive metal ion.

Unchelated DOTAM or a functional variant thereof in step iii) is DOTAM or a functional variant thereof that was not chelated with the Pb radionuclide in step ii).

The non-radioactive metal ion may be an ion of Pb, Ca, Zn, Gd or Cu. In some embodiment, the metal ion is an ion of Ca or Cu. In some embodiment, the metal ion is an ion of Ca, in particular Ca2+.

In some embodiment, the unchelated DOTAM or functional variant thereof remaining after step ii) is at least 90mol%, at least 95mol%, at least 99mol% of the DOTAM or functional variant thereof added to the Pb radionuclide. In one particular embodiment, the unchelated DOTAM or functional variant thereof remaining after step ii) is at least 99mol%.

In some embodiments, the unchelated DOTAM or functional variant thereof remaining after step iii) is less than 5mol%, less than 2mol%, less than 1mol%, less than 0.1mol%, less than 0.01mol%of the DOTAM or functional variant thereof added to the Pb radionuclide. In one particular embodiment, the unchelated DOTAM or functional variant thereof remaining after step iii) is less than 1mol%, less than 0.1mol%, less than 0.01mol%.

Pb radionuclides provided in step a) may be generated by placing radioactive material that decays to the Pb radionuclide of interest in a generator, wherein the radioactive material is bound to a solid material. For example, such radioactive material in the production of $^{212}Pb$ may be 224 Radium. The radionuclide of interest is then extracted from the generator in an aqueous solution which can contain radiological and chemical impurities. The aqueous solution containing the Pb radionuclide of interest and the impurities is purified via a liquid chromatography on a column. The liquid chromatography on a column may be an extraction chromatography or a partition chromatography. An extraction or partition chromatography is based on the distribution of the elements that are to be separated between an organic phase, or extractant, and an aqueous phase, wherein the extractant being bound to an inert support and forming with it the stationary phase, whereas the aqueous phase represents the mobile phase.

The extraction chromatography may use a stationary phase which includes an ether crown as the extractant and, in particular, a dicyclohexano- 1 8-crown-6 or a dibenzo- 1 8-crown-6 whose cyclohexyl or benzyl groups are substituted by one or more C [ to C] 2 alkyl groups, with a straight or branched chain, in solution in an organic diluent not miscible with water, typically a long hydrocarbon chain alcohol, in other words a Cx chain and above.

In particular, a stationary phase may be used which comprises 4,4'(5')-dier - butylcyclohexano- 1 8-crown-6

as the extractant, preferably diluted in octan- 1 -ol. Such a stationary phase has the advantage of selectively retaining over 99% of $^{212}$Pb present in an aqueous solution containing from 1.5 to 2.5 moles/L of a strong acid, which typically corresponds to the types of aqueous solutions that are used to extract $^{212}$Pb from a radium-224 generator. This type of stationary phase is for example available, in bottles but also packaged in ready-to-use columns or cartridges for chromatography, from the company TRISKEM International under the commercial name "Pb resin".

Alternatively, the solution comprising the desired radionuclide and the impurities can also be purified with an ion exchange chromatography, for example, cation exchange chromatography.

A method of producing and purifying $^{212}$Pb is described in WO2013174949.

SEQUENCES

Certain sequences as referred to herein are provided in the table below.

Table 2

| SEQ ID NO | Description | SEQUENCE |
|---|---|---|
| 1 | heavy chain CDR1 <Pb-Dotam> PRIT-213 | gfslstysms |
| 2 | heavy chain CDR2 <Pb-Dotam> PRIT-213 | figsrgdtyyaswakg |
| 3 | heavy chain CDR3 <Pb-Dotam> PRIT-213 | erdpygggaypphl |
| 4 | light chain CDR1 <Pb-Dotam> PRIT-213 | qsshsvysdndla |
| 5 | light chain CDR2 <Pb-Dotam> PRIT-213 | qasklas |
| 6 | light chain CDR3 <Pb-Dotam> PRIT-213 | lggyddesdtyg |
| 7 | heavy chain variable domain 1 of <Pb-Dotam> PRIT-213 | vtlkesgpvl vkptetltlt ctvsgfslst ysmswirqpp gkalewlgfi gsrgdtyyas wakgrltisk dtsksqvvlt mtnmdpvdta tyycarerdp ygggaypphl wgrgtlvtvs s |
| 8 | light chain variable domain <Pb-Dotam> PRIT-213 | iqmtqspssl sasvgdrvti tcqsshsvys dndlawyqqk pgkapklliy qasklasgvp srfsgsgsgt dftltisslq pedfatyycl ggyddesdty gfgggtkvei k |
| 9 | heavy chain variable domain <Pb-Dotam> PRIT-214 | vqlqqwgagl lkpsetlslt cavygfslst ysmswirqpp gkglewigfi gsrgdtyyas wakgrvtisr dtsknqvslk lssvtaadta vyycarerdp ygggaypphl wgrgtlvtvs s |
| 10 | light chain variable domain <Pb-Dotam> PRIT-214 | iqmtqspssl sasvgdrvti tcqsshsvys dndlawyqqk pgkapklliy qasklasgvp srfsgsgsgt dftltisslq pedfatyycl ggyddesdty gfgggtkvei k |
| 11 | heavy chain CDR1 <CEA> | GFNIKDTYMH |
| 12 | heavy chain CDR2 <CEA> | RIDPANGNSKYVPKFQG |
| 13 | heavy chain CDR3 <CEA> | FGYYVSDYAMAY |
| 14 | light chain CDR1 <CEA> | RAGESVDIFGVGFLH |

(continued)

| SE QID NO | Description | SEQUENCE |
|---|---|---|
| 15 | light chain CDR2 <CEA> | RASNRAT |
| 16 | light chain CDR3 <CEA> | QQTNEDPYT |
| 17 | heavy chain variable domain <CEA> 84.66 | QVQLVQSGAEVKKPGSSVKVSCKASGFNIKDTYMHWVRQAPGQGLEWMGRIDPANGNSKY<br>VPKFQGRVTITADTSTSTAYMELSSLRSEDTAVYYCAPFGYYVSDYAMAYWGQGTLVTVSS |
| 18 | Light chain variable domain <CEA> 84.66 | EIVLTQSPATLSLSPGERATLSCRAGESVDIFGVGFLHWYQQKPGQAPRLLIYRASNRATGIPA<br>RFSGSGSGTDFTLTISSLEPEDFAVYYCQQTNEDPYTFGQGTKLEIK |
| 19 | heavy chain 1 of bispecific, trivalent<CEA/Pb-Dotam> PRIT-214 VH_84.66 | 1 qvqlvqsgae vkkpgssvkv sckasgfnik dtymhwvrqa pgqglewmgr<br>51 idpangnsky vpkfqgrvti tadtststay melsslrsed tavyycapfg<br>101 yyvsdyamay wgqgtlvtvs sastkgpsvf plapssksts ggtaalgclv<br>151 kdyfpepvtv swnsgaltsg vhtfpavlqs sglyslssvv tvpssslgtq<br>201 tyicnvnhkp sntkvdkkve pkscdkthtc ppcpapeaag gpsvflfppk<br>251 pkdtlmisrt pevtcvvvdv shedpevkfn wyvdgvevhn aktkpreeqy<br>301 nstyrvvsvl tvlhqdwlng keykckvsnk algapiekti skakgqprep<br>351 qvytlppcrd eltknqvslw clvkgfypsd iavewesngq pennykttpp<br>401 vldsdgsffl yskltvdksr wqqgnvfscs vmhealhnhy tqkslslspg<br>451 ggggsggggs ggggsggggs vqlqqwgagl lkpsetlslt cavygfslst<br>501 ysmswirqpp gkglewigfi gsrgdtyyas wakgrvtisr dtsknqvslk<br>551 lssvtaadta vyycarerdp ygggaypphl wgrgtlvtvs s |
| 20 | heavy chain 2 of bispecific, trivalent<CEA/Pb-Dotam> PRIT-214 VL_84.66 | 1 qvqlvqsgae vkkpgssvkv sckasgfnik dtymhwvrqa pgqglewmgr<br>51 idpangnsky vpkfqgrvti tadtststay melsslrsed tavyycapfg<br>101 yyvsdyamay wgqgtlvtvs sastkgpsvf plapssksts ggtaalgclv<br>151 kdyfpepvtv swnsgaltsg vhtfpavlqs sglyslssvv tvpssslgtq<br>201 tyicnvnhkp sntkvdkkve pkscdkthtc ppcpapeaag gpsvflfppk<br>251 pkdtlmisrt pevtcvvvdv shedpevkfn wyvdgvevhn aktkpreeqy<br>301 nstyrvvsvl tvlhqdwlng keykckvsnk algapiekti skakgqprep<br>351 qvctlppsrd eltknqvsls cavkgfypsd iavewesngq pennykttpp<br>401 vldsdgsffl vskltvdksr wqqgnvfscs vmhealhnhy tqkslslspg<br>451 ggggsggggs ggggsggggs iqmtqspssl sasvgdrvti tcqsshsvys<br>501 dndlawyqqk pgkapklliy qasklasgvp srfsgsgsgt dftltisslq<br>551 pedfatyycl ggyddesdty gfgggtkvei k |

(continued)

| SEQ ID NO | Description | SEQUENCE |
|---|---|---|
| 21 | light chain <CEA> 84.66 | 1 eivltqspat lslspgerat lscragesvd ifgvgflhwy qqkpgqaprl<br>51 liyrasnrat giparfsgsg sgtdftltis slepedfavy ycqqtnedpy<br>101 tfgqgtklei krtvaapsvf ifppsdeqlk sgtasvvcll nnfypreakv<br>151 qwkvdnalqs gnsqesvteq dskdstysls stltlskady ekhkvyacev<br>201 thqglsspvt ksfnrgec |
| 22 | heavy chain 1 of bispecific, trivalent <CEA/Pb-Dotam> PRIT-213 | 1 qvqlvqsgae vkkpgssvkv sckasgfnik dtymhwvrqa pgqglewmgr<br>51 idpangnsky vpkfqgrvti tadtststay melsslrsed tavyycapfg<br>101 yyvsdyamay wgqgtlvtvs sastkgpsvf plapssksts ggtaalgclv |
| | VH_84.66 → knob | 151 kdyfpepvtv swnsgaltsg vhtfpavlqs sglyslssvv tvpssslgtq<br>201 tyicnvnhkp sntkvdkkve pkscdkthtc ppcpapeaag gpsvflfppk<br>251 pkdtlmisrt pevtcvvvdv shedpevkfn wyvdgvevhn aktkpreeqy<br>301 nstyrvvsvl tvlhqdwlng keykckvsnk algapiekti skakgqprep<br>351 qvytlppcrd eltknqvslw clvkgfypsd iavewesngq pennykttpp<br>401 vldsdgsffl yskltvdksr wqqgnvfscs vmhealhnhy tqkslslspg<br>451 ggggsggggs ggggsggggs vtlkesgpvl vkptetltlt ctvsgfslst<br>501 ysmswirqpp gkalewlgfi gsrgdtyyas wakgrltisk dtsksqvvlt<br>551 mtnmdpvdta tyycarerdp ygggayppphl wgrgtlvtvs s |
| 23 | heavy chain 2 of bispecific, trivalent <CEA/Pb-Dotam> PRIT-213 VL_84.66 → hole | 1 qvqlvqsgae vkkpgssvkv sckasgfnik dtymhwvrqa pgqglewmgr<br>51 idpangnsky vpkfqgrvti tadtststay melsslrsed tavyycapfg<br>101 yyvsdyamay wgqgtlvtvs sastkgpsvf plapssksts ggtaalgclv<br>151 kdyfpepvtv swnsgaltsg vhtfpavlqs sglyslssvv tvpssslgtq<br>201 tyicnvnhkp sntkvdkkve pkscdkthtc ppcpapeaag gpsvflfppk<br>251 pkdtlmisrt pevtcvvvdv shedpevkfn wyvdgvevhn aktkpreeqy<br>301 nstyrvvsvl tvlhqdwlng keykckvsnk algapiekti skakgqprep<br>351 qvctlppsrd eltknqvsls cavkgfypsd iavewesngq pennykttpp<br>401 vldsdgsffl vskltvdksr wqqgnvfscs vmhealhnhy tqkslslspg<br>451 ggggsggggs ggggsggggs iqmtqspssl sasvgdrvti tcqsshsvys<br>501 dndlawyqqk pgkapklliy qasklasgvp srfsgsgsgt dftltisslq<br>551 pedfatyycl ggyddesdty gfgggtkvei k |

(continued)

| SEQ ID NO | Description | SEQUENCE |
|---|---|---|
| 24 | heavy chain 1 of bispecific, <CEA/Pb-Dotam> Rabbit Dotam _84.66 | 1 qvqlvqsgae vkkpgssvkv sckasgfnik dtymhwvrqa pgqglewmgr<br>51 idpangnsky vpkfqgrvti tadtststay melsslrsed tavyycapfg<br>101 yyvsdyamay wgqgtlvtvs sastkgpsvf plapssksts ggtaalgclv<br>151 kdyfpepvtv swnsgaltsg vhtfpavlqs sglyslssvv tvpssslgtq<br>201 tyicnvnhkp sntkvdkkve pkscdkthtc ppcpapeaag gpsvflfppk<br>251 pkdtlmisrt pevtcvvvdv shedpevkfn wyvdgvevhn aktkpreeqy<br>301 nstyrvvsvl tvlhqdwlng keykckvsnk algapiekti skakgqprep<br>351 qvctlppsrd eltknqvsls cavkgfypsd iavewesngq pennykttpp<br>401 vldsdgsffl vskltvdksr wqqgnvfscs vmhealhnhy tqkslslspg<br>451 ggggsggggs ggggsggggs avltqtpspv spavggtvti scqsshsvys<br>501 dndlawyqqk lgqppklliy qasklasgvs srfsgsgsgt qftltisgvq<br>551 sddaatyycl ggyddesdty gfgggtevvv k |
| 25 | heavy chain 2 of bispecific, tetravalent <CEA/Pb-Dotam> Rabbit Dotam_84.66 | 1 qvqlvqsgae vkkpgssvkv sckasgfnik dtymhwvrqa pgqglewmgr<br>51 idpangnsky vpkfqgrvti tadtststay melsslrsed tavyycapfg<br>101 yyvsdyamay wgqgtlvtvs sastkgpsvf plapssksts ggtaalgclv<br>151 kdyfpepvtv swnsgaltsg vhtfpavlqs sglyslssvv tvpssslgtq<br>201 tyicnvnhkp sntkvdkkve pkscdkthtc ppcpapeaag gpsvflfppk<br>251 pkdtlmisrt pevtcvvvdv shedpevkfn wyvdgvevhn aktkpreeqy |
| | | 301 nstyrvvsvl tvlhqdwlng keykckvsnk algapiekti skakgqprep<br>351 qvytlppcrd eltknqvslw clvkgfypsd iavewesngq pennykttpp<br>401 vldsdgsffl yskltvdksr wqqgnvfscs vmhealhnhy tqkslslspg<br>451 ggggsggggs ggggsggggs qsveesggrl vtpgtpltlt ctvsgfslst<br>501 ysmswvrqap gkglewigfi gsrgdtyyas wakgrftvsr tsttvdlkit<br>551 spttedtaty fcarerdpyg ggaypphlwg pgtlvtvss |
| 26 | Linker | GGGGSGGGGSGGGGSGGGGS |

(continued)

| SEQ ID NO | Description | SEQUENCE |
|---|---|---|
| 27 | Immunomedics hNM14 | 1 evqlvesggg vvqpgrslrl scsasgfdft tywmswvrqa pgkglewige<br>51 ihpdsstiny apslkdrfti srdnakntlf lqmdslrped tgvyfcasly<br>101 fgfpwfaywg qgtpvtvssa stkgpsvfpl apsskstsgg taalgclvkd<br>151 yfpepvtvsw nsgaltsgvh tfpavlqssg lyslssvvtv pssslgtqty<br>201 icnvnhkpsn tkvdkkvepk scdkthtcpp cpapeaaggp svflfppkpk<br>251 dtlmisrtpe vtcvvvdvsh edpevkfnwy vdgvevhnak tkpreeqyns<br>301 tyrvvsvltv lhqdwlngke ykckvsnkal gapiektisk akgqprepqv<br>351 ytlppcrdel tknqvslwcl vkgfypsdia vewesngqpe nnykttppvl<br>401 dsdgsfflys kltvdksrwq qgnvfscsvm healhnhytq kslslspggg<br>451 ggsggggsgg ggsggggsqs veesggrlvt pgtpltltct vsgfslstys<br>501 mswvrqapgk glewigfigs rgdtyyaswa kgrftvsrts ttvdlkitsp<br>551 ttedtatyfc arerdpyggg aypphlwgpg tlvtvss<br>1 evqlvesggg vvqpgrslrl scsasgfdft tywmswvrqa pgkglewige<br>51 ihpdsstiny apslkdrfti srdnakntlf lqmdslrped tgvyfcasly<br>101 fgfpwfaywg qgtpvtvssa stkgpsvfpl apsskstsgg taalgclvkd<br>151 yfpepvtvsw nsgaltsgvh tfpavlqssg lyslssvvtv pssslgtqty |

(continued)

| SEQ ID NO | Description | SEQUENCE |
|---|---|---|
| | | 201 icnvnhkpsn tkvdkkvepk scdkthtcpp cpapeaaggp svflfppkpk<br>251 dtlmisrtpe vtcvvvdvsh edpevkfnwy vdgvevhnak tkpreeqyns<br>301 tyrvvsvltv lhqdwlngke ykckvsnkal gapiektisk akgqprepqv<br>351 ctlppsrdel tknqvslsca vkgfypsdia vewesngqpe nnykttppvl<br>401 dsdgsfflvs kltvdksrwq qgnvfscsvm healhnhytq kslslspggg<br>451 ggsggggsgg ggsggggsav ltqtpspvsp avggtvtisc qsshsvysdn<br>501 dlawyqqklg qppklliyqa sklasgvssr fsgsgsgtqf tltisgvqsd<br>551 daatyyclgg yddesdtygf gggtevvvk<br>1 diqltqspss lsasvgdrvt itckasqdvg tsvawyqqkp gkapklliyw<br>51 tstrhtgvps rfsgsgsgtd ftftisslqp ediatyycqq yslyrsfgqg<br>101 tkveikrtva apsvfifpps deqlksgtas vvcllnnfyp reakvqwkvd<br>151 nalqsgnsqe svteqdskds tyslsstltl skadyekhkv yacevthqgl<br>201 sspvtksfnr gec |
| 28 | Heavy chain CDR1 <ERBB2> | DTYIH |
| 29 | Heavy chain CDR2 <ERBB2> | RIYPTNGYTRYADSVKG |
| 30 | Heavy chain CDR3 <ERBB2> | WGGDGFYAMDY |
| 31 | Light chain CDR1 <ERBB2> | RASQDVNTAVA |
| 32 | Light chain CDR2 <ERBB2> | SASFLYS |
| 33 | Light chain CDR3 <ERBB2> | QQHYTTPPT |
| 34 | Heavy chain variable domain <ERBB2> | EVQLVESGGGLVQPGGSLRLSCAASGFNIKDTYIHWVRQAPGKGLEWVARIYPTNGYTRYADSVKGRFTISADTSKNTAYLQ<br>MNSLRAEDTAVYYCSRWGGDGFYAMDYWGQGTLVTVSS |

(continued)

| SEQ ID NO | Description | SEQUENCE |
|---|---|---|
| 35 | Light chain variable domain <ERBB2> | DIQMTQSPSSLSASVGDRVTITCRASQDVNTAVAWYQQKPGKAPKLLIYSASFLYSGVPSRFSGSRSGTDFTLTISSLQPED FATYYCQQHYTTPPTFGQGTKVEIK |
| 36 | Heavy chain 1 (knob) of | EVQLVESGGGLVQPGGSLRLSCAASGFNIKDTYIHWVRQAPGKGLEWVARIYPTNGYTRYADSVKGRFTISADTSKNTAYLQ |
| | bispecific, trivalent ERbB2/Pb-Dotam (P1AD9827) | MNSLRAEDTAVYYCSRWGGDGFYAMDYWGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCDKTHTCPPCPAPEAAGGPSVFLF PPKPKDTLMISRTPEVTCVVVDVSHEDPEVKFNWYVDGVEVHNAKTKPREEQYNSTYRVVSVLTVLHQDWLNGKEYKCKVSN KALGAPIEKTISKAKGQPREPQVYTLPPCRDELTKNQVSLWCLVKGFYPSDIAVEWESNGQPENNYKTTPPVLDSDGSFFLY SKLTVDKSRWQQGNVFSCSVMHEALHNHYTQKSLSLSPGGGGGSGGGGSGGGGSGGGGSVTLKESGPVLVKPTETLTLTCTV SGFSLSTYSMSWIRQPPGKALEWLGFIGSRGDTYYASWAKGRLTISKDTSKSQVVLTMTNMDPVDTATYYCARERDPYGGGA YPPHLWGRGTLVTVSS |
| 37 | Heavy chain 2 (hole) of bispecific, trivalent ERbB2/Pb-Dotam (P1AD9827) | EVQLVESGGGLVQPGGSLRLSCAASGFNIKDTYIHWVRQAPGKGLEWVARIYPTNGYTRYADSVKGRFTISADTSKNTAYLQ MNSLRAEDTAVYYCSRWGGDGFYAMDYWGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCDKTHTCPPCPAPEAAGGPSVFLF PPKPKDTLMISRTPEVTCVVVDVSHEDPEVKFNWYVDGVEVHNAKTKPREEQYNSTYRVVSVLTVLHQDWLNGKEYKCKVSN KALGAPIEKTISKAKGQPREPQVCTLPPSRDELTKNQVSLSCAVKGFYPSDIAVEWESNGQPENNYKTTPPVLDSDGSFFLV SKLTVDKSRWQQGNVFSCSVMHEALHNHYTQKSLSLSPGGGGGSGGGGSGGGGSGGGGSIQMTQSPSSLSASVGDRVTITCQ SSHSVYSDNDLAWYQQKPGKAPKLLIYQASKLASGVPSRFSGSGSGTDFTLTISSLQPEDFATYYCLGGYDDESDTYGFGGG TKVEIK |
| 38 | Light chain <ErbB2> (P1AD9827) | DIQMTQSPSSLSASVGDRVTITCRASQDVNTAVAWYQQKPGKAPKLLIYSASFLYSGVPSRFSGSRSGTDFTLTISSLQPED FATYYCQQHYTTPPTFGQGTKVEIKRTVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| 39 | Heavy chain CDR1 <CD20> | YSWIN |
| 40 | Heavy chain CDR2 <CD20> | RIFPGDGDTDYNGKFKG |
| 41 | Heavy chain CDR3 <CD20> | NVFDGYWLVY |
| 42 | Light chain CDR1 <CD20> | RSSKSLLHSNGITYLY |
| 43 | Light chain CDR2 <CD20> | QMSNLVS |
| 44 | Light chain CDR3 CD20> | AQNLELPYT |
| 45 | Heavy chain variable domain <CD20> | QVQLVQSGAEVKKPGSSVKVSCKASGYAFSYSWINWVRQAPGQGLEWMGRIFPGDGDTDYNGKFKGRVTITADKSTSTAYME LSSLRSEDTAVYYCARNVFDGYWLVYWGQGTLVTVSS |

(continued)

| SEQ ID NO | Description | SEQUENCE |
|---|---|---|
| 46 | Light chain variable domain <CD20> | DIVMTQTPLSLPVTPGEPASISCRSSKSLLHSNGITYLYWYLQKPGQSPQLLIYQMSNLVSGVPDRFSGSGSGTDFTLKISR<br>VEAEDVGVYYCAQNLELPYTFGGGTKVEIK |
| 47 | Heavy chain 1 (knob) of bispecific, trivalent CD20/Pb-Dotam(P1AD9826) | QVQLVQSGAEVKKPGSSVKVSCKASGYAFSYSWINWVRQAPGQGLEWMGRIFPGDGDTDYNGKFKGRVTITADKSTSTAYME<br>LSSLRSEDTAVYYCARNVFDGYWLVYWGQGTLVTVSSASTKGPSVFPLAPSSKSTSGGTAALGCLVKDYFPEPVTVSWNSGA<br>LTSGVHTFPAVLQSSGLYSLSSVVTVPSSSLGTQTYICNVNHKPSNTKVDKKVEPKSCDKTHTCPPCPAPEAAGGPSVFLFP<br>PKPKDTLMISRTPEVTCVVVDVSHEDPEVKFNWYVDGVEVHNAKTKPREEQYNSTYRVVSVLTVLHQDWLNGKEYKCKVSNK<br>ALGAPIEKTISKAKGQPREPQVYTLPPCRDELTKNQVSLWCLVKGFYPSDIAVEWESNGQPENNYKTTPPVLDSDGSFFLYS<br>KLTVDKSRWQQGNVFSCSVMHEALHNHYTQKSLSLSPGGGGGSGGGGSGGGGSGGGGSVTLKESGPVLVKPTETLTLTCTVS<br>GFSLSTYSMSWIRQPPGKALEWLGFIGSRGDTYYASWAKGRLTISKDTSKSQVVLTMTNMDPVDTATYYCARERDPYGGGAY<br>PPHLWGRGTLVTVSS |
| 48 | Heavy chain 2 (hole) of bispecific, trivalent CD20/Pb-Dotam (P1AD9826) | QVQLVQSGAEVKKPGSSVKVSCKASGYAFSYSWINWVRQAPGQGLEWMGRIFPGDGDTDYNGKFKGRVTITADKSTSTAYME<br>LSSLRSEDTAVYYCARNVFDGYWLVYWGQGTLVTVSSASTKGPSVFPLAPSSKSTSGGTAALGCLVKDYFPEPVTVSWNSGA<br>LTSGVHTFPAVLQSSGLYSLSSVVTVPSSSLGTQTYICNVNHKPSNTKVDKKVEPKSCDKTHTCPPCPAPEAAGGPSVFLFP |
| | | PKPKDTLMISRTPEVTCVVVDVSHEDPEVKFNWYVDGVEVHNAKTKPREEQYNSTYRVVSVLTVLHQDWLNGKEYKCKVSNK<br>ALGAPIEKTISKAKGQPREPQVCTLPPSRDELTKNQVSLSCAVKGFYPSDIAVEWESNGQPENNYKTTPPVLDSDGSFFLVS<br>KLTVDKSRWQQGNVFSCSVMHEALHNHYTQKSLSLSPGGGGGSGGGGSGGGGSGGGGSIQMTQSPSSLSASVGDRVTITCQS<br>SHSVYSDNDLAWYQQKPGKAPKLLIYQASKLASGVPSRFSGSGSGTDFTLTISSLQPEDFATYYCLGGYDDESDTYGFGGGT<br>KVEIK |
| 49 | Light chain <CD20> (P1AD9826) | DIVMTQTPLSLPVTPGEPASISCRSSKSLLHSNGITYLYWYLQKPGQSPQLLIYQMSNLVSGVPDRFSGSGSGTDFTLKISR<br>VEAEDVGVYYCAQNLELPYTFGGGTKVEIKRTVAAPSVFIFPPSDRKLKSGTASVVCLLNNFYPREAKVQWKVDNALQSGNS<br>QESVTEQDSKDSTYSLSSTLTLSKADYEKHKVYACEVTHQGLSSPVTKSFNRGEC |
| 50 | P1AE1766 >CEA Light Chain RK | DIQMTQSPSSLSASVGDRVTITCKASAAVGTYVAWYQQKPGKAPKLLIYSASYRKRGVPSRFSGSGSGTDFTLTISSLQPED<br>FATYYCHQYYTYPLFTFGQGTKLEIKRTVAAPSVFIFPPSD**RK**LKSGTASVVCLLNNFYPREAKVQWKVDNALQSGNSQESV<br>TEQDSKDSTYSLSSTLTLSKADYEKHKVYACEVTHQGLSSPVTKSFNRGEC |

(continued)

| SEQ ID NO | Description | SEQUENCE |
|---|---|---|
| 51 | P1AE1766 CEA Heavy Chain with DOTAM VL / CH1 | QVQLVQSGAEVKKPGASVKVSCKASGYTFTEFGMNWVRQAPGQGLEWMGWINTKTGEATYVEEFKGRVTFTTDTSTSTAYME<br>LRSLRSDDTAVYYCARWDFAYYVEAMDYWGQGTTVTVSSASTKGPSVFPLAPSSKSTSGGTAALGCLVEDYFPEPVTVSWNS<br>GALTSGVHTFPAVLQSSGLYSLSSVVTVPSSSLGTQTYICNVNHKPSNTKVDEKVEPKSCDKTHTCPPCPAPEAAGGPSVFL<br>FPPKPKDTLMISRTPEVTCVVVDVSHEDPEVKFNWYVDGVEVHNAKTKPREEQYNSTYRVVSVLTVLHQDWLNGKEYKCKVS<br>NKALGAPIEKTISKAKGQPREPQVYTLPPCRDELTKNQVSLWCLVKGFYPSDIAVEWESNGQPENNYKTTPPVLDSDGSFFL<br>YSKLTVDKSRWQQGNVFSCSVMHEALHNHYTQKSLSLSPGGGGGGSGGGGSGGGGSGGGGSIQMTQSPSSLSASVGDRVTIT<br>CQSSHSVYSDNDLAWYQQKPGKAPKLLIYQASKLASGVPSRFSGSGSGTDFTLTISSLQPEDFATYYCLGGYDDESDTYGFG<br>GGTKVEIKSSASTKGPSVFPLAPSSKSTSGGTAALGCLVKDYFPEPVTVSWNSGALTSGVHTFPAVLQSSGLYSLSSVVTVP<br>SSSLGTQTYICNVNHKPSNTKVDKKVEPKSC |
| 52 | P1AE1766 CEA Heavy Chain with DOTAM VH / CK | QVQLVQSGAEVKKPGASVKVSCKASGYTFTEFGMNWVRQAPGQGLEWMGWINTKTGEATYVEEFKGRVTFTTDTSTSTAYME<br>LRSLRSDDTAVYYCARWDFAYYVEAMDYWGQGTTVTVSSASTKGPSVFPLAPSSKSTSGGTAALGCLVEDYFPEPVTVSWNS<br>GALTSGVHTFPAVLQSSGLYSLSSVVTVPSSSLGTQTYICNVNHKPSNTKVDEKVEPKSCDKTHTCPPCPAPEAAGGPSVFL<br>FPPKPKDTLMISRTPEVTCVVVDVSHEDPEVKFNWYVDGVEVHNAKTKPREEQYNSTYRVVSVLTVLHQDWLNGKEYKCKVS<br>NKALGAPIEKTISKAKGQPREPQVCTLPPSRDELTKNQVSLSCAVKGFYPSDIAVEWESNGQPENNYKTTPPVLDSDGSFFL<br>VSKLTVDKSRWQQGNVFSCSVMHEALHNHYTQKSLSLSPGGGGGGSGGGGSGGGGSGGGGSVTLKESGPVLVKPTETLTLTC<br>TVSGFSLSTYSMSWIRQPPGKALEWLGFIGSRGDTYYASWAKGRLTISKDTSKSQVVLTMTNMDPVDTATYYCARERDPYGG<br>GAYPPHLWGRGTLVTVSSASVAAPSVFIFPPSDEQLKSGTASVVCLLNNFYPREAKVQWKVDNALQSGNSQESVTEQDSKDS<br>TYSLSSTLTLSKADYEKHKVYACEVTHQGLSSPVTKSFNRGEC |
| 53 | P1AE1767 >DOTAM "LC" with VH / CK | VTLKESGPVLVKPTETLTLTCTVSGFSLSTYSMSWIRQPPGKALEWLGFIGSRGDTYYASWAKGRLTISKDTSKSQVVLTMT<br>NMDPVDTATYYCARERDPYGGGAYPPHLWGRGTLVTVSSASVAAPSVFIFPPSDEQLKSGTASVVCLLNNFYPREAKVQWKV<br>DNALQSGNSQESVTEQDSKDSTYSLSSTLTLSKADYEKHKVYACEVTHQGLSSPVTKSFNRGEC |
| 54 | P1AE1767 CEA Light Chain RK | DIQMTQSPSSLSASVGDRVTITCKASAAVGTYVAWYQQKPGKAPKLLIYSASYRKRGVPSRFSGSGSGTDFTLTISSLQPED<br>FATYYCHQYYTYPLFTFGQGTKLEIKRTVAAPSVFIFPPSDRKLKSGTASVVCLLNNFYPREAKVQWKVDNALQSGNSQESV<br>TEQDSKDSTYSLSSTLTLSKADYEKHKVYACEVTHQGLSSPVTKSFNRGEC |
| 55 | P1AE1767 CEA Heavy Chain with DOTAM VL / CH1 (knob) | QVQLVQSGAEVKKPGASVKVSCKASGYTFTEFGMNWVRQAPGQGLEWMGWINTKTGEATYVEEFKGRVTFTTDTSTSTAYME<br>LRSLRSDDTAVYYCARWDFAYYVEAMDYWGQGTTVTVSSASTKGPSVFPLAPSSKSTSGGTAALGCLVEDYFPEPVTVSWNS<br>GALTSGVHTFPAVLQSSGLYSLSSVVTVPSSSLGTQTYICNVNHKPSNTKVDEKVEPKSCDKTHTCPPCPAPEAAGGPSVFL |
| | | FPPKPKDTLMISRTPEVTCVVVDVSHEDPEVKFNWYVDGVEVHNAKTKPREEQYNSTYRVVSVLTVLHQDWLNGKEYKCKVS<br>NKALGAPIEKTISKAKGQPREPQVYTLPPCRDELTKNQVSLWCLVKGFYPSDIAVEWESNGQPENNYKTTPPVLDSDGSFFL<br>YSKLTVDKSRWQQGNVFSCSVMHEALHNHYTQKSLSLSPGGGGGGSGGGGSGGGGSGGGGSIQMTQSPSSLSASVGDRVTIT<br>CQSSHSVYSDNDLAWYQQKPGKAPKLLIYQASKLASGVPSRFSGSGSGTDFTLTISSLQPEDFATYYCLGGYDDESDTYGFG<br>GGTKVEIKSSASTKGPSVFPLAPSSKSTSGGTAALGCLVKDYFPEPVTVSWNSGALTSGVHTFPAVLQSSGLYSLSSVVTVP<br>SSSLGTQTYICNVNHKPSNTKVDKKVEPKSC |

(continued)

| SEQ ID NO | Description | SEQUENCE |
|---|---|---|
| 56 | P1AE1767 CEA Heavy Chain (hole) | QVQLVQSGAEVKKPGASVKVSCKASGYTFTEFGMNWVRQAPGQGLEWMGWINTKTGEATYVEEFKGRVTFTTDTSTSTAYME LRSLRSDDTAVYYCARWDFAYYVEAMDYWGQGTTVTVSSASTKGPSVFPLAPSSKSTSGGTAALGCLVEDYFPEPVTVSWNS GALTSGVHTFPAVLQSSGLYSLSSVVTVPSSSLGTQTYICNVNHKPSNTKVDEKVEPKSCDKTHTCPPCPAPEAAGGPSVFL FPPKPKDTLMISRTPEVTCVVVDVSHEDPEVKFNWYVDGVEVHNAKTKPREEQYNSTYRVVSVLTVLHQDWLNGKEYKCKVS NKALGAPIEKTISKAKGQPREPQVCTLPPSRDELTKNQVSLSCAVKGFYPSDIAVEWESNGQPENNYKTTPPVLDSDGSFFL VSKLTVDKSRWQQGNVFSCSVMHEALHNHYTQKSLSLSPGK |
| 57 | P1AE1768 >CEA LC | DIQMTQSPSSLSASVGDRVTITCKASAAVGTYVAWYQQKPGKAPKLLIYSASYRKRGVPSRFSGSGSGTDFTLTISSLQPED FATYYCHQYYTYPLFTFGQGTKLEIKRTVAAPSVFIFPPSDRKLKSGTASVVCLLNNFYPREAKVQWKVDNALQSGNSQESV TEQDSKDSTYSLSSTLTLSKADYEKHKVYACEVTHQGLSSPVTKSFNRGEC |
| 58 | P1AE1768 > DOTAM Heavy Chain with VL / CH1 (hole) | IQMTQSPSSLSASVGDRVTITCQSSHSVYSDNDLAWYQQKPGKAPKLLIYQASKLASGVPSRFSGSGSGTDFTLTISSLQPE DFATYYCLGGYDDESDTYGFGGGTKVEIKSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCPPCPAPEAAGGPSVFLFPPKPKDT LMISRTPEVTCVVVDVSHEDPEVKFNWYVDGVEVHNAKTKPREEQYNSTYRVVSVLTVLHQDWLNGKEYKCKVSNKALGAPI EKTISKAKGQPREPQVYTLPPCRDELTKNQVSLWCLVKGFYPSDIAVEWESNGQPENNYKTTPPVLDSDGSFFLYSKLTVDK SRWQQGNVFSCSVMHEALHNHYTQKSLSLSPGK |
| 59 | P1AE1768 CEA Heavy chain (knob) | QVQLVQSGAEVKKPGASVKVSCKASGYTFTEFGMNWVRQAPGQGLEWMGWINTKTGEATYVEEFKGRVTFTTDTSTSTAYME LRSLRSDDTAVYYCARWDFAYYVEAMDYWGQGTTVTVSSASTKGPSVFPLAPSSKSTSGGTAALGCLVEDYFPEPVTVSWNS GALTSGVHTFPAVLQSSGLYSLSSVVTVPSSSLGTQTYICNVNHKPSNTKVDEKVEPKSCDKTHTCPPCPAPEAAGGPSVFL FPPKPKDTLMISRTPEVTCVVVDVSHEDPEVKFNWYVDGVEVHNAKTKPREEQYNSTYRVVSVLTVLHQDWLNGKEYKCKVS NKALGAPIEKTISKAKGQPREPQVCTLPPSRDELTKNQVSLSCAVKGFYPSDIAVEWESNGQPENNYKTTPPVLDSDGSFFL VSKLTVDKSRWQQGNVFSCSVMHEALHNHYTQKSLSLSPGK |
| 60 | P1AE1768 DOTAM "LC" VH / CK | VTLKESGPVLVKPTETLTLTCTVSGFSLSTYSMSWIRQPPGKALEWLGFIGSRGDTYYASWAKGRLTISKDTSKSQVVLTMT NMDPVDTATYYCARERDPYGGGAYPPHLWGRGTLVTVSSASVAAPSVFIFPPSDEQLKSGTASVVCLLNNFYPREAKVQWKV DNALQSGNSQESVTEQDSKDSTYSLSSTLTLSKADYEKHKVYACEVTHQGLSSPVTKSFNRGEC |
| 61 | P1AE1769 DOTAM "LC" with VH / CK | VTLKESGPVLVKPTETLTLTCTVSGFSLSTYSMSWIRQPPGKALEWLGFIGSRGDTYYASWAKGRLTISKDTSKSQVVLTMT NMDPVDTATYYCARERDPYGGGAYPPHLWGRGTLVTVSSASVAAPSVFIFPPSDEQLKSGTASVVCLLNNFYPREAKVQWKV DNALQSGNSQESVTEQDSKDSTYSLSSTLTLSKADYEKHKVYACEVTHQGLSSPVTKSFNRGEC |
| 62 | P1AE1769 CEA LC | DIQMTQSPSSLSASVGDRVTITCKASAAVGTYVAWYQQKPGKAPKLLIYSASYRKRGVPSRFSGSGSGTDFTLTISSLQPED FATYYCHQYYTYPLFTFGQGTKLEIKRTVAAPSVFIFPPSDRKLKSGTASVVCLLNNFYPREAKVQWKVDNALQSGNSQESV TEQDSKDSTYSLSSTLTLSKADYEKHKVYACEVTHQGLSSPVTKSFNRGEC |

(continued)

| SEQ ID NO | Description | SEQUENCE |
|---|---|---|
| 63 | P1AE1769 CEA HC with CEA VH / CH1 / | QVQLVQSGAEVKKPGASVKVSCKASGYTFTEFGMNWVRQAPGQGLEWMGWINTKTGEATYVEEFKGRVTFTTDTSTSTAYME<br>LRSLRSDDTAVYYCARWDFAYYVEAMDYWGQGTTVTVSSASTKGPSVFPLAPSSKSTSGGTAALGCLVEDYFPEPVTVSWNS |
| | DOTAM VL / CH1 (hole) | GALTSGVHTFPAVLQSSGLYSLSSVVTVPSSSLGTQTYICNVNHKPSNTKVDEKVEPKSCDGGGGSGGGGSIQMTQSPSSLS<br>ASVGDRVTITCQSSHSVYSDNDLAWYQQKPGKAPKLLIYQASKLASGVPSRFSGSGSGTDFTLTISSLQPEDFATYYCLGGY<br>DDESDTYGFGGGTKVEIKSSASTKGPSVFPLAPSSKSTSGGTAALGCLVKDYFPEPVTVSWNSGALTSGVHTFPAVLQSSGL<br>YSLSSVVTVPSSSLGTQTYICNVNHKPSNTKVDKKVEPKSCDKTHTCPPCPAPEAAGGPSVFLFPPKPKDTLMISRTPEVTC<br>VVVDVSHEDPEVKFNWYVDGVEVHNAKTKPREEQYNSTYRVVSVLTVLHQDWLNGKEYKCKVSNKALGAPIEKTISKAKGQP<br>REPQVYTLPPCRDELTKNQVSLWCLVKGFYPSDIAVEWESNGQPENNYKTTPPVLDSDGSFFLYSKLTVDKSRWQQGNVFSC<br>SVMHEALHNHYTQKSLSLSPGK |
| 64 | P1AE1769 CEA HC (knob) | QVQLVQSGAEVKKPGASVKVSCKASGYTFTEFGMNWVRQAPGQGLEWMGWINTKTGEATYVEEFKGRVTFTTDTSTSTAYME<br>LRSLRSDDTAVYYCARWDFAYYVEAMDYWGQGTTVTVSSASTKGPSVFPLAPSSKSTSGGTAALGCLVEDYFPEPVTVSWNS<br>GALTSGVHTFPAVLQSSGLYSLSSVVTVPSSSLGTQTYICNVNHKPSNTKVDEKVEPKSCDKTHTCPPCPAPEAAGGPSVFL<br>FPPKPKDTLMISRTPEVTCVVVDVSHEDPEVKFNWYVDGVEVHNAKTKPREEQYNSTYRVVSVLTVLHQDWLNGKEYKCKVS<br>NKALGAPIEKTISKAKGQPREPQVCTLPPSRDELTKNQVSLSCAVKGFYPSDIAVEWESNGQPENNYKTTPPVLDSDGSFFL<br>VSKLTVDKSRWQQGNVFSCSVMHEALHNHYTQKSLSLSPGK |
| 65 | P1AE1770 CEA LC | DIQMTQSPSSLSASVGDRVTITCKASAAVGTYVAWYQQKPGKAPKLLIYSASYRKRGVPSRFSGSGSGTDFTLTISSLQPED<br>FATYYCHQYYTYPLFTFGQGTKLEIKRTVAAPSVFIFPPSDRKLKSGTASVVCLLNNFYPREAKVQWKVDNALQSGNSQESV<br>TEQDSKDSTYSLSSTLTLSKADYEKHKVYACEVTHQGLSSPVTKSFNRGEC |
| 66 | P1AE1770 CEA HC with DOTAM scFab: DOTAM VL / Ck / Linker / VH CH1 (knob) | QVQLVQSGAEVKKPGASVKVSCKASGYTFTEFGMNWVRQAPGQGLEWMGWINTKTGEATYVEEFKGRVTFTTDTST<br>STAYMELRSLRSDDTAVYYCARWDFAYYVEAMDYWGQGTTVTVSSASTKGPSVFPLAPSSKSTSGGTAALGCLVEDYFPEPV<br>TVSWNSGALTSGVHTFPAVLQSSGLYSLSSVVTVPSSSLGTQTYICNVNHKPSNTKVDEKVEPKSCDKTHTCPPCPAPEAAG<br>GPSVFLFPPKPKDTLMISRTPEVTCVVVDVSHEDPEVKFNWYVDGVEVHNAKTKPREEQYNSTYRVVSVLTVLHQDWLNGKE<br>YKCKVSNKALGAPIEKTISKAKGQPREPQVYTLPPCRDELTKNQVSLWCLVKGFYPSDIAVEWESNGQPENNYKTTPPVLDS<br>DGSFFLYSKLTVDKSRWQQGNVFSCSVMHEALHNHYTQKSLSLSPGGGGGGSGGGGSGGGGSGGGGSIQMTQSPSSLSASVG<br>DRVTITCQSSHSVYSDNDLAWYQQKPGKAPKLLIYQASKLASGVPSRFSGSGSGTDFTLTISSLQPEDFATYYCLGGYDDES<br>DTYGFGGGTKVEIKRTVAAPSVFIFPPSDEQLKSGTASVVCLLNNFYPREAKVQWKVDNALQSGNSQESVTEQDSKDSTYSL<br>SSTLTLSKADYEKHKVYACEVTHQGLSSPVTKSFNRGECGGGGSGGGGSGGGGSGGGGSGGGGSGGGGSGGVTLKESGPVLV<br>KPTETLTLTCTVSGFSLSTYSMSWIRQPPGKALEWLGFIGSRGDTYYASWAKGRLTISKDTSKSQVVLTMTNMDPVDTATYY<br>CARERDPYGGGAYPPHLWGRGTLVTVSSASTKGPSVFPLAPSSKSTSGGTAALGCLVKDYFPEPVTVSWNSGALTSGVHTFP<br>AVLQSSGLYSLSSVVTVPSSSLGTQTYICNVNHKPSNTKVDKKVEPKSC |

(continued)

| SEQ ID NO | Description | SEQUENCE |
|---|---|---|
| 67 | P1AE1770 CEA HC (hole) | QVQLVQSGAEVKKPGASVKVSCKASGYTFTEFGMNWVRQAPGQGLEWMGWINTKTGEATYVEEFKGRVTFTTDTSTSTAYME<br>LRSLRSDDTAVYYCARWDFAYYVEAMDYWGQGTTVTVSSASTKGPSVFPLAPSSKSTSGGTAALGCLVEDYFPEPVTVSWNS<br>GALTSGVHTFPAVLQSSGLYSLSSVVTVPSSSLGTQTYICNVNHKPSNTKVDEKVEPKSCDKTHTCPPCPAPEAAGGPSVFL<br>FPPKPKDTLMISRTPEVTCVVVDVSHEDPEVKFNWYVDGVEVHNAKTKPREEQYNSTYRVVSVLTVLHQDWLNGKEYKCKVS<br>NKALGAPIEKTISKAKGQPREPQVCTLPPSRDELTKNQVSLSCAVKGFYPSDIAVEWESNGQPENNYKTTPPVLDSDGSFFL<br>VSKLTVDKSRWQQGNVFSCSVMHEALHNHYTQKSLSLSPGK |

The invention is now further described with reference to specific examples. It will be understood that various other embodiments may be practiced, given the general description provided above.

EXAMPLES

## Example 1: Description of immunization

Immunization of rabbits

A 1:1 mix of the 2 enantiomeric Pb-DOTAM-alkyl-$PEG_4$-KLH fractions (*MS2-DOTAM KLH Fraction 1* and *MS2-DOTAM KLH Fraction 2*) was used for the immunization of New Zealand White rabbits or transgenic rabbits comprising a human immunoglobulin locus as reported in WO 2000/46251, WO 2002/12437, WO 2005/007696, WO 2006/047367, US 2007/0033661, and WO 2008/027986. Each rabbit was immunized with 500 ug of the immunogen mix, emulsified with complete Freund's adjuvant, at day 0 by intradermal application and 500 ug each at days 7, 14, 28, 56 by alternating intramuscular and subcutaneous applications. Thereafter, rabbits received monthly subcutaneous immunizations of 500 ug, and small samples of blood were taken 7 days after immunization for the determination of serum titers. A larger blood sample (10% of estimated total blood volume) was taken during the third and during the ninth month of immunization (at 5-7 days after immunization), and peripheral mononuclear cells were isolated, which were used as a source of antigen-specific B cells in the B cell cloning process (Example 2).

Determination of serum titers (ELISA)

Each of the 2 enantiomeric Pb-DOTAM fractions (*PJRD05.133F1* or *PJRD05.133F2*) was immobilized on a 96-well NUNC Maxisorp plate at 1 ug/ml, 100 ul/well, in PBS, followed by: blocking of the plate with 2% Crotein C in PBS, 200 ul/well; application of serial dilutions of antisera, in duplicates, in 0.5% Crotein C in PBS, 100 ul/well; detection with HRP-conjugated donkey anti-rabbit IgG antibody (Jackson Immunoresearch/Dianova 711-036-152; 1/16 000) and streptavidin-HRP; each diluted in 0.5% Crotein C in PBS, 100 ul/well. For all steps, plates were incubated for 1 h at 37° C. Between all steps, plates were washed 3 times with 0.05% Tween 20 in PBS. Signal was developed by addition of BM Blue POD Substrate soluble (Roche), 100 ul/well; and stopped by addition of 1 M HCl, 100 ul/well. Absorbance was read out at 450 nm, against 690 nm as reference. Titer was defined as dilution of antisera resulting in half-maximal signal.

## Example 2: B-Cell Cloning from Rabbits

Isolation of rabbit peripheral blood mononuclear cells (PBMCs)

Blood samples were taken of immunized rabbits. EDTA containing whole blood was diluted twofold with 1x PBS (PAA, Pasching, Austria) before density centrifugation using lympholyte mammal (Cedarlane Laboratories, Burlington, Ontario, Canada) according to the specifications of the manufacturer. The PBMCs were washed twice with 1x PBS.

EL-4 B5 medium

RPMI 1640 (Pan Biotech, Aidenbach, Germany) supplemented with 10% FCS (Hyclone, Logan, UT, USA), 2 mM Glutamin, 1% penicillin/streptomycin solution (PAA, Pasching, Austria), 2 mM sodium pyruvate, 10 mM HEPES (PAN Biotech, Aidenbach, Germany) and 0,05 mM b-mercaptoethanole (Gibco, Paisley, Scotland) was used.

Coating of plates

Sterile cell culture 6-well plates were coated with 2 μg/ml KLH in carbonate buffer (0,1 M sodium bicarbonate, 34 mM Disodiumhydrogencarbonate, pH 9,55) overnight at 4°C. Plates were washed in sterile PBS three times before use. Sterile streptavidin coated 6-well plates (Microcoat, Bernried, Germany) were coated with a 1 + 1 enantiomer mixture of biotinylated TCMC-Pb-dPEC3-Biotin Isomer A (1 μg/ml) and B (1 μg/ml) in PBS for 3 h at room temperature. Prior to the panning step these 6-well plates were washed three times with sterile PBS.

Depletion of macrophages/monocytes

The PBMCs were seeded on sterile KLH-coated 6-well-plates to deplete macrophages and monocytes through unspecific adhesion and to remove cells binding to KLH. Each well was filled at maximum with 4 ml medium and up to 6 × 10e6 PBMCs from the immunized rabbit and were allowed to bind for 1 h at 37°C and 5% CO2. The cells in the

supernatant (peripheral blood lymphocytes (PBLs)) were used for the antigen panning step.

Enrichment of B cells on the Pb-containing TCMC enantiomer

6-well plates coated with the enantiomer mixture of TCMC-Pb-dPEC3-Biotin Isomer A and B were seeded with up to 6 × 10e6 PBLs per 4 ml medium and allowed to bind for 1 h at 37 °C and 5% CO2. Non-adherent cells were removed by carefully washing the wells 1-3 times with 1x PBS. The remaining sticky cells were detached by trypsin for 10 min at 37 °C and 5% CO2. Trypsination was stopped with EL-4 B5 medium. The cells were kept on ice until the immune fluorescence staining.

Immune fluorescence staining and Flow Cytometry

The anti-IgG FITC (AbD Serotec, Düsseldorf, Germany) was used for single cell sorting. For surface staining, cells from the depletion and enrichment step were incubated with the anti-IgG FITC antibody in PBS and incubated for 45 min in the dark at 4°C. After staining the PBMCs were washed two times with ice cold PBS. Finally the PBMCs were resuspended in ice cold PBS and immediately subjected to the FACS analyses. Propidium iodide in a concentration of 5 μg/ml (BD Pharmingen, San Diego, CA, USA) was added prior to the FACS analyses to discriminate between dead and live cells.

A Becton Dickinson FACSAria equipped with a computer and the FACSDiva software (BD Biosciences, USA) were used for single cell sort.

B-cell cultivation

The cultivation of the rabbit B cells was prepared by a method described by Lightwood et al (J Immunol Methods, 2006, 316: 133-143). Briefly, single sorted rabbit B cells were incubated in 96-well plates with 200 ul/well EL-4 B5 medium containing Pansorbin Cells (1:100000) (Calbiochem (Merck), Darmstadt, Deutschland), 5% rabbit thymocyte supernatant (MicroCoat, Bernried, Germany) and gamma-irradiated murine EL-4 B5 thymoma cells (5 × 10e5 cells/well) for 7 days at 37 °C in the incubator. The supernatants of the B-cell cultivation were removed for screening and the remaining cells were harvested immediately and were frozen at - 80 °C in 100 μl RLT buffer (Qiagen, Hilden, Germany).

**Example 3: Expression of rabbit antibody**

PCR amplification of V-domains

Total RNA was prepared from B cells lysate (resuspended in RLT buffer - Qiagen - Cat. N° 79216) using the NucleoSpin 8/96 RNA kit (Macherey&Nagel; 740709.4, 740698) according to manufacturer's protocol. RNA was eluted with 60 μl RNase free water. 6μl of RNA was used to generate cDNA by reverse transcriptase reaction using the Superscript III First-Strand Synthesis SuperMix (Invitrogen 18080-400) and an oligo dT-primer according to the manufacturers' instructions. All steps were performed on a Hamilton ML Star System. 4μl of cDNA were used to amplify the immunoglobulin heavy and light chain variable regions (VH and VL) with the AccuPrime Supermix (Invitrogen 12344-040) in a final volume of 50μl using the primers rbHC.up and rbHC.do for the heavy chain and rbLC.up and rbLC.do for the light chain (Table 3). All forward primers were specific for the signal peptide (of respectively VH and VL) whereas the reverse primers were specific for the constant regions (of respectively VH and VL). The PCR conditions for the RbVH+RbVL were as follows: Hot start at 94°C for 5 min; 35 cycles of 20s at 94°C, 20s at 70°C, 45s at 68 °C, and a final extension at 68°C for 7 min.

**Table 3**

| | |
|---|---|
| rbHC.up | AAGCTTGCCACCATGGAGACTGGGCTGCGCTGGCTTC |
| rbHCf.do | CCATTGGTGAGGGTGCCCGAG |
| rbLC.up | AAGCTTGCCACCATGGACAYGAGGGCCCCCACTC |
| rbLC.do | CAGAGTRCTGCTGAGGTTGTAGGTAC |

8μl of 50μl PCR solution were loaded on a 48 E-Gel 2% (Invitrogen G8008-02). Positive PCR reactions were cleaned using the NucleoSpin Extract II kit (Macherey&Nagel; 740609250) according to manufacturer's protocol and eluted in 50μl elution buffer. All cleaning steps were performed on a Hamilton ML Starlet System.

Recombinant expression of rabbit monoclonal bivalent antibodies

For recombinant expression of rabbit monoclonal bivalent antibodies, PCR-products coding for VH or VL were cloned as cDNA into expression vectors by the overhang cloning method (RS Haun et al., Biotechniques (1992) 13, 515-518; MZ Li et al., Nature Methods (2007) 4, 251-256). The expression vectors contained an expression cassette consisting of a 5' CMV promoter including intron A, and a 3' BGH poly adenylation sequence. In addition to the expression cassette, the plasmids contained a pUC18-derived origin of replication and a beta-lactamase gene conferring ampicillin resistance for plasmid amplification in E.coli. Three variants of the basic plasmid were used: one plasmid containing the rabbit IgG constant region designed to accept the VH regions while two additional plasmids containing rabbit or human kappa LC constant region to accept the VL regions. Linearized expression plasmids coding for the kappa or gamma constant region and VL /VH inserts were amplified by PCR using overlapping primers. Purified PCR products were incubated with T4 DNA-polymerase which generated single-strand overhangs. The reaction was stopped by dCTP addition. In the next step, plasmid and insert were combined and incubated with recA which induced site specific recombination. The recombined plasmids were transformed into E.coli. The next day the grown colonies were picked and tested for correct recombined plasmid by plasmid preparation, restriction analysis and DNA-sequencing. For antibody expression, the isolated HC and LC plasmids were transiently co-transfected into 2ml (96well plate) of Freestyle HEK293-F cells (Invitrogen R790-07) by using 239-Free Transfection Reagent (Novagen) following procedure suggested by Reagent supplier. The supernatants were harvested after 1 week and delivered for purification.

**Example 4: Selection of Rabbit Monoclonal Antibodies**

The table below shows properties of various monoclonal bivalent rabbit antibodies. PRIT-0128 was selected as the lead candidate as it has comparable binding to chelated Pb and Bi, reduced binding to other chelated metals, and high affinity (<100pM).

The SET (solution equilibration titration) assay was carried out as described below.

Preparation of assay-plate: 384-well streptavidin plates (Nunc, Microcoat #11974998001) were incubated overnight at 4°C with 25 μl/well of a DOTAM-Biotin-Isomer Mix in PBS-buffer at a concentration of 20 ng/ml.

Equilibration of anti-DOTAM antibody samples with free DOTAM-metal chelates (Pb, Bi, Ca, Cu, Zn, Mg, Fe): 0.01 nM - 1 nM of antibody were titrated with the relevant DOTAM-metal chelates in 1:3, 1:2 or 1:1.7 dilution steps starting at a concentration of 2500 nM, 500 nM or 100 nM of DOTAM-metal chelate. The samples were incubated at 4°C overnight in sealed REMP Storage polypropylene microplates (Brooks).

After overnight incubation, streptavidin plates were washed 3x with 90 μl PBST per well. 15 ul of each sample from the equilibration plate were transferred to the assay plate and incubated for 15 min at RT, followed by 3x 90 μl washing steps with PBST buffer. Detection was carried out by adding 25 μl of a goat anti-human IgG antibody-POD conjugate (Jackson, 109-036-088, 1:4000 in OSEP), followed by 6x 90 μl washing steps with PBST buffer. 25 μl of TMB substrate (Roche Diagnostics GmbH, Cat. No.: 11835033001) were added to each well. Measurement took place at 370/492 nm on a Safire2 reader (Tecan).

**MATERIALS:**

1. DOTAM-Biotin-Isomer Mix:
Mixture of the following components, conc. = 20 ng/ml

- Pb-Dotam-Bn-biotin/ TCMC-Pb-dPEG3-Biotin, isomer A
- Pb-Dotam-Bn-biotin/ TCMC-Pb-dPEG3-Biotin, isomer B
- Pb-Dotam-alkyl-biotin isomer A
- Pb-Dotam-alkyl-biotin isomer B

2. PBS: DPBS, PAN, P04-36500

3. BSA: Roche, 10735086001

4. Tween 20: Polysorbat 20 (usb, #20605, 500ml)

5. PBST: 10x, Roche, #11666789001/0,1% Tween 20

6. OSEP: PBS (10x, Roche, 4 11666789001)/0,5% BSA (Bovine Serum Albumin Fraction V, fatty acid free, Roche, # 10735086001)/0,05% Tween 20

Table 4

| Name | Species | KD [SET-Titration] | | | |
|---|---|---|---|---|---|
| | | Pb | Bi | Ca | Zn |
| PRIT-0135 | WTRa | 0.000 | 0.000 | 0.001 | 0.285 |
| PRIT-0129 | WTRa | 0.001 | 0.024 | 0.013 | 9.251 |
| PRIT-0128 | WTRa | 0.002 | 0.003 | 0.003 | 8.152 |
| PRIT-0132 | WTRa | 0.002 | 0.046 | 0.002 | 0.217 |
| PRIT-0134 | WTRa | 0.002 | 0.059 | 0.003 | 0.443 |
| PRIT-0136 | WTRa | 0.004 | 0.029 | 0.014 | 131.926 |
| PRIT-0127 | WTRa | 0.014 | 0.092 | 0.015 | 222.339 |
| PRIT-0107 | TgRa | 1.1 | 51.0 | 48.0 | >1000 |
| WTRa: Wild Type Rabbits; TgRa: transgenic rabbits | | | | | |

**Example 5: Molecular biology**

Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions.

Gene and oligonucleotide synthesis

Desired gene segments were prepared by chemical synthesis at Geneart GmbH (Regensburg, Germany). The synthesized gene fragments were cloned into an E. coli plasmid for propagation/amplification. The DNA sequences of subcloned gene fragments were verified by DNA sequencing. Alternatively, short synthetic DNA fragments were assembled by annealing chemically synthesized oligonucleotides or via PCR. The respective oligonucleotides were prepared by metabion GmbH (Planegg-Martinsried, Germany)

Protein determination

The protein concentration of purified polypeptides was determined by determining the optical density (OD) at 280 nm, using the molar extinction coefficient calculated on the basis of the amino acid sequence of the polypeptide.

Generation of plasmids for the recombinant expression of antibody heavy or light chains

Desired proteins were expressed by transient transfection of human embryonic kidney cells (HEK 293). For the expression of a desired gene/protein (e.g. full length antibody heavy chain, full length antibody light chain, or a full length antibody heavy chain containing an additional domain (e.g. an immunoglobulin heavy or light chain variable domain at its C-terminus) a transcription unit comprising the following functional elements was used:

- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence (SS),
- a gene/protein to be expressed, and
- the bovine growth hormone polyadenylation sequence (BGH pA).

In addition to the expression unit/cassette including the desired gene to be expressed the basic/standard

mammalian expression plasmid contained an origin of replication from the vector pUC18 which allows replication of this plasmid in E. coli, and a beta-lactamase gene which confers ampicillin resistance in E. coli.

a) Expression plasmid for antibody heavy chains

Antibody heavy chain encoding genes including C-terminal fusion genes comprising a complete and functional antibody heavy chain, followed by an additional antibody V-heavy or V-light domain was assembled by fusing a DNA fragment coding for the respective sequence elements (V-heavy or V-light) separated each by a G4Sx4 linker to the C-terminus of the CH3 domain of a human IgG molecule (VH-CH1-hinge-CH2-CH3-linker-VH or VH-CH1-hinge-CH2-CH3-linker-VL). Recombinant antibody molecules bearing one VH and one VL domain at the C-termini of the two CH3 domains, respectively, were expressed using the knob-into-hole technology.

The expression plasmids for the transient expression of an antibody heavy chain with a C-terminal VH or VL domain in HEK293 cells comprised besides the antibody heavy chain fragment with C-terminal VH or VL domain expression cassette, an origin of replication from the vector pUC18, which allows replication of this plasmid in E. coli, and a beta-lactamase gene which confers ampicillin resistance in E. coli. The transcription unit of the antibody heavy chain fragment with C-terminal VH or VL domain fusion gene comprises the following functional elements:

- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- an antibody heavy chain (VH-CH1-hinge-CH2-CH3-linker-VH or VH-CH1-hinge-CH2-CH3-linker-VL) encoding nucleic acid, and
- the bovine growth hormone polyadenylation sequence (BGH pA).

Expression plasmids coding for all heavy chain polypeptides/proteins mentioned in Table 2 were constructed according to the methods as outlined before.

b) Expression plasmid for antibody light chains

Antibody light chain encoding genes comprising a complete and functional antibody light chain was assembled by fusing a DNA fragment coding for the respective sequence elements.

The expression plasmid for the transient expression of an antibody light chain comprised besides the antibody light chain fragment an origin of replication from the vector pUC18, which allows replication of this plasmid in E. coli, and a beta-lactamase gene which confers ampicillin resistance in E. coli. The transcription unit of the antibody light chain fragment comprises the following functional elements:

- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- an antibody light chain (VL-CL) encoding nucleic acid, and
- the bovine growth hormone polyadenylation sequence (BGH pA).

Expression plasmids coding for all light chain polypeptides/proteins mentioned in table 2 were constructed according to the methods as outlined before

A schematic representation of the format used is depicted in Figure 1. The star refers to PGLALA substitutions: 1 refers to the DOTAM binder and 2 and 3 refer to the anti-target (here CEA) binder.

**Example 6: Transient expression of PRIT Molecules**

Transient expression of the antibody molecules

The antibody molecules were generated in transiently transfected HEK293 cells (human embryonic kidney cell line 293-derived) cultivated in F17 Medium (Invitrogen Corp.). For transfection "293-Free" Transfection Reagent (Novagen) was used. The respective antibody heavy- and light chain molecules as described above were expressed from individual expression plasmids. Transfections were performed as specified in the manufacturer's instructions. Immunoglobulin-containing cell culture supernatants were harvested three to seven (3-7) days after transfection. Supernatants were stored at reduced temperature (e.g. -80°C) until purification.

General information regarding the recombinant expression of human immunoglobulins in e.g. HEK293 cells is

given in: Meissner, P. et al., Biotechnol. Bioeng. 75 (2001) 197-203.

**Example 7: Purification of Proteins**

The harvested cell culture supernatant was applied on a column (1.1cm diameter 5 cm length) filled with 5 ml of Protein A resin (Mab Select Sure) at a flow rate of 5 ml/min. After washing with 20 ml PBS buffer the antibody was eluted with 25 ml Na citrate pH 3.0.

The eluate was then adjusted to pH 5.0 with 1 M Tris pH 9.0 and incubated at 4°C overnight.

After centrifugation 10 min at 10000 × g and filtration over a 0.2μm filter the filtrate was then applied on a size exclusion chromatography on a Superdex 200 column (2.6 cm diameter 60 cm length) pre-equilibrated in a buffer containing 20mM Histidin, 140mM NaCl at pH 6.0 and eluted with the same buffer.

The main elution peak containing the purified antibody was collected and the final purity was analyzed.

Material- Table 5

| | Vendor | stock conc. [mg/mL] | Ident# | Lot# |
|---|---|---|---|---|
| RS-CEA_Il2v | in-house | 10.5 | R06895882 | GMP-batch 1 |
| Chrome Pure Human IgG | Jackson Immuno Research | 12.0 | 009-000-003 | 116598 |
| Pb-DOTAM-FITC | Macrocyclics | 1.0 | FRRD04-37 | |
| goat <hu IgG(H+L) > -AlexaFluor 488 | Invitrogen | 2.0 | A11013 | 1173476 |
| PBS w/o Mg and Ca | PAN Biotech | | P04-36500 | 4780114 |
| FKS Gibco | Gibco | | 10500-064 | 07Q1416K |
| 5ml RoundbottomTube (FACS Tube) | BD Falcon | | 352054 | 1094065 |
| 96-Well PP Plate V-Bottom | Costar | | 3357 | 10113004 |

**Example 8: FACS**

MKN-45 cells were detached from the culture bottle using Trypsin and were counted using a Casy cell counter. After pelleting at 4°C, 300g the cells were resuspended in FACS Buffer (2.5% FCS in PBS), adjusted to 2.0E+06 cells /mL dispensed to 96-well PP V-bottom-Platte (25 μL/well = 5.0E+04Zellen/well).

- FACS staining using DOTAM-FITC

The primary CEA specific antibodies were adjusted to 40 ug/mL in FACS buffer, resulting in a final concentration of 10 μg/mL. RS-CEA-Il2v was used as a reference. Pb-DOTAM labeled with FITC and the primary antibodies were used in equimolar ratios. They were mixed and incubated for 10 min at RT to allow binding of the antibodies to Pb-DOTAM. Subsequently, 20μl of the prepared mix were added to 25μl cell suspension and incubated for 1 h at 4°C. The cells were then washed twice in FACS buffer and resuspended in 70 μl/well FACS buffer for measurement using a FACS Canto (BD, Pharmingen).

- FACS staining using <hu kappa>

The primary CEA specific antibodies were adjusted to 20 μg/mL in FACS buffer, resulting in a final concentration of 10 μg/mL. RS-CEA-Il2v was used as a reference. 20μl were added to 25μl cell suspension and incubated for 1 h at 4°C. The cells were then washed twice in FACS buffer. After washing, the cells were resuspended in 50 μL FACS-buffer containing secondary antibody (<huIgG>-Alexa488, c=10 pg/mL) and incubated 1h at 4°C. The cells were then washed twice in FACS buffer and resuspended in 70 μl/well FACS buffer for measurement using a FACS Canto (BD, Pharmingen).

Depicted in figure 36 is an example that shows binding of one antibody (PRIT-0165) to MKN-45 cells, detecting it either using secondary detection (right panel, Alexa 488) or DOTAM FITC (left panel, FITC-A).

**Example 9: Humanization:**

For the identification of a suitable human acceptor framework during the humanization of the DOTAM binder PRIT-0128 a combination of two methodologies was used. On the one hand a classical approach was taken by searching for an acceptor framework with high sequence homology to the parental antibody and subsequent grafting of the CDR regions onto this acceptor framework. Each amino acid difference of the identified frameworks to the parental antibody was judged for impact on the structural integrity of the binder and backmutations towards the parental sequence were introduced whenever appropriate.

On the other hand, an in house developed in silico tool was used to predict the orientation of the VH and VL domains of the humanized versions towards each other (see WO2016/062734). This was carried out for the virtual grafts of the CDRs on all possible human germline combinations. The results were compared to the VH-VL domain orientation of the parental binder to select for framework combinations which are close in geometry to the starting antibody.

In each case the following CDR regions of the parental antibody were grafted onto the acceptor framework (numbering according to Kabat):

VH_CDR1: 31-35
VH_JCDR2: 50-65
VH_CDR3: 95-102
VL_CDR1: 24-34
VL_CDR2: 50-56
VL_CDR3: 89-97

The humanization variants were produced in the final format with the DOTAM binder fused to the C-terminus of the Fc of the tumor targeting IgG as an VH/VL Fv fusion (without CH1 and Ck respectively). The parental (non-humanized) DOTAM binder PRIT-0128 derived molecule in the final format is called PRIT-0156.

Herceptin framework was included as well due to the suitability in terms of VH/VL prediction and the increased stability of the framework. For all VH humanized variants, the human J element hJH2 was used. For all VK humanized variants, the human J element hJK4 was used.

The HC4 is a grafting of PRIT-128 on the human germline IGHV3-30-02 with one backmutation kabat A49G

To get the variable heavy chain HC5, the CDRs were grafted on human germline hVH_2_26 with A49G as a backmuation and the deletion of the first amino acid to reflect the original rabbit N-terminus.

Grafted on Herceptin V-region (derived from human germline hVH3_66), the variant HC7 is characterized by a few modifications in the acceptor framework: deletion of N-terminus E, A49G, A71R, and S93A.

For HC10, the CDRs of PRIT-128 were grafted on the human germline IGHV4_34_01.

Here, the N-terminus was modified, starting with V2, to reflect the original rabbit antibody starting with Q2. In addition, G29F and F31L were considered as backmutation wrt Kabat nomenclature as well as V71R and F78V in framework 3.

For the light chain LC1, the CDRs were grafted on the human germline IGKV1_39_01 without any backmutation. The start was chosen as 12 to reflect the original rabbit Ab starting with A2.

The light chain variant LC3 was obtained by grafting of the CDRs on human germline hVK1_5. D1 was deleted and a I2A backmutation was considered as a new N-terminus. As additional backmutations, K42Q and A43P were took into account.

Not all possible combinations of the humanization matrix were produced but a selection of defined combinations was chosen based on considerations like VH/VL prediction and sequence risks of the given combination.

The goal of the humanization was to obtain humanized binders which do not lose more than a factor of 10 in terms of affinity to DOTAM and display increased stability if possible. This was achieved with several binders of comparable or even better affinity to DOTAM as well as an increase of thermal stability as measured by DLS of about 10-15°C. See tables 7 and 8, below.

**Example 10: Solution equilibrium based kd determination**

To screen a larger amount of humanization candidates for their affinity to Pb-DOTAM, solution equilibrium titration (SET) was used.

Table 6 details the SET based affinity determination of selected humanized DOTAM binders against Pb-DOTAM. All antibodies in Table 6, are bispecific antibodies that comprise bivalent binding to CEA and monovalent binding to Pb-Dotam (2:1 format, see Figure 1):

Table 6

| Molecule name | Humanized HC / LC combination | SET Pb-DOTAM (pM) |
|---|---|---|
| PRIT-0187-0002 | HC10 LC1 | 0,03 |
| PRIT-0193-0002 | HC5 LC3 | 0,36 |
| PRIT-0195-0004 | HC10 LC3 | 0,40 |
| PRIT-0156-0004 | Parental molecule | 0,43 |
| PRIT-0182-0002 | HC8 LC7 | 5,54 |
| PRIT-0189-0002 | HC7 LC2 | 5,81 |
| PRIT-0185-0002 | HC2 LC1 | 5,87 |
| PRIT-0192-0002 | HC2 LC3 | 8,04 |
| PRIT-0183-0004 | HC2 LC2 | 8,11 |
| PRIT-0197-0002 | HC7 LC1 | 8,69 |
| PRIT-0198-0002 | HC7 LC3 | 9,09 |
| PRIT-0182-0004 | HC8 LC7 | 17,14 |
| PRIT-0183-0002 | HC2 LC2 | 22,45 |
| PRIT-0180-0004 | HC9 LC6 | 26,10 |
| PRIT-0190-0002 | HC9 LC2 | 33,83 |
| PRIT-0194-0002 | HC8 LC3 | 34,63 |
| PRIT-0199-0002 | HC9 LC1 | 43,91 |
| PRIT-0180-0002 | HC9 LC6 | 47,70 |
| PRIT-0188-0002 | HC4 LC2 | 54,34 |
| PRIT-0178-0004 | HC1 LC6 | 60,06 |
| PRIT-0179-0004 | HC4 LC6 | 64,92 |
| PRIT-0181-0002 | HC4 LC7 | 65,11 |
| PRIT-0179-0002 | HC4 LC6 | 65,81 |
| PRIT-0178-0002 | HC1 LC6 | 66,68 |
| PRIT-0187-0004 | HC10 LC1 | 78,09 |
| PRIT-0184-0002 | HC1 LC1 | 80,56 |
| PRIT-0200-0002 | HC9 LC3 | 83,24 |
| PRIT-0191-0002 | HC1 LC3 | 111,10 |

**Example 11: Kinexa based kd determination**

For more detailed analysis and an orthogonal method for affinity determination, Kinexa was used.

Instrumentation and materials

A KinExA 3200 instrument from Sapidyne Instruments (Boise, ID) with autosampler was used. Polymethylmethacrylate (PMMA) beads were purchased from Sapidyne, whereas PBS (phosphate buffered saline), BSA (bovine serum albumin fraction V) and the anti-DOTAM antibodies were prepared in-house (Roche). Dylight650®-conjugated affinity-purified goat anti-human IgG-Fc Fragment cross-adsorbed antibody was purchased from Bethyl Laboratories (Montgomery, TX). The biotinylated Pb-DOTAM antigens (Pb-DOTAM-alkyl-biotin isomer A and B, Pb-DOTAM-Bn-biotin / TCMC-Pb-dPEG3-Biotin, isomer A and B) and the non-biotinylated Pb-DOTAM were obtained from AREVA Med

(Bethesda, MD) .

Preparation of antigen coated beads

PMMA beads were coated according to the KinExA Handbook protocol for biotinylated molecules (Sapidyne). Briefly, first, 10 ug of Biotin-BSA (Thermo Scientific) in 1 ml PBS (pH7.4) was added per vial (200mg) of beads for adsorption coating. After rotating for 2 h at room temperature, the supernatant was removed and beads were washed 5 times with 1 ml PBS. Second, 1 ml of 100 μg of NeutrAvidin Biotin-Binding Protein (Thermo Scientific) in PBS containing 10 mg/ml BSA was added to the beads and incubated at room temperature for additional 2 h to couple NeutrAvidin to the beads and to provide additional biotin binding sites for subsequent binding of biotinylated proteins. The NeutrAvidin-coated-beads were then rinsed 5 times with 1 ml PBS. Finally, the beads were coated with 200 ng/ml biotinylated Pb-DOTAM-Isomer Mix (50 ng for each Isomer) in PBS and incubated for further 2 h at room temperature. Beads were then resuspended in 30 ml PBS and used immediately.

KinExA equilibrium assays

All KinExA experiments were performed at room temperature (RT) using PBS pH 7.4 as running buffer. Samples were prepared in running buffer supplemented with 1 mg/ml BSA ("sample buffer"). A flow rate of 0.25 ml/min was used. A constant amount of anti-DOTAM antibody with 5 pM binding site concentration was titrated with Pb-DOTAM antigen by twofold serial dilution starting at 100 pM (concentration range 0.049 pM - 100 pM). One sample of antibody without antigen served as 100% signal (i.e. without inhibition). Antigen-antibody complexes were incubated at RT for at least 24 h to allow equilibrium to be reached. Equilibrated mixtures were then drawn through a column of Pb-DOTAM-coupled beads in the KinExA system at a volume of 5 ml permitting unbound antibody to be captured by the beads without perturbing the equilibrium state of the solution. Captured antibody was detected using 250 ng/ml Dylight 650©-conjugated anti-human Fc-fragment specific secondary antibody in sample buffer. Each sample was measured in duplicates for all equilibrium experiments.

The KD was obtained from non-linear regression analysis of the data using a one-site homogeneous binding model contained within the KinExA software (Version 4.0.11) using the "standard analysis" method. The software calculates the KD and determines the 95% confidence interval by fitting the data points to a theoretical KD curve. The 95% confidence interval (Sapidyne TechNote TN207R0) is given as KD low and KD high.

Other examples for affinity values as determined by Kinexa are provided below, for PRIT-213. PRIT-0213 is the same molecule as PRIT-0186, except for another CEA binding VH /VL, see Table 8.

PRIT-213

Metal-DOTAM Chelate Affinities of CEA-DOTAM BsAb

| Antigen | KD [pM] | 95% CI[pM] |
|---|---|---|
| Pb-DOTAM | 0.84 | 0.44-1.4 |
| Ca-DOTAM | 0.95 | 0.43-1.7 |
| Bi-DOTAM | 5.7 | 4.6-6.2 |
| Cu-DOTAM | 122000 | 60000 - 206000 |

Additional values are as shown below:

| TheraPS Name | Antigen | KD [pM] | 95% confidence interval [pM] |
|---|---|---|---|
| PRIT-0213-0005 | Ca - DOTAM | 0.95 | 0.43 - 1.7* |
| PRIT-0214-0005 | | 0.52 | 0.34 - 0.74 |
| PRIT-0213-0005 | Bi - DOTAM | 5.7 | 4.6 - 6.2* |
| PRIT-0214-0005 | | 6.0 | 5.5 - 6.4 |

(continued)

| TheraPS Name | Antigen | KD [pM] | 95% confidence interval [pM] |
|---|---|---|---|
| PRIT-0213-0005 | Cu - DOTAM | 122000 | 60000 - 206000*° |
| PRIT-0214-0005 | | 38000 | 19000 - 63000*° |
| *broad confidence interval, indicating the measured $K_D$ not as precise °assay not completely optimized for nM - affinity | | | |

**Example 12: Thermostability measurements for humanized PRIT molecules**

Method and data analysis

Different variants of the humanized PRIT molecules in the final format (in 20 mM Histidine, 140 mM NaCl, pH 6.0) were diluted in the same buffer to 1 mg/ ml. 30 μl of each sample was transferred into a 384-well plate filter device (alongside an anti-HER3 antibody as reference). After centrifugation at 1,000 g for 1 min wells were overlaid with 10 μl of paraffin oil. The plate was centrifuged again (1,000 g for 1 min) and transferred into the DLS pklate reader (Dyna Pro PlateReader-II, Wyatt). Starting at 25 °C the temperature was increased at a speed of 0.05 °C/ min to ’79.9 °C. Scattered light was recorded using the Dynamics Software (V7.0).

Data was transferred to Excel (Microsoft), sorted by sample und temperature and a software add-in used to create melting curves. The temperature, where clear deviation from the baseline occurred, was defined as "onset of aggregation" and the inflection point of the melting curve as "melting temperature".

Results - Table 7

| Sample | onset of aggregation (in °C) | melting temperature (in °C) |
|---|---|---|
| Her 3 | 63 ±1 | 67.5 ±1 |
| PRIT-0156 (parental molecule with rabbit DOTAM binder) | 45 ±1 | 51 ±1 |
| PRIT 205 | 51 ±1 | 51.5 ±1 |
| PRIT 206 | 51 ±1 | 58.1 ±1 |
| PRIT 207 | 49 ±1 | 58.1 ±1 |
| PRIT 208 | 54 ±1 | 57.7 ±1 |
| PRIT 209 | 54 ±1 | 57.7 ±1 |
| PRIT-0213 | 54 ±1 | 57.7 ±1 |

**Example 13: Selection of candidates**

PRIT-0156 is a 2:1 antibody comprising the rabbit DOTAM binder PRIT-0128 combined with the CEA binder CH1A1A. PRIT-0178 to PRIT-0204 are humanized variants in the same format with the same CEA binder. PRIT-0205 up to PRIT-0221 correspond to the PRIT-0178 to PRIT-0204 humanization variants in the DOTAM binding part, but have the CEA binder changed to T84.66.

The table below compares the properties of various PRIT molecules. Preferred compounds were PRIT-213 and PRIT-214.

(a)Table 8: Selection of Candidates

| Antibody | CEA binder | Dotam binder | Format |
|---|---|---|---|
| PRIT-0218 | none | WT | Rabbit antibody |
| PRIT-0156 | CH1A1A | PRIT-0218 | 2:1 antibody with PRIT 0128 dotam binder |
| PRIT-0186 | CH1A1A | HC5/LC1 (humanized PRIT-0218) | 2:1 format |
| PRIT-0213 | T84.86 | HC5/LC1 (humanized PRIT-0218) | 2:1 format |
| PRIT-0187 | CH1A1A | HC10/LC1 (humanized PRIT-0218) | 2:1 format |
| PRIT-0214 | T84.86 | HC10/LC1 (humanized PRIT-0218) | 2:1 format |
| PRIT-0206 | T84.86 | HC5/LC3 (humanized PRIT-0218) | 2:1 format |
| PRIT-0216 | T84.86 | HC5/LC3 (humanized PRIT-0218) | 2:1 format |
| PRIT-0217 | T84.86 | HC5/LC3 (humanized PRIT-0218) | 2:1 format |
| PRIT-0208 | T84.86 | HC7/LC1 (humanized PRIT-0218) | 2:1 format |

| | **CEA BiCEAnder** | **DOTAM Binder** | **SET PbDOTAM [pM]** | **Kinexa PbDOTAM [pM]** | **Yield [mg/l]** | **T Agg 266nm** | **Humanness** | | **Biodistribution** |
|---|---|---|---|---|---|---|---|---|---|
| **PRIT-0156** | CH1A1A | WT Rabbit | 0,01 | 0,25 | 25 | 45 | --- | --- | Ok |
| **PRIT-0186** | CH1A1A | HC5 / LC1 | 0,4 | 0,92 | 38 | 50 | HC 58% | LC 57% | Ok |
| **PRIT-0213** | T84.66 | HC5/LC1 | 0,4 | 0,84 | 4,0 | 54 | HC 58% | LC 57% | Ok |

(continued)

| | CEA BiCEAnder | DOTAM Binder | SET PbDOTAM [pM] | Kinexa PbDOTAM [pM] | Yield [mg/l] | T Agg 266nm | Humanness | | Biodistribution |
|---|---|---|---|---|---|---|---|---|---|
| **PRIT-0187** | CH1A1A | HC10/ LC1 | 0,03 | 0,99 | 17,8 | 57 | HC 40% | LC 57% | Ok |
| **PRIT-0214** | T84.66 | HC10/LC1 | 0,03 | 0,84 | 6,0 | 55 | HC 40% | LC 57% | Ok |
| **PRIT-0206** | T84.66 | HC5/LC3 | 0,36 | 1,0 | 1,32 | 50 | HC 58% | LC 55% | Ok |
| **PRIT-0216** | T84.66 | HC5/LC3 | - | 51 | 7,0 | 50 | HC 58% | LC 55% | Ok |
| **PRIT-0217** | T84.66 | HC5/LC3 | - | 3 | 10,0 | 54 | HC 58% | LC 55% | Ok |
| **PRIT-0208** | T84.66 | HC7/LC1 | 8,7 | 8,3 | 7,6 | 59 | HC 51% | LC 57% | Ok |

Sequences

HC5:
VTLKESGPVLVKPTETLTLTCTVSGFSLSTYSMSWIRQPPGKALEWLG**F**IGSRG**D**T**Y**YASWAKGRLTISKDTSKSQVVLTMTNMDPVDTATYYCAR**ERDPY**GGG**AY**PPHLWGRGTL VTVSS

LC1:
SIQMTQSPSSLSASVGDRVTITCQSSHSV**Y**SDN**D**LAWYQQKPGKAPKLLIYQASKLASGVPSRFSGSGSGTDFTL TISSLQPLDFATYYCLG**GYD**DFSD**TY**GFGGGTKVEIK

LC3: aqmtqspstl sasvgdrvti tcqsshsvys

501 dndlawyqqk pgqppklliy qasklasgvp srfsgsgsgt eftltisslq

551 pddfatyycl ggyddesdty gfgggtkvei k

HC7: vqlvesgggl vqpggslrls caasgfslst

501 ysmswvrqap gkglewvgfi gsrgdtyyas wakgrftisr dtskntaylq

551 mnslraedta vyycarerdp ygggaypphl wgrgtlvtvs s

HC10: vqlqqwgagl lkpsetlslt cavygfslst

501 ysmswirqpp gkglewigfi gsrgdtyyas wakgrvtisr dtsknqvslk

551 lssvtaadta vyycarerdp ygggaypphl wgrgtlvtvs s

T84.66 VH 1 qvqlvqsgae vkkpgssvkv sckasgfnik dtymhwvrqa pgqglewmgr
51 idpangnsky vpkfqgrvti tadtststay melsslrsed tavyycapfg
101 yyvsdyamay wgqgtlvtvs s

T84.66 VL:

EIVLTQSPATLSLSPGERATLSCRAGESVDIFGVGFLHWYQQKPGQAPRLLIYRASNRATGIPA RFSGSGSGTDFTLTISSLEPEDFAVYYCQQTNEDPYTFGQGTKLEIK

CH1A1A VH:

qvqlvqsgae vkkpgasvkv sckasgytft efgmnwvrqa pgqglewmgw

51 intktgeaty veefkgrvtf ttdtststay melrslrsdd tavyycarwd

101 fayyveamdy wgqgttvtvs s

CH1A1A VL:

1 diqmtqspss lsasvgdrvt itckasaavg tyvawyqqkp gkapklliys

51 asyrkrgvps rfsgsgsgtd ftltisslqp edfatyychq yytyplftfg

101 qgtkleik

## Example 14: Crystallization, data collection and structure determination of the Fab P1AA1227 Pb-DOTAM complex

For complex formation the Fab derived from the humanized VH / VL in PRIT-0213, called PIAA1227 at 26mg/ml was mixed with Pb-DOTAM powder in a molar ratio of 1:4.2. After 2 hour incubation at 4°C initial crystallization trials were performed in sitting drop vapor diffusion setups at 21 °C using the JCSG+ screen (Qiagen, Hilden). Crystals appeared within 5 days out of 0.2 M (NH4)2SO4, 0.1 M BIS-TRIS pH 5.5, 25 %w/v PEG3350. Crystals were harvested directly from the screening plate without any further optimization step.

*Data collection and structure determination.* For data collection crystals were flash frozen at 100K in precipitant solution containing 10% ethylenglycol. Diffraction data were collected at a wavelength of 1.0000 Å using a PILATUS 6M detector at the beamline X10SA of the Swiss Light Source (Villigen, Switzerland). Data have been processed with XDS (Kabsch, W. Acta Cryst. D66, 133-144 (2010)) and scaled with SADABS (BRUKER). The crystals of the complex belong to space group C2 with cell axes of a= 135.63 Å, b= 56.42 Å, c= 64.52 Å and β=108.36° and diffract to a resolution of 1.40 Å. The structure was determined by molecular replacement with PHASER (McCoy, A.J, Grosse-Kunstleve, R.W., Adams, P.D., Storoni, L.C., and Read, R.J. J. Appl. Cryst. 40, 658-674 (2007)) using the coordinates of an in house Fab structure as search model. Difference electron density was used to place the Pb-DOTAM and to change amino acids according to the sequence differences by real space refinement. Structures were refined with programs from the CCP4 suite (Collaborative Computational Project, Number 4 Acta Cryst. D50, 760-763 (1994).) and BUSTER (Bricogne, G., Blanc, E., Brandl, M., Flensburg, C., Keller, P., Paciorek, W., Roversi, P., Sharff, A., Smart, O.S., Vonrhein, C., Womack, T.O . (2011). Buster version 2.9.5 Cambridge, United Kingdom : Global Phasing Ltd.). Manual rebuilding was done with COOT (Emsley, P., Lohkamp, B., Scott, W.G. and Cowtan, K. Acta Cryst D66, 486-501 (2010)).

Data collection and refinement statistics are summarized in Table 9. All graphical presentations were prepared with PYMOL (The Pymol Molecular Graphics System, Version 1.7.4. Schrödinger, LLC.).

**Table 9- Data collection and refinement statistics for Fab P1AA1227-Pb-DOTAM complex**

| | | **P1AA1227-Pb-**DOTAM |
|---|---|---|
| **Data collection** | | |
| Space group | | C2 |
| Cell dimensions | | |
| | *a, b, c* (Å) | 135.63, 56.42, 64.52 |
| α, β, γ(°) | | 90, 108.36, 90 |
| Resolution (Å) | | 1.40 |
| $R_{sym}$ or $R_{merge}$ | | 0.041 |
| $I / \delta I$ | | 10.3 (0.94) |
| Completeness (%) | | 94.4 (86.1) |
| Redundancy | | 3.37 (3.33) |
| **Refinement** | | |
| Resolution (Å) | | 48.9 - 1.40 |
| No. reflections | | 81631 |
| $R_{work}$ / $R_{free}$ | | 19 . 21/22 . 38 |
| No. atoms | | |
| | Protein | 3342 |
| | Water | 523 |
| Pb-Dotam | | 29 |
| *B*-factors | | |
| | Protein | 14.81 |
| | Water | 37.46 |
| Pb-Dotam | | 21.09 |
| R.m.s. deviations | | |
| | Bond lengths (Å) | 0.011 |
| | Bond angles (°) | 1.57 |

*Values in parentheses are for highest-resolution shell.

**Structure of Fab P1AA1227 in complex with Pb-Dotam**

In order to characterize the interaction details of the Pb-Dotam with Fab **P1AA1227** we determined the crystal structure of the complex at a resolution of 1.40 Å. The structure reveals Fab **P1AA1227** to bind to Pb-DOTAM by main contributions of the CDR1 and CDR3 of the light chain and by CDR2 and CDR3 of the heavy chain.

Analysis of the binding interface with the program PISA reveals an interaction pattern of Fab **P1AA1227** with the Pb-DOTAM via 3 hydrogen bonds, polar interactions and van der Waals contacts. The Pb-DOTAM is bound in a pocket formed by heavy and light chain. This pocket has the shape of a box which is open on one side. Side walls and bottom of the pocket contribute apolar interactions whereas at the rim of the walls polar interactions dominate. Side chain hydrogen bonds are formed between CDR3 residues of heavy chain Glu95 and Asp97 with DOTAM carbamoyl nitrogen atoms N7 and N8. An additional hydrogen bond is established via the main chain carbonyl atom of Arg96 with atom N7 of DOTAM. The complex is further stabilized through apolar interactions of heavy chain CDR2 Phe50 and Tyr58 side chains which are oriented edge to face to the azacyclododecane ring. The light chain contributes mostly the "bottom" of the pocket with CDR3 residues Gly91-Tyr96 providing apolar contacts to the tetracyclododecane ring. Asp32 entertains a hydrogen bond to the carbamoyl nitrogen atom N6 of DOTAM. (Numbering according to Kabat).

Figure 2 shows the structure of P1AA1227 in complex with Pb-DOTAM.

Figure 3 shows the view on the interaction site.

The table below shows the heavy chain paratope residues, based on analysis with the program PISA.

Table 10

| **Heavy Chain Residues (Kabat number)** | **Type of interaction** | **Pb-DOTAM** |
|---|---|---|
| Phe50 | apolar | Edge to face to azacyclododecane ring |
| Asp56 | polar | N8 |
| Tyr58 | apolar | Edge to face to azacyclododecane ring |
| Glu95 | H-bond | N7 |
| Arg96 | H-bond (mc carbonyl) | N7 |
| Asp97 | H-bond | N8 |
| Pro98 | Edge-face | Above Pb-Dotam, 4Å distance |
| Tyr99 | polar (but distance 6A) | N5 |
| Alal00C | apolar | C12 |
| Tyr100D | polar (mc atoms) | N7 |

The Table below shows the light chain paratope residues, based on analysis with the program PISA.

Table 11

| **Light chain residues (Kabat number)** | **Type of interaction** | **Pb-DOTAM** |
|---|---|---|
| Tyr28 | apolar | Edge to face to azacyclododecane ring |
| Asp32 | polar, H-bond | N6 |
| Gly91 | apolar | below azacyclododecane ring |
| Tyr92 | apolar | below azacyclododecane ring |
| Asp 9 3 | apolar | below azacyclododecane ring |
| Thr95C | apolar | below azacyclododecane ring |
| Tyr96 | apolar | Edge to face to azacyclododecane ring |

Paratope residues are also underlined in the sequences below:

> **P1AA1227_**HC

VTLKESGPVLVKPTETLTLTCTVSGFSLSTYSMSWIRQPPGKALEWLG**F**IGSRG**D**T**Y**YASWAKGRLTISKDTSKS QVVLTMTNMDPVDTATYYCAR**ERDPY**GGG**AY**PPHLWGRGTLVTVSSASTKGPSVFPLAPSSKSTSGGTAALGCLV KDYFPEPVTVSWNSGALTSGVHTFPAVLQSSGLYSLSSVVTVPSSSLGTQTYICNVNHKPSNTKVDKKVEPKSC

> **P1AA1227_**LC

SIQMTQSPSSLSASVGDRVTITCQSSHSV**Y**SDN**D**LAWYQQKPGKAPKLLIYQASKLASGVPSRFSGSGSGTDFTL TISSLQPEDFATYYCLG**GYD**DESD**TY**GFGGGTKVEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC

**Example 15: In vivo biodistribution and efficacy: material and methods**

Glossary for the following examples

| | |
|---|---|
| BD | Biodistribution |
| bsAb | Bispecific antibody |
| CA | Clearing agent |
| CEA | Carcinoembryonic antigen |
| Dex500 | Dextran500-TCMC-Pb |
| Dex[n] | Dextran[n]-TCMC-Pb |
| DOTAM | 1,4,7,10-tetrakis(carbamoylmethyl)-1,4,7,10-tetraazacyclododecane |
| H&E | Hematoxylin and eosin |
| ID | Injected dose |
| ELISA | Enzyme-linked immunosorbent assay |
| i.p. | Intraperitoneal |
| i.v. | Intravenous |
| MIRD | Medical Internal Radiation Dose |
| MW | Molecular weight |
| NBF | Neutral buffered formalin |
| OCT | Optimum cutting temperature |
| OS | Overall survival |
| PBS | Phosphate-buffered saline |
| p.i. | Post injection |
| PK | Pharmacokinetic |
| PRIT | Pretargeted radioimmunotherapy |
| RBE | Relative biological effectiveness |
| RT | Room temperature |
| PSCA | Prostate stem cell antigen |
| s. c. | Subcutaneous |
| SCID | Severe combined immunodeficiency |
| SD | Standard deviation |

(continued)

| | |
|---|---|
| SOPF | Specific and opportunistic pathogen-free |
| SPF | Specific pathogen-free |
| TA | Target antigen |
| TCMC | 1,4,7,10-tetrakis(carbamoylmethyl)-1,4,7,10-tetraazacyclododecane |
| TGI | Tumor growth inhibition |
| TR | Tumor regression |

Material and Methods of protocols

General

All experimental protocols were reviewed and approved by the local authorities (Comité Régional d'Ethique de l'Expérimentation Animate du Limousin (CREEAL), Laboratoire Départemental d'Analyses et de Recherches de la Haute-Vienne). Female severe combined immunodeficiency (SCID) mice (Charles River) were maintained under specific-pathogen-free conditions with daily cycles of light and darkness (12 h/12 h), in line with ethical guidelines. No manipulations were performed during the first week after arrival, to allow the animals to acclimatize to the new environment. All mice were monitored daily for assessment of physical condition and general well-being.

Tumor volumes were estimated through calipering, calculated according to the formula: $volume = 0.5 \times length \times width^2$. Blood was collected at termination from the venous sinus using retro-orbital bleeding, followed by additional tissue harvest for radioactive measurements and/or histological analysis, as mandated by the protocols. Unexpected or abnormal conditions were documented.

Statistical analysis was performed using GraphPad Prism 6 (GraphPad Software, Inc.) and JMP 8 (SAS Institute Inc.).

Reagents

Bispecific antibodies were provided by Roche Diagnostics GmbH, Pharma Research Penzberg (Penzberg, Germany) and stored at -80°C until the day of injection. They were then thawed and diluted in standard vehicle buffer (20 mM Histidine/Histidine HCl, 140 mM NaCl; pH 6.0).

Bispecific antibodies

| Compound | Target | Protocols | Supplier |
|---|---|---|---|
| PRIT-0155 | PSCA | 83, 87 | Roche Pharma Research PZ |
| PRIT-0156 | CH1A1A | 80, 91 | Roche Pharma Research PZ |
| PRIT-0165 | T84.66 | 80, 85, 90, 91, 95 | Roche Pharma Research PZ |
| PRIT-0175 | Digoxigenin | 80, 91, 93 | Roche Pharma Research PZ |
| PRIT-0186 | CH1A1A | 80 | Roche Pharma Research PZ |
| PRIT-0187 | CH1A1A | 80 | Roche Pharma Research PZ |
| PRIT-0205 | T84.66 | 80 | Roche Pharma Research PZ |
| PRIT-0206 | T84.66 | 80 | Roche Pharma Research PZ |
| PRIT-0207 | T84.66 | 80 | Roche Pharma Research PZ |
| PRIT-0208 | T84.66 | 80 | Roche Pharma Research PZ |
| PRIT-0209 | T84.66 | 80 | Roche Pharma Research PZ |
| PRIT-0213 = CEA-PRIT | T84.66 | 93, 105, 106 | Roche Pharma Research PZ |
| PRIT-0214 | T84.66 | 93 | Roche Pharma Research PZ |

Clearing reagents were provided by Macrocyclics (Plano, TX, USA). They were stored at -80°C until the day of injection when they were thawed and diluted in PBS to the desired concentration.

Clearing agents

| Compound | TCMC substitution | Protocols | Supplier |
|---|---|---|---|
| Dex500-TriGalNAc 3:1† | 81-1 | 44 | Macrocyclics |
| Dex500-TriGalNAc 9:1† | 101-1 | 44, 70 | Macrocyclics |
| Dex20 | 14-1 | 83, 85, 87 | Macrocyclics |
| Dex20t | 16-1 | 70 | Macrocyclics |
| Dex7 0 | 8-1 | 83, 85, 87 | Macrocyclics |
| Dex70† | 10-1 | 44, 70 | Macrocyclics |
| Dex70-TriGalNac† | | 70 | Macrocyclics |
| Dex250 | 19-1 | 83, 87 | Macrocyclics |
| Dex250t | 24-1 | 44, 70 | Macrocyclics |
| Dex5 001 * | | 70 | Macrocyclics |
| Dex500* | 84-1 | 80, 83, 85, 87, 90, 91 | Macrocyclics |
| CDex500-(Glu)3* | 95-1 | 83, 87 | Macrocyclics |
| CDex500-(Glu)2* | 84-1 | 83, 87 | Macrocyclics |
| CDex500-(Glu)4* | 78-1 | 83, 85, 87 | Macrocyclics |
| Dex500-M(Glu)2* | 11-1 | 83, 85, 87 | Macrocyclics |
| Dex20-M(Glu)2 | 2-1 | 83, 87 | Macrocyclics |
| Dex500-(10%)** | 9-1 | 95 | Macrocyclics |
| Dex500-(20%)** | 20-1 | 95 | Macrocyclics |
| Dex500-(40%)** | 39-1 | 95 | Macrocyclics |
| Dex500-(100%)** | 84-1 | 90, 95 | Macrocyclics |
| Dex500-(50%)** | 47-1 | 105, 106 | Macrocyclics |

tNo Pb-quench; *30-k Da filtration cutoff; **100-kDa fi ltration cutoff

DOTAM chelates for radiolabeling were provided by Macrocyclics and maintained at -20°C before radiolabeling. Subsequent labeling with either lead-203 ($^{203}$Pb) or lead-212 ($^{212}$Pb) was performed by AREVA Med (Razès, France). Mice were injected intravenously (i.v.) with 100 μL of the respective Pb-DOTAM solutions, diluted with PBS to obtain the desired Pb dose/activity concentration. $^{203}$Pb-DOTAM was used pre-bound with bispecific antibodies, whereas $^{212}$Pb-DOTAM was administered after PRIT and clearing agent. Radioactive measurements were performed using a 2470 WIZARD$^2$ automatic gamma counter (PerkinElmer).

Radiolabeled chelates

| Compound | Formulation buffer | Supplier |
|---|---|---|
| $^{212}$Pb-DOTAM | PBS | Macrocyclics, AREVA Med |
| $^{203}$Pb-DOTAM | PBS | Macrocyclics, AREVA Med |

BxPC3 is a human primary pancreatic adenocarcinoma cell line, naturally expressing CEA. BxPC3 cells were cultured in RPMI-1640 medium (Gibco, ref. No. 42401-018) enriched with 10% fetal bovine serum and 1% GlutaMAX (Gibco, ref. No. 35050-061). LS174T is a human colorectal adenocarcinoma cell line, naturally expressing CEA. LS174T cells were cultured in DMEM medium (Gibco, ref. No. 42430-082) enriched with 10% fetal bovine serum. MKN45 is a human gastric adenocarcinoma cell line, naturally expressing CEA. MKN45 cells were cultured in RPMI-1640 medium (Gibco, ref. No. 42401-018) enriched with 20% fetal bovine serum. Solid xenografts were established by subcutaneous injection of cells in RPMI or DMEM media, mixed 1:1 with Corning® Matrigel® basement membrane matrix (growth factor reduced; cat No. 354230), into the right flank.

Cell lines

| Cell line | Cells per mouse | Injected volume | Supplier |
|---|---|---|---|
| BxPC3 | $5\times10^6$ | 100 µL | ECACC* |
| LS174T | $1\times10^6$ | 100 µL | ATCC** |
| MKN45 | $0.5\times10^6$ | 100 µL | DSMZ *** |

*European Collection of Authenticated Cell Cultures (Salisbury, UK)

**American Type Culture Collection (Manassas, VA, USA)

***Leibniz-Institut DSMZ - Deutsche Sammlung von Mikroorganismen und Zellkulturen GmbH (Braunschweig, Germany)

**Example 16: Pretargeting with various CEA-targeting bispecific antibodies (Protocol 80 (a, b, c))**

This study aimed to evaluate the Pb accumulation in pancreatic adenocarcinoma xenografts in mice after pretargeting with various CEA-targeting bispecific antibodies, in order to optimize the regimen and select the most suitable candidate for transition to clinical trials. The experiments were divided into three separate protocols, performed in the order i) 80b, ii) 80c, and iii) 80a. Protocol 80b assessed the tumor uptake of five fully humanized bispecific antibody constructs, pre-bound with $^{203}$Pb-DOTAM. Protocol 80c assessed the tumor uptake of bispecific antibody constructs pre-bound with $^{203}$Pb-DOTAM at three different time points after injection, to optimize the timing between PRIT injection and CA/chelate injection in pretargeting regimens. Finally, protocol 80a assessed the tumor uptake of $^{212}$Pb-DOTAM in a standard pretargeting setting, using five fully humanized bispecific antibody constructs, targeting either T84.66 or CH1A1A.

Study design, protocol 80a

| Study day | Date | Experimental procedure |
|---|---|---|
| 1 | 2016-02-10 | S.c. injection* of BxPC3 cells |
| 6 | 2016-02-15 | I.v. injection* of PRIT bsAb |
| 9 | 2016-02-18 | I.v. injection* of CA |
| 9 | 2016-02-18 | Elution of $^{212}$Pb-DOTAM |
| 9 | 2016-02-18 | I.v. injection* of $^{212}$Pb-DOTAM |
| 10 | 2016-02-19 | Euthanasia and necropsy; gamma counting of tissues |

*Injection volume 100 µL

Study design, protocol 80b

| Study day | Date | Experimental procedure |
|---|---|---|
| 1 | 2016-01-13 | S.c. injection* of BxPC3 cells |
| 6 | 2016-01-18 | Elution of $^{203}$Pb-DOTAM and pre-binding with PRIT bsAb |
| 6 | 2016-01-18 | I.v. injection* of $^{203}$Pb-DOTAM-bsAb |
| 10 | 2016-01-22 | Euthanasia and necropsy; gamma counting of tissues |

*Injection volume 100 µL

Study design, protocol 80c

| Study day | Date | Experimental procedure |
|---|---|---|
| 1 | 2016-01-20 | S.c. injection* of BxPC3 cells |
| 6 | 2016-01-25 | Elution of $^{203}$Pb-DOTAM and pre-binding with PRIT bsAb |
| 6 | 2016-01-25 | I.v. injection* of $^{203}$Pb-DOTAM-bsAb |
| 7 | 2016-01-26 | Euthanasia and necropsy; gamma counting of tissues |
| 9 | 2016-01-28 | Euthanasia and necropsy; gamma counting of tissues |
| 13 | 2016-02-01 | Euthanasia and necropsy; gamma counting of tissues |
| *Injection volume 100 μL | | |

Each mouse (age 6-7 weeks) was injected subcutaneously (s.c.) with BxPC3 cells (passage 30) in 100 μL RPMI/Matrigel into the right flank. Five days after tumor cell injection, mice were sorted into experimental groups with an average tumor volume of 160-170 $mm^3$. Antibodies were diluted to a final concentration of 30 μg per 100 μL, and subsequently administered i.v., either alone (80a), or pre-bound with $^{203}$Pb-DOTAM (80b, 80c). PRIT-0165 and PRIT-0156 were used as positive CEA-binding contro-s, targeting T84.66 and CH1A1A, respectively. PRIT-0175 was used as a non-CEA-binding control.

In protocol 80a, a clearing agent was intravenously injected at a concentration of 30 μg per 100 μL three days after the bispecific antibody, followed two hours later by $^{212}$Pb-DOTAM.

Study groups, protocol 80a ($n_{tot}$ = 24)

| **Group** | **A** | **B** | **C** | **D** | **E** | **F** | **G** | **H** |
|---|---|---|---|---|---|---|---|---|
| **bsAb** | PRIT-0206 | PRIT-0207 | PRIT-0208 | PRIT-0165 * | PRIT-0186 | PRIT-0187 | PRIT-0156 ** | PRIT-0175 *** |
| **bsAb dose** (μg) | 30 | 30 | 30 | 30 | 30 | 30 | 30 | 30 |
| **CA** | Dex500 | Dex500 | Dex500 | Dex500 | Dex500 | Dex500 | Dex500 | Dex500 |
| **CA dose** (μg) | 30 | 30 | 30 | 30 | 30 | 30 | 30 | 30 |
| **Chelate** | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM |
| **Pb activity** (μCi) | 13 | 13 | 13 | 13 | 13 | 13 | 13 | 13 |
| **n** | 3 | 3 | 3 | 3 | 3 | 3 | 3 | 3 |
| *Positive control (T84.66); **Positive control (CH1A1A); ***Negative control | | | | | | | | |

Study groups, protocol 80b ($n_{tot}$ = 21)

| Group | A | B | C | D | E | F | G |
|---|---|---|---|---|---|---|---|
| bsAb | PRIT- 0205 | PRIT- 0206 | PRIT- 0207 | PRIT- 0208 | PRIT- 0209 | PRIT- 0165 * | PRIT-0175 ** |
| bsAb dose (μg) | 30 | 30 | 30 | 30 | 30 | 30 | 30 |
| CA | - | - | - | - | - | - | - |
| CA dose (μg) | - | - | - | - | - | - | - |

(continued)

| Group bsAb | A PRIT- 0205 | B PRIT- 0206 | C PRIT- 0207 | D PRIT- 0208 | E PRIT- 0209 | F PRIT- 0165 * | G PRIT-0175 ** |
|---|---|---|---|---|---|---|---|
| Pre-bound chelate | $^{203}$Pb-DOTAM | $^{203}$Pb-DOTAM | $^{203}$Pb-DOTAM | $^{203}$Pb-DOTAM | $^{203}$Pb-DOTAM | $^{203}$Pb-DOTAM | $^{203}$Pb-DOTAM |
| Pb activity (μCi) | 4 | 4 | 4 | 4 | 4 | 4 | 4 |
| n | 3 | 3 | 3 | 3 | 3 | 3 | 3 |
| *Positive control (T84.66); **Negative control | | | | | | | |

Study groups, protocol 80c ($n_{tot}$ = 27)

| Group bsAb | A PRIT-0206 | B PRIT-0165 * | C PRIT-0175 ** |
|---|---|---|---|
| bsAb dose (μg) | 30 | 30 | 30 |
| CA | - | - | - |
| CA dose (μg) | - | - | - |
| Pre-bound chelate | $^{203}$Pb-DOTAM | $^{203}$Pb-DOTAM | $^{203}$Pb-DOTAM |
| Pb activity (μCi) | 1-2 | 1-2 | 1-2 |
| Necropsy time points (h p.i.) | 24, 72, 168 | 24, 72, 168 | 24, 72, 168 |
| n (per time point) | 9 (3) | 9 (3) | 9 (3) |
| *Positive control (T84.66); **Negative control | | | |

Mice were sacrificed for biodistribution purposes after 24 h (80a); 96 h (80b); or 24, 72, or 168 h (80c). From all mice in protocol 80a were harvested: blood, bladder, spleen, kidneys, liver, lung, heart, muscle, and tumor. From all mice in protocols 80b and 80c were harvested: blood, bladder, small intestine, colon, spleen, pancreas, kidneys, liver, lung, heart, femoral bone, muscle, and tumor. Collected samples were weighed and put in plastic tubes for immediate radioactivity measurement. The percent injected dose per gram of tissue (%ID/g) was then calculated, including corrections for radioactive decay and background.

Results, 80a

All CEA-binding bispecific antibodies resulted in specific tumor targeting of $^{212}$Pb-DOTAM, with little or no uptake in normal tissues 24 hours after DOTAM injection. For PRIT-0206, PRIT-0207, and PRIT-0208 the average tumor uptake ± SD was 8.62 ± 1.05, 7.30 ± 3.84, and 7.75 ± 2.61 %ID/g, respectively, with their corresponding T84.66-binding positive control PRIT-0165 at 9.13 ± 1.82 %ID/g. The CH1A1A-binders PRIT-0186 and PRIT-0187 resulted in tumor values of 17.44 ± 1.39 and 16.50 ± 3.25 %ID/g, their positive control PRIT-0156 at 18.98 ± 1.89 %ID/g. The non-CEA binding PRIT-0175 resulted in 0.41 ± 0.42 %ID/g in the tumor.

Overall, the bispecific antibodies targeting CH1A1A resulted in significantly higher tumor uptakes than those targeting T84.66 (unpaired t-test, p<0.0001); CH1A1A and T84.66 both resulted in significantly higher uptake compared with the negative control (one-way ANOVA, p<0.0001). The results are shown in figure 4.

Results, 80b

Specific targeting of tumors was achieved with all pre-bound CEA-binding bispecific antibodies, although the %ID/g was slightly lower for the fully humanized versions compared with the positive control PRIT-0165. The non-CEA-binding control resulted in comparatively negligible tumor accumulation.

Overall, the calculated %ID/g in this pre-bound experiment setting reached levels that were approximately tenfold higher than those of corresponding PRIT regimens; however, the output data reflected the gamma counter activity measurements and no calculation errors were found. Importantly, the tumor-to-normal tissue ratios remained within the

expected range. Specifically, the tumor-to-blood ratio (± SD, n = 3) was 6.76 ± 2.95 for PRIT-0205, 7.56 ± 2.27 for PRIT-0206, 9.33 ± 0.91 for PRIT-0207, 10.77 + 0.84 for PRIT-0208, 11.71 ± 0.84 for PRIT-0209, 10.78 ± 0.88 for PRIT-0165, and 0.85 ± 0.12 for PRIT-0175. The results are shown in figure 5.

Results, 80c

Both CEA-binding antibodies resulted in significant tumor accumulation as compared with the negative control. Statistical analysis showed no significant difference between tumor targeting using PRIT-0206 or PRIT-0165 for any of the studied time points, neither was there any significant difference in %ID/g between day 3 and 7 for any of the studied antibodies (two-way ANOVA, $p < 0.05$). Analogous with protocol 80b, the calculated %ID/g values were high overall; however, the tumor-to-blood ratios remained within the expected range. No significant differences in tumor-to-blood ratios were seen between day 1 and 3, but for PRIT-0165 and PRIT-0206, waiting 7 days significantly increase PRIT-0213 d the ratio (two-way ANOVA, $p < 0.05$). The results are shown in figure 6.

Tumor-to-blood ratios (± SD; n=3) of $^{203}$Pb-DOTAM-bsAb at various time points after injection.

| Days after injection | PRIT-0206 | PRIT-0165 | PRIT-0175 |
|---|---|---|---|
| 1 | 4.48 ± 0.38 | 4.28 ± 1.31 | 0.84 ± 0.22 |
| 3 | 8.73 ± 4.07 | 7.13 ± 0.33 | 0.75 ± 0.30 |
| 7 | 29.67 ± 10.52 | 17.87 ± 12.15 | 0.76 ± 0.25 |

Summary and conclusion

All fully humanized bispecific antibodies targeting either T84.66 or CH1A1A resulted in significant accumulation of radioactivity in BxPC3 tumors, comparable to those of their respective positive controls.

The %ID/g of $^{203}$Pb-DOTAM-bsAb in tumor did not differ significantly between 3 and 7 days, but the corresponding tumor-to-blood ratio favored the later time point due to the decrease in blood radioactivity.

**Example 17: Biodistribution of clearing agents (Protocol 44)**

The aim of this study was to address the biodistribution of a selection of clearing agents with different properties (e.g. molecular backbone, size, and charge) and, more specifically, their presence and/or accumulation in tumors. This was of interest seeing as clearing agents may potentially enter into tumors, bind to tumor-bound antibodies and/or pull them out of the tumor, negatively affecting subsequent binding of radioligands.

Study design, protocol 44

| Study day | Date | Experimental procedure |
|---|---|---|
| 1 | 2015-03 | I.v. injection* of $^{212}$Pb-labeled CA |
| 1 | 2015-03 | Euthanasia and necropsy; gamma counting of tissues (2 h p.i.) |
| 2 | 2015-03 | Euthanasia and necropsy; gamma counting of tissues (24 h p.i.) |
| *Injection volume 100 μL | | |

Study groups, protocol 44 ($n_{tot}$ = 24)

| Group | CAt | CA dose | $^{212}$Pb | Time | n |
|---|---|---|---|---|---|
| | | | | | |
| | | (μg) | activity (μCi) | point (h p.i.) | |
| A | Dex70 | 30 | 5 | 2 | 3 |

(continued)

| | | (μg) | activity (μCi) | point (h p.i.) | |
|---|---|---|---|---|---|
| B | Dex250 | 30 | 5 | 2 | 3 |
| C | Dex500-TriGalNAc 3:1 | 30 | 5 | 2 | 3 |
| D | Dex500-TriGalNAc 9:1 | 30 | 5 | 2 | 3 |
| E | Dex70 | 30 | 5 | 24 | 3 |
| F | Dex250 | 30 | 5 | 24 | 3 |
| G | Dex500-TriGalNAc 3:1 | 30 | 5 | 24 | 3 |
| H | Dex500-TriGalNAc 9:1 | 30 | 5 | 24 | 3 |
| tAll clearing agents without Pb-quench before $^{212}$Pb-labeling | | | | | |

30 μg of Dex70, Dex250, and TriGalNac-modified clearing agents were quenched with 5 μCi $^{212}$Pb and diluted in PBS to obtain 30 μg per 100 μL total volume for i.v. injection.

Mice were sacrificed and necropsied 2 or 24 hours after injection of $^{212}$Pb-CA. Blood, urinary bladder, heart, lung, liver, spleen, kidneys, intestine (duodenum, jejunum, ileum), colon, pancreas, stomach, ovaries, brain, femur with bone marrow, and tumor were collected, weighed and measured for radioactivity content, and the %ID and %ID/g subsequently calculated. In addition, urine was sampled at the 24-h time point.

Results, 44

All clearing agents were rapidly cleared from the blood stream, accumulating mainly in liver and colon already at 2 hours after injection. About 50% of the injected $^{212}$Pb was found in the liver 24 hours after injection, as demonstrated by the organ-wise (%ID) radioactivity distribution. TriGalNAc-modified clearing agents also accumulated to a certain extent in the spleen, explained by the presence of TriGalNAc molecules. In general, little radioactivity was found in the urine after 24 hours. However, the smallest clearing agent (Dex70) was excreted slower than expected.

Figure 7 shows radioactivity distribution in selected tissues 2 hours after injection of $^{212}$Pb-labeled clearing agents in MKN45 tumor-bearing mice (%ID/g ± SD, n=3).

Figure 8 shows Radioactivity distribution in selected tissues and urine, 24 hours after injection of $^{212}$Pb-labeled clearing agents in MKN45 tumor-bearing mice (%ID/g ± SD, n=3).

Figure 9 shows organ-wise radioactivity distribution in selected tissues and urine, 24 hours after injection of $^{212}$Pb-labeled clearing agents in MKN45 tumor-bearing mice (%ID ± SD, n=3).

Summary and conclusion

None of the radiolabeled CAs resulted in tumor uptake of $^{212}$Pb, administered at a dose of 30 μg per mouse. Modification of Dex500 with TriGalNac was not beneficial compared with non-modified Dex70 and Dex250 clearing agents.

**Example 18: Long-term biodistribution of clearing agents (Protocol 70)**

This study compared the long-term biodistribution of six different clearing agents. The in vivo tracking was performed through radiolabeling with $^{203}$Pb.

Study design, protocol 70

| **Study day** | **Date** | Experimental procedure |
|---|---|---|
| 1 | 2015-09 | I.v. injection* of $^{203}$Pb-labeled CA |
| 8 | 2015-09 | Euthanasia and necropsy; gamma counting of tissues |
| *Injection volume 100 μL | | |

Study groups, protocol 70 ($n_{tot}$ = 18)

| Group | CA† | CA dose (μg) | $^{203}$Pb activity (μCi) | n |
|---|---|---|---|---|
| A | Dex500 | 30 | 50 | 3 |
| B | Dex500-TriGalNAc 9:1 | 30 | 50 | 3 |
| C | Dex250 | 30 | 50 | 3 |
| D | Dex70 | 30 | 50 | 3 |
| E | Dex70-TriGalNac | 30 | 50 | 3 |
| F | Dex20 | 30 | 50 | 3 |
| tAll clearing agents without Pb-quench before $^{203}$Pb-labeling<br>Results, 70 | | | | |

Figure 10 shows Radioactivity distribution in selected tissues 1 week after injection of $^{203}$Pb-labeled clearing agents in tumor-free mice (%ID/g ± SD, n=3).

Figure 11 shows organ-wise radioactivity distribution in selected tissues 1 week after injection of $^{203}$Pb-labeled clearing agents in tumor-free mice (%ID ± SD, n=3).

**Example 19: Clearing agent in blood (Protocols 83 and 87)**

This part covers two studies, of which the first includes an initial screen comparing nine different dextran-based clearing agents with PBS, in terms of residence time in blood. The aim was to identify promising candidates for future pretargeting experiments, allowing repeated treatments separated by three weeks. The hypothesis was that clearing agents which remain in circulation for a prolonged time bind to administered bispecific antibodies, effectively blocking binding of subsequently injected radiolabeled DOTAM. The clearing reagents varied in terms of size, charge, and 1,4,7,10-tetrakis(carbamoylmethyl)-1,4,7,10-tetraazacyclododecane (TCMC) load.

The second study assessed the nine clearing agents in terms of antibody clearing efficiency. The experiment was designed as a standard singleinjection PRIT regimen in tumor-free mice, evaluating the $^{212}$Pb-DOTAM retention in blood as an indication of retained bsAb after CA administration.

Study design, protocol 83

| Study day | Date | Experimental procedure |
|---|---|---|
| 1 | 2016-01-19 | I.v. injection* cf PRIT bsAb |
| 2 | 2016-01-20 | I.v. injection* cf CA or PBS |
| 24 | 2016-02-11 | I.v. injection* of PRIT bsAb |
| 25 | 2016-02-12 | Elution of $^{212}$Pb-DOTAM |
| 25 | 2016-02-12 | I.v. injection* of $^{212}$Pb-DOTAM |
| 25 | 2016-02-12 | Euthanasia and blood sampling + gamma counting |
| *Injection volume 100 μL | | |

Study design, protocol 87

| Study day | Date | Experimental procedure |
|---|---|---|
| 6 | 2016-02-29 | I.v. injection* of PRIT bsAb |
| 9 | 2016-03-01 | I.v. injection* of CA or PBS |
| 9 | 2016-03-01 | Elution of $^{212}Pb$-DOTAM |
| 9 | 2016-03-01 | I.v. injection* of $^{212}Pb$-DOTAM |
| 10 | 2016-03-02 | Euthanasia and blood sampling + gamma counting |
| *Injection volume 100 µL | | |

Study groups in protocol 83 and 87 ($n_{tot}$ = 30 per protocol)

| Group | bsAb | bsAb dose (µg) | CA | CA dose 83/87 (µg) | Chelate | Pb activity (µCi) | n |
|---|---|---|---|---|---|---|---|
| A | PRIT-0155 | 30 | Dex20 | 30/60 | $^{212}Pb$-DOTAM | 10 | 3/3 |
| B | PRIT-0155 | 30 | Dex70 | 30/60 | $^{212}Pb$-DOTAM | 10 | 3/3 |
| C | PRIT-0155 | 30 | Dex250 | 30/60 | $^{212}Pb$-DOTAM | 10 | 3/3 |
| D | PRIT-0155 | 30 | Dex500 | 30/60 | $^{212}Pb$-DOTAM | 10 | 3/3 |
| J | PRIT-0155 | 30 | CDex500-(Glu)3 | 30/60 | $^{212}Pb$-DOTAM | 10 | 3/3 |
| K | PRIT-0155 | 30 | CDex500-(Glu)2 | 30/60 | $^{212}Pb$-DOTAM | 10 | 3/3 |
| L | PRIT-0155 | 30 | CDex500-(Glu)4 | 30/60 | $^{212}Pb$-DOTAM | 10 | 3/3 |
| M | PRIT-0155 | 30 | Dex500-M(Glu)2 | 30/60 | $^{212}Pb$-DOTAM | 10 | 3/3 |
| N | PRIT-0155 | 30 | Dex20-M(Glu)2 | 30/60 | $^{212}Pb$-DOTAM | 10 | 3/3 |
| O | PRIT-0155 | 30 | - | 0/0 | $^{212}Pb$-DOTAM | 10 | 3/3 |

At the time of the first PRIT injection, mice were 7-9 weeks old. All mice in the study were tumor-free; therefore, any DOTAM-binding bispecific antibody could be used for screening purposes. The clearing agents were administered intravenously one day after PRIT-0155, diluted in PBS to a final concentration of 30 (protocol 83) or 60 (protocol 87) µg per 100 µL. Group O received PBS instead of clearing agent. The compounds in groups A-D were based on dextran sizes of 20, 70, 250, or 500 kDa, with varying TCMC substitution. Compounds in groups J-N were based on capped (i.e. with neutralization of excess amines) dextran-500 (CDex) with varying charge (Glu), or dextrane-500 and dextrane-20 with mono versions of Glu (M(Glu)). Capped dextran with -(Glu)4, -(Glu)3, and -(Glu)2 corresponded to a very negative, negative, and neutral net charge, respectively; the mono version -M(Glu)2 corresponded to a negative-to-slightly positive net charge.

Results, 83

Group averages of radioactivity content (%ID/g) in blood that were significantly different from that of the PBS control group (41.1 ± 1.4 %ID/g) indicated retention of clearing agent in circulation, three weeks after administration. Three compounds did not differ significantly from the control: capped Dex500-(Glu)4, Dex500-mono-(Glu)2, and Dex20-mono-(Glu)3; others differed to a varying degree.

Figure 12 shows Radioactivity content in blood 4 h after injection of $^{212}$Pb-DOTAM (%ID/g ± SD, n = 3). The striped bar represents the no-CA control, with which all candidate reagents were compared. Asterisks mark the level of statistical significance, from lower (*) to higher (***).

Results, 87

The tested clearing agents performed well in general, with one exception: Dex500-M(Glu)2, which stood out with 8.07 ± 0.61 %ID/g remaining in blood after 24 hours.

Figure 13 shows average radioactivity content in blood 24 h after injection of $^{212}$Pb-DOTAM (%ID/g ± SD, n = 3). The striped bar represents the no-CA control, with which all candidate reagents were compared.

Summary and conclusion

The screened reagents performed well overall in terms of self-clearance and achieved antibody clearance from circulation. Repeated treatments with three weeks between CA injections proved feasible without risking the $^{212}$Pb-DOTAM binding to bispecific antibodies. In addition, bispecific antibodies were cleared within 2 hours after CA injection using a majority of the tested compounds.

**Example 20: Tumor penetration of clearing agents (Protocol 85)**

Tumor penetration of the clearing agent is a potential problem in PRIT regimens, as penetrating DOTAM-bound CA fragments would compete with $^{212}$Pb-DOTAM in binding to antibody-pretargeted tumor cells. In this study, six different clearing agents were compared in terms of inhibition of $^{212}$Pb-DOTAM association to tumors. Candidates were chosen based on the results from the baseline CA screen (protocol 83) in order to assess the impact on tumor-associated radioactivity from i) dextran size and ii) charge of the molecule.

Study design, protocol 85

| Study day | Date | Experimental procedure |
|---|---|---|
| 1 | 2016-02-17 | I.v. injection* of BxPC3 cells |
| 6 | 2016-02-22 | I.v. injection* of PRIT bsAb |
| 9 | 2016-02-25 | I.v. injection* of CA or PBS |
| 9 | 2016-02-25 | Elution of $^{212}$Pb-DOTAM |
| 9 | 2016-02-25 | I.v. injection* of $^{212}$Pb-DOTAM |
| 10 | 2016-02-26 | Euthanasia and necropsy; gamma counting of tissues |
| *Injection volume 100 μL | | |

Study groups in protocol 85 ($n_{tot}$ = 18)

| Group | bsAb | bsAb dose (μg) | CA | CA dose (μg) | Chelate | Pb activity (μCi) | n |
|---|---|---|---|---|---|---|---|
| A | PRIT-0165 | 30 | Dex20 | 30 | $^{212}$Pb-DOTAM | 10 | 3 |

(continued)

| Group | bsAb | bsAb dose (μg) | CA | CA dose (μg) | Chelate | Pb activity (μCi) | n |
|---|---|---|---|---|---|---|---|
| B | PRIT-0165 | 30 | Dex70 | 30 | $^{212}$Pb-DOTAM | 10 | 3 |
| D | PRIT-0165 | 30 | Dex500 | 30 | $^{212}$Pb-DOTAM | 10 | 3 |
| E | PRIT-0165 | 30 | CDex500-(Glu) 4 | 30 | $^{212}$Pb-DOTAM | 10 | 3 |
| F | PRIT-0165 | 30 | Dex500-M(Glu) 2 | 30 | $^{212}$Pb-DOTAM | 10 | 3 |
| G | PRIT-0165 | 30 | - | 0 | $^{212}$Pb-DOTAM | 10 | 3 |

Each mouse (age 7 weeks) was injected s.c. with BxPC3 cells (passage 33) in 100 μL RPMI/Matrigel into the right flank. Five days after tumor cell injection, mice were sorted into experimental groups with an average tumor volume of 200 $mm^3$.

The clearing agents were based on dextran sizes of 20, 70, or 500 kDa, with varying TCMC substitution. CDex500-(Glu)4 was based on capped (i.e. with neutralization of excess amines) dextran-500 (CDex) with a negative net charge; Dex500-M(Glu)2 had the mono version of Glu (M(Glu)) and neutral-to-slightly positive net charge. All were administered intravenously, 30 μg per 100 μL, three days after PRIT-0165. Group G received PBS instead of clearing agent.

Mice were sacrificed 24 hours after injection of $^{212}$Pb-DOTAM. Blood and tumors were collected, and their respective %ID/g calculated from sample weights and radioactivity content.

Results, 85

Three of the candidates displayed very little or no tumor uptake: Dex20 (0.26 ± 0.03 %ID/g), Dex70 (4.06 ± 2.06 %ID/g), and CDex500-(Glu)4 (2.98 + 0.73 %ID/g), indicating extensive CA penetration into the tumors. In contrast, Dex500-M(Glu)2 resulted in high radioactivity in both tumor (69.39 ± 9.70 %ID/g) and blood (20.68 ± 1.22 %ID/g), at levels that were similar to those of the PBS control (59.02 ± 15.53 and 27.92 ± 2.38 %ID/g in tumor and blood, respectively), interpreted as a bsAb clearance failure. Dex500 displayed a considerable accumulation of radioactivity in tumor (20.78 ± 3.76 %ID/g), indicating little tumor penetration, whereas the blood clearance was essentially complete (0.17 ± 0.01 %ID/g). Nonetheless, the tumor uptake achieved in the no-CA control indicated that a certain degree of low-molecular weight (MW) DOTAM-dextran fragments were present also in the Dex500 batch.

Figure 14 shows radioactivity content in blood and tumors 24 h after injection of $^{212}$Pb-DOTAM (%ID/g + SD, n = 3).

Summary and conclusion

This experiment confirmed the notion that the presence of low-MW CA species can interfere substantially with tumor accumulation of $^{212}$Pb-DOTAM. It also demonstrated the wide MW range of molecules that are present in any batch of CA, regardless of the specified size. Because of this, the greater the specified CA size, the lower the risk of introducing low-MW fragments that can penetrate the tumors. However, even for Dex500 a certain level of penetration was revealed, indicated by the difference in tumor uptake compared with the no-CA control. Consequently, this solution should be diafiltered using a higher MW cutoff in the future, to remove as much as possible of these interfering fragments. A decision was made to increase the MW cutoff from 30 to 100 kDa for Dex500.

**Example 21: clearing agent manufacturing process for Dex500**

Amino dextran (20.0 g) was dissolved in a mixture of 0.1 M $Na_2CO_3$ in $H_2O$ (400 mL) and 0.1 M $NaHCO_3$ in $H_2O$ (400 mL). After a clear colorless solution was obtained, p-SCN-Bn-DOTAM·4HCl (S-2-(4-Isothiocyanatobenzyl)-1,4,7,10-tetraaza-1,4,7,10-tetra (2-carbamoylmethyl)cyclododecane tetra hydrochloride salt, 2.03 g) was added under stirring . The resulting slightly turbid solution was stirred at room temperature for 4 hours before the reaction mixture was neutralized to pH 6-7 by adding 2 M HCl. The resulting solution was purified by tangential flow filtration with a 100

kDa cut-off (Sartorius Hydrosart, Slice 200 100kDa 0.02m$^2$, stabilized cellulose based membrane, Ultrafiltration Cassette) to remove low molecular weight impurities. The resulting solution was lyophilized under reduced pressure to give 17.9 g of the desired intermediate.

16.1 g of the solid obtained from freeze-drying was dissolved in a mixture of 0.1 M AcOH in $H_2O$ (60 mL) and 0.1 M $NaOAc{\cdot}3H_2O$ (540 mL). To the clear colorless solution $Pb(OAc)_2{\cdot}3H_2O$ (744 mg) was added as a solid. The clear colorless solution was stirred for 60 min before a solution of xylenol orange (1% in $H_2O$, 250 uL) was added. The purple color indicated the presence of free Pb(II) in the solution. A solution of EDTA (0.01 M in $H_2O$) was added until a color change to yellow was observed (65.2 mL). The resulting solution was purified by tangential flow filtration with a 100 kDa cut-off (Sartorius Hydrosart, Slice 200 100kDa 0.02m$^2$, stabilized cellulose based membrane, Ultrafiltration Cassette) to remove low molecular weight impurities. The resulting solution was lyophilized under reduced pressure to give 14.0 g of the desired clearing agent.

The resulting clearing agent is substituted with multiple Pb-DOTAM moieties of the form shown schematically below:

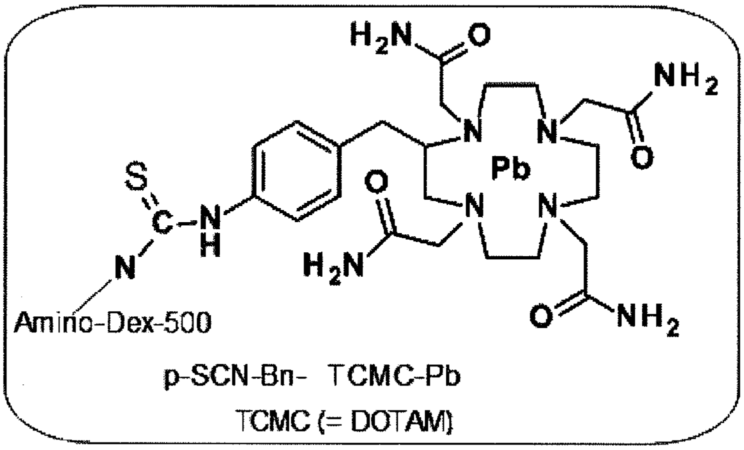

**Example 22: Clearing agent dose on tumor accumulation (Protocol 90)**

This study aimed to investigate the impact on clearing agent dose on tumor accumulation in a subcutaneous tumor model, hypothesizing that a higher amount of clearing agent could lead to a higher degree of penetration of clearing agent into tumors, thereby decreasing the subsequent uptake of labeled chelate by blocking the DOTAM-binding arm of tumor-bound bispecific antibodies. However, too low a dose would lead to less efficient accumulation of tumor-associated radioactivity, due to higher levels of DOTAM-binding bispecific antibodies in circulation.

In addition, a comparison was made between clearing agent batches that had been diafiltered to remove low-MW components, with cutoff at either 30 or 100 kDa, in an effort to decrease the tumor penetration of DOTAM-bound dextran fragments.

Study design, protocol 90

| Study day | Date | Experimental procedure |
|---|---|---|
| 1 | 2016-03-21 | S.c. injection* of BxPC3 cells |
| 6 | 2016-03-26 | I.v. injection* of PRIT bsAb |
| 10 | 2016-03-30 | I.v. injection* of Dex500 |
| 10 | 2016-03-30 | Elution of $^{212}$Pb-DOTAM |
| 10 | 2016-03-30 | I.v. injection* of $^{212}$Pb-DOTAM |
| 11 | 2016-03-31 | Euthanasia and necropsy; gamma counting of tissues |
| *Injection volume 100 $\mu$L | | |

Study groups in protocol 90 ($n_{tot}$ = 24)

| Group | A | B | C | D | E | F | G | H |
|---|---|---|---|---|---|---|---|---|
| bsAb | PRIT-0165 | PRIT-0165 | PRIT-0165 | PRIT-0165 | PRIT-0165 | PRIT-0165 | PRIT-0165 | PRIT-0165 |
| bsAbdose (μg) | 100 | 100 | 100 | 100 | 30 | 30 | 30 | 100 |
| CA | Dex500 * | Dex500 * | Dex500 * | Dex500 * | Dex500 * | Dex500 ** | 0 | 0 |
| CA dose (μg) | 10 | 25 | 50 | 100 | 30 | 30 | 0 | 0 |
| Chelate | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM |
| Pb activity (μCi) | 10 | 10 | 10 | 10 | 10 | 10 | 10 | 10 |
| n | 3 | 3 | 3 | 3 | 3 | 3 | 3 | 3 |
| *100-kDa filtration cutoff; **30-kDa filtration cutoff | | | | | | | | |

Solid xenografts were established by subcutaneous injection of BxPC3 cells (passage 30) in RPMI medium mixed 1:1 with Corning® Matrigel® basement membrane matrix (growth factor reduced; cat No. 354230). Each mouse (age 11 weeks) was injected s.c. with 5×$10^6$ cells in 100 μL RPMI/Matrigel into the right flank. Five days after tumor cell injection, mice were sorted into experimental groups with an average tumor volume of 150 $mm^3$.

PRIT-0165 was injected i.v. at a concentration of either 30 or 100 pg per 100 μL, followed four days later by clearing agent (groups A-F) at a concentration of 10, 25, 30, 50, or 100 μg per 100 μL. $^{212}$Pb-DOTAM was injected two hours after the CA. Groups G and H received no clearing agent between administrations of bispecific antibody and radiolabeled chelate.

Mice were sacrificed for biodistribution purposes 24 hours after injection of $^{212}$Pb-DOTAM, and blood, bladder, spleen, kidneys, liver, lung, muscle, tail, and tumor were collected. Samples were weighed and measured for radioactivity, and the %ID/g subsequently calculated for each organ, including corrections for decay and background.

Results, 90

The $^{212}$Pb accumulation in normal tissues was low for all groups to which a clearing agent was administered; contrastingly, groups who received no clearing agent displayed significant levels of radioactivity overall.

The biodistribution data demonstrate the effect of varying the amount of clearing agent (100-kDa cutoff). Linear regression of the individual tumor data yielded a significant slope, indicating higher tumor uptake with decreasing amounts of Dex500 ($p = 0.03$, $R^2 = 0.31$). The effect on radioactivity content in blood was drastic, going from 31.5 ± 0.2 %ID/g using PBS to 1.6 ± 0.5 %ID/g using 10 μg of Dex500.

Two groups received PRIT-0165 and Dex500 at equal (1:1) ratio; either 100 or 30 μg of each agent. The resulting %ID/g in tumor was not significantly different between the two groups (unpaired t-test, $p = 0.726$). Importantly, the comparison between equal amounts of Dex500 with different filtration cutoffs showed a significant gain in tumor accumulation using the higher-MW cutoff: 54.9 ± 6.9 instead of 32.5 ± 4.4 %ID/g (unpaired t-test, $p = 0.009$).

Figure 15 shows distribution of $^{212}$Pb 24 h after injection of radiolabeled DOTAM (%ID/g ± SD, n = 3), using 30 or 100 μg of bispecific antibody and 10-100 μg of clearing agents with 100- or 30-kDa filtration cutoffs, or no clearing agent at all (PBS).

Figure 16 shows the effect on activity concentration of $^{212}$Pb in blood and tumor with increasing amounts of clearing agent (0-100 μg). Tumors were pretargeted using 100 μg of PRIT-0165, followed 4 days later by Dex500 diafiltered with a 100-kDa cutoff, or PBS. $^{212}$Pb-DOTAM was administered 2 hours after the CA. The symbols represent the %ID/g 24 h after the radioactive injection, and the line the linear regression of the tumor data.

Summary and conclusion

The study demonstrated a radical increase in tumor accumulation of $^{212}Pb$, while maintaining low levels of circulating radioactivity. Two key findings were concluded: 1) diafiltration with a 100-kDa cutoff significantly decreased the tumor penetration of DOTAM-bound dextran fragments, and 2) the ratio of CA to bsAb impacted the outcome more than the absolute CA amount. An excellent tumor-to-blood ratio was achieved using an antibody:CA ratio of 10:1 after administration of 100 μg of bispecific antibody. Furthermore, it was concluded that all future studies using Dex500-based clearing agents should use reagents diafiltered using a 100-kDa cutoff.

**Example 23: Impact on tumor-associated radioactivity (Protocol 95)**

In this study, nine different clearing agents based on dextran-500 were compared in terms of inhibition of $^{212}Pb$-DOTAM association to tumors. More specifically, the experiment assessed the impact on tumor-associated radioactivity from i) TCMC saturation, ii) clearing agent-to-antibody ratio, and iii) production/purification method. In addition, the tumor uptake was compared after injection of 10 or 30 μCi of $^{212}Pb$-DOTAM, without pretargeting or CA, to assess whether radioactivity saturation of the tumor was achieved already at the lower dose.

Study design, protocol 95

| Study day | Date | Experimental procedure |
|---|---|---|
| 1 | 2016-06-14 | I.v. injection* of BxPC3 cells |
| 13 | 2016-06-26 | I.v. injection* of PRIT bsAb |
| 16 | 2016-06-29 | I.v. injection* of CA or PBS |
| 16 | 2016-06-29 | Elution of $^{212}Pb$-DOTAM |
| 16 | 2016-06-29 | I.v. injection* of $^{212}Pb$-DOTAM |
| 17 | 2016-06-30 | Euthanasia and necropsy; gamma counting of tissues |
| *Injection volume 100 μL | | |

Study groups in protocol 95 ($n_{tot}$ = 27)

| Group | bsAb | bsAb dose (μg) | CA | CA dose (μg) | Chelate | Pb activity (μCi) | n |
|---|---|---|---|---|---|---|---|
| C | PRIT-0165 | 100 | Dex500-(10%) | 10 | $^{212}Pb$-DOTAM | 10 | 3 |
| D | PRIT-0165 | 100 | Dex500-(10%) | 25 | $^{212}Pb$-DOTAM | 10 | 3 |
| E | PRIT-0165 | 100 | Dex500-(10%) | 50 | $^{212}Pb$-DOTAM | 10 | 3 |
| F | PRIT-0165 | 100 | Dex500-(10%) | 100 | $^{212}Pb$-DOTAM | 10 | 3 |
| G | PRIT-0165 | 100 | Dex500-(20%) | 40 | $^{212}Pb$-DOTAM | 10 | 3 |
| H | PRIT-0165 | 100 | Dex500-(40%) | 20 | $^{212}Pb$-DOTAM | 10 | 3 |
| I | PRIT-0165 | 100 | Dex500-(100%) | 10 | $^{212}Pb$-DOTAM | 10 | 3 |

(continued)

| Group | bsAb | bsAb dose (μg) | CA | CA dose (μg) | Chelate | Pb activity (μCi) | n |
|---|---|---|---|---|---|---|---|
| J | PRIT-0165 | 100 | - | 0 | $^{212}$Pb-DOTAM | 10 | 3 |
| K | PRIT-0165 | 100 | - | 0 | $^{212}$Pb-DOTAM | 30 | 3 |

Solid xenografts were established by subcutaneous injection of BxPC3 cells (passage 20) in RPMI medium mixed 1:1 with Corning® Matrigel® basement membrane matrix (growth factor reduced; cat No. 354230). Each mouse (age 8 weeks) was injected s.c. with $5\times10^6$ cells in 100 μL RPMI/Matrigel into the right flank. Fifteen days after tumor cell injection, mice were sorted into experimental groups with an average tumor volume of 210 $mm^3$.

PRIT-0165 (100 μg per 100 μL) was administered i.v., followed three days later by dextran-500-based clearing reagents (10-170 μg per 100 μL) with varying TCMC substitution (10-100%). Groups J and K received PBS instead of clearing agent. Two hours later, mice were injected i.v. with 100 μL of the respective $^{212}$Pb-DOTAM solutions (10 or 30 pCi).

Mice were sacrificed and necropsied 24 hours after injection of $^{212}$Pb-DOTAM. Blood, bladder, spleen, kidneys, liver, lung, muscle, tail, and tumors were collected, weighed and measured for radioactivity content, and the %ID/g subsequently calculated.

Results, 95

The biodistribution data revealed an apparent trend in average radioactivity content (%ID/g ± SD) in collected tissues 24 hours after $^{212}$Pb-DOTAM injection depending on administered amount, with higher $^{212}$Pb concentration in blood and normal tissues with lower CA amount. In addition, higher $^{212}$Pb activity resulted in higher $^{212}$Pb accumulation in tumor, without a corresponding increase in normal tissue uptake.

Figure 17 shows radioactivity distribution in selected tissues 24 h after injection of $^{212}$Pb-DOTAM (%ID/g + SD, n = 3). The dark grey and black bars represent no-CA positive controls, with which the candidate reagents were compared.

One-way ANOVA with correction for multiple comparisons revealed that only Dex500-(40%) (76.79 ± 33.28, $p < 0.0001$) and Dex500-(100%) (43.26 ± 24.66, $p = 0.0115$) differed significantly from the 10-pCi negative control (2.26 ± 0.25) in terms of tumor-to-blood ratio. The perceived difference between the clearing reagent with the highest tumor-to-blood ratio (Dex500-(40%)) and the previous standard (Dex500-(100%)) was not statistically significant (unpaired t-test, $p = 0.2336$).

Figure 18 shows $^{212}$Pb content in blood and tumors 24 h after injection of $^{212}$Pb-DOTAM (%ID/g ± SD, n = 3), and the corresponding tumor-to-blood ratios. The dark grey and black bars represent no-CA positive controls, with which the candidate reagents were compared.

Linear regression and polynomial (cubic) curve fitting was performed to analyze the impact from increasing clearing agent (Dex500-(10%)) amounts and TCMC load (no CA, Dex500-(10%), Dex500-(20%), Dex500-(40%), Dex500-(100%)) on the tumor-to-blood ratio. The slope of the linear curve was statistically significant ($p < 0.0001$) in favor of larger amounts of Dex500-(10%) for increased tumor-to-blood ratio, whereas for a certain amount of TCMCs injected, based on 100 μg of Dex500-(10%), a maximum was indicated at a TCMC-to-Dex500 ratio of around 60. It is important to note that these results should not be taken out of their context to produce general conclusions about reagent amounts or TCMC saturation; they are valid only for the applied settings.

Figure 19 shows the tumor-to-blood ratio 24 h after injection of $^{212}$Pb-DOTAM as a function of CA amount (PJRD08-46) and TCMC saturation (9-, 20-, 39-, or 84-to-1). The dashed lines represent linear regression ($R^2 = 0.82$) and nonlinear curve fit ($R^2 = 0.74$) of the respective data.

The final test compared 10 versus 30 μCi of $^{212}$Pb-DOTAM, without injection of antibodies or clearing agents. The content in blood was similar for the two study groups, but 30 μCi resulted in higher tumor accumulation than 10 μCi: 107.74 ± 14.71 versus 72.38 ± 10.83 %ID/g (+ SD, n = 3). The resulting tumor-to-blood ratios were significantly different: 3.20 ± 0.20 versus 2.26 ± 0.25 for 30 and 10 μCi, respectively (unpaired t-test, $p = 0.007$).

Summary and conclusion

In conclusion, 20 μg of Dex500 with 39 TCMCs per molecule (Dex500-(40%)) generated very high accumulation of $^{212}$Pb in pretargeted tumors combined with low retention in blood, 24 hours after administration of $^{212}$Pb-DOTAM.

It was the best performing clearing agent, side-by-side with 10 μg of the previous standard CA, Dex500-(100%),

with 84 TCMCs per molecule. Due to considerable intra-group variability and small sample size, the difference in tumor-to-blood ratio was not statistically different between the two reagents, but the averages indicated that Dex500-(100%) may be favorable. In addition, it was concluded that 30 μCi of $^{212}$Pb-DOTAM may yield higher tumor accumulation than 10 μCi, i.e. saturation was not reached at the lower dose.

**Example 24: Impact on bispecific antibody dose on tumor accumulation (Protocol 91)**

This study aimed to investigate the impact on bispecific antibody dose on tumor accumulation in a subcutaneous tumor model. The hypothesis was that a higher amount of bispecific antibody could more easily saturate the available binding sites on tumor cells, thereby increasing the subsequent uptake of labeled chelate. On the other hand, overly increasing the dose could lead to less efficient accumulation of tumor-associated radioactivity, due to higher levels of circulating bispecific antibodies. Study design, protocol 91

| Study day | Date | Experimental procedure |
|---|---|---|
| 1 | 2016-03-29 | S.c. injection* of BxPC3 cells |
| 6 | 2016-04-03 | I.v. injection* of PRIT bsAb |
| 10 | 2016-04-07 | I.v. injection* of Dex500 |
| 10 | 2016-04-07 | Elution of $^{212}$Pb-DOTAM |
| 10 | 2016-04-07 | I.v. injection* of $^{212}$Pb-DOTAM |
| 11 | 2016-04-08 | Euthanasia and necropsy; gamma counting of tissues |
| *Injection volume 100 μL | | |

Study groups in protocol 91 ($n_{tot}$ = 21)

| Group | A | B | C | D | E | F | G |
|---|---|---|---|---|---|---|---|
| bsAb | PRIT-0165 | PRIT-0165 | PRIT-0165 | PRIT-0156 | PRIT-0156 | PRIT-0156 | PRIT-0175 * |
| bsAb dose (μg) | 30 | 100 | 200 | 30 | 100 | 200 | 100 |
| CA | Dex500 | Dex500 | Dex500 | Dex500 | Dex500 | Dex500 | Dex500 |
| CA dose | 3 | 10 | 20 | 3 | 10 | 20 | 10 |
| (μg) | | | | | | | |
| Chelate | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM | $^{212}$Pb-DOTAM |
| Pb activity (μCi) | 10 | 10 | 10 | 10 | 10 | 10 | 10 |
| n | 3 | 3 | 3 | 3 | 3 | 3 | 3 |
| *Non-CEA-binding control | | | | | | | |

Solid xenografts were established by subcutaneous injection of BxPC3 cells (passage 34) in RPMI medium mixed 1:1 with Corning® Matrigel® basement membrane matrix (growth factor reduced; cat No. 354230). Each mouse (age 7-12 weeks) was injected s.c. with 5×10$^6$ cells in 100 μL RPMI/Matrigel into the right flank. Five days after tumor cell injection, mice were sorted into experimental groups with an average tumor volume of 220 mm$^3$.

Mice were i.v. administered CEA-binding bispecific antibodies PRIT-0165 or PRIT-0156 at a concentration of 30-200 μg per 100 μL, or 100 μg of the non-CEA-binding control antibody PRIT-0175. After four days, all groups were injected i.v. with a clearing agent at a concentration of 3, 10, or 20 μg per 100 μL (1/10 of the injected antibody dose), followed two hours later by 10 μCi of $^{212}$Pb-DOTAM. Mice were sacrificed 24 hours later, and necropsies performed.

Blood, bladder, spleen, kidneys, liver, lung, muscle, tail, and tumor were collected. Samples were weighed and measured for radioactivity, and the %ID/g calculated for each organ.

Results, 91

Pretargeting using 100 μg of either CEA-binding bispecific antibody resulted in significant accumulation of $^{212}$Pb in tumors, as compared with 100 μg of the non-CEA binding control (unpaired t-test, p = 0.027 and 0.008 for comparison with PRIT-0156 and PRIT-0165, respectively). No significant difference was seen in tumor uptake when increasing the antibody dose from 30 to 200 μg for either CEA-binding construct. However, the overall radioactivity levels were slightly elevated in most normal tissues for antibody doses above 30 ug. Importantly, the %ID/g in blood increased significantly for each dose increment, except between 100 and 200 μg of PRIT-0156 (unpaired t-test, $p<0.05$).

Figure 20 shows distribution of $^{212}$Pb 24 h after injection of radiolabeled DOTAM (%ID/g ± SD, n = 3). Bars with white and grey background represent targeting of T84.66 and CH1A1A, respectively; the black bar represents the non-CEA-binding control.

Summary and conclusion

All three antibody dose levels (30, 100, and 200 μg) resulted in high, specific uptake of radioactivity in tumors for both tested bispecific constructs. The lack of difference in tumor accumulation with increased dose indicated that the binding sites were already saturated at the lower dosage, or that the time between PRIT administration and clearing agent injection (four days) was too short to allow further tumor accumulation. As expected, the radioactivity levels in blood were increased with increasing dose, slightly narrowing the therapeutic window of the treatment.

**Example 25: Efficacy of CEA-PRIT (Protocol 93 (a, b))**

In this study, the efficacy of CEA-PRIT using two clinical bsAb candidates (PRIT-0213 and PRIT-0214) was assessed in two parallel subcutaneous tumor models: BxPC3 and LS174T. The bispecific antibodies were compared side-by-side in single- and double-cycle treatment regimens.

Study design, protocol 93a (BxPC3)

| Study day | Date | Experimental procedure |
|---|---|---|
| 1 | 2016-04-13 | I.v. injection* of BxPC3 cells |
| 13 | 2016-04-25 | I.v. injection* of PRIT bsAb |
| 17 | 2016-04-29 | I.v. injection* of CA |
| 17 | 2016-04-29 | Elution of $^{212}$Pb-DOTAM |
| 17 | 2016-04-29 | I.v. injection* of $^{212}$Pb-DOTAM |
| 18 | 2016-04-30 | Euthanasia and necropsy; gamma counting of tissues |
| 41 | 2016-05-23 | I.v. injection* of PRIT bsAb |
| 45 | 2016-05-24 | I.v. injection* of CA |
| 45 | 2016-05-24 | Elution of $^{212}$Pb-DOTAM |
| 45 | 2016-05-24 | I.v. injection* of $^{212}$Pb-DOTAM |
| 46 | 2016-05-25 | Euthanasia and necropsy; gamma counting of tissues |
| *Injection volume 100 μL | | |

Study design, protocol 93b (LS174T)

| Study | Date | Experimental procedure |
|---|---|---|
| | | |
| day | | |
| 1 | 2016-04-25 | I.v. injection* of LS174T cells |
| 5 | 2016-04-29 | I.v. injection* of PRIT bsAb |
| 9 | 2016-05-03 | I.v. injection* of CA |
| 9 | 2016-05-03 | Elution of $^{212}$Pb-DOTAM |
| 9 | 2016-05-03 | I.v. injection* of $^{212}$Pb-DOTAM |
| 10 | 2016-05-04 | Euthanasia and necropsy; gamma counting of tissues |
| 33 | 2016-05-27 | I.v. injection* of PRIT bsAb |
| 37 | 2016-05-31 | I.v. injection* of CA |
| 37 | 2016-05-31 | Elution of $^{212}$Pb-DOTAM |
| 37 | 2016-05-31 | I.v. injection* of $^{212}$Pb-DOTAM |
| 38 | 2016-06-01 | Euthanasia and necropsy; gamma counting of tissues |
| *Injection volume 100 μL | | |

Groups A-G represent the treated mice that were followed up for assessment of efficacy, whereas groups H-L comprise mice that were sacrificed for biodistribution purposes 24 h after their last $^{212}$Pb-DOTAM injection.

Study groups in protocol 93a (BxPC3; $n_{tot}$ = 71)

| Group | bsAb | bsAb dose (μg) | CA | CA dose (μg) | Chelate | Pb activi ty (μCi) | Cycle s | n |
|---|---|---|---|---|---|---|---|---|
| A | PRIT-0213 | 100 | Dex500 | 25 | $^{212}$Pb-DOTAM | 30/10 | 2 | 8 |
| B | PRIT-0213 | 100 | Dex500 | 25 | $^{212}$Pb-DOTAM | 30/30 | 2 | 8 |
| C | PRIT-0214 | 100 | Dex500 | 25 | $^{212}$Pb-DOTAM | 30/10 | 2 | 8 |
| D | PRIT-0214 | 100 | Dex500 | 25 | $^{212}$Pb-DOTAM | 30/30 | 2 | 8 |
| E | PRIT-0175 | 100 | Dex500 | 25 | $^{212}$Pb-DOTAM | 30/30 | 2 | 8 |
| F | - | 0 | - | 0 | $^{212}$Pb-DOTAM | 30/30 | 2 | 8 |
| G | - | 0 | - | 0 | - | 0 | 2 | 8 |
| H | PRIT-0213 | 100 | Dex500 | 25 | $^{212}$Pb-DOTAM | 30 | 1 | 3 |
| I | PRIT- | 100 | Dex500 | 25 | $^{212}$Pb-DOTAM | 30/30 | 2 | 3 |
| | 0213 | | | | | | | |

(continued)

| Group | bsAb | bsAb dose ($\mu$g) | CA | CA dose ($\mu$g) | Chelate | Pb activi ty ($\mu$Ci) | Cycle s | n |
|---|---|---|---|---|---|---|---|---|
| J | PRIT-0214 | 100 | Dex500 | 25 | $^{212}$Pb-DOTAM | 30 | 1 | 3 |
| K | PRIT-0214 | 100 | Dex500 | 25 | $^{212}$Pb-DOTAM | 30/30 | 2 | 3 |
| L | PRIT-0175 | 100 | Dex500 | 25 | $^{212}$Pb-DOTAM | 30 | 1 | 3 |

Study groups in protocol 93b (LS174T; $n_{tot}$ = 71)

| Group | bsAb | bsAb dose ($\mu$g) | CA | CA dose ($\mu$g) | Chelate | Pb activi ty ($\mu$Ci) | Cycle s | n |
|---|---|---|---|---|---|---|---|---|
| A | PRIT-0213 | 100 | Dex500 | 25 | $^{212}$Pb-DOTAM | 30 | 1 | 8 |
| B | PRIT-0213 | 100 | Dex500 | 25 | $^{212}$Pb-DOTAM | 30/30 | 2 | 8 |
| C | PRIT-0214 | 100 | Dex500 | 25 | $^{212}$Pb-DOTAM | 30 | 1 | 8 |
| D | PRIT-0214 | 100 | Dex500 | 25 | $^{212}$Pb-DOTAM | 30/30 | 2 | 8 |
| E | PRIT-0175 | 100 | Dex500 | 25 | $^{212}$Pb-DOTAM | 30/30 | 2 | 8 |
| F | - | 0 | - | 0 | $^{212}$Pb-DOTAM | 30/30 | 2 | 8 |
| G | - | 0 | - | 0 | - | 0 | 2 | 8 |
| H | PRIT-0213 | 100 | Dex500 | 25 | $^{212}$Pb-DOTAM | 30 | 1 | 3 |
| I | PRIT-0213 | 100 | Dex500 | 25 | $^{212}$Pb-DOTAM | 30/30 | 2 | 3 |
| J | PRIT-0214 | 100 | Dex500 | 25 | $^{212}$Pb-DOTAM | 30 | 1 | 3 |
| K | PRIT-0214 | 100 | Dex500 | 25 | $^{212}$Pb-DOTAM | 30/30 | 2 | 3 |
| L | PRIT-0175 | 100 | Dex500 | 25 | $^{212}$Pb-DOTAM | 30 | 1 | 3 |

Solid xenografts were established by subcutaneous injection of cells in RPMI (BxPC3) or DMEM (LS174T) media mixed 1:1 with Corning® Matrigel® basement membrane matrix (growth factor reduced; cat No. 354230).

For protocol 93a, mice (age 7 weeks) were injected s.c. into the right flank with $5\times10^6$ BxPC3 cells (passage 33) in 100 $\mu$L RPMI/Matrigel. Twelve days after tumor cell injection, mice were sorted into experimental groups with an average tumor volume of 235 mm$^3$.

For protocol 93b, mice (age 9 weeks) were injected s.c. into the right flank with $1\times10^6$ LS174T cells (passage 26) in 100 $\mu$L DMEM/Matrigel. Three days after tumor cell injection, mice were sorted into experimental groups with an average tumor volume of 150 mm$^3$.

Each treatment cycle started with injection of humanized bsAbs (PRIT-0213, PRIT-0214, or PRIT-0175). After four days, the clearing agent (Dex500) was administered, followed two hours later by $^{212}$Pb-DOTAM. To minimize

reingesLion of radioactive urine/feces after treatment cycle 1, cages were changed 4 hours after $^{212}$Pb-DOTAM administration, and then again at 24 h p.i. For cycle 2, mice were placed in cages with grilled floors for 4 hours after $^{212}$Pb-DOTAM administration, before being transferred to new cages with standard bedding. All cages were then changed at 24 h p.i., as after cycle 1.

The tumor development in the mice was followed through repeated calipering three times a week. If needed, additional calipering was performed in the gaps between scheduled measurements. The body weight of the animals was measured repeatedly in the same fashion. Mice whose tumor volume reached 3000 mm$^3$ were immediately euthanized. Other factors taken into account for euthanasia for ethical reasons were body weight loss, tumor status (e.g. ulceration) and general appearance of the animal. Wet food was provided to all animals starting from five days after the radioactive injection if mandated by an acute loss of body weight (collective or individual) due to radiation-induced toxicity, until all individuals had recovered sufficiently.

The following organs and tissues were harvested from groups A-G at the time of euthanasia: bladder, ovaries, liver, spleen, kidneys, femur (including bone marrow), colon, jejunum, stomach, and tumor. Unexpected or abnormal conditions were noted and photographed. Tissues were immediately put in 10% neutral buffered formalin (4°C) and then transferred to PBS (4°C) after 5 days. The formalin-fixed samples were then shipped to Roche Pharma Research and Early Development, Roche Innovation Center Basel, for further processing and analysis.

Mice in groups H, J, and L were sacrificed and necropsied 24 hours after their first and only injection of $^{212}$Pb-DOTAM; groups I and K were sacrificed and necropsied 24 hours after their second $^{212}$Pb-DOTAM injection. The following organs and tissues were harvested: blood, bladder, small intestine, colon, spleen, pancreas, kidneys, liver, lung, heart, femoral bone, muscle, tail, and tumor. Collected samples were weighed and measured for radioactivity, and the percent injected dose per gram of tissue (%ID/g) subsequently calculated, including corrections for decay and background.

Results, 93a

The results after treatment cycle 1 corresponded well with previously achieved tissue uptakes in this model, with high tumor accumulation (25.0 + 9.7 and 19.5 ± 6.4 %ID/g for PRIT-0213 and PRIT-0214, respectively) and low accumulation in normal tissues. However, cycle 2 resulted in a different $^{212}$Pb distribution profile, with increased radioactive content in all collected tissues.

Figure 21 shows Radioactivity distribution in selected tissues 24 h after injection of $^{212}$Pb-DOTAM (%ID/g ± SEM, n = 3), for treatment cycles 1 and 2 in the BxPC3 model.

All mice that were injected with 30 μCi of $^{212}$Pb-DOTAM experienced an initial drop in body weight that was mitigated by day 10 after the first radioactive injection. Groups that were administered a second 30-μCi injection suffered a more pronounced, acute weight loss, likely due to the slower clearance of radioactivity and resulting accumulation of $^{212}$Pb in normal tissues observed in the associated cycle-2 biodistribution. No such effect was observed for the groups that received a second cycle of 10 μCi, after which the weight loss was moderate.

Figure 22 shows average body weights in groups A-G (n = 8) after CEA-PRIT in the BxPC3 model. Curves were truncated at the first death in each group. Dotted vertical lines indicate $^{212}$Pb-DOTAM administration for some or all groups, according to the study design.

Figure 23 shows average weight change in groups A-G (n = 8) after CEA-PRIT in the BxPC3 model, expressed as the percentage of initial body weight. Curves were truncated at the first death in each group. Dotted vertical lines indicate $^{212}$Pb-DOTAM administration for some or all groups, according to the study design.

All groups doubled their tumor volume by day 14-19 compared with baseline. The first $^{212}$Pb-DOTAM treatment was given on day 16, after which tumors in the PRIT-0213 and PRIT-0214 groups continued to grow for about a week before exhibiting a regression in volume. No tumors were completely regressed, but the volumes remained relatively constant at the low level until day 40-47, when they once again reached the double volume compared with baseline. The second 30-μCi $^{212}$Pb-DOTAM injection was administered at this point (day 44), which, as previously discussed, resulted in acute weight loss and subsequent euthanasia for most affected mice within the following week. Mice that were given a second treatment cycle with 10 instead of 30 μCi of $^{212}$Pb-DOTAM (day 55) experienced less radiation-induced toxicity. The 10-pCi injection resulted in a second phase of tumor regression, lasting between approximately days 58 and 75. PRIT with the nonspecific bsAb resulted in significant but limited tumor growth inhibition compared with $^{212}$Pb-DOTAM alone or PBS. Means comparisons using Dunnet's method revealed that all treatment regimens using PRIT-0213 and PRIT-0214 resulted in equal treatment-to-control ratios, all significantly different compared with either control group from day 23-26 and onwards.

On day 47, the last day on which all treatment groups were still represented, the tumor growth inhibition (TGI) was 87.3, 88.8, 84.1, 88.7, 57.5, and 15.8% for groups "PRIT-0213, 30 + 10 μCi", "PRIT-0213, 30 + 30 μCi", "PRIT-0214, 30 + 10 μCi", "PRIT-0214, 30 + 30 μCi", "PRIT-0175, 30 + 30 μCi", and "DOTAM alone, 30 + 30 μCi", respectively, compared with PBS. The last mouse in the vehicle control group was accounted for on day 66, at which time the TGI was 87.8, 84.5, and 87.3% for the three remaining groups: "PRIT-0213, 30 + 10 μCi", "PRIT-0214, 30 + 10 μCi", and

"PRIT-0214, 30 + 30 μCi", respectively. A total of six mice survived until the end of the experiment (day 84): one from group "PRIT-0213, 30 + 10 μCi", three from group "PRIT-0214, 30 + 10 μCi", and two from group "PRIT-0214, 30 + 30 μCi".

Figure 24 shows tumor growth averages with standard error for groups A-G in the BxPC3 model (n=8). Curves were truncated at the first death in each group. Dotted vertical lines indicate $^{212}Pb$-DOTAM administration for some or all groups, according to the study design.

Figure 25 shows individual tumor growth curves for groups A-G in the BxPC3 model. The dotted vertical lines indicate administration of $^{212}Pb$-DOTAM.

Figure 26 shows Kaplan-Meier curves showing the survival in groups A-G in the BxPC3 model (n=8). The dotted vertical lines indicate administration of $^{212}Pb$-DOTAM.

Pairwise tests were performed to specify which groups were significantly different in terms of survival: the Log-Rank test (more weight on later survival events), and the Wilcoxon test (more weight on early survival times), both using Bonferroni correction for multiple testing. Due to the radiation-induced toxicity, "PRIT-0213, 30 + 30 μCi" performed equal to or worse than the control groups. The corresponding PRIT-0214 treatment achieved slightly better results, increasing survival compared with PBS and the non-specific antibody. The two groups with 10 μCi of $^{212}Pb$-DOTAM in the second cycle significantly increased the survival compared with all groups, except for each other and "PRIT-0214, 30 + 30 μCi".

Pairwise Log-Rank Test (multiple test level = 0.00238)

| Group | Vehicle (PBS) | DOTAM alone 30 + 30 μCi | PRIT- 0175 30 + 30 μCi | PRIT-0213 30 + 10 μCi | PRIT- 0213 30 + 30 μCi | PRIT-0214 30 + 10 μCi | PRIT-0214 30 + 30 μCi |
|---|---|---|---|---|---|---|---|
| Vehicle (PBS) | 1.0000 | 0.5271 | 0.9420 | 0.0002* | 0.6178 | 0.0003* | 0.0425* |
| DOTAM alone 30 + 30 μCi | 0.5271 | 1.0000 | 0.0032* | 0.0001* | 0.8474 | <.0001* | 0.0685 |
| PRIT-0175 30 + 30 μCi | 0.9420 | 0.0032* | 1.0000 | 0.0001* | 0.0007* | 0.0001* | 0.0001* |
| PRIT-0213 30 + 10 μCi | 0.0002* | 0.0001* | 0.0001* | 1.0000 | <.0001* | 0.6377 | 0.2894 |
| PRIT-0213 30 + 30 μCi | 0.6178 | 0.8474 | 0.0007* | <.0001* | 1.0000 | <.0001* | 0.0302* |
| PRIT-0214 30 + 10 μCi | 0.0003* | <.0001* | 0.0001* | 0.6377 | <.0001* | 1.0000 | 0.1392 |
| PRIT-0214 30 + 30 | 0.0425* | 0.0685 | 0.0001* | 0.2894 | 0.0302* | 0.1392 | 1.0000 |

Pairwise Log-Rank Test (multiple test level = 0.00238)

| Group | Vehicle (PBS) | alone 30 + 30 μCi | PRIT-0175 30 + 30 μCi | PRIT-0213 30 + 10 μCi | PRIT-0213 30 + 30 μCi | PRIT-0214 30 + 10 μCi | PRIT-0214 30 + 30 μCi |
|---|---|---|---|---|---|---|---|
| μCi | | | | | | | |

Pairwise Wilcoxon Test (multiple test level = 0.00238)

| Group | Vehicle (PBS) | DOTAM alone 30 + 30 μCi | PRIT- 0175 30 + 30 μCi | PRIT-0213 30 + 10 μCi | PRIT-0213 30 + 30 μCi | PRIT-0214 30 + 10 μCi | PRIT-0214 30 + 30 μCi |
|---|---|---|---|---|---|---|---|
| Vehicle (PBS) | 1.0000 | 0.1439 | 0.6563 | 0.0004* | 0.1290 | 0.0006* | 0.0219* |
| DOTAM alone 30 + 30 μCi | 0.1439 | 1.0000 | 0.0042* | 0.0004* | 0.8704 | 0.0002* | 0.0827 |
| PRIT-0175 30 + 30 μCi | 0.6563 | 0.0042* | 1.0000 | 0.0002* | 0.0008* | 0.0002* | 0.0002* |
| PRIT-0213 30 + 10 μCi | 0.0004* | 0.0004* | 0.0002* | 1.0000 | 0.0002* | 0.7489 | 0.0790 |
| PRIT-0213 30 + 30 μCi | 0.1290 | 0.8704 | 0.0008* | 0.0002* | 1.0000 | 0.0002* | 0.0648 |
| PRIT-0214 30 + 10 μCi | 0.0006* | 0.0002* | 0.0002* | 0.7489 | 0.0002* | 1.0000 | 0.0350* |
| PRIT-0214 30 + 30 μCi | 0.0219* | 0.0827 | 0.0002* | 0.0790 | 0.0648 | 0.0350* | 1.0000 |

Results, 93b

Both treatment cycles resulted in high $^{212}Pb$ tumor accumulation and low $^{212}Pb$ accumulation in normal tissues. The tumor uptake after cycle 1 was 30.9 + 2.9 and 21.4 + 1.9 %ID/g for PRIT-0213 and PRIT-0214, respectively; after cycle 2 the corresponding numbers were 33.2 ± 0.7 and 40.1 + 6.5 %ID/g.

Figure 27 shows radioactivity distribution in selected tissues 24 h after injection of $^{212}Pb$-DOTAM (%ID/g ± SD, n = 3), for treatment cycle 1 and 2 in the LS174T model.

All mice that were injected with $^{212}Pb$-DOTAM experienced a moderate decrease in body weight, similarly after both 30-μCi treatment cycles. Many animals were however prematurely euthanized for ethical reasons because of poor tumor status.

Figure 28 shows average body weights in groups A-G (n = 8) after CEA-PRIT in the LS174T model. Curves were truncated at the first death in each group. Dotted vertical lines indicate $^{212}Pb$-DOTAM administration for some or all groups, according to the study design.

Figure 29 shows average weight change in groups A-G (n = 8) after CEA-PRIT in the LS174T model, expressed as the percentage of initial body weight. Curves were truncated at the first death in each group. Dotted vertical lines indicate $^{212}Pb$-DOTAM administration for some or all groups, according to the study design.

The first $^{212}Pb$-DOTAM treatment was given on day 8. No tumors regressed compared with baseline, but the PRIT-0213 and PRIT-0214 group averages remained relatively constant, increasing very slowly. The second 30-μCi $^{212}Pb$-DOTAM injection was administered on day 36; however, due to rapid tumor growth in the control groups and the previously discussed problem with poor tumor status across all groups, many mice were already euthanized by that time. Those that did receive a second 30-μCi injection of $^{212}Pb$-DOTAM regained or retained their tumor control, but no tumors regressed completely. Means comparisons using Dunnet's method revealed that all treatment regimens using PRIT-0213 and PRIT-0214 resulted in significantly different treatment-to-control ratios compared with the PBS group, starting from day 14. Also the PRIT-0175 and DOTAM controls differed significantly from the vehicle control, starting from day 16 and 22, respectively.

On day 22, the last day on which all treatment groups were still represented, the TGI was 83.1, 88.8, 87.5, 91.0, 53.0, and 64.7% for groups "PRIT-0213, 30 μCi", "PRIT-0213, 30 + 30 μCi", "PRIT-0214, 30 μCi", "PRIT-0214, 30 + 30 μCi", "PRIT-0175, 30 + 30 μCi", and "DOTAM alone, 30 + 30 μCi", respectively, compared with PBS. The last mouse in the vehicle control group was accounted for on day 30, at which time the TGI was 92.5, 89.5, 90.8, 92.6, and 79.9% for the remaining groups "PRIT-0213, 30 μCi", "PRIT-0213, 30 + 30 μCi", "PRIT-0214, 30 μCi", "PRIT-0214, 30 + 30 μCi", and "PRIT-0175, 30 + 30 μCi", respectively. Two mice survived until the end of the experiment (day 101), both from group "PRIT-0214, 30 + 30 μCi".

Figure 30 shows tumor growth averages with standard error for groups A-G (n=8) in the LS174T model. Curves were truncated at the first death in each group. Dotted vertical lines indicate $^{212}Pb$-DOTAM administration for some or

all groups, according to the study design.

Figure 31 shows individual tumor growth curves for groups A-G in the LS174T model. The dotted vertical lines indicate administration of $^{212}$Pb-DOTAM.

Figure 32 shows Kaplan-Meier curves showing the survival in groups A-G in the LS174T model (n=8). The dotted vertical lines indicate administration of $^{212}$Pb-DOTAM.

The Log-Rank and Wilcoxon tests were performed with Bonferroni correction, to specify which groups were significantly different in terms of survival. The key findings were that "PRIT-0214, 30 + 30 μCi" significantly increased the survival compared with all groups except "PRIT-0213, 30 μCi". The overall survival in group "PRIT-0213, 30 + 30 μCi" was, however, not significantly different from that of "PRIT-0213, 30 μCi", thus differing only slightly from that of "PRIT-0214, 30 + 30 μCi".

Pairwise Log-Rank Test (multiple test level = 0.00238)

| Group | Vehicle (PBS) | DOTAM alone 30 + 30 μCi | PRIT-0175 30 + 30 μCi | PRIT-0213 30 + 30 μCi | PRIT-0213 30 μCi | PRIT-0214 30 + 30 μCi | PRIT-0214 30 μCi |
|---|---|---|---|---|---|---|---|
| Vehicle (PBS) | 1.0000 | 0.0034* | 0.4728 | 0.0002* | <. 0001* | <.0001* | 0.0399* |
| DOTAM alone 30 + 30 μCi | 0.0034* | 1.0000 | 0.0090* | <.0001* | <. 0001* | <.0001* | 0.0091* |
| PRIT-0175 30 + 30 μCi | 0.4728 | 0.0090* | 1.0000 | 0.0051* | <. 0001* | <.0001* | 0.3974 |
| PRIT-0213 | 0.0002* | <.0001* | 0.0051* | 1.0000 | 0.9883 | 0.0353* | 0.0357* |

Pairwise Log-Rank Test (multiple test level = 0.00238)

| Group | Vehicle (PBS) | DOTAM alone 30 + 30 μCi | PRIT-0175 30 + 30 μCi | PRIT-0213 30 + 30 μCi | PRIT-0213 30 μCi | PRIT-0214 30 + 30 μCi | PRIT-0214 30 μCi |
|---|---|---|---|---|---|---|---|
| 30 + 30 μCi | | | | | | | |
| PRIT-0213 30 μCi | <.0001* | <.0001* | <.0001* | 0.9883 | 1.0000 | 0.1428 | 0.0002* |
| PRIT-0214 30 + 30 μCi | <.0001* | <.0001* | <.0001* | 0.0353* | 0.1428 | 1.0000 | <.0001* |
| PRIT-0214 30 μCi | 0.0399* | 0.0091* | 0.3974 | 0.0357* | 0.0002* | <.0001* | 1.0000 |

Pairwise Wilcoxon Test (multiple test level = 0.00238)

| Group | Vehicle (PBS) | DOTAM alone 30 + 30 μCi | PRIT-0175 30 + 30 μCi | PRIT-0213 30 + 30 μCi | PRIT-0213 30 μCi | PRIT-0214 30 + 30 μCi | PRIT-0214 30 μCi |
|---|---|---|---|---|---|---|---|
| Vehicle (PBS) | 1.0000 | 0.0111* | 0.8734 | 0.0005* | 0.0002* | 0.0002* | 0.1330 |
| DOTAM alone 30 + 30 μCi | 0.0111* | 1.0000 | 0.0417* | 0.0002* | 0.0002* | 0.0002* | 0.0160* |

(continued)

| Group | Vehicle (PBS) | DOTAM alone 30 + 30 μCi | PRIT-0175 30 + 30 μCi | PRIT-0213 30 + 30 μCi | PRIT-0213 30 μCi | PRIT-0214 30 + 30 μCi | PRIT-0214 30 μCi |
|---|---|---|---|---|---|---|---|
| PRIT-0175 30 + 30 μCi | 0.8734 | 0.0417* | 1.0000 | 0.0047* | 0.0002* | 0.0002* | 0.3994 |
| PRIT-0213 30 + 30 μCi | 0.0005* | 0.0002* | 0.0047* | 1.0000 | 0.5630 | 0.0193* | 0.0541 |
| PRIT-0213 30 μCi | 0.0002* | 0.0002* | 0.0002* | 0.5630 | 1.0000 | 0.1100 | 0.0004* |
| PRIT-0214 30 + 30 μCi | 0.0002* | 0.0002* | 0.0002* | 0.0193* | 0.1100 | 1.0000 | 0.0002* |
| PRIT-0214 30 μCi | 0.1330 | 0.0160* | 0.3994 | 0.0541 | 0.0004* | 0.0002* | 1.0000 |

Summary and conclusion

CEA-PRIT using either of the two bispecific antibodies PRIT-0213 and PRIT-0214, with one or two treatment cycles, resulted in significant tumor growth inhibition and increase in survival for both studied tumor models. The troubleshooting efforts following the unexpected second-cycle $^{212}$Pb biodistribution and subsequent radiation-induced toxicity in the BxPC3 study concluded that the situation was likely caused by an unidentified injection-related issue. The BxPC3 efficacy study should be repeated to confirm that the problem is not inherent in the treatment regimen. Future experiments in the LS174T model should not be performed until the problem of poor tumor status is resolved. To increase the efficiency of the second treatment cycle, it is proposed that the timing between the two cycles is shortened in order to avoid tumor regrowth. In addition, adding a third treatment cycle to the regimen could also be an option for further evaluation.

Finally, studies of the underlying mechanisms behind the tumor growth are of great interest to clarify why, although their growth is clearly inhibited, the tumors do not regress completely, but instead start to regrow after a certain amount of time.

**Example 26: Clearing agent doses (Protocol 105)**

In this study, a range of doses of the dextran-500-based clearing agent with 50% TCMC saturation was assessed in terms of effect on $^{212}$Pb-DOTAM association to pretargeted tumors.

Study design, protocol 105

| Study day | Date | Experimental procedure |
|---|---|---|
| 1 | 2016-08-30 | I.v. injection* of BxPC3 cells |
| 13 | 2016-09-12 | I.v. injection* of PRIT bsAb |
| 16 | 2016-09-15 | I.v. injection* of CA or PBS |
| 16 | 2016-09-15 | Elution of $^{212}$Pb-DOTAM |
| 16 | 2016-09-15 | I.v. injection* of $^{212}$Pb-DOTAM |
| 17 | 2016-09-16 | Euthanasia and necropsy; gamma counting of tissues |
| *Injection volume 100 μL | | |

Study groups in protocol 105 ($n_{tot}$ = 18)

| Group | bsAb | bsAb | CA | CA dose | Chelate | Pb | n |
|---|---|---|---|---|---|---|---|
| | | | | | | | |
| | | dose (μg) | | (μg) | | activity (μCi) | |
| A | CEA-DOTAM | 100 | Dex500-(50°) | 5 | $^{212}Pb$-DOTAM | 10 | 3 |
| B | CEA-DOTAM | 100 | Dex500- (50%) | 10 | $^{212}Pb$-DOTAM | 10 | 3 |
| C | CEA-DOTAM | 100 | Dex500- (50%) | 30 | $^{212}Pb$-DOTAM | 10 | 3 |
| D | CEA-DOTAM | 100 | Dex500- (50%) | 75 | $^{212}Pb$-DOTAM | 10 | 3 |
| E | CEA-DOTAM | 100 | Dex500- (50%) | 250 | $^{212}Pb$-DOTAM | 10 | 3 |
| F | CEA-DOTAM | 100 | - | 0 | $^{212}Pb$-DOTAM | 10 | 3 |

Solid xenografts were established by subcutaneous injection of BxPC3 cells (passage 26) in RPMI medium mixed 1:1 with Corning® Matrigel® basement membrane matrix (growth factor reduced; cat No. 354230). Each mouse (age 6 weeks) was injected s.c. with $5 \times 10^6$ cells in 100 μL RPMI/Matrigel into the right flank. Fifteen days after tumor cell injection, mice were sorted into experimental groups with an average tumor volume of 222 $mm^3$.

CEA-PRIT (100 μg per 100 μL) was administered i.v., followed three days later by Dex500-(50%) (5-250 μg per 100 μL). Group F received PBS instead of clearing agent. Two hours later, mice were injected i.v. with 100 μL of $^{212}Pb$-DOTAM (10 μCi).

Mice were sacrificed and necropsied 24 hours after injection of $^{212}Pb$-DOTAM. Blood, bladder, spleen, kidneys, liver, lung, muscle, tail, and tumors were collected, weighed and measured for radioactivity content, and the %ID/g subsequently calculated.

Results, 105

The tumor accumulation of $^{212}Pb$ was generally high for all CA doses, except 250 μg. The blood clearance of the CEA-PRIT bispecific antibody was most efficient for CA doses of 30, 75, and 250 μg. Consequently, the highest tumor-to blood ratios were achieved for 30, 75, and 250 μg of CA: 187.7, 180.2, and 243.5, respectively. The corresponding tumor uptake of $^{212}Pb$ was 91.8 ± 18.9, 70.5 ± 11.1, and 35.2 + 17.0 %ID/g (±SD, n=3).

Figure 33 shows radioactivity distribution in selected tissues 24 h after injection of $^{212}Pb$-DOTAM (%ID/g + SD, n = 3). The grey bars represent the tissue accumulation after injection of various amounts of Dex500-(50%) CA; the black bar represents the no-CA control.

Figure 34 shows $^{212}Pb$ content in blood and tumors 24 h after injection of $^{212}Pb$-DOTAM (%ID/g ± SD, n = 3), and the corresponding tumor-to-blood ratios. The grey bars represent the tissue accumulation after injection of various amounts of the Dex500-(50%) CA; the black bar represents the no-CA control.

Summary and conclusion

Based on the achieved $^{212}Pb$ accumulation in tumors, the blood clearance, and the subsequent tumor-to-blood ratio, a dose of 30 μg of Dex500-(50%) appeared favorable for CEA-PRIT in the BxPC3 model.

**Example 27: Residence time in blood of dextran-500-based clearing agent (Protocol 106)**

In this study, the dextran-500-based clearing agent with 50% TCMC saturation was assessed in terms of residence time in blood. The aim was to identify a suitable time frame for repeated treatments (1-4 weeks), ensuring that little or no clearing agent remain in circulation that could bind to administered bispecific antibodies, effectively blocking binding of subsequently injected radiolabeled DOTAM.

Study design, protocol 106

| Study day | Date | Experimental procedure |
|---|---|---|
| 1 | 2016-09-19 | I.v. injection* of PRIT bsAb |
| 2 | 2016-09-12 | I.v. injection* of CA or PBS |
| 9 | 2016-09-27 | I.v. injection* of PRIT bsAb (1 week) |
| 10 | 2016-09-28 | Elution of $^{212}$Pb-DOTAM |
| 10 | 2016-09-28 | I.v. injection* of $^{212}$Pb-DOTAM (1 week) |
| 10 | 2016-09-28 | Euthanasia and blood sampling + gamma counting (1 week) |
| 16 | 2016-10-04 | I.v. injection* of PRIT bsAb (2 weeks) |
| 17 | 2016-10-05 | Elution of $^{212}$Pb-DOTAM |
| 17 | 2016-10-05 | I.v. injection* of $^{212}$Pb-DOTAM (2 weeks) |
| 17 | 2016-10-05 | Euthanasia and blood sampling + gamma counting (2 weeks) |
| 23 | 2016-10-11 | I.v. injection* of PRIT bsAb (3 weeks) |
| 24 | 2016-10-12 | Elution of $^{212}$Pb-DOTAM |
| 24 | 2016-10-12 | I.v. injection* of $^{212}$Pb-DOTAM (3 weeks) |
| 24 | 2016-10-12 | Euthanasia and blood sampling + gamma counting (3 weeks) |
| 30 | 2016-10-18 | I.v. injection* of PRIT bsAb (4 weeks) |
| 31 | 2016-10-19 | Elution of $^{212}$Pb-DOTAM |
| 31 | 2016-10-19 | I.v. injection* of $^{212}$Pb-DOTAM (4 weeks) |
| 31 | 2016-10-19 | Euthanasia and blood sampling + gamma counting (4 weeks) |
| *Injection volume 100 μL | | |

Study groups in protocol 106 ($n_{tot}$ = 24)

| Group | bsAb | bsAb dose (μg) | CA | CA dose (μg) | Interv al (week) | Chelate | Pb activi ty (μCi) | n |
|---|---|---|---|---|---|---|---|---|
| A | CEA-DOTAM | 100 | Dex500-(50%) | 25 | 1 | $^{212}$Pb-DOTAM | 10 | 3 |
| B | CEA-DOTAM | 100 | - | 0 | 1 | $^{212}$Pb-DOTAM | 10 | 3 |
| C | CEA-DOTAM | 100 | Dex500-(50%) | 25 | 2 | $^{212}$Pb-DOTAM | 10 | 3 |
| D | CEA-DOTAM | 100 | - | 0 | 2 | $^{212}$Pb-DOTAM | 10 | 3 |
| E | CEA-DOTAM | 100 | Dex500-(50%) | 25 | 3 | $^{212}$Pb-DOTAM | 10 | 3 |
| F | CEA-DOTAM | 100 | - | 0 | 3 | $^{212}$Pb-DOTAM | 10 | 3 |
| G | CEA-DOTAM | 100 | Dex500-(50%) | 25 | 4 | $^{212}$Pb-DOTAM | 10 | 3 |

(continued)

| Group | bsAb | bsAb dose (μg) | CA | CA dose (μg) | Interv al (week) | Chelate | Pb activi ty (μCi) | n |
|---|---|---|---|---|---|---|---|---|
| H | CEA-DOTAM | 100 | - | 0 | 4 | $^{212}$Pb-DOTAM | 10 | 3 |

Mice (9 weeks) were administered i.v. with CEA-PRIT (100 μg per 100 μL), followed one day later by Dex500-(50%) (25 μg per 100 μL). Groups B, D, F, and H received PBS instead of clearing agent. After 1, 2, 3, or 4 weeks mice were re-injected i.v. with CEA-PRIT (100 μg per 100 μL), followed one day later by 100 μL of $^{212}$Pb-DOTAM (10 μCi).

Mice were sacrificed 4 hours after injection of $^{212}$Pb-DOTAM. Blood was collected at the time of euthanasia, and samples were weighed and measured for radioactivity content. The percent injected dose per gram of blood (%ID/g) was subsequently calculated, including corrections for decay and background.

Results, 106

There was no statistically significant difference in average radioactivity content between mice that received Dex500-(50%) or PBS for either of the studied time points (1-way ANOVA, Sidak's multiple comparisons test, $p>0.05$).

Figure 35 shows Radioactivity content in blood 4 h after injection of $^{212}$Pb-DOTAM (%ID/g ± SD, n = 3).

Summary and conclusion

The results show that a repeated CEA-PRIT treatment cycle can be initiated already one week after the last injection of Dex500-(50%), without blocking binding of subsequently administered $^{212}$Pb-DOTAM to CEA-DOTAM bsAb.

**Example 28: Additional formats of bispecific antibodies**

Generation of plasmids for the recombinant expression of antibody heavy or light chains

Desired proteins were expressed by transient transfection of human embryonic kidney cells (HEK 293). For the expression of a desired gene/protein (e.g. full length antibody heavy chain, full length antibody light chain, or a full length antibody heavy chain containing an additional domain (e.g. an immunoglobulin heavy or light chain variable domain at its C-terminus) a transcription unit comprising the following functional elements was used:

- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence (SS),
- a gene/protein to be expressed, and
- the bovine growth hormone polyadenylation sequence (BGH pA).

In addition to the expression unit/cassette including the desired gene to be expressed the basic/standard mammalian expression plasmid contained

- an origin of replication from the vector pUC18 which allows replication of this plasmid in E. coli, and

- a beta-lactamase gene which confers ampicillin resistance in E. coli.

P1AE1766

Antibody heavy chain encoding genes including C-terminal fusion genes comprising a complete and functional antibody heavy chain, followed by an additional antibody VL-CH1 or VH-C-kappa domain was assembled by fusing a DNA fragment coding for the respective sequence elements separated each by a G4Sx4 linker to the C-terminus of the CH3 domain of a human IgG molecule (VH-CH1-hinge-CH2-CH3-linker-VL-CH1 or VH-CH1-hinge-CH2-CH3-linker-VH-Ck). Recombinant antibody molecules bearing one VL-CH1 and one VH-Ck domain at the C-termini of the two CH3 domains, respectively, were expressed using the knob-into-hole technology.

Antibody light chain encoding genes comprising a complete and functional antibody light chain was assembled

by fusing a DNA fragment coding for the respective sequence elements.

P1AE1768

Antibody heavy chain encoding genes comprising a complete and functional antibody heavy chain targeting either CEA or DOTAM, were assembled by using the knob-into-hole technology.
Antibody light chain encoding genes comprising a complete and functional antibody light chain was assembled by fusing a DNA fragment coding for the respective sequence elements. Correct association was ensured using CrossMab technology (swapping the CL and CH1 domains for one of the arms).

P1AE1769

A fusion gene was assembled by fusing a DNA fragment coding for a VH-CH1 domain via a G4Sx4 linker to the N-terminus of the VL domain of a human IgG molecule containing an exchange of the VL domain for the VH domain (thus, making a fusion gene encoding the structure VH-CH1-Linker-VL-CH1-hinge-CH2-CH3). Recombinant antibody molecules bearing one VH-CH1 and no fusion at the N-termini, respectively, were expressed using the knob-into-hole technology.
Antibody VH-C kappa encoding genes comprising a complete and functional antibody light chain was assembled by fusing a DNA fragment coding for the respective sequence elements.
Antibody light chain encoding genes comprising a complete and functional antibody light chain was assembled by fusing a DNA fragment coding for the respective sequence elements.
Correct association was ensured using CrossMab technology.

P1AE1767

Antibody heavy chain encoding genes including C-terminal fusion genes comprising a complete and functional antibody heavy chain, followed by an additional antibody V-light-CH1 domain was assembled by fusing a DNA fragment coding for the respective sequence elements separated each by a G4Sx4 linker to the C-terminus of the CH3 domain of a human IgG molecule (VH-CH1-hinge-CH2-CH3-linker-VL-CH1). Recombinant antibody molecules bearing one VL-CH1 and no fusion at the C-termini of the two CH3 domains, respectively, were expressed using the knob-into-hole technology.
Antibody VH-C kappa encoding genes comprising a complete and functional antibody light chain was assembled by fusing a DNA fragment coding for the respective sequence elements.
Antibody light chain encoding genes comprising a complete and functional antibody light chain was assembled by fusing a DNA fragment coding for the respective sequence elements.
Correct association was ensured using CrossMab technology.

P1AE1770

Antibody heavy chain encoding genes including C-terminal fusion genes comprising a complete and functional antibody heavy chain, followed by an additional antibody V-light-C-Kappa-Linker-V-heavy-CH1 domain was assembled by fusing a DNA fragment coding for the respective sequence elements separated each by a G4Sx4 linker to the C-terminus of the CH3 domain of a human IgG molecule (VH-CH1-hinge-CH2-CH3-linker-VL-Ck-Linker-VH-CH1). Recombinant antibody molecules bearing one single chain Fab and no fusion at the C-termini of the two CH3 domains, respectively, were expressed using the knob-into-hole technology.
Antibody light chain encoding genes comprising a complete and functional antibody light chain was assembled by fusing a DNA fragment coding for the respective sequence elements.

Transient expression of the antibody molecules

The antibody molecules were generated in transiently transfected HEK293 cells (human embryonic kidney cell line 293-derived) cultivated in F17 Medium (Invitrogen Corp.). For transfection "293-Free" Transfection Reagent (Novagen) was used. The respective antibody heavy- and light chain molecules as described above were expressed from individual expression plasmids. Transfections were performed as specified in the manufacturer's instructions. Immunoglobulin-containing cell culture supernatants were harvested three to seven (3-7) days after transfection. Supernatants were stored at reduced temperature (e.g. -80°C) until purification.
General information regarding the recombinant expression of human immunoglobulins in e.g. HEK293 cells is given in: Meissner, P. et al., Biotechnol. Bioeng. 75 (2001) 197-203.

| TapirID | Expression Volume | Yield [mg] | Monomer Content (SEC) |
|---|---|---|---|
| P1AE1766 | 2 l | 2,5 | >98% |
| P1AE1767 | 2 l | 2 | >98% |
| P1AE1768 | 2 l | 20 | >95% |
| P1AE1769 | 2 l | 2 | >96% |
| P1AE1770 | 2 l | 0,4 | >96% |

The molecules have been purified by a MabSelect Sure (Affinity Chromatography) and followed by Superdex 200 (Size Exclusion Chromatography).

Kinexa assessment of the DOTAM binding properties of the different formats

For detailed analysis of affinity determination, Kinexa was used.

*Instrumentation and materials*

A KinExA 3200 instrument from Sapidyne Instruments (Boise, ID) with autosampler was used. Polymethylmethacrylate (PMMA) beads were purchased from Sapidyne, whereas PBS (phosphate buffered saline), BSA (bovine serum albumin fraction V) and the anti-DOTAM antibodies were prepared in-house (Roche). Dylight650®-conjugated affinity-purified goat anti-human IgG-Fc Fragment cross-adsorbed antibody was purchased from Bethyl Laboratories (Montgomery, TX). The biotinylated Pb-DOTAM antigens (Pb-DOTAM-alkyl-biotin isomer A and B, Pb-DOTAM-Bn-biotin / TCMC-Pb-dPEG3-Biotin, isomer A and B) and the non-biotinylated Pb-DOTAM were obtained from AREVA Med (Bethesda, MD) .

*Preparation of antigen coated beads*

PMMA beads were coated according to the KinExA Handbook protocol for biotinylated molecules (Sapidyne). Briefly, first, 10 μg of Biotin-BSA (Thermo Scientific) in 1 ml PBS (pH7.4) was added per vial (200mg) of beads for adsorption coating. After rotating for 2 h at room temperature, the supernatant was removed and beads were washed 5 times with 1 ml PBS. Second, 1 ml of 100 μg of NeutrAvidin Biotin-Binding Protein (Thermo Scientific) in PBS containing 10 mg/ml BSA was added to the beads and incubated at room temperature for additional 2 h to couple NeutrAvidin to the beads and to provide additional biotin binding sites for subsequent binding of biotinylated proteins. The NeutrAvidin-coated-beads were then rinsed 5 times with 1 ml PBS. Finally, the beads were coated with 200 ng/ml biotinylated Pb-DOTAM-Isomer Mix (50 ng for each Isomer) in PBS and incubated for further 2 h at room temperature. Beads were then resuspended in 30 ml PBS and used immediately.

*KinExA equilibrium assays*

All KinExA experiments were performed at room temperature (RT) using PBS pH 7.4 as running buffer. Samples were prepared in running buffer supplemented with 1 mg/ml BSA ("sample buffer"). A flow rate of 0.25 ml/min was used. A constant amount of anti-DOTAM antibody with 5 pM binding site concentration was titrated with Pb-DOTAM antigen by twofold serial dilution starting at 100 pM (concentration range 0.049 pM - 100 pM). One sample of antibody without antigen served as 100% signal (i.e. without inhibition). Antigen-antibody complexes were incubated at RT for at least 24 h to allow equilibrium to be reached. Equilibrated mixtures were then drawn through a column of Pb-DOTAM-coupled beads in the KinExA system at a volume of 5 ml permitting unbound antibody to be captured by the beads without perturbing the equilibrium state of the solution. Captured antibody was detected using 250 ng/ml Dylight 650©-conjugated anti-human Fc-fragment specific secondary antibody in sample buffer. Each sample was measured in duplicates for all equilibrium experiments.

The KD was obtained from non-linear regression analysis of the data using a one-site homogeneous binding model contained within the KinExA software (Version 4.0.11) using the "standard analysis" method. The software calculates the KD and determines the 95% confidence interval by fitting the data points to a theroretical KD curve. The 95% confidence interval (Sapidyne TechNote TN207R0) is given as KD low and KD high.

| Sample | KD ( pM) | Confidence Interval (pM) |
|---|---|---|
| P1AE1766 | 1,4 | 0,4-3 |
| P1AE1767 | 1,0 | 0,8-1,4 |
| P1AE1768 | 0,8 | 0,5-1,3 |
| P1AE1769 | 0,9 | 0,4-1,5 |
| P1AE1770 | 1,3 | 0,9-1,8 |
| PRIT-213 | 1, | 0,6-1,5 |

All constructs are comparable in KD as confidence intervals overlap.

Sequences of molecules in different formats:

Optional charge modifications are shown in bold text and underline: EQ->RK, or KK->EE P1AE1766

>CEA Light Chain RK

DIQMTQSPSSLSASVGDRVTITCKASAAVGTYVAWYQQKPGKAPKLLIYSASYRKRGVPSRFSGSGSGTDFTLTI
SSLQPEDFATYYCHQYYTYPLFTFGQGTKLEIKRTVAAPSVFIFPPSD**<u>RK</u>**LKSGTASVVCLLNNFYPREAKVQWK
VDNALQSGNSQESVTEQDSKDSTYSLSSTLTLSKADYEKHKVYACEVTHQGLSSPVTKSFNRGEC

>CEA Heavy Chain with DOTAM VL / CH1

QVQLVQSGAEVKKPGASVKVSCKASGYTFTEFGMNWVRQAPGQGLEWMGWINTKTGEATYVEEFKGRVTFTTDTS
TSTAYMELRSLRSDDTAVYYCARWDFAYYVEAMDYWGQGTTVTVSSASTKGPSVFPLAPSSKSTSGGTAALGCLV
**<u>E</u>**DYFPEPVTVSWNSGALTSGVHTFPAVLQSSGLYSLSSVVTVPSSSLGTQTYICNVNHKPSNTKVD**<u>E</u>**KVEPKSCD
KTHTCPPCPAPEAAGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALGAPIEKTISKAKGQPREPQVYTLPPCRDELTKNQVSLWCLVKG
FYPSDIAVEWESNGQPENNYKTTPPVLDSDGSFFLYSKLTVDKSRWQQGNVFSCSVMHEALHNHYTQKSLSLSPG
GGGGGSGGGGSGGGGSGGGGSIQMTQSPSSLSASVGDRVTITCQSSHSVYSDNDLAWYQQKPGKAPKLLIYQASK
LASGVPSRFSGSGSGTDFTLTISSLQPEDFATYYCLGGYDDESDTYGFGGGTKVEIKSSASTKGPSVFPLAPSSK
STSGGTAALGCLVKDYFPEPVTVSWNSGALTSGVHTFPAVLQSSGLYSLSSVVTVPSSSLGTQTYICNVNHKPSN
TKVDKKVEPKSC

> CEA Heavy Chain with DOTAM VH / CK

QVQLVQSGAEVKKPGASVKVSCKASGYTFTEFGMNWVRQAPGQGLEWMGWINTKTGEATYVEEFKGRVTFTTDTS
TSTAYMELRSLRSDDTAVYYCARWDFAYYVEAMDYWGQGTTVTVSSASTKGPSVFPLAPSSKSTSGGTAALGCLV
**<u>E</u>**DYFPEPVTVSWNSGALTSGVHTFPAVLQSSGLYSLSSVVTVPSSSLGTQTYICNVNHKPSNTKVD**<u>E</u>**KVEPKSCD
KTHTCPPCPAPEAAGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALGAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVMHEALHNHYTQKSLSLSPG
GGGGGSGGGGSGGGGSGGGGSVTLKESGPVLVKPTETLTLTCTVSGFSLSTYSMSWIRQPPGKALEWLGFIGSRG
DTYYASWAKGRLTISKDTSKSQVVLTMTNMDPVDTATYYCARERDPYGGGAYPPHLWGRGTLVTVSSASVAAPSV
FIFPPSDEQLKSGTASVVCLLNNFYPREAKVQWKVDNALQSGNSQESVTEQDSKDSTYSLSSTLTLSKADYEKHK
VYACEVTHQGLSSPVTKSFNRGEC

P1AE1767

>DOTAM "LC" with VH / CK

VTLKESGPVLVKPTETLTLTCTVSGFSLSTYSMSWIRQPPGKALEWLGFIGSRGDTYYASWAKGRLTISKDTSKS
QVVLTMTNMDPVDTATYYCARERDPYGGGAYPPHLWGRGTLVTVSSASVAAPSVFIFPPSDEQLKSGTASVVCLL

NNFYPREAKVQWKVDNALQSGNSQESVTEQDSKDSTYSLSSTLTLSKADYEKHKVYACEVTHQGLSSPVTKSFNR GEC

> CEA Light Chain RK

DIQMTQSPSSLSASVGDRVTITCKASAAVGTYVAWYQQKPGKAPKLLIYSASYRKRGVPSRFSGSGSGTDFTLTI SSLQPEDFATYYCHQYYTYPLFTFGQGTKLEIKRTVAAPSVFIFPPSD**RK**LKSGTASVVCLLNNFYPREAKVQWK VDNALQSGNSQESVTEQDSKDSTYSLSSTLTLSKADYEKHKVYACEVTHQGLSSPVTKSFNRGEC

> CEA Heavy Chain with DOTAM VL / CH1

QVQLVQSGAEVKKPGASVKVSCKASGYTFTEFGMNWVRQAPGQGLEWMGWINTKTGEATYVEEFKGRVTFTTDTS TSTAYMELRSLRSDDTAVYYCARWDFAYYVEAMDYWGQGTTVTVSSASTKGPSVFPLAPSSKSTSGGTAALGCLV **E**DYFPEPVTVSWNSGALTSGVHTFPAVLQSSGLYSLSSVVTVPSSSLGTQTYICNVNHKPSNTKVD**E**KVEPKSCD KTHTCPPCPAPEAAGGPSVFLFPPKPKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREPQVYTLPPCRDELTKNQVSLWCLVKG FYPSDIAVEWESNGQPENNYKTTPPVLDSDGSFFLYSKLTVDKSRWQQGNVFSCSVMHEALHNHYTQKSLSLSPG GGGGGSGGGGSGGGGSGGGGSIQMTQSPSSLSASVGDRVTITCQSSHSVYSDNDLAWYQQKPGKAPKLLIYQASK LASGVPSRFSGSGSGTDFTLTISSLQPEDFATYYCLGGYDDESDTYGFGGGTKVEIKSSASTKGPSVFPLAPSSK STSGGTAALGCLVKDYFPEPVTVSWNSGALTSGVHTFPAVLQSSGLYSLSSVVTVPSSSLGTQTYICNVNHKPSN TKVDKKVEPKSC

>CEA Heavy Chain

QVQLVQSGAEVKKPGASVKVSCKASGYTFTEFGMNWVRQAPGQGLEWMGWINTKTGEATYVEEFKGRVTFTTDTS TSTAYMELRSLRSDDTAVYYCARWDFAYYVEAMDYWGQGTTVTVSSASTKGPSVFPLAPSSKSTSGGTAALGCLV **E**DYFPEPVTVSWNSGALTSGVHTFPAVLQSSGLYSLSSVVTVPSSSLGTQTYICNVNHKPSNTKVD**E**KVEPKSCD KTHTCPPCPAPEAAGGPSVFLFPPKPKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREPQVCTLPPSRDELTKNQVSLSCAVKG FYPSDIAVEWESNGQPENNYKTTPPVLDSDGSFFLVSKLTVDKSRWQQGNVFSCSVMHEALHNHYTQKSLSLSPG K

P1AE1768

>CEA LC

DIQMTQSPSSLSASVGDRVTITCKASAAVGTYVAWYQQKPGKAPKLLIYSASYRKRGVPSRFSGSGSGTDFTLTI SSLQPEDFATYYCHQYYTYPLFTFGQGTKLEIKRTVAAPSVFIFPPSD**RK**LKSGTASVVCLLNNFYPREAKVQWK VDNALQSGNSQESVTEQDSKDSTYSLSSTLTLSKADYEKHKVYACEVTHQGLSSPVTKSFNRGEC

> DOTAM Heavy Chain with VL / CH1

IQMTQSPSSLSASVGDRVTITCQSSHSVYSDNDLAWYQQKPGKAPKLLIYQASKLASGVPSRFSGSGSGTDFTLT ISSLQPEDFATYYCLGGYDDESDTYGFGGGTKVEIKSSASTKGPSVFPLAPSSKSTSGGTAALGCLVKDYFPEPV TVSWNSGALTSGVHTFPAVLQSSGLYSLSSVVTVPSSSLGTQTYICNVNHKPSNTKVDKKVEPKSCDKTHTCPPC PAPEAAGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALGAPIEKTISKAKGQPREPQVYTLPPCRDELTKNQVSLWCLVKGFYPSDIAV EWESNGQPENNYKTTPPVLDSDGSFFLYSKLTVDKSRWQQGNVFSCSVMHEALHNHYTQKSLSLSPGK

> CEA Heavy chain

QVQLVQSGAEVKKPGASVKVSCKASGYTFTEFGMNWVRQAPGQGLEWMGWINTKTGEATYVEEFKGRVTFTTDTS TSTAYMELRSLRSDDTAVYYCARWDFAYYVEAMDYWGQGTTVTVSSASTKGPSVFPLAPSSKSTSGGTAALGCLV **E**DYFPEPVTVSWNSGALTSGVHTFPAVLQSSGLYSLSSVVTVPSSSLGTQTYICNVNHKPSNTKVD**E**KVEPKSCD KTHTCPPCPAPEAAGGPSVFLFPPKPKDTLMISRTPEVTCVVVDVSHEDPEVKFNWYVDGVEVHNAKTKPREEQY NSTYRVVSVLTVLHQDWLNGKEYKCKVSNKALGAPIEKTISKAKGQPREPQVCTLPPSRDELTKNQVSLSCAVKG FYPSDIAVEWESNGQPENNYKTTPPVLDSDGSFFLVSKLTVDKSRWQQGNVFSCSVMHEALHNHYTQKSLSLSPG K

>DOTAM "LC" VH / CK

VTLKESGPVLVKPTETLTLTCTVSGFSLSTYSMSWIRQPPGKALEWLGFIGSRGDTYYASWAKGRLTISKDTSKS
QVVLTMTNMDPVDTATYYCARERDPYGGGAYPPHLWGRGTLVTVSSASVAAPSVFIFPPSDEQLKSGTASVVCLL
NNFYPREAKVQWKVDNALQSGNSQESVTEQDSKDSTYSLSSTLTLSKADYEKHKVYACEVTHQGLSSPVTKSFNR
GEC

P1AE1769

➢ DOTAM "LC" with VH / CK

VTLKESGPVLVKPTETLTLTCTVSGFSLSTYSMSWIRQPPGKALEWLGFIGSRGDTYYASWAKGRLTISKDTSKS
QVVLTMTNMDPVDTATYYCARERDPYGGGAYPPHLWGRGTLVTVSSASVAAPSVFIFPPSDEQLKSGTASVVCLL
NNFYPREAKVQWKVDNALQSGNSQESVTEQDSKDSTYSLSSTLTLSKADYEKHKVYACEVTHQGLSSPVTKSFNR
GEC

> CEA LC

DIQMTQSPSSLSASVGDRVTITCKASAAVGTYVAWYQQKPGKAPKLLIYSASYRKRGVPSRFSGSGSGTDFTLTI
SSLQPEDFATYYCHQYYTYPLFTFGQGTKLEIKRTVAAPSVFIFPPSD**RK**LKSGTASVVCLLNNFYPREAKVQWK
VDNALQSGNSQESVTEQDSKDSTYSLSSTLTLSKADYEKHKVYACEVTHQGLSSPVTKSFNRGEC

> CEA HC with CEA VH / CH1 / DOTAM VL / CH1

QVQLVQSGAEVKKPGASVKVSCKASGYTFTEFGMNWVRQAPGQGLEWMGWINTKTGEATYVEEFKGRVTFTTDTS
TSTAYMELRSLRSDDTAVYYCARWDFAYYVEAMDYWGQGTTVTVSSASTKGPSVFPLAPSSKSTSGGTAALGCLV
**E**DYFPEPVTVSWNSGALTSGVHTFPAVLQSSGLYSLSSVVTVPSSSLGTQTYICNVNHKPSNTKVD**E**KVEPKSCD
GGGGSGGGGSIQMTQSPSSLSASVGDRVTITCQSSHSVYSDNDLAWYQQKPGKAPKLLIYQASKLASGVPSRFSG
SGSGTDFTLTISSLQPEDFATYYCLGGYDDESDTYGFGGGTKVEIKSSASTKGPSVFPLAPSSKSTSGGTAALGC
LVKDYFPEPVTVSWNSGALTSGVHTFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALGAPIEKTISKAKGQPREPQVYTLPPCRDELTKNQVSLWCLV
KGFYPSDIAVEWESNGQPENNYKTTPPVLDSDGSFFLYSKLTVDKSRWQQGNVFSCSVMHEALHNHYTQKSLSLS
PGK

>CEA HC

QVQLVQSGAEVKKPGASVKVSCKASGYTFTEFGMNWVRQAPGQGLEWMGWINTKTGEATYVEEFKGRVTFTTDTS
TSTAYMELRSLRSDDTAVYYCARWDFAYYVEAMDYWGQGTTVTVSSASTKGPSVFPLAPSSKSTSGGTAALGCLV
**E**DYFPEPVTVSWNSGALTSGVHTFPAVLQSSGLYSLSSVVTVPSSSLGTQTYICNVNHKPSNTKVD**E**KVEPKSCD
KTHTCPPCPAPEAAGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALGAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVMHEALHNHYTQKSLSLSPG
K

P1AE1770

> CEA LC

DIQMTQSPSSLSASVGDRVTITCKASAAVGTYVAWYQQKPGKAPKLLIYSASYRKRGVPSRFSGSGSGTDFTLTI
SSLQPEDFATYYCHQYYTYPLFTFGQGTKLEIKRTVAAPSVFIFPPSD**RK**LKSGTASVVCLLNNFYPREAKVQWK
VDNALQSGNSQESVTEQDSKDSTYSLSSTLTLSKADYEKHKVYACEVTHQGLSSPVTKSFNRGEC

> CEA HC with DOTAM scFab: DOTAM VL / Ck / Linker / VH CH1

QVQLVQSGAEVKKPGASVKVSCKASGYTFTEFGMNWVRQAPGQGLEWMGWINTKTGEATYVEEFKGRVTFTTDTS
TSTAYMELRSLRSDDTAVYYCARWDFAYYVEAMDYWGQGTTVTVSSASTKGPSVFPLAPSSKSTSGGTAALGCLV
**E**DYFPEPVTVSWNSGALTSGVHTFPAVLQSSGLYSLSSVVTVPSSSLGTQTYICNVNHKPSNTKVD**E**KVEPKSCD
KTHTCPPCPAPEAAGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALGAPIEKTISKAKGQPREPQVYTLPPCRDELTKNQVSLWCLVKG
FYPSDIAVEWESNGQPENNYKTTPPVLDSDGSFFLYSKLTVDKSRWQQGNVFSCSVMHEALHNHYTQKSLSLSPG
GGGGGSGGGGSGGGGSGGGGSIQMTQSPSSLSASVGDRVTITCQSSHSVYSDNDLAWYQQKPGKAPKLLIYQASK
LASGVPSRFSGSGSGTDFTLTISSLQPEDFATYYCLGGYDDESDTYGFGGGTKVEIKRTVAAPSVFIFPPSDEQL
KSGTASVVCLLNNFYPREAKVQWKVDNALQSGNSQESVTEQDSKDSTYSLSSTLTLSKADYEKHKVYACEVTHQG
LSSPVTKSFNRGECGGGGSGGGGSGGGGSGGGGSGGGGSGGGGSGGVTLKESGPVLVKPTETLTLTCTVSGFSLS
TYSMSWIRQPPGKALEWLGFIGSRGDTYYASWAKGRLTISKDTSKSQVVLTMTNMDPVDTATYYCARERDPYGGG
AYPPHLWGRGTLVTVSSASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSC

> CEA HC

QVQLVQSGAEVKKPGASVKVSCKASGYTFTEFGMNWVRQAPGQGLEWMGWINTKTGEATYVEEFKGRVTFTTDTS
TSTAYMELRSLRSDDTAVYYCARWDFAYYVEAMDYWGQGTTVTVSSASTKGPSVFPLAPSSKSTSGGTAALGCLV
**E**DYFPEPVTVSWNSGALTSGVHTFPAVLQSSGLYSLSSVVTVPSSSLGTQTYICNVNHKPSNTKVD**E**KVEPKSCD
KTHTCPPCPAPEAAGGPSVFLFPPKPKDTLMISRTPEVTCVVVDVSHEDPEVKFNWYVDGVEVHNAKTKPREEQY
NSTYRVVSVLTVLHQDWLNGKEYKCKVSNKALGAPIEKTISKAKGQPREPQVCTLPPSRDELTKNQVSLSCAVKG
FYPSDIAVEWESNGQPENNYKTTPPVLDSDGSFFLVSKLTVDKSRWQQGNVFSCSVMHEALHNHYTQKSLSLSPG
K

**Example 29: Bispecific antibodies binding to Pb-DOTAM and CD20 or Her2**

Generation of plasmids for the recombinant expression of antibody heavy or light chains

Desired proteins were expressed by transient transfection of human embryonic kidney cells (HEK 293). For the expression of a desired gene/protein (e.g. full length antibody heavy chain, full length antibody light chain, or a full length antibody heavy chain containing an additional domain (e.g. an immunoglobulin heavy or light chain variable domain at its C-terminus) a transcription unit comprising the following functional elements was used:

- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence (SS),
- a gene/protein to be expressed, and
- the bovine growth hormone polyadenylation sequence (BGH pA).

In addition to the expression unit/cassette including the desired gene to be expressed the basic/standard mammalian expression plasmid contained

- an origin of replication from the vector pUC18 which allows replication of this plasmid in E. coli, and

- a beta-lactamase gene which confers ampicillin resistance in E. coli.

a) Expression plasmid for antibody heavy chains

Antibody heavy chain encoding genes including C-terminal fusion genes comprising a complete and functional antibody heavy chain, followed by an additional antibody V-heavy or V-light domain was assembled by fusing a DNA fragment coding for the respective sequence elements (V-heavy or V-light) separated each by a G4Sx4 linker to the C-terminus of the CH3 domain of a human IgG molecule (VH-CH1-hinge-CH2-CH3-linker-VH or VH-CH1-hinge-CH2-CH3-linker-VL). Recombinant antibody molecules bearing one VH and one VL domain at the C-termini of the two CH3 domains, respectively, were expressed using the knob-into-hole technology.

The expression plasmids for the transient expression of an antibody heavy chain with a C-terminal VH or VL domain in HEK293 cells comprised besides the antibody heavy chain fragment with C-terminal VH or VL domain expression cassette, an origin of replication from the vector pUC18, which allows replication of this plasmid in E. coli, and a beta-lactamase gene which confers ampicillin resistance in E. coli. The transcription unit of the antibody heavy chain fragment with C-terminal VH or VL domain fusion gene comprises the following functional elements:

- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- an antibody heavy chain (VH-CH1-hinge-CH2-CH3-linker-VH or VH-CH1-hinge-CH2-CH3-linker-VL) encoding nucleic acid, and
- the bovine growth hormone polyadenylation sequence (BGH pA).

The amino acid sequence of the mature antibody heavy chain fragment with C-terminal VH or VL domain fusion protein is

P1AD9826 → CD20-DOTAM

QVQLVQSGAEVKKPGSSVKVSCKASGYAFSYSWINWVRQAPGQGLEWMGRIFPGDGDTDYNGKFKGRVTITADKS
TSTAYMELSSLRSEDTAVYYCARNVFDGYWLVYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALGAPIEKTISKAKGQPREPQVCTLPPSRDELTKNQVSLSCAVKGFY
PSDIAVEWESNGQPENNYKTTPPVLDSDGSFFLVSKLTVDKSRWQQGNVFSCSVMHEALHNHYTQKSLSLSPGGG
GGSGGGGSGGGGSGGGGSIQMTQSPSSLSASVGDRVTITCQSSHSVYSDNDLAWYQQKPGKAPKLLIYQASKLAS
GVPSRFSGSGSGTDFTLTISSLQPEDFATYYCLGGYDDESDTYGFGGGTKVEIK

QVQLVQSGAEVKKPGSSVKVSCKASGYAFSYSWINWVRQAPGQGLEWMGRIFPGDGDTDYNGKFKGRVTITADKS
TSTAYMELSSLRSEDTAVYYCARNVFDGYWLVYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKT
HTCPPCPAPEAAGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALGAPIEKTISKAKGQPREPQVYTLPPCRDELTKNQVSLWCLVKGFY
PSDIAVEWESNGQPENNYKTTPPVLDSDGSFFLYSKLTVDKSRWQQGNVFSCSVMHEALHNHYTQKSLSLSPGGG
GGSGGGGSGGGGSGGGGSVTLKESGPVLVKPTETLTLTCTVSGFSLSTYSMSWIRQPPGKALEWLGFIGSRGDTY
YASWAKGRLTISKDTSKSQVVLTMTNMDPVDTATYYCARERDPYGGGAYPPHLWGRGTLVTVSS

P1AD9827 → ERBB2-DOTAM

EVQLVESGGGLVQPGGSLRLSCAASGFNIKDTYIHWVRQAPGKGLEWVARIYPTNGYTRYADSVKGRFTISADTS
KNTAYLQMNSLRAEDTAVYYCSRWGGDGFYAMDYWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKKVEPKSCDK
THTCPPCPAPEAAGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALGAPIEKTISKAKGQPREPQVCTLPPSRDELTKNQVSLSCAVKGF
YPSDIAVEWESNGQPENNYKTTPPVLDSDGSFFLVSKLTVDKSRWQQGNVFSCSVMHEALHNHYTQKSLSLSPGG
GGGSGGGGSGGGGSGGGGSIQMTQSPSSLSASVGDRVTITCQSSHSVYSDNDLAWYQQKPGKAPKLLIYQASKLA
SGVPSRFSGSGSGTDFTLTISSLQPEDFATYYCLGGYDDESDTYGFGGGTKVEIK

EVQLVESGGGLVQPGGSLRLSCAASGFNIKDTYIHWVRQAPGKGLEWVARIYPTNGYTRYADSVKGRFTISADTS
KNTAYLQMNSLRAEDTAVYYCSRWGGDGFYAMDYWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNHKPSNTKVDKKVEPKSCDK
THTCPPCPAPEAAGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALGAPIEKTISKAKGQPREPQVYTLPPCRDELTKNQVSLWCLVKGF
YPSDIAVEWESNGQPENNYKTTPPVLDSDGSFFLYSKLTVDKSRWQQGNVFSCSVMHEALHNHYTQKSLSLSPGG

GGGSGGGGSGGGGSGGGGSVTLKESGPVLVKPTETLTLTCTVSGFSLSTYSMSWIRQPPGKALEWLGFIGSRGDT
YYASWAKGRLTISKDTSKSQVVLTMTNMDPVDTATYYCARERDPYGGGAYPPHLWGRGTLVTVSS

b) Expression plasmid for antibody light chains

Antibody light chain encoding genes comprising a complete and functional antibody light chain was assembled by fusing a DNA fragment coding for the respective sequence elements.

The expression plasmid for the transient expression of an antibody light chain comprised besides the antibody light chain fragment an origin of replication from the vector pUC18, which allows replication of this plasmid in E. coli, and a beta-lactamase gene which confers ampicillin resistance in E. coli. The transcription unit of the antibody light chain fragment comprises the following functional elements:

- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- an antibody light chain (VL-CL) encoding nucleic acid, and
- the bovine growth hormone polyadenylation sequence (BGH pA).

P1AD9827 → ERBB2-DOTAM

DIQMTQSPSSLSASVGDRVTITCRASQDVNTAVAWYQQKPGKAPKLLIYSASFLYSGVPSRFSGSRSGTDFTLTI
SSLQPEDFATYYCQQHYTTPPTFGQGTKVEIKRTVAAPSVFIFPPSDEQLKSGTASVVCLLNNFYPREAKVQWKV
DNALQSGNSQESVTEQDSKDSTYSLSSTLTLSKADYEKHKVYACEVTHQGLSSPVTKSFNRGEC

P1AD9826 → CD20-DOTAM

DIVMTQTPLSLPVTPGEPASISCRSSKSLLHSNGITYLYWYLQKPGQSPQLLIYQMSNLVSGVPDRFSGSGSGTD
FTLKISRVEAEDVGVYYCAQNLELPYTFGGGTKVEIKRTVAAPSVFIFPPSDRKLKSGTASVVCLLNNFYPREAK
VQWKVDNALQSGNSQESVTEQDSKDSTYSLSSTLTLSKADYEKHKVYACEVTHQGLSSPVTKSFNRGEC

Transient expression of the antibody molecules

The antibody molecules were generated in transiently transfected HEK293 cells (human embryonic kidney cell

line 293-derived) cultivated in F17 Medium (Invitrogen Corp.). For transfection "293-Free" Transfection Reagent (Novagen) was used. The respective antibody heavy- and light chain molecules as described above were expressed from individual expression plasmids. Transfections were performed as specified in the manufacturer's instructions. Immunoglobulin-containing cell culture supernatants were harvested three to seven (3-7) days after transfection. Supernatants were stored at reduced temperature (e.g. -80°C) until purification.

General information regarding the recombinant expression of human immunoglobulins in e.g. HEK293 cells is given in: Meissner, P. et al., Biotechnol. Bioeng. 75 (2001) 197-203.

Purification of the antibody molecules P1AD8926 and P1AD8927

The PRIT molecules have been purified by a MabSelect Sure (Affinity Chromatography) and followed by Superdex 200 (Size Exclusion Chromatography).

| **TapirID** | **Expression Volume** | Concentration Pool [mg/mL] | Amount Pool [mg] | **Monomer Content** | **Purity** |
|---|---|---|---|---|---|
| **P1AD8926** | 21 | 2,03 | 22 | > 99% | > 95% |
| **P1AD8927** | 21 | 2,07 | 69 | > 99% | > 95% |

Mass Analysis:

To confirm the identity of the PRIT molecules, ESI-MS was used.

| **TapirID** | **Identity** | **Purity** | **Comment** |
|---|---|---|---|
| **P1AD8926** | confirmed | Side products A, A2C2 (small) | Oxylosation detected |
| **P1AD8927** | confirmed | Side products A, A2C2 (small) | Oxylosation detected |

FACS Analysis of P1AD8927 Functionality

To assess the functionality of P1AD8927 KPL-4 cells were detached from the culture vessel using accutase at 37°C for 10 minutes. Subequently, the cells were washed twice in PBS, and seeded into 96 well v-bottom plates to a final density of 4x106 Cells/well .

The antibody was prelabelled with Zenon<human IgG>A488, added to the cells in concentrations as indicated in Fig 38. Subsequently, the cells were incubated for 1 h on ice and washed twice in PBS and resuspended in 200µl PBS / 5% FCS for measurement of FITC fluorescence using a FACS canto.

The results are shown in figure 38

To assess the binding capability of antibody to DOTAM, after binding to KPL-4 cells, the cells were washed to remove unbound antibdy. Subsequently, Pb-DOTAM-FITC was added to detect DOTAM binding competent cell bound antibodies (Figure 39). P1AD8927 shows a dose dependent FITC signal that was isotype corrected. This experiment shows that the DOTAM binding is functional in this antibody.

FACS Analysis of P1AD8926 Functionality

To assess the functionality of P1AD8926 Raji cells were washed twice in PBS, and seeded into 96 well v-bottom plates to a final density of 4x106 cells/well.

The antibody was prelabelled with Zenon<human IgG>A488, added to the cells in concentrations as indicated in Fig 40. Subsequently, the cells were incubated for 1 h on ice and washed twice in PBS and resuspended in 200µl PBS / 5% FCS for measurement of FITC fluorescence using a FACS canto.

To assess the binding capability of antibody to DOTAM, after binding to Raji cells, the cells were washed to remove unbound antibody. Subsequently, Pb-DOTAM-FITC was added to detect DOTAM binding competent cell bound antibodies (Figure 41). P1AD8927 shows a dose dependent FITC signal that was isotype corrected. This experiment shows that the DOTAM binding is functional in this antibody.

**Example 30: Material and methods of examples 31 to 37**

This example sets out materials and methods for the studies of examples 31-37.

Three-step PRIT (one cycle)

Step 1: Adminstration of a BsAb (i.v. or i.p.): The BsAb used in the studies binds Pb-DOTAM with high affinity and targets the tumor e.g. via CEA.

Step 2: Administration of CA (i.v. or i.p.): To allow efficient tumor accumulation of $^{212}$Pb-DOTAM, circulating BsAb needs to be neutralized in the blood using a CA that blocks $^{212}$Pb-DOTAM binding to unbound BsAb, without penetrating into the tumor and consequently blocking the pretargeted sites. The CA is administered once the BsAb has accumulated to a sufficient degree in the tumor, generally after 4-10 days. The Pb-DOTAM-dextran-500 CA was developed based on an amino dextran with a mean molecular weight of 500 kDa, to which DOTAM is conjugated via a thiourea linker, as shown below.

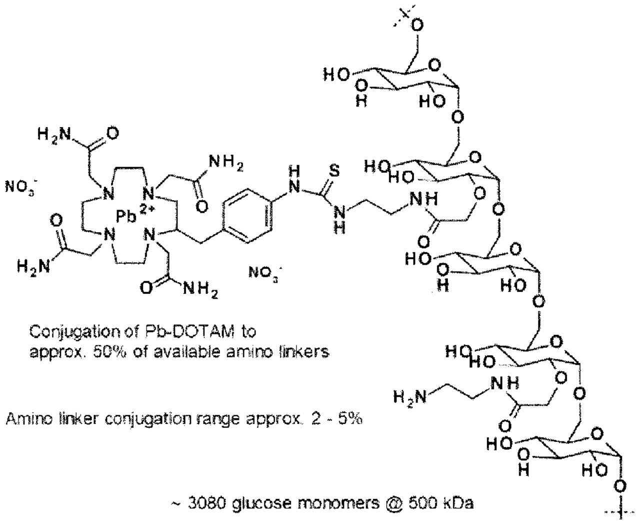


Step 3: Administration of $^{212}$Pb-DOTAM (i.v.): The radioactive injection is performed in the last step, generally 24 to 48 hours after the CA injection, allowing the $^{212}$Pb-DOTAM to preferentially bind the pretargeted tumor sites, efficiently reducing the systemic radiation exposure.

General materials and methods

Experimental protocols performed at ARCoLab were reviewed and approved by the local authorities (Comité Régional d'Ethique de l'Expérimentation Animale du Limousin (CREEAL), Laboratoire Départemental d'Analyses et de Recherches de la Haute-Vienne). Severe combined immunodeficiency (SCID) and CD1 mice were provided by Charles River and maintained under specific and opportunistic pathogen-free (SOPF) conditions with daily cycles of light and darkness (12 h/12 h), in line with ethical guidelines. Study 121 employed specific pathogen-free (SPF) conditions, with mice provided by Envigo. No manipulations were performed during the first 5 days after arrival, to allow the animals to acclimatize to the new environment. All mice were controlled daily for clinical symptoms and detection of adverse events.

Solid xenografts were established by subcutaneous (s.c.) injection of CEA-expressing tumor cells in cell culture media mixed 1:1 with Corning® Matrigel® basement membrane matrix (growth factor reduced; cat No. 354230). Tumor volumes were estimated through manual calipering, calculated according to the formula: $volume = 0.5 \times length \times width^2$.

To minimize re-ingestion of radioactive urine/feces, all efficacy study mice were placed in cages with grilled floors for 4 hours after $^{212}$Pb-DOTAM administration, before being transferred to new cages with standard bedding. All cages were then changed at 24 hours post injection (p.i.). This procedure was not performed for mice being euthanized for biodistribution purposes up to 24 hours after the radioactive injection.

The body weight (BW) of the study animals was measured at least 3 times per week, with additional measurements as needed depending on the health status. Mice whose BW loss exceeded 25% of their initial BW or whose tumor volume reached 3000 mm$^3$ were euthanized immediately. Other factors taken into account for euthanasia for ethical reasons were tumor status (e.g. necrotic areas, blood/liquid leaking out, signs of automutilation) and general appearance of the animal (e.g. fur, posture, movement). Wet food was provided to all mice starting from 5 days after the radioactive

injection, if mandated by an acute BW loss (collective or individual), until all individuals had recovered sufficiently.

Blood was collected at termination from the venous sinus using retro-orbital bleeding, followed by additional tissue harvest for radioactive measurements and/or histological analysis, as mandated by the protocols. Unexpected or abnormal conditions were documented. Tissues collected for formalin fixation were immediately put in 10% neutral buffered formalin (4°C) and then transferred to phosphate-buffered saline (PBS; 4°C) after 5 days. Organs and tissues collected for biodistribution purposes were weighed and measured for radioactivity using a 2470 WIZARD$^2$ automatic gamma counter (PerkinElmer), and the percent injected dose per gram of tissue (% ID/g) subsequently calculated, including corrections for decay and background.

Statistical analysis was performed using GraphPad Prism 6 (GraphPad Software, Inc.) and JMP 8 (SAS Institute Inc.). Curve analysis of tumor growth inhibition (TGI) was performed based on mean tumor volumes using the formula:

$$TGI = 100 - \frac{\overline{v_{treatment,d} - v_{treatment,0}}}{\overline{v_{ref,d} - v_{ref,0}}} \times 100$$

where d indicates treatment day and 0 the baseline value. Vehicle (PBS) was selected as the reference group. Tumor regression (TR) was calculated according to:

$$TR = \frac{\overline{v_{treatment,0} - v_{treatment,d}}}{v_{treatment,0}}$$

where positive values indicated tumor regression, and values below -1 growth beyond the double baseline value.

Pairwise tests were performed to specify which groups were significantly different in terms of survival: the Log-Rank test (more weight on later survival events), and the Wilcoxon test (more weight on early survival times), both using Bonferroni correction for multiple testing.

Test compounds

The compounds utilized in the described studies are presented in the tables headed "bispecific antibodies", "clearing agents", and "radiolabeled chelates", below.

CEA-DOTAM (PRIT-0213) is a fully humanized BsAb targeting the T84.66 epitope of CEA, whereas DIG-DOTAM (PRIT-0175) is a non-CEA-binding BsAb used as a negative control. P1AE1766, P1AE1767, P1AE1768, P1AE1769, and P1AE1770 are humanized CEA-DOTAM BsAbs targeting the CH1A1A epitope of CEA, as described in example 28 above. The antibody constructs were stored at -80°C until the day of injection when they were thawed and diluted in standard vehicle buffer (20 mM Histidine, 140 mM NaCl; pH 6.0) or 0.9% NaCl to their final respective concentrations for intravenous (i.v.) or intraperitoneal (i.p.) administration.

The Ca-DOTAM-dextran-500 and Pb-DOTAM-dextran-500 CA were stored at -20°C until the day of injection when they were thawed and diluted in phosphate-buffered saline (PBS) for i.v. or i.p. administration.

The DOTAM chelate for radiolabeling was provided by Macrocyclics and maintained at -20°C before radiolabeling. $^{212}$Pb-DOTAM was generated by elution with DOTAM from a thorium generator, and subsequently quenched with Cu, Ca, Zn, Gd, or Pb after labeling. The $^{212}$Pb-DOTAM solution was diluted with PBS or 0.9% NaCl to obtain the desired $^{212}$Pb activity concentration for i.v. injection.

Mice in vehicle control groups received multiple injections of PBS instead of BsAb, CA, and $^{212}$Pb-DOTAM.

**Bispecific antibodies**

| Compound | Target | Protocols |
|---|---|---|
| CEA-DOTAM (PRIT-0213) | T84.66 | 103, 116, 131, 146, 154 |
| DIG-DOTAM (PRIT-0175) | Digoxigen in | 103, 151, 152, 154 |
| CD20-DOTAM (P1AD9826) | CD20 | 151, 162 |
| HER2-DOTAM (P1AD9827) | HER2 | 152 |
| P1AE1766 | CH1A1A | 154 |

(continued)

| Compound | Target | Protocols |
|---|---|---|
| P1AE1767 | CH1A1A | 154 |
| P1AE1768 | CH1A1A | 154 |
| P1AE1769 | CH1A1A | 154 |
| P1AE1770 | CH1A1A | 154 |

**Clearing agents**

| Compound | Protocols |
|---|---|
| Pb-DOTAM-dextran-500 | 103, 116, 131, |
| Ca-DOTAM-dextran-500 | 146, 151, 152, 154, 162 |

**Radiolabeled chelates (Supplier: Orano Med)**

| Compound | Quenching | Protocols |
|---|---|---|
| $^{212}$Pb-DOTAM | Cu | 103, 116, 131 |
| $^{212}$Pb-DOTAM | Ca | 131, 146, 151, 152, 154, 162 |
| $^{212}$Pb-DOTAM | Zn | 131 |
| $^{212}$Pb-DOTAM | Gd | 131 |
| $^{212}$Pb-DOTAM | Pb | 131 |
| $^{203}$Pb-DOTAM-CEA-DOTAM | Cu | 121 |
| $^{203}$Pb-DOTAM-DIG-DOTAM | Cu | 121 |
| $^{212}$Pb-DOTAM-CD2 0- | Ca | 162 |
| DOTAM | | |

Tumour models

The tumor cell lines used and the injected amount for inoculation in mice are described in the table "tumour cells lines" below. BxPC3 is a human primary pancreatic adenocarcinoma cell line, naturally expressing CEA. BxPC3 cells were cultured in RPMI-1640 Medium, GlutaMAX™ Supplement, HEPES (Gibco, ref. No. 72400-021)enriched with 10% fetal bovine serum (GE Healthcare Hyclone SH30088.03). HPAF-II (CRL-1997) is a human pancreatic adenocarcinoma cell line, naturally expressing CEA. HPAF-II cells were cultured in EMEM medium (Gibco 31095-029) enriched with 10% standard fetal bovine serum, 1% GlutaMAX 100X, 1% MEM NEAA (Minimum Essential Medium Non-Essential Amino Acids) 100X (Gibco 11140-035), and 1% sodium pyruvate (100 mM; Gibco 11360-070). WSU-DLCL2 is a human B cell lymphoma cell line, naturally expressing CD20. WSU-DLCL2 cells were cultured in RPMI-1640 medium (Gibco, ref. No. 72400-021) enriched with 10% fetal bovine serum. NCI-N87 is a gastric cancer cell line, naturally expressing HER2. NCI-N87 cells were cultured in RPMI-1640 medium (Gibco, ref. No. 72400-021) enriched with 10% fetal bovine serum. Solid xenografts were established in each SCID mouse on study day 0 by subcutaneous injection of cells in RPMI or DMEM mediamixed 1:1 with Corning® Matrigel® basement membrane matrix (growth factor reduced; cat No. 354230), into the right flank.

**Tumor cell lines**

| Cell line | Cells per mouse | Injected volume | Protocols | Supplier |
|---|---|---|---|---|
| BxPC3 | $5\times10^6$ | 100 μL | 103, 116, 121, 131, 146 | ECACC* |
| HPAF-II (CRL-1997) | $1\times10^6$ | 100 μL | 114, 154 | ATCC** |
| WSU-DLCL2 | $1.5\times10^6$ | 100 μL | 151, 162 | DSMZ*** |
| NCI-N87 | $1.5\times10^6$ | 100 μL | 152 | ATCC** |
| *European Collection of Authenticated Cell Cultures (Salisbury, UK) "American Type Culture Collection (Manassas, VA, USA)<br>***Leibniz-Institut DSMZ - Deutsche Sammlung von Mikroorganismen und Zellkulturen GmbH (Braunschweig, Germany) | | | | |

STUDIES

**Example 31: Efficacy of CEA-PRIT in tumor model (Protocol 103)**

This study assessed the efficacy of CEA-PRIT using the CEA-DOTAM BsAb for treatment of s.c. BxPC3 tumors in mice. The therapy was administered either as a single treatment with 10 or 30 μCi of $^{212}$Pb-DOTAM, or in three repeated cycles for each of the two radioactivity levels. Comparisons were made with PRIT using a non-CEA binding control antibody (DIG-DOTAM), the CEA-DOTAM BsAb alone (no radioactivity), and no treatment (PBS). Biodistributions were performed after the first and second cycle to confirm $^{212}$Pb-DOTAM targeting and clearance, and the treatment efficacy was assessed in terms of TGI, TR, and survival. The mice were carefully monitored throughout the study to assess the tolerability of the different treatment schedules. The study outline is shown in figure 42.

Study design

The time course and design of protocol 103 are shown in the tables below.

**Time course of protocol 103**

| Study day | Experimental procedure |
|---|---|
| 0 | S.c. injection of BxPC3 cells |
| 20 | I.v. injection of PRIT BsAb (groups C-G, J-L) |
| 21 | I.v. injection of PRIT BsAb (groups A, B, H, I) |
| 24 | I.v. injection of CA (groups C-G, J-L) |
| 24 | Elution of $^{212}$Pb-DOTAM |
| 24 | I.v. injection of $^{212}$Pb-DOTAM (groups C-G, J-L) |
| 25 | Euthanasia and tissue harvest + gamma counting (group J) |
| 25 | I.v. injection of CA (groups A, B, H, I) |
| 25 | Elution of $^{212}$Pb-DOTAM |
| 25 | I.v. injection of $^{212}$Pb-DOTAM (groups A, B, H, I) |
| 26 | Euthanasia and tissue harvest + gamma counting (group H) |
| 34 | I.v. injection of PRIT BsAb (groups B, D-G, I, K, L) |
| 38 | I.v. injection of CA (groups B, D-G, I, K, L) |

(continued)

| Study day | Experimental procedure |
|---|---|
| 38 | Elution of $^{212}$Pb-DOTAM |
| 38 | I.v. injection of $^{212}$Pb-DOTAM (groups B, D-G, I, K, L) |
| 39 | Euthanasia and tissue harvest + gamma counting (groups I, K, L) |
| 48 | I.v. injection of PRIT BsAb (groups B, D-G) |
| 52 | I.v. injection of CA (groups B, D-G) |
| 52 | Elution of $^{212}$Pb-DOTAM |
| 52 | I.v. injection of $^{212}$Pb-DOTAM (groups B, D-G) |

**Study groups in protocol 103**

| Group | n | BsAb | BsAb dose (μg) | Pb-DOTAM-Dextran-500 dose (μg) | Chelate | $^{212}$Pb activi ty (μCi) | Cycles |
|---|---|---|---|---|---|---|---|
| **A** | 10 | CEA-DOTAM | 100 | 25 | $^{212}$Pb-DOTAM | 10 | 1 |
| **B** | 10 | CEA-DOTAM | 100 | 25 | $^{212}$Pb-DOTAM | 10 | 3 |
| **C** | 10 | CEA-DOTAM | 100 | 25 | $^{212}$Pb-DOTAM | 30 | 1 |
| **D** | 10 | CEA-DOTAM | 100 | 25 | $^{212}$Pb-DOTAM | 30 | 3 |
| **E** | 10 | CEA-DOTAM | 100 | 0 | - | 0 | 3 |
| **F** | 10 | DIG-DOTAM | 100 | 25 | $^{212}$Pb-DOTAM | 30 | 3 |
| **G** | 10 | - | 0 | 0 | - | 0 | 3 |
| **H** | 3 | CEA-DOTAM | 100 | 25 | $^{212}$Pb-DOTAM | 10 | 1 |
| **I** | 3 | CEA-DOTAM | 100 | 25 | $^{212}$Pb-DOTAM | 10 | 2 |
| **J** | 3 | CEA-DOTAM | 100 | 25 | $^{212}$Pb-DOTAM | 30 | 1 |
| **K** | 3 | CEA-DOTAM | 100 | 25 | $^{212}$Pb-DOTAM | 30 | 2 |
| **L** | 3 | DIG-DOTAM | 100 | 25 | $^{212}$Pb-DOTAM | 30 | 2 |

Solid xenografts were established in each SCID mouse on study day 0 by s.c. injection of $5\times10^6$ cells (passage 24) in RPMI/Matrigel into the right flank. Twenty days after tumor cell injection, mice were sorted into experimental groups with an average tumor volume of 290 mm$^3$.

Due to logistical reasons, the treatments were started during the course of 2 days, with BsAb or PBS injections of groups C, D, E, F, G, J, K, and L on the first day, as described in the table above, followed by BsAb injections of groups A, B, H, and I on the next day. Four days later, the CA was injected, followed 2 hours later by $^{212}$Pb-DOTAM or

PBS. Groups receiving 30 or 0 μCi were injected first, followed the next day by those receiving 10 μCi. Groups B, D, E, F, G, I, K, and L received multiple treatments with 2 weeks between the radioactive injections. All repeated treatment cycles (2nd and 3rd) were commenced simultaneously, i.e. all mice received the BsAb or PBS injection on the same day, followed by CA and $^{212}$Pb-DOTAM, or PBS, 4 days later. The following organs and tissues were harvested from groups A-G at the time of euthanasia: bladder, ovaries, liver, spleen, kidneys, femur (including bone marrow), colon, jejunum, stomach, and tumor.

Mice in groups H and J were sacrificed and necropsied 24 hours after their first and only injection of $^{212}$Pb-DOTAM; groups I, K, and L were sacrificed and necropsied 24 hours after their second $^{212}$Pb-DOTAM injection. Blood, bladder, small intestine, colon, spleen, pancreas, kidneys, liver, lung, heart, femoral bone, muscle, tail, and tumor were harvested for radioactive measurement at euthanasia.

Results

The in vivo distribution of $^{212}$Pb 24 hours p.i. demonstrated high uptake in subcutaneous BxPC3 tumors and low accumulation in normal tissues, as seen in Figure 43. The significant difference between tumors pretargeted by CEA-DOTAM and those pretargeted by the non-CEA-binding BsAb DIG-DOTAM confirmed the high specificity of the treatment. One-way analysis of variance (ANOVA) with Sidak's multiple comparisons test showed that there was no significant difference in average tumor uptake of $^{212}$Pb, neither between 1 (50.3% ID/g) and 2 (43.0% ID/g) 10-pCi injections, nor between 1 (37.6% ID/g) and 2 (24.1% ID/g) 30-μCi injections. Likewise, there was no significant difference between administering 1 cycle of 10 μCi and 1 cycle of 30 μCi (p>0.05). However, the tumor uptake was significantly lower after 2 cycles of 30 μCi compared with 2 cycles of 10 μCi, as was the tumor uptake after 2 cycles of 30 μCi in tumors pretargeted with DIG-DOTAM (2.9% ID/g) compared with either CEA-DOTAM treatment. Panel B of Figure 43 shows that the % ID/g was comparable for tumors of different sizes, within the various treatment groups.

The average tumor development and the individual tumor growth curves are shown in Figure 44 and Figure 45, respectively. All groups doubled their tumor volume by day 20-24. The first $^{212}$Pb-DOTAM treatment was given on day 24, after which tumors in the CEA-DOTAM groups continued to grow for approximately 1 week before starting to shrink. No tumors regressed completely, but volumes for the multiple-treated groups (B and D) remained relatively constant until the last treatment on day 52. Tumors in mice injected only once with either 10 or 30 μCi started to regrow on day 44, although those receiving the higher activity had a slightly slower growth rate. Nonspecific PRIT with DIG-DOTAM resulted in statistically significant but limited TGI compared with CEA-DOTAM alone or vehicle. The two latter control groups exhibited identical tumor development.

On day 63, the last day on which all treatment groups could be analyzed based on means, the TGI was 57.2, 89.8, 77.7, 96.6, -6.2, and 67.3% for groups A, B, C, D, E, and F, respectively, compared with no treatment. The last mouse in the vehicle control group was accounted for on day 74, at which time the TGI was 48.5, 83.3, 63.5, and 95.7% for the four remaining groups: A, B, C, and D, respectively. The study was terminated on day 118 after cell injection, by euthanasia of the last remaining mouse (group D).

Log-Rank and Wilcoxon tests were performed to specify which groups were significantly different in terms of survival, the results shown in the two tables below. All CEA-DOTAM PRIT regimens significantly increased the survival compared with the three control groups. The overall survival was slightly better after 3 cycles of 10 μCi than after a single 10-μCi cycle (p = 0.0110), but there was no significant difference between 3 cycles of 10 μCi and either of the 30-μCi regimens with CEA-DOTAM pretargeting. Kaplan-Meier survival curves are shown in Figure 46.

**Pairwise Log-Rank test (multiple test level = 0.00238)**

| Group | Vehicle (PBS) | CEA-DOTAM alone | CEA-DOTAM 10 μCi ×1 | CEA-DOTAM 10 μCi ×3 | CEA-DOTAM 30 μCi ×1 | CEA-DOTAM 30 μCi ×3 | DIG-DOTAM 30 μCi ×3 |
|---|---|---|---|---|---|---|---|
| **Vehicle (PBS)** | 1.0000 | 0.3197 | 0.0005* | <.0001* | <.0001* | 0.0043* | 0.5588 |
| **CEA-DOTAM alone** | 0.3197 | 1.0000 | <.0001* | <.0001* | <.0001* | 0.0024* | 0.8491 |

(continued)

| Group | Vehicle (PBS) | CEA-DOTAM alone | CEA-DOTAM 10 μCi ×1 | CEA-DOTAM 10 μCi ×3 | CEA-DOTAM 30 μCi ×1 | CEA-DOTAM 30 μCi ×3 | DIG-DOTAM 30 μCi ×3 |
|---|---|---|---|---|---|---|---|
| CEA-DOTAM 10 μCi ×1 | 0.0005* | <.0001* | 1.0000 | 0.0110* | 0.1114 | 0.1562 | 0.0002* |
| CEA-DOTAM 10 μCi ×3 | <.0001* | <.0001* | 0.0110* | 1.0000 | 0.0980 | 0.8798 | <.0001* |
| CEA-DOTAM 30 μCi ×1 | <.0001* | <.0001* | 0.1114 | 0.0980 | 1.0000 | 0.3839 | <.0001* |
| CEA-DOTAM 30 μCi x3 | 0.0043* | 0.0024* | 0.1562 | 0.8798 | 0.3839 | 1.0000 | 0.0034* |
| DIG-DOTAM 30 μCi x3 | 0.5588 | 0.8491 | 0.0002* | <.0001* | <.0001* | 0.0034* | 1.0000 |

**Pairwise Wilcoxon test (multiple test level = 0.00238)**

| Group | Vehicle (PBS) | CEA-DOTAM alone | CEA-DOTAM 10 μCi ×1 | CEA-DOTAM 10 μCi ×3 | CEA-DOTAM 30 μCi ×1 | CEA-DOTAM 30 μCi ×3 | DIG-DOTAM 30 μCi ×3 |
|---|---|---|---|---|---|---|---|
| Vehicle (PBS) | 1.0000 | 0.3792 | 0.0009* | <.0001* | <.0001* | 0.0255* | 0.4931 |
| CEA-DOTAM alone | 0.3792 | 1.0000 | 0.0001* | <.0001* | <.0001* | 0.0130* | 1.0000 |
| CEA-DOTAM 10 μCi ×1 | 0.0009* | 0.0001* | 1.0000 | 0.0033* | 0.0370* | 0.3590 | 0.0005* |
| CEA-DOTAM 10 μCi ×3 | <.0001* | <.0001* | 0.0033* | 1.0000 | 0.1007 | 0.5963 | <.0001* |
| CEA-DOTAM 30 μCi ×1 | <.0001* | <.0001* | 0.0370* | 0.1007 | 1.0000 | 0.8797 | <.0001* |
| CEA-DOTAM 30 μCi ×3 | 0.0255* | 0.0130* | 0.3590 | 0.5963 | 0.8797 | 1.0000 | 0.0122* |
| DIG-DOTAM 30 μCi ×3 | 0.4931 | 1.0000 | 0.0005* | <.0001* | <.0001* | 0.0122* | 1.0000 |

Adverse events and toxicity

The observed adverse events motivating sacrifice of mice for ethical reasons can be sorted into two groups: 1) tumor status, and 2) radiation-induced toxicity. The first refers to necrotic and/or ulcerating tumors, prompting mice to "clean" the tumor area on themselves or their cage mates. Mice who reached this stage were immediately euthanized to avoid suffering.

The second group of adverse events comprised typical symptoms of radiation-induced toxicity, e.g. BW loss, diarrhea, and lethargy. Figure 47 shows the BW development in the therapy groups. All mice that were injected with 30 μCi of $^{212}$Pb-DOTAM experienced an initial BW drop that was mitigated by day 8 after the first radioactive injection. Groups that were administered multiple 30-μCi injections (D and F) suffered a more prolonged BW loss. The BW loss was mild in groups that received 10 μCi (A and B), even at multiple injections. A clear difference was thus perceived between mice that received 10 and 30 μCi of $^{212}$Pb-DOTAM. Even after repeated 10-pCi treatments the mice displayed, in general, no major signs of adverse toxicity. Mice treated with one cycle of 30 μCi expressed transient weight loss, but recovered. However, the second and third 30-μCi cycles resulted in more extensive adverse toxicity, resulting in a number of animals sacrificed due to a combination of BW loss and signs of pain and/or discomfort. From this can be concluded that 10 μCi is a safe radioactivity level under the applied conditions, whereas 30 μCi is approaching the maximum tolerated activity.

Conclusion

We conclude that given as a monotherapy regimen, repeated CEA-PRIT with 10 or 30 μCi of $^{212}$Pb-DOTAM provided significant increase in survival and TGI, but neither complete tumor eradication nor sustained tumor control in this setting. To avoid radiation-induced toxicity while maintaining tumor control over time, these results suggest using less than 30 but more than 10 μCi of $^{212}$Pb-DOTAM.

**Example 32: Selection of an appropriate time between BsAb and CA injection for CEA-PRIT (Protocols 116 and 121)**

Protocols 116 and 121 were designed to guide the selection of an appropriate time between BsAb and CA injection for CEA-PRIT, on the basis of high tumor uptake and homogeneous intratumoral BsAb distribution. Protocol 116 assessed the intratumoral BsAb distribution compared with the CEA expression in the BxPC3 model at day 4, 7, and 14 after i.v. injection, detected by immunofluorescence staining. Protocol 121 assessed the accumulation of BsAb directly labelled with lead-203 ($^{203}$Pb) in BxPC3 tumors after 1, 4, 7, and 10 days. The BsAb was labelled with the Pb isotope $^{203}$Pb (half-life 2.2 days), to be able to follow the development over a longer period of time compared with $^{212}$Pb (half-life 10.6 hours).

Study design

The time courses and designs of protocols 116 and 121 are shown in the four tables below.

**Time course of protocol 116**

| Study day | Experimental procedure |
|---|---|
| 0 | S.c. injection of BxPC3 cells (groups A, B, C, D, E) |
| 0* | S.c. injection of BxPC3 cells (groups F, G) |
| 20 | I.v. injection of PRIT BsAb (groups A, B, C, D, E) |
| 18* | I.v. injection of PRIT BsAb (groups F, G) |
| 24 | I.v. injection of CA (group C) |
| 24 | Euthanasia and necropsy (groups A, B, C; 4 d p.i.) |
| 22* | I.v. injection of CA and $^{212}$Pb-DOTAM (groups F, G) |
| 27 | Euthanasia and necropsy (group D; 7 d p.i.) |

(continued)

| Study day | Experimental procedure |
|---|---|
| 34 | Euthanasia and necropsy (group E; 14 d p.i.) |
| 32* | I.v. injection of PRIT BsAb (group F) |
| 36* | Euthanasia and necropsy (groups F, G) |

**Study groups in protocol 116**

| Group | n | BsAb | BsAb (μg) | Time (d) | CA (μg) | Time (h) | $^{212}$Pb (μCi) | PRIT cycles | Sample time (d) |
|---|---|---|---|---|---|---|---|---|---|
| **A** | 2 | - | 0 | - | 0 | - | 0 | - | 4 |
| **B** | 3 | CEA-DOTAM | 100 | - | 0 | - | 0 | - | 4 |
| **C** | 3 | CEA-DOTAM | 100 | 4 | 25 | 2 | 0 | - | 4 |
| **D** | 3 | CEA-DOTAM | 100 | - | 0 | - | 0 | - | 7 |
| **E** | 3 | CEA-DOTAM | 100 | - | 0 | - | 0 | - | 14 |
| **F** | 3 | CEA-DOTAM | 100 | 4 | 25 | 2 | 10 | 2 | 4* |
| **G** | 3 | CEA-DOTAM | 100 | 4 | 25 | 2 | 10 | 1 | 4* |
| *Days after second BsAb injection (initiated 1.5 weeks after $^{212}$Pb-DOTAM) | | | | | | | | | |

**Time course of protocol 121**

| Study day | Experimental procedure |
|---|---|
| 0 | S.c. injection of BxPC3 cells |
| 20 | I.v. injection of $^{203}$Pb-DOTAM-BsAb |
| 21 | Euthanasia and necropsy (group A; 1 d p.i.); gamma counting of tissues |
| 24 | Euthanasia and necropsy (groups B, E; 4 d p.i.); gamma counting of tissues |
| 27 | Euthanasia and necropsy (group C; 7 d p.i.); gamma counting of tissues |
| 30 | Euthanasia and necropsy (group D; 10 d p.i.); gamma counting of tissues |

**Study groups in protocol 121**

| Group | n | $^{203}$Pb-BsAb | BsAb (μg) | Time (d) | CA (μg) | Time (h) | $^{203}$Pb (μCi) | Sample time (d) |
|---|---|---|---|---|---|---|---|---|
| **A** | 5 | CEA-DOTAM | 100 | - | 0 | - | 20 | 1 |

(continued)

| Group | n | $^{203}$Pb-BsAb | BsAb (μg) | Time (d) | CA (μg) | Time (h) | $^{203}$Pb (μCi) | Sample time (d) |
|---|---|---|---|---|---|---|---|---|
| B | 5 | CEA-DOTAM | 100 | - | 0 | - | 20 | 4 |
| C | 5 | CEA-DOTAM | 100 | - | 0 | - | 20 | 7 |
| D | 5 | CEA-DOTAM | 100 | - | 0 | - | 20 | 10 |
| E | 5 | DIG-DOTAM | 100 | - | 0 | - | 20 | 4 |

Solid xenografts were established through s.c. injection of BxPC3 cells. Nineteen days after inoculation, mice in protocol 116 were sorted into experimental groups with an average tumor volume of 227 mm$^3$. In protocol 121, the average tumor volume was 203 mm$^3$ after 20 days.

Immunofluorescence staining

Mice in protocol 116 were necropsied after euthanasia, and spleen, kidneys, liver, muscle, and tumor harvested. Collected tissues were split in two pieces: one was put in a cryomold containing Tissue-Tek® optimum cutting temperature (OCT) embedding compound, and put on dry ice for rapid freezing, and the other fixed in 10% neutral buffered formalin (NBF) for 24 hours and then transferred to PBS for storage until paraffin-embedding. Frozen samples in OCT were maintained at -80°C before sectioning. Histological staining was performed using reagents listed in the table below.

**Reagents used for histological staining in protocol 116**

| Compound | Lot/Ref. No. | Conc./ diluti on | Supplier |
|---|---|---|---|
| Rabbit IgG anti-human CEA (clone T84.66) | P1AD5732 | 1 μg/mL | Roche Glycart |
| Goat anti-Rabbit IgG (H+L) Highly Cross-Adsorbed Secondary Antibody, Alexa Fluor 488 | A11034 | 1/400 | Fisher |
| Goat anti-Human IgG (H+L) Cross-Adsorbed Secondary Antibody, Alexa Fluor 555 | A21433 | 1/300 | Fisher |
| Blocking serum (goat) | G9023-10ML | 5% | Sigma |
| Hoechst stain | 94403-1ML | 1/1000 | Sigma |

*Staining for CEA*

Frozen tumors were sectioned into 12-μm slices using a Leica CM1850 UV cryotome, and the slides stored at -20°C. Before staining, the slides were thawed for 1 hour at room temperature (RT), and then washed for 5 minutes with PBS (1X, pH 7.4), followed by 5 minutes in cold acetone (-20°C), and once more with PBS for 5 minutes. The slides were dried with a piece of paper around the tissue, and a circle drawn around the tissue using a hydrophobic pen (Dako; Agilent). Incubation of sections were performed with 400 μL of blocking serum (goat) during 1 hour at RT. The blocking serum was removed and 200 μL of primary antibody (rabbit IgG anti-human CEA) added to the sections, followed by incubation overnight at 4°C in a dark chamber. On day 2, the sections were washed twice with PBS for 10 minutes, and 400 μL of blocking serum added. After incubation during 1 hour at RT, the blocking serum was removed and 200 μL of secondary antibody (Alexa Fluor 488-labelled goat anti-rabbit IgG) and counterstaining (Hoechst) added to the sections, followed by incubation in a dark chamber for 2 hours at RT. The slides were then washed twice with PBS for 10 minutes. Finally, the fluorescence mounting medium (Dako S3023; Agilent) and coverslip were added to the slides, which were subsequently air dried and stored in darkness at -20°C. Analysis of the stained slides was performed using a Zeiss Axio Scope.A1 modular microscope.

*Staining for CEA--DOTAM*

Frozen tumor sections were stored and processed as described for CEA-staining above, but with PBS instead

of primary antibody. The secondary antibody was a goat anti-human IgG labelled with Alexa Fluor 555.

*H&E staining*

Hematoxylin and eosin (H&E) staining of frozen tumor sections from protocol 116 was performed using a Leica Autostainer XL automated slide stainer.

*Biodistribution*

Mice in protocol 121 were sacrificed for biodistribution purposes and their blood collected through retro-orbital bleeding before termination. In addition, bladder, small intestine, colon, spleen, pancreas, kidneys, stomach, liver, lung, heart, brain, femoral bone, muscle, skin, tail, and tumor were harvested after euthanasia and measured for radioactivity.

Results

*Immunofluorescence staining - protocol 116*

The untreated control displayed high levels of CEA uniformly distributed in collected tumors. Control staining for CEA-DOTAM BsAb resulted in no signal, as expected.

Tumors from mice injected with CEA-DOTAM BsAb were taken at 4, 7, and 14 days p.i. At 4 days, the BsAb was fully covering the boundaries of the tumor cell nodules within the tissue, but had not entirely penetrated into the larger nodules. This is shown by the presence of darker, Hoechst-stained areas, surrounded by brightly red bordering cell layers. After 7 days, the signal from CEA-DOTAM staining appeared more homogeneously distributed, and there were fewer tumor cell structures with dark interior areas. One week later, 14 days p.i., the distribution of the fluorescent signal remained similar to the 7-day time point, but with lower signal overall.

Tumor samples from the two groups that were administered the entire PRIT cycle, including irradiation with $^{212}$Pb-DOTAM, displayed areas of necrosis due to radiation-induced cell death. Findings included cell swelling, loss of cellular detail (ghost cells), some nuclear atypia, and a potential increase of interstitial fibrosis. Tumors from mice in group F that received a second dose of CEA-DOTAM 2 weeks after the first BsAb injection retained a high and homogeneous CEA expression, and the CEA-DOTAM distribution 4 days after the second BsAb injection resembled that of the non-irradiated tumors, i.e. BsAb distributed to all CEA-expressing parts of the tumor but with limited penetration into certain larger structures, resulting in darker areas on the images. Corresponding control samples were acquired at the same time from group G, which received no second BsAb injection after the initial PRIT cycle. In these tumor sections, a faint signal could be seen for CEA-DOTAM staining, indicating that a certain amount of BsAb was still tumor-bound 18 days after injection.

*Biodistribution - protocol 121*

The average $^{203}$Pb accumulation and clearance in BxPC3 tumor-bearing SCID mice is displayed in Figure 48. Blood clearance was slow, as expected from an antibody without the aid of a CA. No unexpected uptake of the labelled BsAb was revealed in normal organs or tissues. The tumor accumulation over time is shown in Figure 49, starting at an average of 49% ID/g 1 day after injection, increasing to 130, 189, and 197% ID/g at day 4, 7, and 10, respectively. The labelled negative control resulted in no tumor accumulation, with 3% ID/g on day 4 after injection.

Statistical analysis was performed by one-way analysis of variance (ANOVA) using Sidak's multiple comparisons test, to assess whether the perceived differences in tumor uptake at different time points were significant. According to the test, significant increase in $^{203}$Pb accumulation was achieved only between day 1 and 7, and day 1 and 10; no other time points were significantly different from each other. Testing merely 4 versus 7 days using an unpaired t-test resulted in a statistically significant increase at 7 days ($p = 0.0468$), whereas performing the corresponding test for 7 versus 10 days did not ($p = 0.8316$).

Conclusion

The results showed an overall increase in 203Pb-BsAb uptake in tumor between 4 and 7 days after injection, but no further improvement was achieved when extending the time interval to 10 days. Microscopically, the BsAb penetration into tumors was improved after 7 days compared with 4. No benefit was seen at the 14-day time point, by which time the overall fluorescent signal seemed to decrease, without improving the intratumoural distribution. The studies support choosing a BsAb-CA time interval of 7 days over 4 days.

**Example 33: Biodistribution (in vivo $^{212}$Pb-DOTAM distribution data for estimations of the absorbed radioactive dose to tumor and normal tissues) (Protocol 146)**

Protocol 146 was designed to provide in vivo $^{212}$Pb-DOTAM distribution data after PRIT for estimations of the absorbed radioactive dose to tumor and normal tissues, in SCID mice carrying s.c. BxPC3 tumors. The quenching of $^{212}$Pb-DOTAM was performed using Ca (see example 34).

Pretargeting was performed by injection of CEA-DOTAM BsAb, followed 7 days later by the CA. After 24 hours, the $^{212}$Pb-DOTAM was administered. Groups of mice were sacrificed at multiple time points from 5 minutes to 48 hours after the radioactive injection, and blood and organs harvested for radioactive measurement. Excrements were sampled at selected time points by use of metabolic cages to assess the excretion rate of the radioactive compound. Absorbed doses were finally calculated through established methods using the resulting time-activity curves.

Study design

The time course and design of protocol 146 are shown in the two tables below.

**Time course of protocol 146**

| Study day | Experimental procedure |
|---|---|
| 0 | S.c. injection of BxPC3 cells |
| 21 | I.v. injection of PRIT BsAb |
| 28 | I.v. injection of CA |
| 29 | Elution of $^{212}$Pb-DOTAM |
| 29 | I.v. injection of $^{212}$Pb-DOTAM |
| 29 | Euthanasia and necropsy (5 min-6 h p.i.); gamma counting of tissues |
| 30 | Euthanasia and necropsy (24 h p.i.); gamma counting of tissues |
| 31 | Euthanasia and necropsy (48 h p.i.); gamma counting of tissues |

**Study groups in protocol 146**

| **Group** | **n** | **BsAb** (μg) | **Time** (d) | **CA** (μg) | **Time** (h) | **$^{212}$Pb** (μCi) | **DOTAM quench** | **Sample time** |
|---|---|---|---|---|---|---|---|---|
| **A** | 5 | 100 | 7 | 25 | 24 | 20 | Ca | 5 min |
| **B** | 5 | 100 | 7 | 25 | 24 | 20 | Ca | 30 min |
| **C** | 5 | 100 | 7 | 25 | 24 | 20 | Ca | 2 h |
| **D** | 5 | 100 | 7 | 25 | 24 | 20 | Ca | 6 h |
| **E** | 5 | 100 | 7 | 25 | 24 | 20 | Ca | 24 h |
| **F** | 5 | 100 | 7 | 25 | 24 | 20 | Ca | 48 h |

Solid xenografts were established by s.c. injection of BxPC3 cells into SCID mice (Envigo) aged 8 weeks. Twenty-eight days after inoculation, mice were sorted into experimental groups with an average tumor volume of 310 $mm^3$.

Mice in group E and F were individually housed (i.e. one mouse per cage) in grilled-floor metabolic cages. Urine and feces were collected from each cage 2, 6, and 24 hours after the radioactive injection, and the individual samples subsequently measured for radioactivity. Mice in group F were transferred to a regular cage 24 hours p.i.

Blood/serum was collected from all mice through retro-orbital bleeding before termination. After euthanasia, the following additional organs and tissues were harvested for radioactivity measurement and calculation of % ID/g:

bladder, uterus, small intestine, colon, spleen, pancreas, kidneys, stomach, liver, lung, heart, brain, femoral bone, skin, muscle, abdominal fat, tail, and tumor.

Radiation dosimetry

Absorbed doses were calculated following the formalism outlined in Pamphlet No. 21 from the Medical Internal Radiation Dose (MIRD) Committee (Bolch WE, Eckerman KF, Sgouros G, and Thomas SR. J Nucl Med 2009; 50:477-484).

Formally, the absorbed dose $D(r_T)$ in a target tissue or region $r_T$ is calculated as the time-integral of the activity $A(r_S,t)$ multiplied by the absorbed dose rate per unit activity $S(r_T \leftarrow r_S)$, summed over all source regions $r_S$. Given that the vast majority of energy released by the $^{212}Pb$ decay chain comes from alpha radiation and furthermore by the much higher biological effectiveness of alpha radiation (as characterized by the relative biological effectiveness, RBE) two approximations were made:

1. the energy is absorbed locally due to the short range of the alpha particles, which means that only the term $S(r_T \leftarrow r_T)$ is considered, and

2. the contribution from beta and gamma radiation is neglected.

With $\Delta_\alpha$ being the energy released by alpha decays, or rather the sum of the mean energies released by alpha decays in the decay chain of $^{212}Pb$, here expressed in units $J/(\mu Ci \cdot h)$, the absorbed dose in a target tissue or region $r_T$ is calculated by the formula:

$$D_\alpha(r_T) = \sum_{r_S} \int_0^\infty A(r_S,t) \cdot S(r_T \leftarrow r_S) \cdot dt = \Delta_\alpha \cdot \int_0^\infty \frac{A(r_T,t)}{M(r_T)} \cdot dt$$

The calculations are based on the composite (average) decay-corrected radioactive concentration at time $t$, expressed as $[\%ID/g]_t$. In a first step, these are transformed into radioactive concentrations as $\mu Cl$ per $kg$ tissue weight for the target tissue $r_T$:

$$\frac{A(r_T,t)}{M(r_T)} = \frac{A_0[\mu Ci]}{100\%} \cdot e^{-\lambda \cdot t} \cdot [\%ID/g]_t \cdot 1000\frac{g}{kg}$$

$A_0[\mu Ci]$ is the injected activity and $\lambda$ is the exponential decay rate for $^{212}Pb$, 0.0651 $h^{-1}$ (corresponding to the 10.6-hour half-life).

In a second step, the radioactive concentration was integrated over time for each tissue. This was achieved using the "Linear Log Trapezoidal calculation method" in the pharmacokinetic software Phoenix WinNonlin 6.4 (Certara USA, Inc., 100 Overlook Center, Suite 101, Princeton, NJ 08540 USA). The model type "Plasma" was used with uniform weighting.

In the final step, absorbed doses were calculated by multiplying the time integrals of the radioactive concentration by the energy release $\Delta_\alpha = 170 \cdot 10^{-6}$ $J/(\mu Ci \cdot h)$. Additionally, RBE-weighted absorbed doses were calculated using a RBE of 5, as suggested in MIRD Pamphlet No. 22 (Sgouros G, Roeske JC, McDevitt MR, et al. J Nucl Med 2010; 51:311-328).

Results

*Biodistribution*

The average $^{212}Pb$ accumulation and clearance in tumor-bearing SCID mice is displayed in figure 50. The accumulation of radioactivity was high in tumor, with 17% ID/g already at 5 minutes after injection, increasing to over 40 % by 6 hours p.i. and remaining at that level for at least 48 hours. No major differences were discovered in normal tissues compared with the tumor-free condition.

*Absorbed doses*

The mean absorbed doses in Gy calculated for protocol 146 are shown in Table 24, both as absolute values (RBE = 1) and corrected for the higher cytotoxicity of alpha emitters (RBE = 5). For the SCID mice, the average weight was 18.5 g; with an injected $^{212}Pb$ activity of 20 μCi this resulted in normalized injected activities of 1.1 μCi/g body weight.

An absorbed dose of approximately 20 Gy was achieved in tumor while the absorbed dose remained well below 2 Gy in most normal tissues, except bladder. The $^{212}Pb$ content in bladder differed significantly between individual mice, indicating a high variability for this tissue. RBE-corrected doses were provided to enable comparison with radiotherapy using external gamma radiation, resulting in an estimated absorbed dose to tumor of approximately 100 Gy per 20 μCi of $^{212}Pb$-DOTAM injected.

**Mean absorbed doses (Gy) to collected organs and tissues after injection of 20 pCi $^{212}Pb$-DOTAM**

| Organ/tissue | Procotol 146 Pretargeted $^{212}Pb$-DOTAM (tumor-bearing SCID) | |
|---|---|---|
| | RBE = 1 | RBE = 5 |
| Abdominal fat | 1.25 | 6.25 |
| Bladder | 6.53 | 32.64 |
| Blood | 0.72 | 3.60 |
| Brain | 0.04 | 0.19 |
| Colon | 0.43 | 2.17 |
| Femoral bone | 0.27 | 1.33 |
| Heart | 0.33 | 1.63 |
| Kidneys | 1.43 | 7.14 |
| Liver | 0.22 | 1.09 |
| Lung | 1.34 | 6.69 |
| Muscle | 0.29 | 1.47 |
| Pancreas | 0.36 | 1.80 |
| Serum | 1.64 | 8.20 |
| Skin | 0.47 | 2.33 |
| Small intestine | 0.22 | 1.08 |
| Spleen | 0.27 | 1.36 |
| Stomach | 0.24 | 1.20 |
| Tail | 0.54 | 2.69 |
| Uterus | 0.61 | 3.04 |
| Tumor (BxPC3) | 19.91 | 99.55 |

Conclusion

An absorbed dose of approximately 20 Gy was achieved in tumor while the absorbed dose in most normal tissues remained well below 2 Gy. Overall, the study indicated no major risks of dose-limiting toxicity using the evaluated PRIT regimen.

**Example 34: Quenching of unchelated DOTAM (Protocol 131)**

The alpha emitter $^{212}Pb$ is chelated to DOTAM after elution from a thorium-containing resin, producing the pharmacologically active $^{212}Pb$-DOTAM. After the radiolabeling, an excess (>99%) of free unchelated DOTAM remains in solution, readily capturing metal ions from the environment. Upon injection into a patient, it would rapidly bind to circulating $Ca^{2+}$, forming the pharmacologically inactive Ca-DOTAM. Although the CEA-DOTAM BsAb is designed to preferentially bind Pb-DOTAM, it will also form stable complexes with Ca-DOTAM at a comparable degree. As a consequence, blocking of $^{212}Pb$-DOTAM from preLargeted tumor sites could occur at saturated conditions, potentially decreasing the efficacy of the PRIT treatment. To avoid potential competition, a quenching step is therefore added to the $^{212}Pb$-DOTAM elution process, in which a metal ion ("X") is introduced to control the formation of "X-DOTAM" with a significantly higher dissociation constant (Kd). The affinity of the DOTAM binder towards various X-DOTAM chelates as determined by KinExA (kinetic exclusion assay) equilibrium measurements is shown in the table below.

**Metal-DOTAM chelate affinities of CEA-DOTAM BsAb**

| **Chelate** | **Kd** [pM] | **95% CI** [pM] |
|---|---|---|
| Pb-DOTAM | 0.84 | 0.44-1.4 |
| Ca-DOTAM | 0.95 | 0.43-1.7 |
| Cu-DOTAM | 122 000 | 60 000-206 000 |
| Zn-DOTAM | 15 000 | 9 000-19 000 |

The initial choice for quenching was Cu, thus replacing unchelated DOTAM with quenched, non-competing Cu-DOTAM, and a large number of studies within the CEA-PRIT program were executed using this condition. However, it was later discovered that the Cu-DOTAM complex was, in fact, not stable in vivo, and a study was undertaken to assess its stability compared with alternative quenching ions in human and mouse serum, plasma, and blood. The selected ions were, on the one hand, Pb and Ca, with similar affinity, and on the other hand, Zn and Cu, with significantly lower affinity. The study demonstrated significantly higher stability of Zn-DOTAM compared with Cu-DOTAM in human and mouse blood, as measured by spontaneous Ca-DOTAM formation 35 minutes p.i., reaching approximately 25% and 30% in mouse and human blood, respectively, for initially injected Cu-DOTAM, compared with 0% and 0% for Zn-DOTAM, and 4% and 4% for Pb-DOTAM

Protocol 131 was designed to assess the DOTAM quenching candidate metals in vivo, comparing their effect on tumor and normal tissue accumulation of $^{212}Pb$-DOTAM after pretargeting with CEA-DOTAM and clearing with Pb-DOTAM-dextran-500. Zn and Gd, which have reasonable in vitro complex stability with DOTAM, were assessed side-by-side with Cu. In addition, Ca and stable Pb were used as controls. The study outline is shown in Figure 51.

The time course and design of Protocol 131 is shown in the two tables below.

**Time course of Protocol 131**

| **Study day** | **Experimental procedure** |
|---|---|
| 0 | Preparation of BxPC3 cells and filling of syringes |
| 0 | S.c. injection of BxPC3 cells |
| 18 | I.v. injection of BsAb |
| 25 | I.v. injection of CA |
| 26 | Elution of $^{212}Pb$-DOTAM and filling of syringes |
| 26 | I.v. injection of $^{212}Pb$-DOTAM |
| 26 | Euthanasia and tissue harvest (2 h p.i.) + gamma counting |

**Study groups in Protocol 131**

| **Group** | **n** | **BsAb** | **BsAb** (μg) | **Time** (d) | **CA** (μg) | **Time** (h) | **$^{212}$Pb** (μCi ) | **Quench** | **BD** (h p.i.) |
|---|---|---|---|---|---|---|---|---|---|
| **A** | 4 | CEA-DOTAM | 100 | 7 | 25 | 24 | 10 | Zn | 2 |
| **B** | 4 | CEA-DOTAM | 100 | 7 | 25 | 24 | 10 | Gd | 2 |
| **C** | 4 | CEA-DOTAM | 100 | 7 | 25 | 24 | 10 | Cu | 2 |
| **D** | 4 | CEA-DOTAM | 100 | 7 | 25 | 24 | 10 | Ca | 2 |
| **E** | 4 | CEA-DOTAM | 100 | 7 | 25 | 24 | 10 | Pb | 2 |

Solid xenografts were established by s.c. injection of BxPC3 cells into the right flank of SCID mice aged 5-7 weeks. Eighteen days after tumor cell injection, mice were sorted into experimental groups with an average tumor volume of 200 $mm^3$. The $^{212}$Pb-DOTAM was injected on day 26 after inoculation, at which point the average tumor volume was 350 $mm^3$.

The antibody was diluted in 20 mM His/His-HCl, 140 mM NaCl, pH 6.0 to a final concentration of 100 μg per 100 μL for i.v. administration according to the table "Study groups in Protocol 131" and Figure 51. The CA was thawed and diluted in PBS to a final concentration of 25 μg per 100 μL for i.v. administration, 7 days after the BsAb injection. After another 24 hours, $^{212}$Pb-DOTAM, quenched using either of the 5 different metals, was injected i.v. (10 μCi in 100 μL 0.9% NaCl).

Mice were sacrificed for biodistribution purposes 2 hours after injection of $^{212}$Pb-DOTAM, and the following tissues and organs harvested: blood, bladder, spleen, kidneys, liver, lung, muscle, tail, and tumor.

Results

The average $^{212}$Pb accumulation and clearance in all collected tissues 2 hours after $^{212}$Pb-DOTAM injection is displayed in Figure 52. There were no statistically significant differences in average % ID/g between any of the quenching metals, compared with the Pb control (two-way ANOVA with Dunnett's multiple comparisons test). Individual values for tumors and selected normal tissues are shown in Figure 53. A larger individual variation in $^{212}$Pb content was observed in spleen and muscle after quenching with Zn and Gd compared with the other metals, although the difference in average % ID/g between the different treatment groups was not significant (one-way ANOVA with Tukey's multiple comparisons test).

Conclusion

Protocol 131 assessed the effect on tumor and normal tissue accumulation of pretargeted $^{212}$Pb-DOTAM from Zn-, Gd-, or Cu-quenching of DOTAM, compared with Ca- or Pb-quenching. No difference in tumor uptake of $^{212}$Pb was observed, indicating no significant blocking of pretargeted binding sites using the controls Ca and Pb under the applied experimental conditions. An increased variation in $^{212}$Pb content in spleen and muscle was observed after quenching with Zn and Gd, indicating that metal-DOTAM complexes that compete with $^{212}$Pb-DOTAM might contribute to the neutralization of non-CA-bound CEA-DOTAM in non-targeted tissues. Quenching with Cu resulted in comparable results to Ca quenching, further confirming the indicated in vivo instability of Cu-DOTAM. Ca was eventually chosen for quenching of DOTAM, for three main reasons: increased control of the in vivo formulation of the $^{212}$Pb-DOTAM solution, compared with Cu quenching; the potential to augment the neutralization of non-CA-bound BsAb, reducing the variability in normal tissue uptake of $^{212}$Pb; and the potential to reduce the effect of the hypothetical "binding site barrier" phenomenon, by which the penetration of molecules with high affinity towards an antigen is restricted due to immediate binding to easily accessible targets under sub-saturating conditions.

## Example 35: Evaluation of CD20-DOTAM and HER2-DOTAM for PRIT (Protocols 151 and 152)

Protocols 151 and 152 aimed to evaluate the binding of $^{212}$Pb-DOTAM to subcutaneous tumors in mice pre-targeted by the fully humanized BsAbs CD20-DOTAM and HER2-DOTAM, respectively. Three-step PRIT was performed by injection of the antibody constructs, followed after 7 days by the Ca-DOTAM-dextran-500 CA and finally $^{212}$Pb-DOTAM, 24 hours later. Mice were sacrificed 24 hours after the radioactive injection, and blood and organs harvested for radioactive measurement.

Study design

The time courses and designs of Protocols 151 and 152 are shown in the following four tables.

**Time course of Protocol 151**

| Study day | Experimental procedure |
|---|---|
| 0 | Preparation of WSU-DLCL2 cells and filling of syringes |
| 0 | S.c. injection of WSU-DLCL2 cells |
| 5 | I.v. injection of CD20-DOTAM BsAb |
| 12 | I.v. injection of CA (groups A, B) |
| 12 | Tumor harvest and formalin fixation (groups C, D) |
| 13 | Elution of $^{212}$Pb-DOTAM and filling of syringes |
| 13 | I.v. injection of $^{212}$Pb-DOTAM (groups A, B) |
| 14 | Euthanasia and tissue harvest (24 h p.i.) + gamma counting (groups A, B) |

**Study groups in Protocol 151**

| Group | n | BsAb | BsAb (μg) | Time (d) | CA (μg ) | Time (h) | $^{212}$Pb (μCi) | BD (h p.i. ) |
|---|---|---|---|---|---|---|---|---|
| **A** | 3 | CD20-DOTAM | 100 | 7 | 25 | 24 | 20 | 6 |
| **B** | 3 | DIG-DOTAM | 100 | 7 | 25 | 24 | 20 | 6 |
| **C** | 2 | CD20-DOTAM | 100 | 7 | 0 | - | 0 | - |
| **D** | 2 | DIG-DOTAM | 100 | 7 | 0 | - | 0 | - |
| **E** | 5 | - | 0 | - | 0 | - | 0 | - |

**Time course of Protocol 152**

| Study day | Experimental procedure |
|---|---|
| 0 | Preparation of NCI-N87 cells and filling of syringes |
| 0 | S.c. injection of NCI-N87 cells |
| 14 | I.v. injection of HER2-DOTAM BsAb |
| 21 | I.v. injection of CA (groups A, B) |
| 21 | Tumor harvest and formalin fixation (groups C, D) |

(continued)

| Study day | Experimental procedure |
|---|---|
| 22 | Elution of $^{212}$Pb-DOTAM and filling of syringes |
| 22 | I.v. injection of $^{212}$Pb-DOTAM (groups A, B) |
| 23 | Euthanasia and tissue harvest (24 h p.i.) + gamma counting (groups A, B) |

**Study groups in Protocol 152**

| **Group** | **n** | **BsAb** | **BsAb** (μg) | **Time** (d) | **CA** (μg) | **Time** (h) | $^{212}$**Pb** (μCi) | **BD** (h p.i. ) |
|---|---|---|---|---|---|---|---|---|
| **A** | 3 | HER2-DOTAM | 100 | 7 | 25 | 24 | 20 | 6 |
| **B** | 3 | DIG-DOTAM | 100 | 7 | 25 | 24 | 20 | 6 |
| **C** | 2 | HER2-DOTAM | 100 | 7 | 0 | - | 0 | - |
| **D** | 2 | DIG-DOTAM | 100 | 7 | 0 | - | 0 | - |
| **E** | 5 | - | 0 | - | 0 | - | 0 | - |

Solid xenografts were established in protocol 151 by s.c. injection of CD20-expressing WSU-DLCL2 human B cell lymphoma cells in DMEM media into SCID mice aged 9 weeks. Four days after tumor cell injection, mice were sorted into experimental groups with an average tumor volume of 105 $mm^3$. The $^{212}$Pb-DOTAM was injected on day 13 after inoculation, at which point the average tumor volume was 242 $mm^3$.

In protocol 152, SCID mice aged 10 weeks were xenografted by s.c. injection of HER2-expressing NCI-N87 human gastric carcinoma cells in RPMI 1640 media. Fourteen days after tumor cell injection, mice were sorted into experimental groups with an average tumor volume of 74 $mm^3$. The $^{212}$Pb-DOTAM was injected on day 22 after inoculation; the average tumor volume being 61 $mm^3$ on day 21.

Biodistribution mice were sacrificed 24 hours after the radioactive injection. Blood was collected before termination through retro-orbital bleeding under anesthesia. All mice were necropsied after euthanasia, with additional collection of skin, bladder, stomach, small intestine, colon, spleen, pancreas, kidneys, liver, lung, heart, femoral bone, muscle, tail, and tumor.

Results

*Biodistribution - protocol 151*

The average $^{212}$Pb content in all collected tissues 24 hours after injection is displayed in figure 54. The negative control BsAb DIG-DOTAM rendered no accumulation of radioactivity in WSU-DLCL2 tumors (0.4 ± 0.3% ID/g), whereas the CD20-DOTAM BsAb resulted in 25.0 ± 3.7% ID/g. No significant $^{212}$Pb uptake was seen in normal tissues/organs, except for bladder (12.5 + 4.5% ID/g), kidneys (3.1 ± 0.3% ID/g), and lung (2.2 + 0.2% ID/g).

*Biodistribution - protocol 152*

The average $^{212}$Pb content in all collected tissues 24 hours after injection is displayed in Figure 55. The negative control BsAb DIG-DOTAM rendered no accumulation of radioactivity in NCI-N87 tumors (0.8 ± 0.4% ID/g), whereas the HER2-DOTAM BsAb resulted in 24.6 ± 1.5% ID/g. No significant $^{212}$Pb uptake was seen in normal tissues/organs, except for very low values in kidneys (1.6 ± 0.1% ID/g) and lung (1.4 ± 0.2% ID/g)

Conclusion

The results of Protocols 151 and 152 demonstrated specific targeting and in vivo proof-of-concept of CD20-PRIT and HER2-PRIT using the three-step pretargeting approach developed for CEA-PRIT.

**Example 36: In vivo distribution and tumor accumulation data for other formats (Protocol 154)**

Protocol 154 aimed to evaluate the use of 5 novel CEA-DOTAM BsAb constructs for PRIT. It was designed to provide in vivo distribution and tumor accumulation data of $^{212}$Pb-DOTAM in SCID mice carrying subcutaneous HPAF-II xenografts. Three-step PRIT was performed by injection of the CEA-DOTAM constructs, followed 7 days later by a Ca-DOTAM-dextran-500 CA and finally $^{212}$Pb-DOTAM, 24 hours after the CA. Mice were sacrificed 6 hours after the radioactive injection, and blood and organs harvested for radioactive measurement. Comparisons were made with PRIT using the standard CEA-DOTAM BsAb and a non-CEA-binding BsAb. The study outline is shown in Figure 56.

Study design

The time course and design of Protocol 154 is shown in the two tables below.

**Time course of Protocol 154**

| Study day | Experimental procedure |
|---|---|
| 0 | Preparation of HPAF-II cells and filling of syringes |
| 0 | S.c. injection of HPAF-II cells |
| 12 | I.v. injection of BsAb |
| 19 | I.v. injection of CA |
| 19 | Elution of $^{212}$Pb-DOTAM and filling of syringes |
| 20 | I.v. injection of $^{212}$Pb-DOTAM |
| 20 | Euthanasia and tissue harvest (6 h p.i.) + gamma counting |

**Study groups in Protocol 154**

| **Group** | **n** | **BsAb** | **BsAb** (μg) | **Time** (d) | **CA** (μg ) | **Time** (h) | **$^{212}$Pb** (μCi) | **BD** (h p.i.) |
|---|---|---|---|---|---|---|---|---|
| **A** | 3 | DIG-DOTAM | 100 | 7 | 25 | 24 | 10 | 6 |
| **B** | 3 | CEA-DOTAM | 100 | 7 | 25 | 24 | 10 | 6 |
| **C** | 3 | P1AE1766 | 100 | 7 | 25 | 24 | 10 | 6 |
| **D** | 3 | P1AE1767 | 100 | 7 | 25 | 24 | 10 | 6 |
| **E** | 3 | P1AE1768 | 100 | 7 | 25 | 24 | 10 | 6 |
| **F** | 3 | P1AE1769 | 100 | 7 | 25 | 24 | 10 | 6 |
| **G** | 3 | P1AE1770 | 100 | 7 | 25 | 24 | 10 | 6 |

Solid xenografts were established by s.c. injection of HPAF-II cells into the right flank of mice aged 9 weeks. Eleven days after tumor cell injection, mice were sorted into experimental groups with an average tumor volume of 79 $mm^3$. The $^{212}$Pb-DOTAM was injected on day 20 after inoculation, at which point the average tumor volume was 230 $mm^3$.

All antibodies were diluted in 20 mM His/His-HCl, 140 mM NaCl, pH 6.0 to a final concentration of 100 μg per 100 μL for i.v. administration according to the table "Study groups in Protocol 154" and Figure 56. The CA was thawed and diluted in PBS to a final concentration of 25 μg per 100 μL for i.v. administration, 7 days after the BsAb injection. After another 24 hours, $^{212}$Pb-DOTAM quenched with Ca was injected i.v. (10 μCi in 100 μL 0.9% NaCl).

Mice were sacrificed for biodistribution purposes 6 hours after injection of $^{212}$Pb-DOTAM, and the following tissues and organs harvested: blood, skin, bladder, stomach, small intestine, colon, spleen, pancreas, kidneys, liver, lung, heart, femoral bone, muscle, brain, tail, and tumor.

Results

The average $^{212}Pb$ accumulation and clearance in all collected tissues 6 hours after injection is displayed in Figure 57. The blood content and tumor accumulation is shown in larger detail in Figure 58. The negative control BsAb DIG-DOTAM rendered no accumulation of radioactivity in tumors (1.4 ± 0.1% ID/g), whereas the standard CEA-DOTAM BsAb resulted in 36.8 ± 3.4% ID/g. For the novel constructs, the corresponding numbers were 31.8 ± 3.1% ID/g (P1AE1766), 35.0 ± 8.5% ID/g (P1AE1767), 14.2 ± 6.7% ID/g (P1AE1768), 38.8 ± 10.1% ID/g (P1AE1769), and 39.5 ± 6.8% ID/g (P1AE1770). The significantly lower tumor accumulation of P1AE1768 can be explained by the CEA monovalency of this antibody construct.

No significant $^{212}Pb$ uptake was seen in normal tissues/organs, except for bladder, in which the radioactivity reached levels generally seen at 6 h p.i. for this PRIT regimen.

Conclusion

The results of the study demonstrated in vivo proof-of-concept of all tested CEA-DOTAM antibody constructs (P1AE1766, P1AE1767, P1AE1768, P1AE1769, and P1AE1770) for three-step PRIT.

**Example 37: CD20-PRIT efficacy (Protocol 162)**

The aim of this study was to show proof-of-concept of the treatment regimen developed for CEA-PRIT directed against an alternative target: cluster of differentiation 20 (CD20). The therapeutic efficacy was evaluated after 3 cycles of CD20-PRIT in mice bearing subcutaneous WSU-DLCL2 tumors. Comparisons were also made with: 1-step CD20-RIT, using BsAbs that were pre-incubated with $^{212}Pb$-DOTAM before injection; with rituximab, a type I monoclonal antibody directed against CD20 and a reference in treatment of CD20+ diseases; and with GA101, a type II monoclonal antibody also directed against CD20.

Study design, protocol 162

| Study day | Experimental procedure |
|---|---|
| 0 | Preparation of WSU-DLCL2 cells and filling of syringes |
| 0 | S.c. injection of WSU-DLCL2 cells |
| 7 | I.p. injection of PRIT BsAb or histidine buffer (groups A, B, C, E, H, I, J, K) |
| 14 | I.p. injection of CA or histidine buffer (groups A, B, C, H, I, J, K) |
| 15 | Elution of $^{212}Pb$-DOTAM and filling of syringes |
| 15 | I.v. injection of $^{212}Pb$-DOTAM-CD20-DOTAM (groups D, L) |
| 15 | I.v. injection of $^{212}Pb$-DOTAM or 0.9% NaCl (groups A, B, C, H, I, J, K) |
| 15 | I.v. injection of GA101 or rituximab (groups F, G) |
| 16 | Euthanasia and tissue harvest (24 h p.i.) + gamma counting (groups H, I, L) |
| 21 | I.p. injection of PRIT BsAb or histidine buffer (groups A, B, C, E, J, K) |
| 28 | I.p. injection of CA or histidine buffer (groups A, B, C, J, K) |
| 29 | Elution of $^{212}Pb$-DOTAM and filling of syringes |
| 29 | I.v. injection of $^{212}Pb$-DOTAM-CD20-DOTAM (group D) |
| 29 | I.v. injection of $^{212}Pb$-DOTAM or 0.9% NaCl (groups A, B, C, J, K) |
| 29 | I.v. injection of GA101 or rituximab (groups F, G) |
| 30 | Euthanasia and tissue harvest (24 h p.i.) + gamma counting (group J) |
| 35 | I.p. injection of PRIT BsAb or histidine buffer (groups A, B, C, E, K) |
| 42 | I.p. injection of CA or histidine buffer (groups A, B, C, K) |

(continued)

| Study day | Experimental procedure |
|---|---|
| 43 | Elution of $^{212}$Pb-DOTAM and filling of syringes |
| 43 | I.v. injection of $^{212}$Pb-DOTAM-CD20-DOTAM (group D) |
| 43 | I.v. injection of $^{212}$Pb-DOTAM or 0.9% NaCl (groups A, B, C, K) |
| 43 | I.v. injection of GA101 or rituximab (groups F, G) |
| 44 | Euthanasia and tissue harvest (24 h p.i.) + gamma counting (group K) |

Study groups in protocol 162 ($n_{tot}$ = 85)

| Group | BsAb | BsAb per cycle (μg) | CA per cycle (μg) | $^{212}$Pb-DOTAM per cycle (μCi) | Treatment cycles (#) | n (mice) |
|---|---|---|---|---|---|---|
| A | - | 0 | 0 | 0 | 3 | 10 |
| B | DIG-DOTAM | 100 | 25 | 20 | 3 | 10 |
| C | CD20-DOTAM | 100 | 25 | 20 | 3 | 10 |
| D | $^{212}$Pb-DOTAM-CC20-DOTAM | 20 | 0 | 10 | 3 | 10 |
| E | CD20-DOTAM | 100 | 0 | 0 | 3 | 10 |
| F | GA101 | 600 | 0 | 0 | 3 | 10 |
| G | Rituximab | 600 | 0 | 0 | 3 | 10 |
| H | DIG-DOTAM | 100 | 25 | 20 | 1 | |
| I | CD20-DOTAM | 100 | 25 | 20 | 1 | 3 |
| J | CD20-DOTAM | 100 | 25 | 20 | 2 | 3 |
| K | CD20-DOTAM | 100 | 25 | 20 | 3 | 3 |
| L | $^{212}$Pb-DOTAM-CEA-DOTAM | 20 | 0 | 10 | 1 | 3 |

Animals were treated according to the experimental schedule illustrated in figure 59. Solid WSU-DLCL2 xenografts were established in SCID mice aged 8 weeks on study day 0 by subcutaneous injection of $1.5\times10^5$ cells into the right flank. Seven days after tumor cell injection, mice were sorted into experimental groups with an average tumor volume of 144 $mm^3$. The $^{212}$Pb-DOTAM was injected on day 15 after inoculation; the average tumor volume was 306 $mm^3$ on day 14.

Mice in groups A-G were followed to evaluate the efficacy of the treatments. For PRIT, the administered activity was 20 μCi per cycle, whereas the corresponding activity for 1-step RIT was 10 μCi, to avoid acute radiation-induced toxicity following from the longer retention time in blood and normal tissues using this regimen.

Mice in groups H, I, and L were sacrificed and necropsied for biodistribution purposes 24 hours after their first and only injection of $^{212}$Pb-DOTAM or $^{212}$Pb-DOTAM-BsAb; groups J and K were sacrificed and necropsied 24 hours after their second and third $^{212}$Pb-DOTAM injection, respectively. The following organs and tissues were harvested from these mice: blood, bladder, spleen, kidneys, liver, lung, muscle, tail, skin, and tumor. Collected samples were weighed and then measured for radioactivity using a 2470 $WIZARD^2$ automatic gamma counter (PerkinElmer), and the percent injected dose per gram of tissue (% ID/g) subsequently calculated, including corrections for decay and background.

For PRIT, either CEA-DOTAM BsAb or DIG-DOTAM (negative control BsAb) was diluted in 20 mM Histidine, 140 mM NaCl (pH 6.0) to a final concentration of 100 μg per 200 μL for i.p. administration. The Ca-DOTAM-dextran-500 CA was administered i.p. (25 μg per 200 μL) 7 days after the BsAb injection, followed 24 hours later by Ca-quenched $^{212}$Pb-DOTAM (20 μCi in 100 μL).

Mice treated with 1-step RIT received pre-bound $^{212}$Pb-DOTAM-CD20-DOTAM, prepared by incubating the $^{212}$Pb-DOTAM with the CD20-DOTAM BsAb for 10 minutes at 37°C before injecting i.v. (20 pCi/20 μg BsAb in 100 μL of 0.9% NaCl) .

Mice treated with Rituximab (MabThera®) or GA101 (obinutuzumab) were injected i.v. with the respective antibodies, diluted in 20 mM Histidine, 140 mM NaCl (pH 6.0) to a final concentration of 600 μg in 200 μL. The injections were performed on the same day as the radioactive injections for the PRIT- and RIT-treated groups.

Preliminary results, 162

The study is still underway at the time of writing this example, but preliminary results can be reported accordingly: The average $^{212}$Pb accumulation and clearance in all collected tissues 24 hours after injection is shown for the first treatment cycle in Figure 60. The negative PRIT control resulted in no uptake (0.7% ID/g) in tumor. The tumor uptake was 22% ID/g for both CD20-PRIT and CD20-RIT.

The average tumor development for the assessed treatments is shown in Figure 61. Tumors in the non-treated vehicle group, the DIG-DOTAM group, and the antibody-based treatment groups grew steadily. In contrast, tumors in the CD20-RIT and CD20-PRIT groups decreased in size after the first treatment cycle.

The BW development in all therapy groups is shown in figure 62. The multiple injections of 20 μCi of $^{212}$Pb-DOTAM were well tolerated but the 10 μCi of pre-bound $^{212}$Pb-DOTAM-CD20-DOTAM resulted in a significant drop in BW.

Summary and Conclusion

The study showed proof of concept of CD20-PRIT, with indications of comparable tumor growth inhibition to that previously demonstrated for CEA-PRIT. The CD20-PRIT treatment was well tolerated, which was not the case for the 1-step CD20-RIT treatment.

## Claims

1. An antibody comprising an antigen binding site specific for DOTAM-lead (Pb) chelate, wherein said antigen binding site comprises at least:

   a) a heavy chain CDR1;
   b) heavy chain CDR2 comprising the amino acid sequence FIGSRGDTYYASWAKG (SEQ ID NO:2), or a variant thereof having up to 1, 2, or 3 substitutions in SEQ ID NO: 2, wherein these substitutions do not include Phe50, Asp56 and Tyr58, and optionally also do not include Gly52 and/or Arg 54;
   c) heavy chain CDR3 comprising the amino acid sequence ERDPYGGGAYPPHL (SEQ ID NO:3), or a variant thereof having up to 1, 2, or 3 substitutions in SEQ ID NO: 3, wherein these substitutions do not include Glu95, Arg96, Asp97, Pro98, and optionally also do not include Ala100C, Tyr100D, and/or Pro100E and/or optionally also do not include Tyr99;
   d) light chain CDR1 comprising the amino acid sequence QSSHSVYSDNDLA (SEQ ID NO:4) or a variant thereof having up to 1, 2, or 3 substitutions in SEQ ID NO: 4, wherein these substitutions do not include Tyr28 and Asp32;
   e) a light chain CDR2; and
   f) light chain CDR3 comprising the amino acid sequence LGGYDDESDTYG (SEQ ID NO:6) or a variant thereof having up to 1, 2, or 3 substitutions in SEQ ID NO: 6, wherein these substitutions do not include Gly91, Tyr92, Asp93, Thr95c and Tyr96,

   wherein numbering is according to Kabat.

2. The antibody of claim 1, wherein:

   a) the heavy chain CDR1 comprises the amino acid sequence GFSLSTYSMS (SEQ ID NO:1) or a variant thereof having up to 1, 2, or 3 substitutions in SEQ ID NO: 1, optionally conservative substitutions; and/or
   e) the light chain CDR2 comprises the amino acid sequence QASKLAS (SEQ ID NO: 5) or a variant thereof having at least 1, 2 or 3 substitutions in SEQ ID NO: 5, optionally conservative substitutions.

3. The antibody of claim 1 or claim 2, wherein the antigen binding site comprises at least one, two, three, four, five, or six CDRs selected from:

a) heavy chain CDR1 comprising the amino acid sequence GFSLSTYSMS (SEQ ID NO:1);
b) heavy chain CDR2 comprising the amino acid sequence FIGSRGDTYYASWAKG (SEQ ID NO:2);
c) heavy chain CDR3 comprising the amino acid sequence ERDPYGGGAYPPHL (SEQ ID NO:3);
d) light chain CDR1 comprising the amino acid sequence QSSHSVYSDNDLA (SEQ ID NO:4);
e) light chain CDR2 comprising the amino acid sequence QASKLAS (SEQ ID NO: 5); and
f) light chain CDR3 comprising the amino acid sequence LGGYDDESDTYG (SEQ ID NO:6).

**4.** The antibody of claim 1 or claim 2, wherein the antigen binding site comprises:

b) heavy chain CDR2 comprising the amino acid sequence FIGSRGDTYYASWAKG (SEQ ID NO:2)
c) heavy chain CDR3 comprising the amino acid sequence ERDPYGGGAYPPHL (SEQ ID NO:3);
d) light chain CDR1 comprising the amino acid sequence QSSHSVYSDNDLA (SEQ ID NO:4); and
f) light chain CDR3 comprising the amino acid sequence LGGYDDESDTYG (SEQ ID NO:6).

**5.** The antibody of claim 1, wherein the antigen binding site comprises:

a) heavy chain CDR1 comprising the amino acid sequence GFSLSTYSMS (SEQ ID NO:1);
b) heavy chain CDR2 comprising the amino acid sequence FIGSRGDTYYASWAKG (SEQ ID NO:2);
c) heavy chain CDR3 comprising the amino acid sequence ERDPYGGGAYPPHL (SEQ ID NO:3);
d) light chain CDR1 comprising the amino acid sequence QSSHSVYSDNDLA (SEQ ID NO:4);
e) light chain CDR2 comprising the amino acid sequence QASKLAS (SEQ ID NO: 5); and
f) light chain CDR3 comprising the amino acid sequence LGGYDDESDTYG (SEQ ID NO:6).

**6.** The antibody according to any one of the preceding claims, which is human, chimeric or humanized.

**7.** An antibody according to any one of the preceding claims, wherein

i) the antigen binding site comprises a heavy chain variable domain comprising an amino acid sequence selected from the group consisting of SEQ ID NO: 7 and SEQ ID NO 9, or a variant thereof comprising an amino acid sequence having at least 90, 91, 92, 93, 94, 95, 96, 97, 98, or 99% identity to SEQ ID NO: 7 or SEQ ID NO: 9; and/or
ii) the antigen binding site comprises a light chain variable domain comprising an amino acid sequence selected from the group consisting of SEQ ID NO: 8 and SEQ ID NO: 10, or a variant thereof comprising an amino acid sequence having at least 90, 91, 92, 93, 94, 95, 96, 97, 98, or 99% identity to SEQ ID NO: 8 or 10.

**8.** An antibody according to any one of the preceding claims, wherein

i) the antigen binding site comprises a heavy chain variable domain comprising an amino acid sequence of SEQ ID No. 7 and a light chain variable domain comprising an amino acid sequence of SEQ ID NO. 8; or
ii) the antigen binding site comprises a heavy chain variable domain comprising an amino acid sequence of SEQ ID No. 9 and a light chain variable domain comprising an amino acid sequence of SEQ ID NO. 10.

**9.** An antibody according to any one of the preceding claims, wherein the antigen binding site binds to the Pb-DOTAM chelate with a Kd value of 100pM, 50pM, 20pM, 10pM, 5pM, 1pM or less, as measured by KinExA (kinetic exclusion) assay.

**10.** An antibody according to any one of the preceding claims, wherein the antigen binding site binds to the Pb-DOTAM chelate and to a Bi-DOTAM chelate, and wherein the ratio of Kd values for the Bi-DOTAM chelate/Pb-DOTAM chelate is in the range of 0.1-10 or 1-10, as measured by KinExA (kinetic exclusion) assay.

**11.** An antibody according to any one of the preceding claims, which is a whole antibody, or which is an antibody fragment selected from the group consisting of an Fv, Fab, Fab', Fab'-SH, $F(ab')_2$, diabody, linear antibody, or single-chain antibody molecule.

**12.** An antibody according to any one of the preceding claims, wherein the antibody is coupled to a moiety which binds specifically to a target antigen.

**13.** The antibody according to claim 12, wherein the target antigen is a tumour specific antigen.

**14.** The antibody according to claim 12 or 13, which is a multispecific or bispecific antibody.

**15.** The multispecific or bispecific antibody of claim 14, wherein the tumour specific antigen is selected from the group consisting of CEA, HER2 and CD20.

**16.** The multispecific or bispecific antibody of claim 14 or 15, wherein the multispecific or bispecific antibody comprises at least one antigen binding site specific for the Pb-DOTAM chelate and at least one antigen binding site specific for CEA, and wherein the antigen binding site specific for CEA comprises a heavy chain comprising at least one, two or three heavy chain CDRs: wherein:

a) heavy chain CDR1 comprises the amino acid sequence of SEQ ID NO: 11
b) heavy chain CDR2 comprises the amino acid sequence of SEQ ID NO: 12
c) heavy chain CDR3 comprises the amino acid sequence of SEQ ID NO: 13;

and/or the antigen binding site specific for CEA comprises a light chain comprising at least one, two or three light chain CDRs: wherein:

d) light chain CDR1 comprises the amino acid sequence SEQ ID NO: 14;
e) light chain CDR2 comprises the amino acid sequence SEQ ID NO:15;
f) light chain CDR3 comprises the amino acid sequence SEQ ID NO: 16.

**17.** The multispecific or bispecific antibody of claim 14 or 15, wherein the multispecific or bispecific antibody comprises at least one antigen binding site specific for the Pb-DOTAM chelate and at least one antigen binding site specific for CEA, and wherein the antigen binding site specific for CEA comprise

i) a heavy chain variable domain comprising an amino acid sequence of SEQ ID NO: 17, or a variant thereof comprising an amino acid sequence having at least 90, 91, 92, 93, 94, 95, 96, 97, 98, or 99% identity to SEQ ID NO: 17; and/or
ii) a light chain variable domain comprising an amino acid sequence of SEQ ID NO: 18, or a variant thereof comprising an amino acid sequence having at least 90, 91, 92, 93, 94, 95, 96, 97, 98, or 99% identity to SEQ ID NO: 18.

**18.** The multispecific or bispecific antibody according to claim 14 or claim 15, wherein the multispecific or bispecific antibody comprises at least one antigen binding site specific for a Pb-DOTAM chelate and at least one antigen binding site specific for HER2, and wherein the antigen binding site specific for HER2 comprises at least one, two, three, four, five, or six CDRs selected from (a)CDR-H1 comprising the amino acid sequence of SEQ ID NO:28; (b) CDR-H2 comprising the amino acid sequence of SEQ ID NO:29; (c) CDR-H3 comprising the amino acid sequence of SEQ ID NO:30; (d) CDR-L1 comprising the amino acid sequence of SEQ ID NO:31; (e) CDR-L2 comprising the amino acid sequence of SEQ ID NO:32; and (f) CDR-L3 comprising the amino acid sequence of SEQ ID NO:33.

**19.** The multispecific or bispecific antibody according to claim 14 or claim 15, wherein the multispecific or bispecific antibody comprises at least one antigen binding site specific for the Pb-DOTAM chelate and at least one antigen binding site specific for HER2, and wherein the antigen binding site specific for HER2 comprises

i) a heavy chain variable domain comprising an amino acid sequence of SEQ ID NO: 34, or a variant thereof comprising an amino acid sequence having at least 90, 91, 92, 93, 94, 95, 96, 97, 98, or 99% identity to SEQ ID NO: 34; and/or
ii) a light chain variable domain comprising an amino acid sequence of SEQ ID NO: 35, or a variant thereof comprising an amino acid sequence having at least 90, 91, 92, 93, 94, 95, 96, 97, 98, or 99% identity to SEQ ID NO: 35.

**20.** The multispecific or bispecific antibody according to claim 14 or claim 15, wherein the multispecific or bispecific antibody comprises at least one antigen binding site specific for a Pb-DOTAM chelate and at least one antigen binding site specific for CD20, and wherein the antigen binding site specific for CD20 comprises at least one, two, three, four, five, or six CDRs selected from (a)CDR-H1 comprising the amino acid sequence of SEQ ID NO:39; (b) CDR-H2 comprising the amino acid sequence of SEQ ID NO:40; (c) CDR-H3 comprising the amino acid sequence of SEQ ID NO:41; (d) CDR-L1 comprising the amino acid sequence of SEQ ID NO:42; (e) CDR-L2 comprising the amino acid sequence of SEQ ID NO:43; and (f) CDR-L3 comprising the amino acid sequence of SEQ ID NO:44.

**21.** The multispecific or bispecific antibody according to claim 14, wherein the multispecific or bispecific antibody comprises at least one antigen binding site specific for the Pb-DOTAM chelate and at least one antigen binding site specific for CD20, and wherein the antigen binding site specific for CD20 comprises

i) a heavy chain variable domain comprising an amino acid sequence of SEQ ID NO: 45, or a variant thereof comprising an amino acid sequence having at least 90, 91, 92, 93, 94, 95, 96, 97, 98, or 99% identity to SEQ ID NO: 45; and/or
ii) a light chain variable domain comprising an amino acid sequence of SEQ ID NO: 46, or a variant thereof comprising an amino acid sequence having at least 90, 91, 92, 93, 94, 95, 96, 97, 98, or 99% identity to SEQ ID NO: 46.

**22.** The multispecific or bispecific antibody of any one of claims 14 to 21, which comprises an Fc region.

**23.** The multispecific or bispecific antibody of claim 22, wherein the Fc region is engineered to reduce effector function, optionally by substitution of one or more of Fc region residues 234, 235, 238, 265, 269, 270, 297, 327 and/or 329.

**24.** The multispecific or bispecific antibody of claim 22 or 23, comprising:

i) a full length antibody comprising an antigen binding site for a first antigen; and
ii) at least a second heavy chain variable domain and second light chain variable domain which together form an antigen binding site for a second antigen,

wherein either the first or the second antigen is the Pb-DOTAM chelate, and the other is the target antigen, optionally wherein the first antigen is the target antigen and the second antigen is the Pb-DOTAM chelate.

**25.** The multispecific or bispecific antibody of claim 24, comprising a full length antibody comprising an antigen binding site for the first antigen, wherein the N- or C-terminus of one of the heavy chains is linked via a polypeptide linker to a first polypeptide and wherein the first polypeptide associates with a second polypeptide to form a Fab or a cross-Fab comprising a binding site for the second antigen.

**26.** The multispecific or bispecific antibody of claim 25, which comprises:

i) a first polypeptide consisting of a VH domain and a CH1 domain, which is associated with a second polypeptide consisting of a VL and CL domain; or
ii) a first polypeptide consisting of a VL domain and a CH1 domain, which is associated with a second polypeptide consisting of a VH and CL domain; or
iii) a first polypeptide consisting of a VH domain and a CL domain, which is associated with a second polypeptide consisting of a VL and CH1 domain; such that the first and second polypeptide together form an antigen binding site for the second antigen.

**27.** The multispecific or bispecific antibody of claim 26, which comprises a full length antibody comprising an antigen binding site for the first antigen, wherein the C-terminus of one of the heavy chains is linked via a polypeptide linker to a first polypeptide consisting of a VL domain and a CH1 domain, which is associated with a second polypeptide consisting of a VH and CL domain.

**28.** An isolated polynucleotide or an isolated set of polynucleotides encoding an antibody according to any one of the preceding claims.

**29.** An expression vector or set of expression vectors comprising the polynucleotide or the set of polynucleotides according to claim 28.

**30.** A prokaryotic or eukaryotic host cell comprising an isolated polynucleotide or an isolated set of polynucleotides according to claim 28, optionally comprising the vector or set of vectors of claim 29.

**31.** A method of producing an antibody according to any one of claims 1 to 27, comprising expressing the antibody from the host cell of claim 30.

## Patentansprüche

1. Antikörper, umfassend eine Antigenbindungsstelle, die für ein DOTAM-Blei- (Pb-) Chelat spezifisch ist, wobei die Antigenbindungsstelle zumindest Folgendes umfasst:

   a) eine Schwerketten-CDR1;
   b) eine Schwerketten-CDR2, umfassend die Aminosäuresequenz FIGSRGDTYYASWAKG (SEQ ID NO: 2) oder eine Variante davon mit bis zu 1, 2 oder 3 Substitutionen in SEQ ID NO: 2, wobei diese Substitutionen Phe50, Asp56 und Tyr58 nicht einschließen und gegebenenfalls auch Gly52 und/oder Arg54 nicht einschließen;
   c) eine Schwerketten-CDR3, umfassend die Aminosäuresequenz ERDPYGGGAYPPHL (SEQ ID NO: 3) oder eine Variante davon mit bis zu 1, 2 oder 3 Substitutionen in SEQ ID NO: 3, wobei diese Substitutionen Glu95, Arg96, Asp97, Pro98 nicht einschließen und gegebenenfalls auch Ala100C, Tyr1000 und/oder Pro100E nicht einschlie-βen und/oder gegebenenfalls auch Tyr99 nicht einschließen;
   d) Leichtketten-CDR1, umfassend die Aminosäuresequenz QSSHSVYSDNDLA (SEQ ID NO: 4) oder eine Variante davon mit bis zu 1, 2 oder 3 Substitutionen in SEQ ID NO: 4, wobei diese Substitutionen Tyr28 und Asp32 nicht einschließen;
   e) eine Leichtketten-CDR2; und
   f) eine Leichtketten-CDR3, umfassend die Aminosäuresequenz LGGYDDESDTYG (SEQ ID NO: 6) oder eine Variante davon mit bis zu 1, 2 oder 3 Substitutionen in SEQ ID NO: 6, wobei diese Substitutionen Gly91, Tyr92, Asp93, Thr95c und Tyr96 nicht einschließen,

   wobei die Nummerierung gemäß Kabat bereitgestellt ist.

2. Antikörper nach Anspruch 1, wobei:

   a) die Schwerketten-CDR1 die Aminosäuresequenz GFSLSTYSMS (SEQ ID NO: 1) oder eine Variante davon mit bis zu 1, 2 oder 3 Substitutionen in SEQ ID NO: 1, gegebenenfalls konservativen Substitutionen, umfasst; und/oder
   e) die Leichtketten-CDR2 die Aminosäuresequenz QASKLAS (SEQ ID NO: 5) oder eine Variante davon mit zumindest 1, 2 oder 3 Substitutionen in SEQ ID NO: 5, gegebenenfalls konservativen Substitutionen, umfasst.

3. Antikörper nach Anspruch 1 oder Anspruch 2, wobei die Antigenbindungsstelle zumindest eine, zwei, drei, vier, fünf oder sechs CDRs umfasst, die aus Folgenden ausgewählt sind:

   a) Schwerketten-CDR1, umfassend die Aminosäuresequenz GFSLSTYSMS (SEQ ID NO: 1);
   b) Schwerketten-CDR2, umfassend die Aminosäuresequenz FIGSRGDTYYASWAKG (SEQ ID NO: 2);
   c) Schwerketten-CDR3, umfassend die Aminosäuresequenz ERDPYGGGAYPPHL (SEQ ID NO: 3);
   d) Leichtketten-CDR1, umfassend die Aminosäuresequenz QSSHSVYSDNDLA (SEQ ID NO: 4);
   e) Leichtketten-CDR2, umfassend die Aminosäuresequenz QASKLAS (SEQ ID NO: 5); und
   f) Leichtketten-CDR3, umfassend die Aminosäuresequenz LGGYDDESDTYG (SEQ ID NO: 6).

4. Antikörper nach Anspruch 1 oder Anspruch 2, wobei die Antigenbindungsstelle Folgendes umfasst:

   b) Schwerketten-CDR2, umfassend die Aminosäuresequenz FIGSRGDTYYASWAKG (SEQ ID NO: 2);
   c) Schwerketten-CDR3, umfassend die Aminosäuresequenz ERDPYGGGAYPPHL (SEQ ID NO: 3);
   d) Leichtketten-CDR1, umfassend die Aminosäuresequenz QSSHSVYSDNDLA (SEQ ID NO: 4); und
   f) Leichtketten-CDR3, umfassend die Aminosäuresequenz LGGYDDESDTYG (SEQ ID NO: 6).

5. Antikörper nach Anspruch 1, wobei die Antigenbindungsstelle Folgendes umfasst:

   a) Schwerketten-CDR1, umfassend die Aminosäuresequenz GFSLSTYSMS (SEQ ID NO: 1);
   b) Schwerketten-CDR2, umfassend die Aminosäuresequenz FIGSRGDTYYASWAKG (SEQ ID NO: 2);
   c) Schwerketten-CDR3, umfassend die Aminosäuresequenz ERDPYGGGAYPPHL (SEQ ID NO: 3);
   d) Leichtketten-CDR1, umfassend die Aminosäuresequenz QSSHSVYSDNDLA (SEQ ID NO: 4);
   e) Leichtketten-CDR2, umfassend die Aminosäuresequenz QASKLAS (SEQ ID NO: 5); und
   f) Leichtketten-CDR3, umfassend die Aminosäuresequenz LGGYDDESDTYG (SEQ ID NO: 6).

6. Antikörper nach einem der vorangegangenen Ansprüche, der menschlich, chimär oder humanisiert ist.

**7.** Antikörper nach einem der vorangegangenen Ansprüche, wobei

i) die Antigenbindungsstelle eine variable Schwerkettendomäne, umfassend eine Aminosäuresequenz, ausgewählt aus der aus SEQ ID NO: 7 und SEQ ID NO: 9 bestehenden Gruppe, oder eine Variante davon, umfassend eine Aminosäuresequenz mit einer Identität von zumindest 90, 91, 92, 93, 94, 95, 96, 97, 98 oder 99 % mit SEQ ID NO: 7 oder SEQ ID NO: 9, umfasst; und/oder
ii) die Antigenbindungsstelle eine variable Leichtkettendomäne, umfassend eine Aminosäuresequenz, ausgewählt aus der aus SEQ ID NO: 8 und SEQ ID NO: 10 bestehenden Gruppe, oder eine Variante davon, umfassend eine Aminosäuresequenz mit einer Identität von zumindest 90, 91, 92, 93, 94, 95, 96, 97, 98 oder 99 % mit SEQ ID NO: 8 oder SEQ ID NO: 10, umfasst.

**8.** Antikörper nach einem der vorangegangenen Ansprüche, wobei

i) die Antigenbindungsstelle eine variable Schwerkettendomäne, umfassend eine Aminosäuresequenz von SEQ ID NO: 7, und eine variable Leichtkettendomäne, umfassend eine Aminosäuresequenz von SEQ ID NO: 8, umfasst; oder
ii) die Antigenbindungsstelle eine variable Schwerkettendomäne, umfassend eine Aminosäuresequenz von SEQ ID NO: 9, und eine variable Leichtkettendomäne, umfassend eine Aminosäuresequenz von SEQ ID NO: 10, umfasst.

**9.** Antikörper nach einem der vorangegangenen Ansprüche, wobei die Antigenbindungsstelle mit einem Kd-Wert von 100 pM, 50 pM, 20 pM, 10 pM, 5 pM, 1 pM oder weniger, wie durch den KinExA- (kinetischer Ausschluss-) Assay gemessen, an das Pb-DOTAM-Chelat bindet.

**10.** Antikörper nach einem der vorangegangenen Ansprüche, wobei die Antigenbindungsstelle an das Pb-DOTAM-Chelat und an ein Bi-DOTAM-Chelat bindet und wobei das Verhältnis von Kd-Werten für das Bi-DOTAM-Chelat/Pb-DOTAM-Chelat im Bereich von 0,1 bis 10 oder 1 bis 10 liegt, wie durch den KinExA- (kinetischer Ausschluss-) Assay gemessen.

**11.** Antikörper nach einem der vorangegangenen Ansprüche, der ein ganzer Antikörper ist oder der ein Antikörperfragment ist, das aus der aus einem Fv, Fab, Fab', Fab'-SH, $F(ab')_2$, Diabody, linearen Antikörper oder Einzelketten-Antikörpermolekül bestehenden Gruppe ausgewählt ist.

**12.** Antikörper nach einem der vorangegangenen Ansprüche, wobei der Antikörper an eine Gruppierung gekoppelt ist, die spezifisch an ein Zielantigen bindet.

**13.** Antikörper nach Anspruch 12, wobei das Zielantigen ein tumorspezifisches Antigen ist.

**14.** Antikörper nach Anspruch 12 oder 13, der ein multispezifischer oder bispezifischer Antikörper ist.

**15.** Multispezifischer oder bispezifischer Antikörper nach Anspruch 14, wobei das tumorspezifische Antigen aus der aus CEA, HER2 und CD20 bestehenden Gruppe ausgewählt ist.

**16.** Multispezifischer oder bispezifischer Antikörper nach Anspruch 14 oder 15, wobei der multispezifische oder bispezifische Antikörper zumindest eine Antigenbindungsstelle umfasst, die für das Pb-DOTAM-Chelat spezifisch ist, und zumindest eine Antigenbindungsstelle umfasst, die für CEA spezifisch ist, und wobei die für CEA spezifische Antigenbindungsstelle eine Schwerkette umfasst, die zumindest eine, zwei oder drei Schwerketten-CDRs umfasst: wobei:

a) Schwerketten-CDR1 die Aminosäuresequenz von SEQ ID NO: 11 umfasst;
b) Schwerketten-CDR2 die Aminosäuresequenz von SEQ ID NO: 12 umfasst;
c) Schwerketten-CDR3 die Aminosäuresequenz von SEQ ID NO: 13 umfasst;

und/oder die für CEA spezifische Antigenbindungsstelle eine Leichtkette umfasst, die zumindest eine, zwei oder drei Leichtketten-CDRs umfasst: wobei:

d) Leichtketten-CDR1 die Aminosäuresequenz SEQ ID NO: 14 umfasst;
e) Leichtketten-CDR2 die Aminosäuresequenz SEQ ID NO: 15 umfasst;

f) Leichtketten-CDR3 die Aminosäuresequenz SEQ ID NO: 16 umfasst.

**17.** Multispezifischer oder bispezifischer Antikörper nach Anspruch 14 oder 15, wobei der multispezifische oder bispezifische Antikörper zumindest eine Antigenbindungsstelle umfasst, die für das Pb-DOTAM-Chelat spezifisch ist, und zumindest eine Antigenbindungsstelle umfasst, die für CEA spezifisch ist, und wobei die für CEA spezifische Antigenbindungsstelle Folgendes umfasst:

i) eine variable Schwerkettendomäne, umfassend eine Aminosäuresequenz von SEQ ID NO: 17, oder eine Variante davon, umfassend eine Aminosäuresequenz mit einer Identität von zumindest 90, 91, 92, 93, 94, 95, 96, 97, 98 oder 99 % mit SEQ ID NO: 17; und/oder
ii) eine variable Leichtkettendomäne, umfassend eine Aminosäuresequenz von SEQ ID NO: 18, oder eine Variante davon, umfassend eine Aminosäuresequenz mit einer Identität von zumindest 90, 91, 92, 93, 94, 95, 96, 97, 98 oder 99 % mit SEQ ID NO: 18.

**18.** Multispezifischer oder bispezifischer Antikörper nach Anspruch 14 oder 15, wobei der multispezifische oder bispezifische Antikörper zumindest eine Antigenbindungsstelle umfasst, die für das Pb-DOTAM-Chelat spezifisch ist, und zumindest eine Antigenbindungsstelle umfasst, die für HER2 spezifisch ist, und wobei die für HER2 spezifische Antigenbindungsstelle zumindest eine, zwei, drei, vier, fünf oder sechs CDRs umfasst, die aus (a) CDR-H1, umfassend die Aminosäuresequenz von SEQ ID NO: 28; (b) CDR-H2, umfassend die Aminosäuresequenz von SEQ ID NO: 29; (c) CDR-H3, umfassend die Aminosäuresequenz von SEQ ID NO: 30; (d) CDR-L1, umfassend die Aminosäuresequenz von SEQ ID NO: 31; (e) CDR-L2, umfassend die Aminosäuresequenz von SEQ ID NO: 32; und (f) CDR-L3, umfassend die Aminosäuresequenz von SEQ ID NO: 33, ausgewählt sind.

**19.** Multispezifischer oder bispezifischer Antikörper nach Anspruch 14 oder 15, wobei der multispezifische oder bispezifische Antikörper zumindest eine Antigenbindungsstelle umfasst, die für das Pb-DOTAM-Chelat spezifisch ist, und zumindest eine Antigenbindungsstelle umfasst, die für HER2 spezifisch ist, und wobei die für HER2 spezifische Antigenbindungsstelle Folgendes umfasst:

i) eine variable Schwerkettendomäne, umfassend eine Aminosäuresequenz von SEQ ID NO: 34, oder eine Variante davon, umfassend eine Aminosäuresequenz mit einer Identität von zumindest 90, 91, 92, 93, 94, 95, 96, 97, 98 oder 99 % mit SEQ ID NO: 34; und/oder
ii) eine variable Leichtkettendomäne, umfassend eine Aminosäuresequenz von SEQ ID NO: 35, oder eine Variante davon, umfassend eine Aminosäuresequenz mit einer Identität von zumindest 90, 91, 92, 93, 94, 95, 96, 97, 98 oder 99 % mit SEQ ID NO: 35.

**20.** Multispezifischer oder bispezifischer Antikörper nach Anspruch 14 oder 15, wobei der multispezifische oder bispezifische Antikörper zumindest eine Antigenbindungsstelle umfasst, die für das Pb-DOTAM-Chelat spezifisch ist, und zumindest eine Antigenbindungsstelle umfasst, die für CD20 spezifisch ist, und wobei die für CD20 spezifische Antigenbindungsstelle zumindest eine, zwei, drei, vier, fünf oder sechs CDRs umfasst, die aus (a) CDR-H1, umfassend die Aminosäuresequenz von SEQ ID NO: 39; (b) CDR-H2, umfassend die Aminosäuresequenz von SEQ ID NO: 40; (c) CDR-H3, umfassend die Aminosäuresequenz von SEQ ID NO: 41; (d) CDR-L1, umfassend die Aminosäuresequenz von SEQ ID NO: 42; (e) CDR-L2, umfassend die Aminosäuresequenz von SEQ ID NO: 43; und (f) CDR-L3, umfassend die Aminosäuresequenz von SEQ ID NO: 44, ausgewählt sind.

**21.** Multispezifischer oder bispezifischer Antikörper nach Anspruch 14, wobei der multispezifische oder bispezifische Antikörper zumindest eine Antigenbindungsstelle umfasst, die für das Pb-DOTAM-Chelat spezifisch ist, und zumindest eine Antigenbindungsstelle umfasst, die für CD20 spezifisch ist, und wobei die für CD20 spezifische Antigenbindungsstelle Folgendes umfasst:

i) eine variable Schwerkettendomäne, umfassend eine Aminosäuresequenz von SEQ ID NO: 45, oder eine Variante davon, umfassend eine Aminosäuresequenz mit einer Identität von zumindest 90, 91, 92, 93, 94, 95, 96, 97, 98 oder 99 % mit SEQ ID NO: 45; und/oder
ii) eine variable Leichtkettendomäne, umfassend eine Aminosäuresequenz von SEQ ID NO: 46, oder eine Variante davon, umfassend eine Aminosäuresequenz mit einer Identität von zumindest 90, 91, 92, 93, 94, 95, 96, 97, 98 oder 99 % mit SEQ ID NO: 46.

**22.** Multispezifischer oder bispezifischer Antikörper nach einem der Ansprüche 14 bis 21, der eine Fc-Region umfasst.

**23.** Multispezifischer oder bispezifischer Antikörper nach Anspruch 22, wobei die Fc-Region verändert ist, um die Effektor-Funktion zu vermindern, gegebenenfalls durch Substitution eines oder mehrerer der Reste 234, 235, 238, 265, 269, 270, 297, 327 und/oder 329 der Fc-Region.

**24.** Multispezifischer oder bispezifischer Antikörper nach Anspruch 22 oder 23, umfassend:

i) einen Antikörper voller Länge, umfassend eine Antigenbindungsstelle für ein erstes Antigen; und
ii) zumindest eine zweite variable Schwerkettendomäne und eine zweite variable Leichtkettendomäne, die zusammen eine Antigenbindungsstelle für ein zweites Antigen bilden,

wobei entweder das erste oder das zweite Antigen das Pb-DOTAM-Chelat ist und das andere das Zielantigen ist, wobei gegebenenfalls das erste Antigen das Zielantigen ist und das zweite Antigen das Pb-DOTAM-Chelat ist.

**25.** Multispezifischer oder bispezifischer Antikörper nach Anspruch 24, umfassend einen Antikörper voller Länge, umfassend eine Antigenbindungsstelle für das erste Antigen, wobei der N- oder der C-Terminus einer der Schwerketten über einen Polypeptidlinker an ein erstes Polypeptid gebunden ist und wobei das erste Polypeptid mit einem zweiten Polypeptid assoziiert, um ein Fab oder ein Kreuz-Fab, umfassend eine Bindungsstelle für das zweite Antigen, zu bilden.

**26.** Multispezifischer oder bispezifischer Antikörper nach Anspruch 25, der Folgendes umfasst:

i) ein erstes Polypeptid, das aus einer VH-Domäne und einer CH1-Domäne besteht, das mit einem zweiten Polypeptid assoziiert ist, das aus einer VL- und einer CL-Domäne besteht; oder
ii) ein erstes Polypeptid, das aus einer VL-Domäne und einer CH1-Domäne besteht, das mit einem zweiten Polypeptid assoziiert ist, das aus einer VH- und einer CL-Domäne besteht; oder
iii) ein erstes Polypeptid, das aus einer VH-Domäne und einer CL-Domäne besteht, das mit einem zweiten Polypeptid assoziiert ist, das aus einer VL- und einer CH1-Domäne besteht;

sodass das erste und das zweite Polypeptid zusammen eine Antigenbindungsstelle für das zweite Antigen bilden.

**27.** Multispezifischer oder bispezifischer Antikörper nach Anspruch 26, der einen Antikörper voller Länge, umfassend eine Antigenbindungsstelle für das erste Antigen, umfasst, wobei der C-Terminus einer der Schwerketten über einen Polypeptidlinker mit einem ersten Polypeptid verbunden ist, das aus einer VL-Domäne und einer CH1-Domäne besteht, das mit einem zweiten Polypeptid assoziiert ist, das aus einer VH- und einer CL-Domäne besteht.

**28.** isoliertes Polynucleotid oder isolierter Satz von Polynucleotiden, das/die für einen Antikörper nach einem der vorangegangenen Ansprüche kodiert/kodieren.

**29.** Expressionsvektor oder Satz von Expressionsvektoren, umfassend ein Polynucleotid oder einen Satz von Polynucleotiden nach Anspruch 28.

**30.** Prokaryotische oder eukaryotische Wirtszelle, umfassend ein isoliertes Polynucleotid oder einen isolierten Satz von Polynucleotiden nach Anspruch 28, gegebenenfalls umfassend einen Vektor oder einen Satz von Vektoren nach Anspruch 29.

**31.** Verfahren zur Herstellung eines Antikörpers nach einem der Ansprüche 1 bis 27, umfassend das Exprimieren des Antikörpers aus einer Wirtszelle nach Anspruch 30.

## Revendications

**1.** Anticorps comprenant un site de liaison à l'antigène spécifique pour un chélate DOTAM-plomb (Pb), dans lequel ledit site de liaison à l'antigène comprend au moins :

a) une CDR1 de chaîne lourde ;
b) une CDR2 de chaîne lourde comprenant la séquence d'acides aminés FIGSRGDTYYASWAKG (SEQ ID NO: 2), ou un variant de celle-ci comportant jusqu'à 1, 2, ou 3 substitutions dans SEQ ID NO: 2, dans lequel ces substitutions ne comprennent pas Phe50, Asp56 et Tyr58, et facultativement également ne comprennent

pas Gly52 et/ou Arg54 ;

c) une CDR3 de chaîne lourde comprenant la séquence d'acides aminés ERDPYGGGAYPPHL (SEQ ID NO: 3), ou un variant de celle-ci comportant jusqu'à 1, 2, ou 3 substitutions dans SEQ ID NO: 3, dans lequel ces substitutions ne comprennent pas Glu95, Arg96, Asp97, Pro98, et facultativement également ne comprennent pas Ala100C, Tyr100D, et/ou Pro100E et/ou facultativement également ne comprennent pas Tyr99 ;

d) une CDR1 de chaîne légère comprenant la séquence d'acides aminés QSSHSVYSDNDLA (SEQ ID NO: 4) ou un variant de celle-ci comportant jusqu'à 1, 2, ou 3 substitutions dans SEQ ID NO: 4, dans lequel ces substitutions ne comprennent pas Tyr28 et Asp32 ;

e) une CDR2 de chaîne légère ; et

f) une CDR3 de chaîne légère comprenant la séquence d'acides aminés LGGYDDESDTYG (SEQ ID NO: 6) ou un variant de celle-ci comportant jusqu'à 1, 2, ou 3 substitutions dans SEQ ID NO: 6, dans lequel ces substitutions ne comprennent pas Gly91, Tyr92, Asp93, Thr95c et Tyr96,

dans lequel la numérotation est selon Kabat.

**2.** Anticorps selon la revendication 1, dans lequel :

a) la CDR1 de chaîne lourde comprend la séquence d'acides aminés GFSLSTYSMS (SEQ ID NO: 1) ou un variant de celle-ci comportant jusqu'à 1, 2, ou 3 substitutions dans SEQ ID NO: 1, facultativement des substitutions conservatrices ; et/ou

e) la CDR2 de chaîne légère comprend la séquence d'acides aminés QASKLAS (SEQ ID NO: 5) ou un variant de celle-ci comportant au moins 1, 2, ou 3 substitutions dans SEQ ID NO: 5, facultativement des substitutions conservatrices.

**3.** Anticorps selon la revendication 1 ou la revendication 2, dans lequel le site de liaison à l'antigène comprend au moins une, deux, trois, quatre, cinq, ou six CDR sélectionnées parmi :

a) une CDR1 de chaîne lourde comprenant la séquence d'acides aminés GFSLSTYSMS (SEQ ID NO: 1) ;

b) une CDR2 de chaîne lourde comprenant la séquence d'acides aminés FIGSRGDTYYASWAKG (SEQ ID NO: 2) ;

c) une CDR3 de chaîne lourde comprenant la séquence d'acides aminés ERDPYGGGAYPPHL (SEQ ID NO: 3) ;

d) une CDR1 de chaîne légère comprenant la séquence d'acides aminés QSSHSVYSDNDLA (SEQ ID NO: 4) ;

e) une CDR2 de chaîne légère comprenant la séquence d'acides aminés QASKLAS (SEQ ID NO: 5) ; et

f) une CDR3 de chaîne légère comprenant la séquence d'acides aminés LGGYDDESDTYG (SEQ ID NO: 6).

**4.** Anticorps selon la revendication 1 ou la revendication 2, dans lequel le site de liaison à l'antigène comprend :

b) une CDR2 de chaîne lourde comprenant la séquence d'acides aminés FIGSRGDTYYASWAKG (SEQ ID NO: 2) ;

c) une CDR3 de chaîne lourde comprenant la séquence d'acides aminés ERDPYGGGAYPPHL (SEQ ID NO: 3) ;

d) une CDR1 de chaîne légère comprenant la séquence d'acides aminés QSSHSVYSDNDLA (SEQ ID NO: 4) ; et

f) une CDR3 de chaîne légère comprenant la séquence d'acides aminés LGGYDDESDTYG (SEQ ID NO: 6).

**5.** Anticorps selon la revendication 1, dans lequel le site de liaison à l'antigène comprend :

a) une CDR1 de chaîne lourde comprenant la séquence d'acides aminés GFSLSTYSMS (SEQ ID NO: 1) ;

b) une CDR2 de chaîne lourde comprenant la séquence d'acides aminés FIGSRGDTYYASWAKG (SEQ ID NO: 2) ;

c) une CDR3 de chaîne lourde comprenant la séquence d'acides aminés ERDPYGGGAYPPHL (SEQ ID NO: 3) ;

d) une CDR1 de chaîne légère comprenant la séquence d'acides aminés QSSHSVYSDNDLA (SEQ ID NO: 4) ;

e) une CDR2 de chaîne légère comprenant la séquence d'acides aminés QASKLAS (SEQ ID NO: 5) ; et

f) une CDR3 de chaîne légère comprenant la séquence d'acides aminés LGGYDDESDTYG (SEQ ID NO: 6).

**6.** Anticorps selon l'une quelconque des revendications précédentes, qui est humain, chimérique ou humanisé.

**7.** Anticorps selon l'une quelconque des revendications précédentes, dans lequel

i) le site de liaison à l'antigène comprend un domaine variable de chaîne lourde comprenant une séquence

d'acides aminés sélectionnée dans le groupe consistant en SEQ ID NO: 7 et SEQ ID NO: 9, ou un variant de celles-ci comprenant une séquence d'acides aminés présentant au moins 90, 91, 92, 93, 94, 95, 96, 97, 98, ou 99 % d'identité avec SEQ ID NO: 7 ou SEQ ID NO: 9 ; et/ou

ii) le site de liaison à l'antigène comprend un domaine variable de chaîne légère comprenant une séquence d'acides aminés sélectionnée dans le groupe consistant en SEQ ID NO: 8 et SEQ ID NO: 10, ou un variant de celles-ci comprenant une séquence d'acides aminés présentant au moins 90, 91, 92, 93, 94, 95, 96, 97, 98, ou 99 % d'identité avec SEQ ID NO: 8 ou 10.

**8.** Anticorps selon l'une quelconque des revendications précédentes, dans lequel

i) le site de liaison à l'antigène comprend un domaine variable de chaîne lourde comprenant une séquence d'acides aminés de SEQ ID NO: 7 et un domaine variable de chaîne légère comprenant une séquence d'acides aminés de SEQ ID NO: 8 ; ou

ii) le site de liaison à l'antigène comprend un domaine variable de chaîne lourde comprenant une séquence d'acides aminés de SEQ ID NO: 9 et un domaine variable de chaîne légère comprenant une séquence d'acides aminés de SEQ ID NO: 10.

**9.** Anticorps selon l'une quelconque des revendications précédentes, dans lequel le site de liaison à l'antigène se lie au chélate Pb-DOTAM avec une valeur Kd de 100 pM, 50 pM, 20 pM, 10 pM, 5 pM, 1 pM ou moins, tel que mesuré par un essai KinExA (exclusion cinétique).

**10.** Anticorps selon l'une quelconque des revendications précédentes, dans lequel le site de liaison à l'antigène se lie au chélate Pb-DOTAM et à un chélate Bi-DOTAM, et dans lequel le rapport des valeurs Kd pour le chélate Bi-DOTAM/chélate Pb-DOTAM est dans la plage de 0,1-10 ou 1-10, tel que mesuré par un essai KinExA (exclusion cinétique).

**11.** Anticorps selon l'une quelconque des revendications précédentes, qui est un anticorps entier, ou qui est un fragment d'anticorps sélectionné dans le groupe consistant en un Fv, Fab, Fab', Fab'-SH, $F(ab')_2$, un dianticorps, un anticorps linéaire, ou une molécule d'anticorps à chaîne unique.

**12.** Anticorps selon l'une quelconque des revendications précédentes, dans lequel l'anticorps est couplé à un fragment qui se lie spécifiquement à un antigène cible.

**13.** Anticorps selon la revendication 12, dans lequel l'antigène cible est un antigène tumoral spécifique.

**14.** Anticorps selon la revendication 12 ou 13, qui est un anticorps multispécifique ou bispécifique.

**15.** Anticorps multispécifique ou bispécifique selon la revendication 14, dans lequel l'antigène tumoral spécifique est sélectionné dans le groupe consistant en l'ACE, HER2 et CD20.

**16.** Anticorps multispécifique ou bispécifique selon la revendication 14 ou 15, dans lequel l'anticorps multispécifique ou bispécifique comprend au moins un site de liaison à l'antigène spécifique pour le chélate Pb-DOTAM et au moins un site de liaison à l'antigène spécifique pour l'ACE, et dans lequel le site de liaison à l'antigène spécifique pour l'ACE comprend une chaîne lourde comprenant au moins une, deux ou trois CDR de chaîne lourde : dans lequel :

a) la CDR1 de chaîne lourde comprend la séquence d'acides aminés de SEQ ID NO: 11
b) la CDR2 de chaîne lourde comprend la séquence d'acides aminés de SEQ ID NO: 12
c) la CDR3 de chaîne lourde comprend la séquence d'acides aminés de SEQ ID NO: 13 ;

et/ou le site de liaison à l'antigène spécifique pour l'ACE comprend une chaîne légère comprenant au moins une, deux ou trois CDR de chaîne légère : dans lequel :

d) la CDR1 de chaîne légère comprend la séquence d'acides aminés SEQ ID NO: 14 ;
e) la CDR2 de chaîne légère comprend la séquence d'acides aminés SEQ ID NO: 15 ;
f) la CDR3 de chaîne légère comprend la séquence d'acides aminés SEQ ID NO: 16.

**17.** Anticorps multispécifique ou bispécifique selon la revendication 14 ou 15, dans lequel l'anticorps multispécifique ou bispécifique comprend au moins un site de liaison à l'antigène spécifique pour le chélate Pb-DOTAM et au moins

un site de liaison à l'antigène spécifique pour l'ACE, et dans lequel le site de liaison à l'antigène spécifique pour l'ACE comprend

i) un domaine variable de chaîne lourde comprenant une séquence d'acides aminés de SEQ ID NO: 17, ou un variant de celle-ci comprenant une séquence d'acides aminés présentant au moins 90, 91, 92, 93, 94, 95, 96, 97, 98, ou 99 % d'identité avec SEQ ID NO: 17 ; et/ou
ii) un domaine variable de chaîne légère comprenant une séquence d'acides aminés de SEQ ID NO: 18, ou un variant de celle-ci comprenant une séquence d'acides aminés présentant au moins 90, 91, 92, 93, 94, 95, 96, 97, 98, ou 99 % d'identité avec SEQ ID NO: 18.

**18.** Anticorps multispécifique ou bispécifique selon la revendication 14 ou la revendication 15, dans lequel l'anticorps multispécifique ou bispécifique comprend au moins un site de liaison à l'antigène spécifique pour un chélate Pb-DOTAM et au moins un site de liaison à l'antigène spécifique pour HER2, et dans lequel le site de liaison à l'antigène spécifique pour HER2 comprend au moins une, deux, trois, quatre, cinq, ou six CDR sélectionnées parmi (a) une CDR-H1 comprenant la séquence d'acides aminés de SEQ ID NO: 28 ; (b) une CDR-H2 comprenant la séquence d'acides aminés de SEQ ID NO: 29 ; (c) une CDR-H3 comprenant la séquence d'acides aminés de SEQ ID NO: 30 ; (d) une CDR-L1 comprenant la séquence d'acides aminés de SEQ ID NO: 31 ; (e) une CDR-L2 comprenant la séquence d'acides aminés de SEQ ID NO: 32 ; et (f) une CDR-L3 comprenant la séquence d'acides aminés de SEQ ID NO: 33.

**19.** Anticorps multispécifique ou bispécifique selon la revendication 14 ou la revendication 15, dans lequel l'anticorps multispécifique ou bispécifique comprend au moins un site de liaison à l'antigène spécifique pour le chélate Pb-DOTAM et au moins un site de liaison à l'antigène spécifique pour HER2, et dans lequel le site de liaison à l'antigène spécifique pour HER2 comprend

i) un domaine variable de chaîne lourde comprenant une séquence d'acides aminés de SEQ ID NO: 34, ou un variant de celle-ci comprenant une séquence d'acides aminés présentant au moins 90, 91, 92, 93, 94, 95, 96, 97, 98, ou 99 % d'identité avec SEQ ID NO: 34 ; et/ou
ii) un domaine variable de chaîne légère comprenant une séquence d'acides aminés de SEQ ID NO: 35, ou un variant de celle-ci comprenant une séquence d'acides aminés présentant au moins 90, 91, 92, 93, 94, 95, 96, 97, 98, ou 99 % d'identité avec SEQ ID NO: 35.

**20.** Anticorps multispécifique ou bispécifique selon la revendication 14 ou la revendication 15, dans lequel l'anticorps multispécifique ou bispécifique comprend au moins un site de liaison à l'antigène spécifique pour un chélate Pb-DOTAM et au moins un site de liaison à l'antigène spécifique pour CD20, et dans lequel le site de liaison à l'antigène spécifique pour CD20 comprend au moins une, deux, trois, quatre, cinq, ou six CDR sélectionnées parmi (a) une CDR-H1 comprenant la séquence d'acides aminés de SEQ ID NO: 39 ; (b) une CDR-H2 comprenant la séquence d'acides aminés de SEQ ID NO: 40 ; (c) une CDR-H3 comprenant la séquence d'acides aminés de SEQ ID NO: 41 ; (d) une CDR-L1 comprenant la séquence d'acides aminés de SEQ ID NO: 42 ; (e) une CDR-L2 comprenant la séquence d'acides aminés de SEQ ID NO: 43 ; et (f) une CDR-L3 comprenant la séquence d'acides aminés de SEQ ID NO: 44.

**21.** Anticorps multispécifique ou bispécifique selon la revendication 14, dans lequel l'anticorps multispécifique ou bispécifique comprend au moins un site de liaison à l'antigène spécifique pour le chélate Pb-DOTAM et au moins un site de liaison à l'antigène spécifique pour CD20, et dans lequel le site de liaison à l'antigène spécifique pour CD20 comprend

i) un domaine variable de chaîne lourde comprenant une séquence d'acides aminés de SEQ ID NO: 45, ou un variant de celle-ci comprenant une séquence d'acides aminés présentant au moins 90, 91, 92, 93, 94, 95, 96, 97, 98, ou 99 % d'identité avec SEQ ID NO: 45 ; et/ou
ii) un domaine variable de chaîne légère comprenant une séquence d'acides aminés de SEQ ID NO: 46, ou un variant de celle-ci comprenant une séquence d'acides aminés présentant au moins 90, 91, 92, 93, 94, 95, 96, 97, 98, ou 99 % d'identité avec SEQ ID NO: 46.

**22.** Anticorps multispécifique ou bispécifique selon l'une quelconque des revendications 14 à 21, qui comprend une région Fc.

**23.** Anticorps multispécifique ou bispécifique selon la revendication 22, dans lequel la région Fc est manipulée pour

réduire une fonction effectrice, facultativement par une substitution d'un ou de plusieurs des résidus de région Fc 234, 235, 238, 265, 269, 270, 297, 327 et/ou 329.

**24.** Anticorps multispécifique ou bispécifique selon la revendication 22 ou 23, comprenant :

i) un anticorps de pleine longueur comprenant un site de liaison à l'antigène pour un premier antigène ; et
ii) au moins un deuxième domaine variable de chaîne lourde et un deuxième domaine variable de chaîne légère qui forment ensemble un site de liaison à l'antigène pour un deuxième antigène,

dans lequel soit le premier soit le deuxième antigène est le chélate Pb-DOTAM, et l'autre est l'antigène cible, facultativement dans lequel le premier antigène est l'antigène cible et le deuxième antigène est le chélate Pb-DOTAM.

**25.** Anticorps multispécifique ou bispécifique selon la revendication 24, comprenant un anticorps de pleine longueur comprenant un site de liaison à l'antigène pour le premier antigène, dans lequel l'extrémité N ou C-terminale d'une des chaînes lourdes est liée via un segment de liaison polypeptidique à un premier polypeptide et dans lequel le premier polypeptide s'associe à un deuxième polypeptide pour former un Fab ou un Fab croisé comprenant un site de liaison pour le deuxième antigène.

**26.** Anticorps multispécifique ou bispécifique selon la revendication 25, qui comprend :

i) un premier polypeptide consistant en un domaine VH et un domaine CH1, qui est associé à un deuxième polypeptide consistant en un domaine VL et CL ; ou
ii) un premier polypeptide consistant en un domaine VL et un domaine CH1, qui est associé à un deuxième polypeptide consistant en un domaine VH et CL ; ou
iii) un premier polypeptide consistant en un domaine VH et un domaine CL, qui est associé à un deuxième polypeptide consistant en un domaine VL et CH1 ;

de sorte que le premier et le deuxième polypeptide forment ensemble un site de liaison à l'antigène pour le deuxième antigène.

**27.** Anticorps multispécifique ou bispécifique selon la revendication 26, qui comprend un anticorps de pleine longueur comprenant un site de liaison à l'antigène pour le premier antigène, dans lequel l'extrémité C-terminale d'une des chaînes lourdes est liée via un segment de liaison polypeptidique à un premier polypeptide consistant en un domaine VL et un domaine CH1, qui est associé à un deuxième polypeptide consistant en un domaine VH et CL.

**28.** Polynucléotide isolé ou ensemble de polynucléotides isolé codant pour un anticorps selon l'une quelconque des revendications précédentes.

**29.** Vecteur d'expression ou ensemble de vecteurs d'expression comprenant le polynucléotide ou l'ensemble de polynucléotides selon la revendication 28.

**30.** Cellule hôte procaryote ou eucaryote comprenant un polynucléotide isolé ou un ensemble de polynucléotides isolé selon la revendication 28, facultativement comprenant le vecteur ou l'ensemble de vecteurs selon la revendication 29.

**31.** Procédé de production d'un anticorps selon l'une quelconque des revendications 1 à 27, comprenant l'expression de l'anticorps à partir de la cellule hôte selon la revendication 30.

**Figure 1**

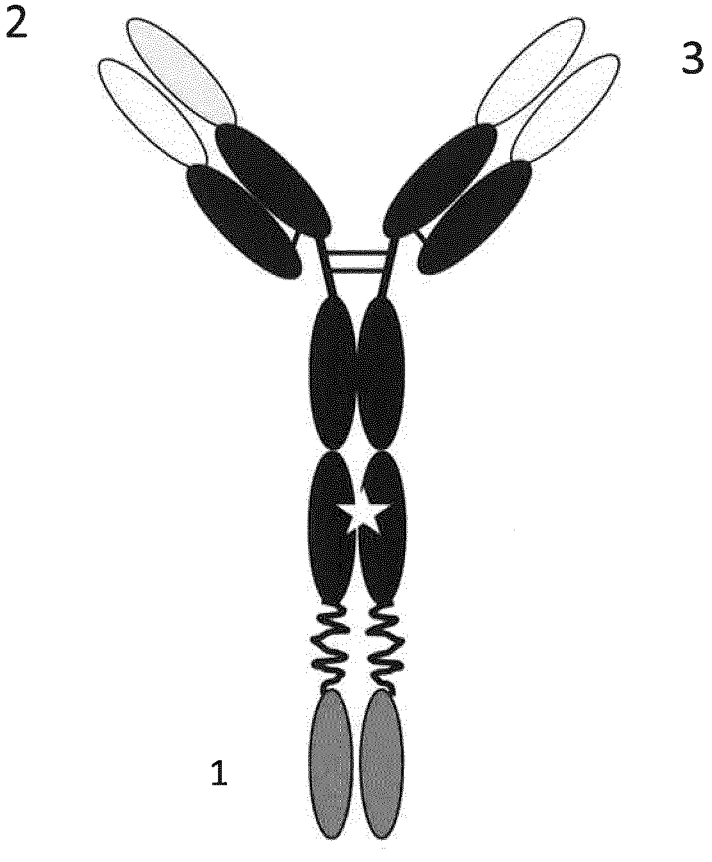

Figure 2

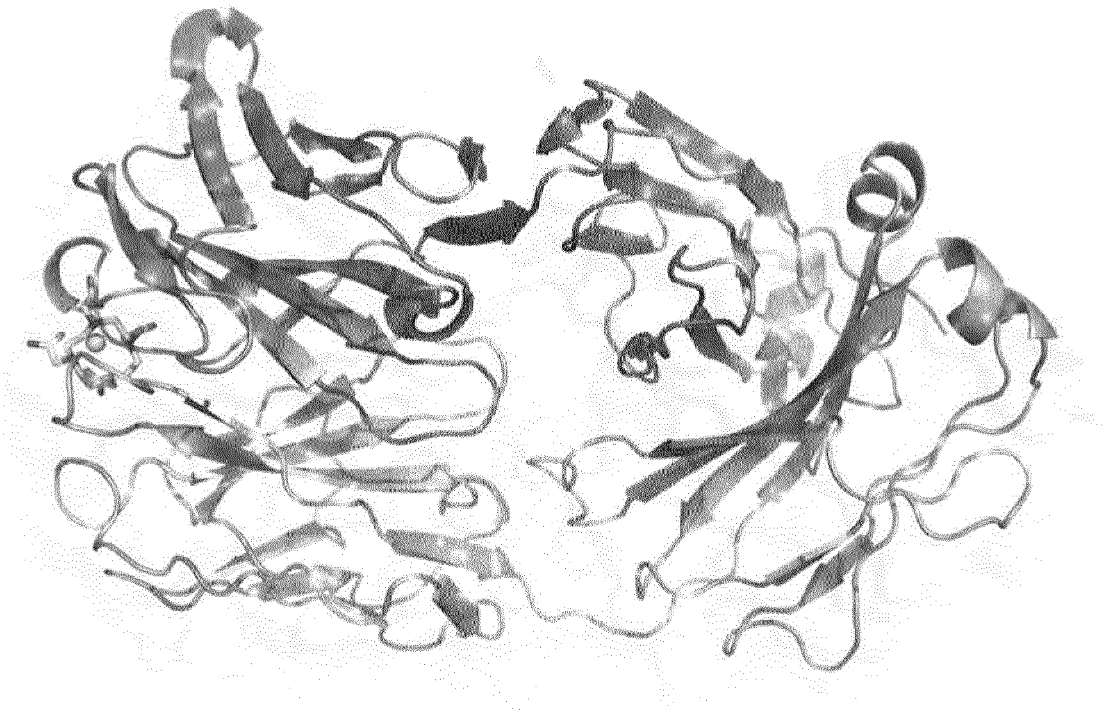

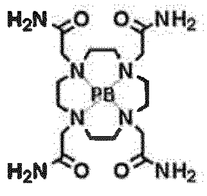

Fab PRIT-0213

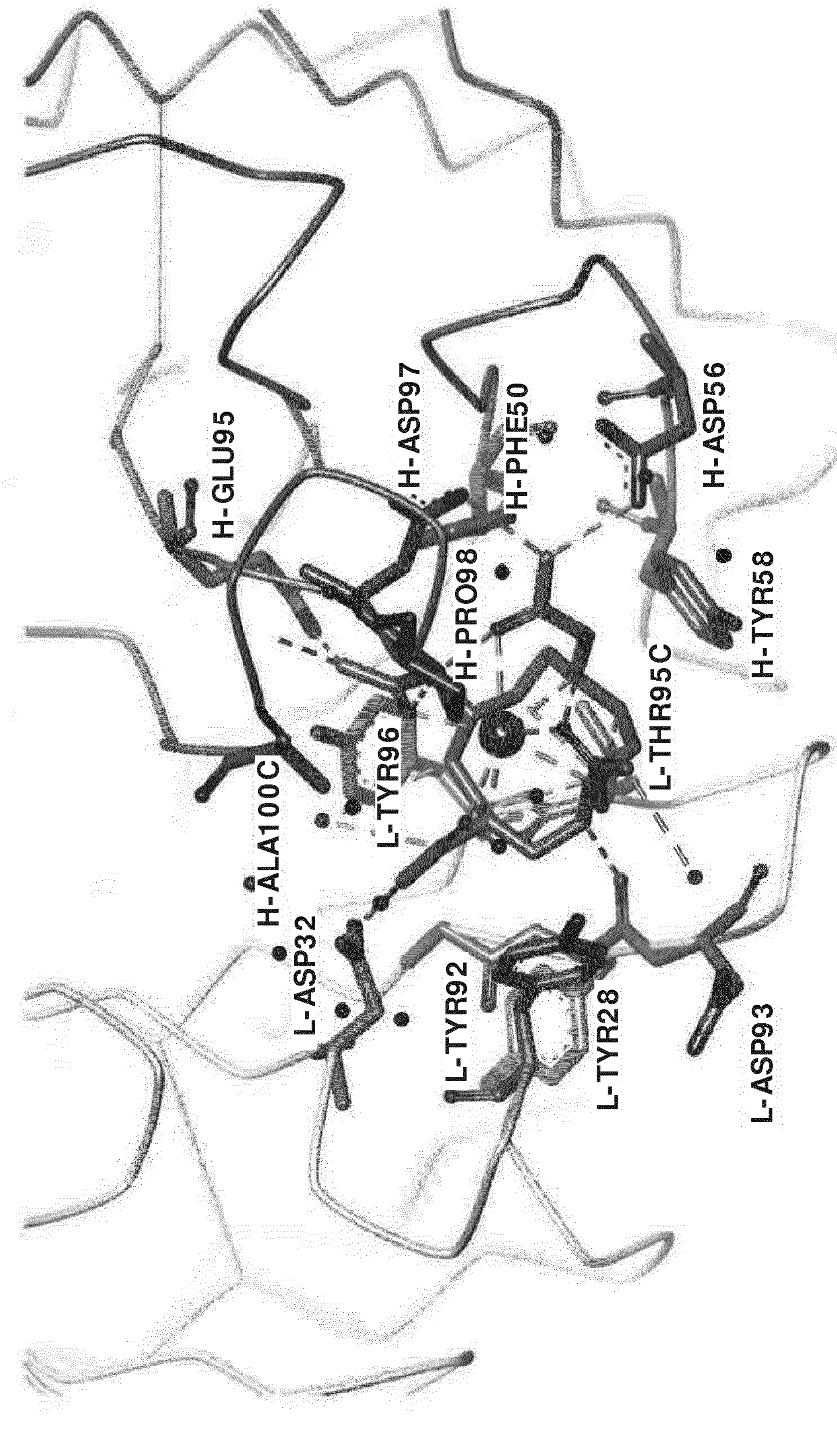
H-GLU95
H-ASP97
H-PHE50
H-ASP56
H-TYR58
H-PRO98
L-THR95C
L-TYR96
H-ALA100C
L-ASP32
L-TYR92
L-TYR28
L-ASP93

FIG. 3

Figure 4

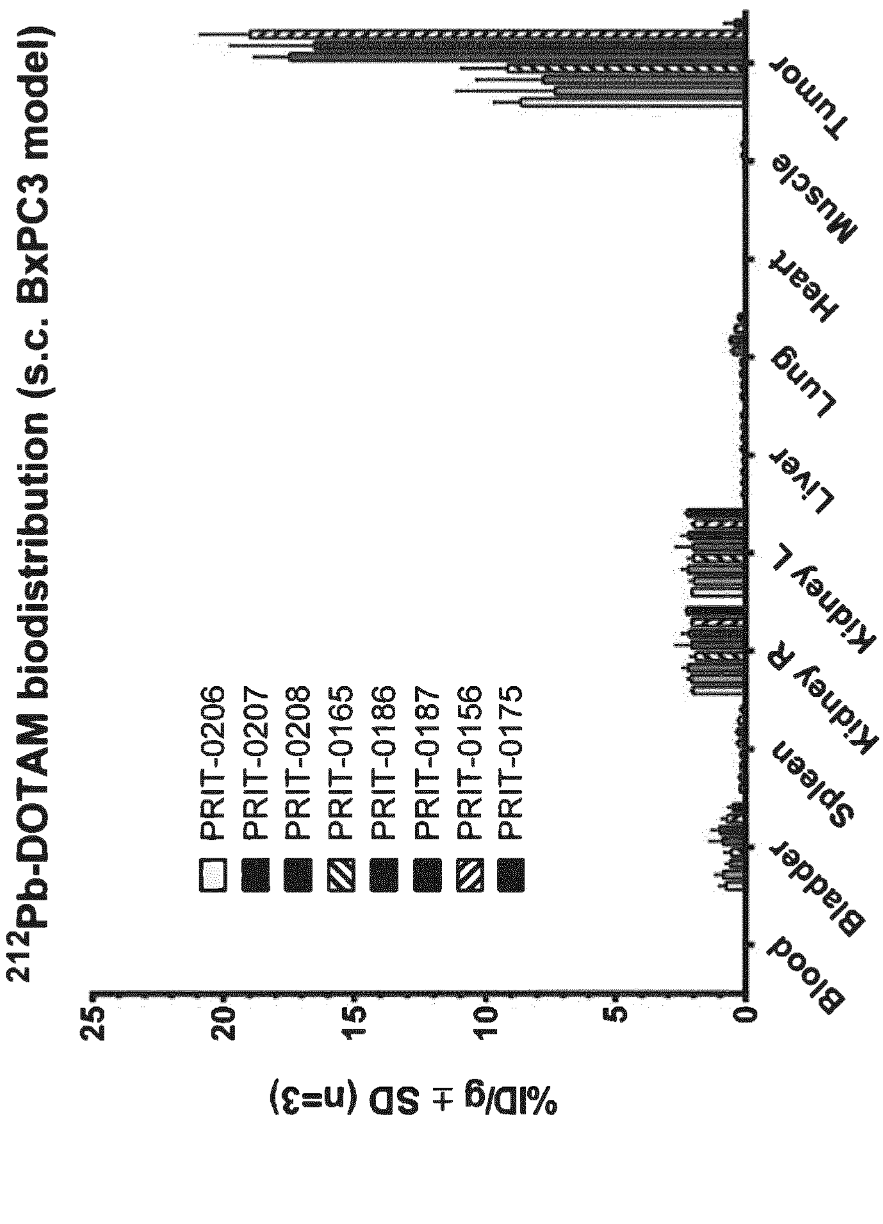

212Pb-DOTAM biodistribution (s.c. BxPC3 model)
%ID/g ± SD (n=3)
0
5
10
15
20
25
PRIT-0206
PRIT-0207
PRIT-0208
PRIT-0165
PRIT-0186
PRIT-0187
PRIT-0156
PRIT-0175
Blood
Bladder
Spleen
Kidney R
Kidney L
Liver
Lung
Heart
Muscle
Tumor

Figure 5

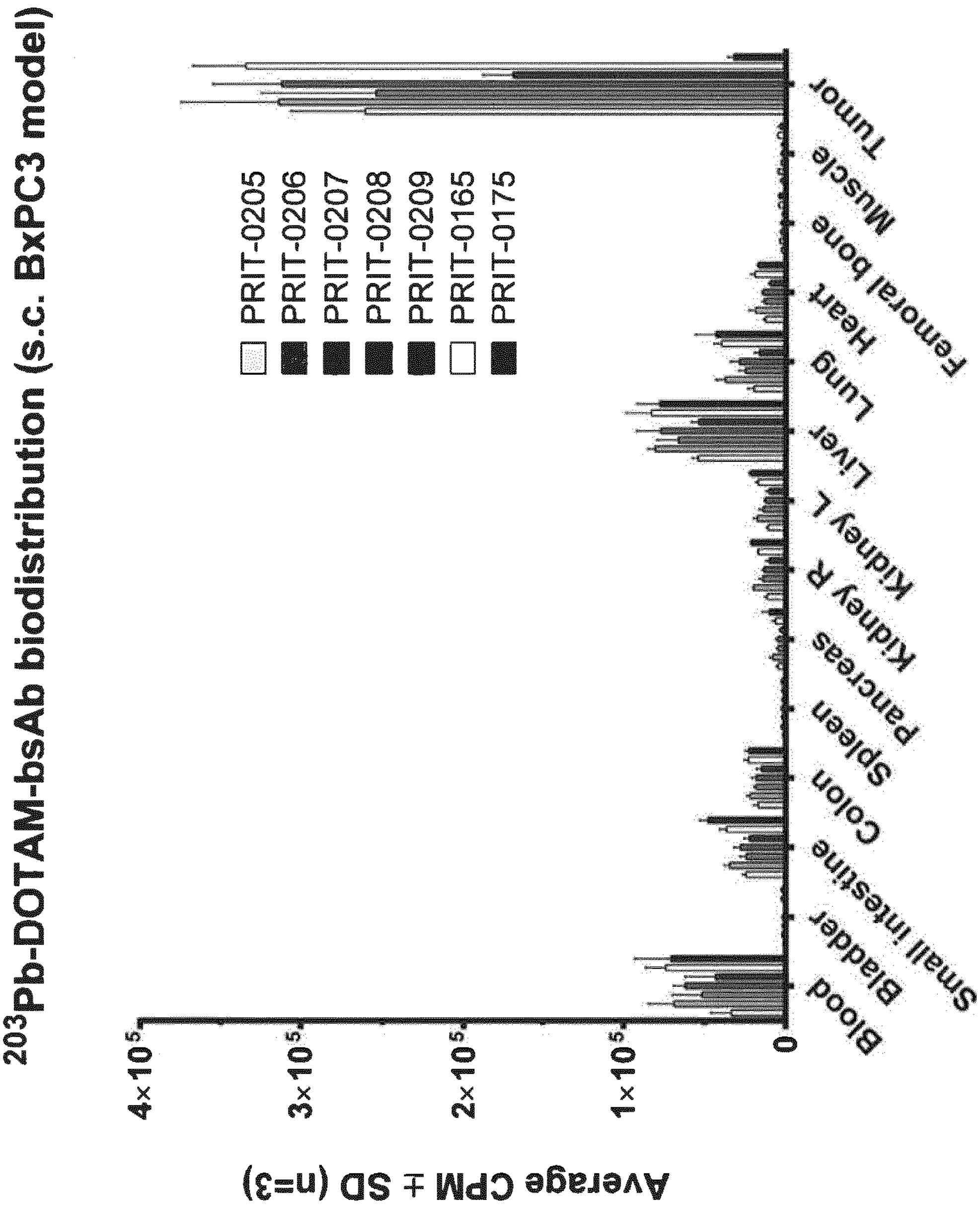

$^{203}$Pb-DOTAM-bsAb biodistribution (s.c. BxPC3 model)
Average CPM ± SD (n=3)
4×10⁵
3×10⁵
2×10⁵
1×10⁵
0
PRIT-0205
PRIT-0206
PRIT-0207
PRIT-0208
PRIT-0209
PRIT-0165
PRIT-0175
Blood
Bladder
Small intestine
Colon
Spleen
Pancreas
Kidney R
Kidney L
Liver
Lung
Heart
Femoral bone
Muscle
Tumor

**Figure 6**

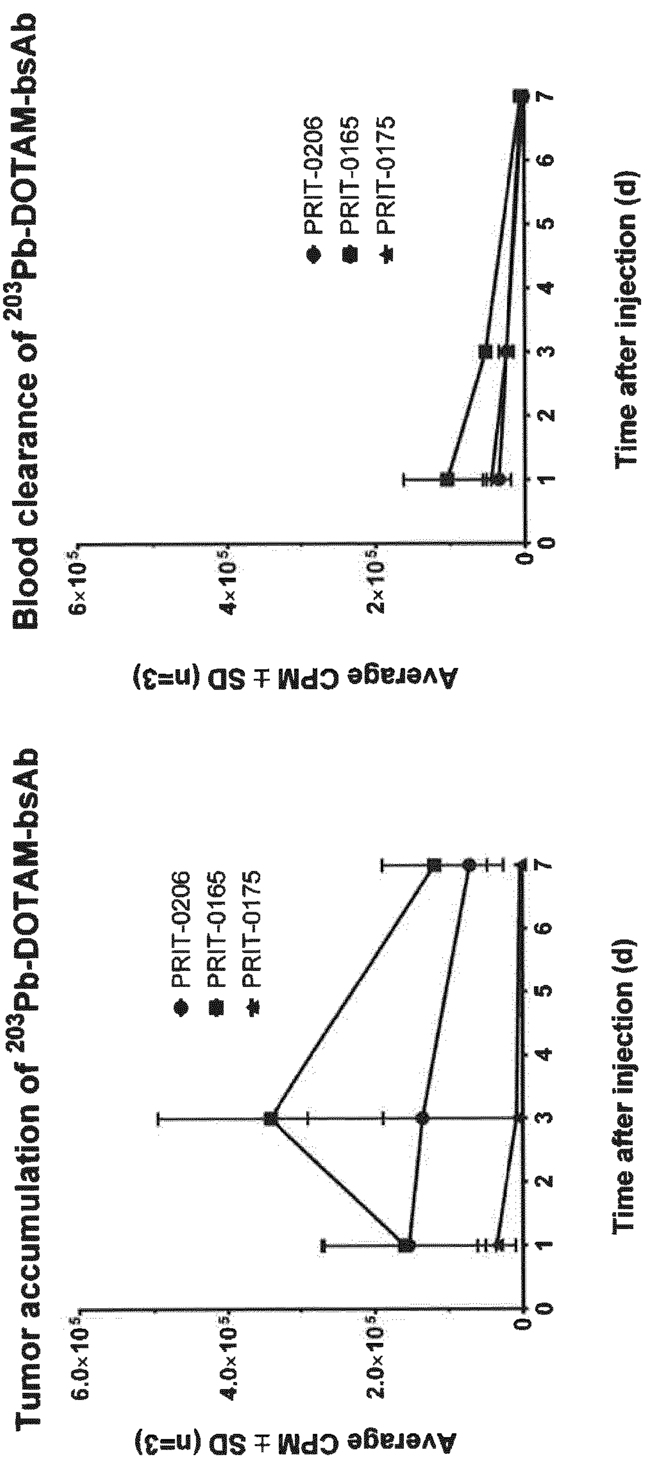

Tumor accumulation of $^{203}$Pb-DOTAM-bsAb
Average CPM ± SD (n=3)
6.0×10$^5$
4.0×10$^5$
2.0×10$^5$
0
0 1 2 3 4 5 6 7
Time after injection (d)
PRIT-0206
PRIT-0165
PRIT-0175
Blood clearance of $^{203}$Pb-DOTAM-bsAb
Average CPM ± SD (n=3)
6×10$^5$
4×10$^5$
2×10$^5$
0
0 1 2 3 4 5 6 7
Time after injection (d)
PRIT-0206
PRIT-0165
PRIT-0175

Figure 7

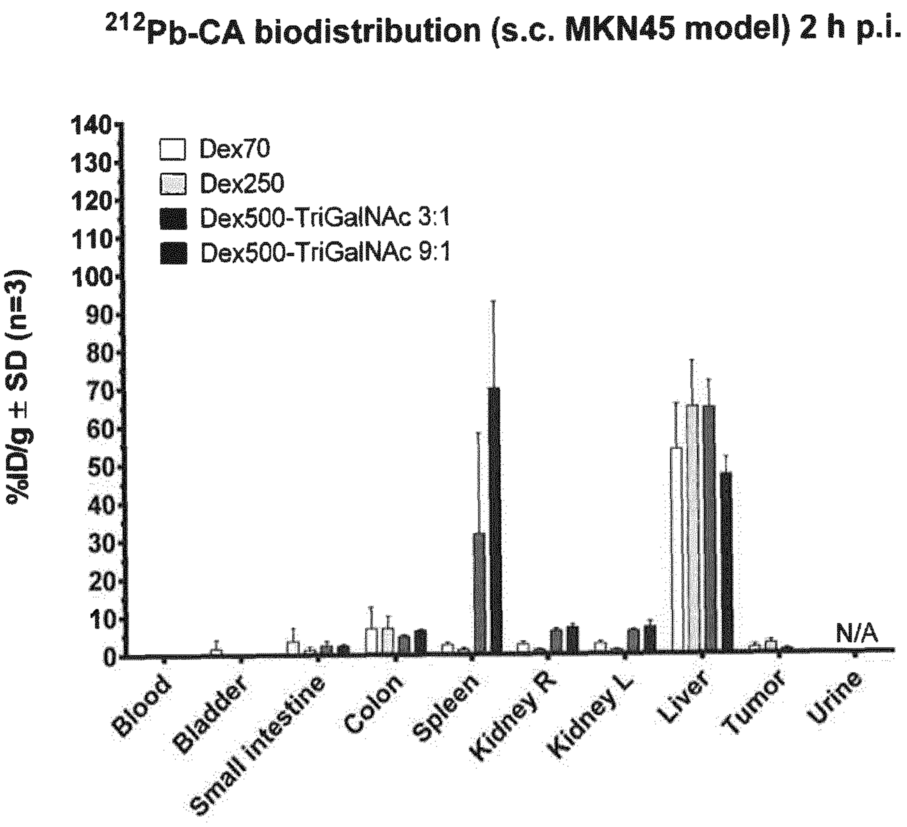

$^{212}$Pb-CA biodistribution (s.c. MKN45 model) 2 h p.i.
%ID/g ± SD (n=3)
140
130
120
110
100
90
80
70
60
50
40
30
20
10
0
Dex70
Dex250
Dex500-TriGalNAc 3:1
Dex500-TriGalNAc 9:1
N/A
Blood
Bladder
Small intestine
Colon
Spleen
Kidney R
Kidney L
Liver
Tumor
Urine

# Figure 8

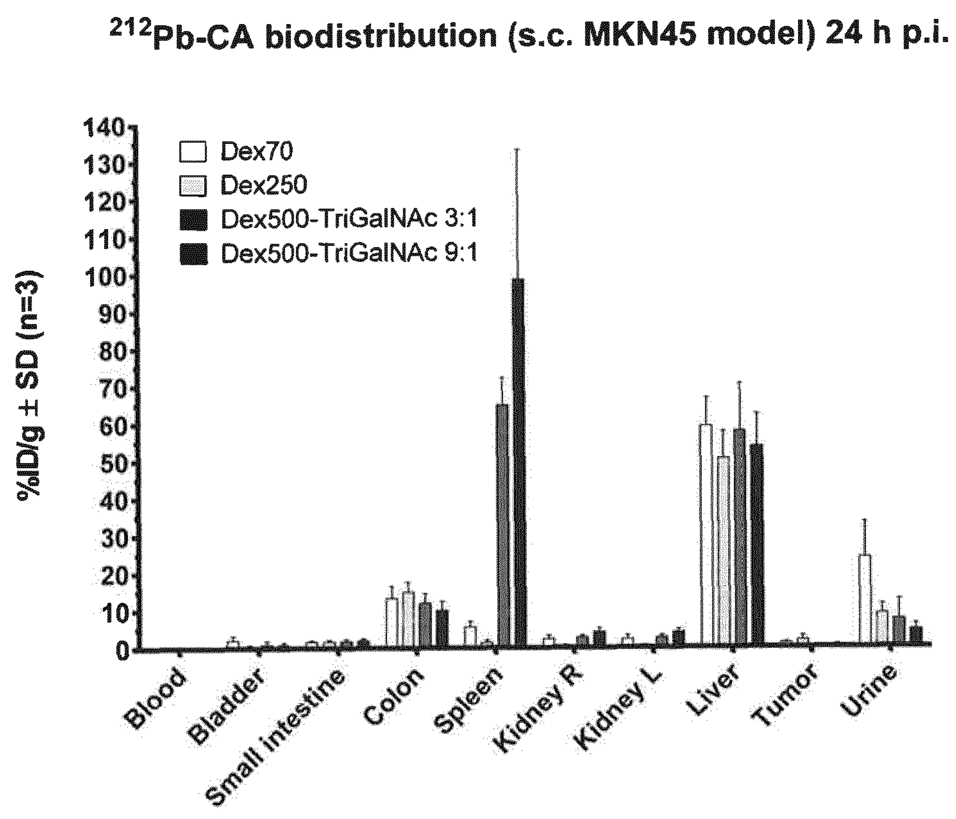
212Pb-CA biodistribution (s.c. MKN45 model) 24 h p.i.
%ID/g ± SD (n=3)
Dex70
Dex250
Dex500-TriGalNAc 3:1
Dex500-TriGalNAc 9:1
0
10
20
30
40
50
60
70
80
90
100
110
120
130
140
Blood
Bladder
Small intestine
Colon
Spleen
Kidney R
Kidney L
Liver
Tumor
Urine

Figure 9

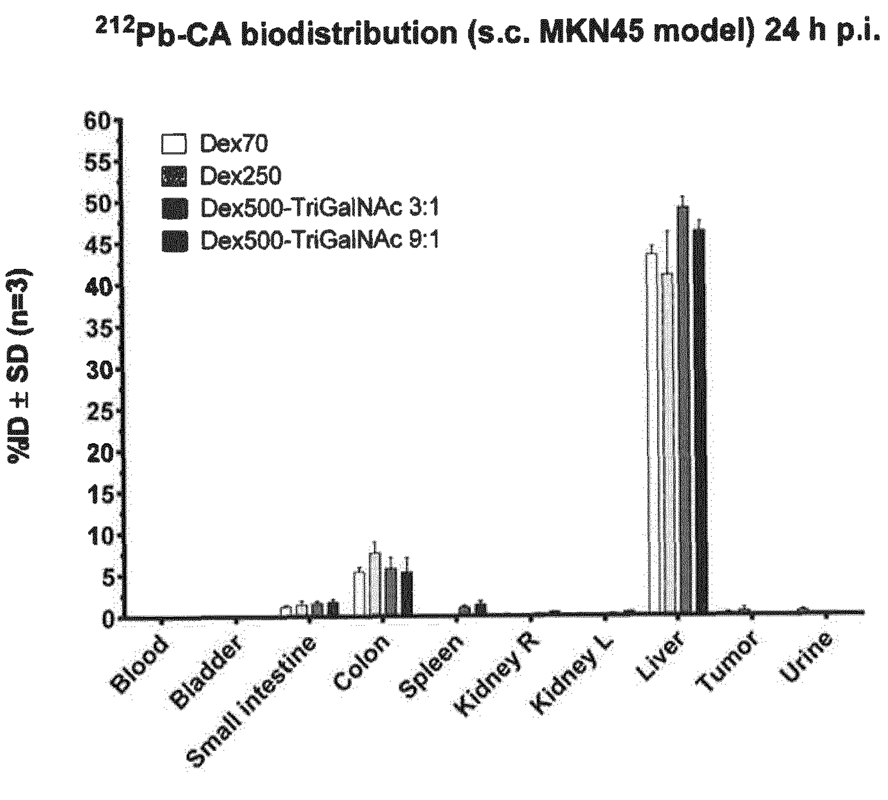

$^{212}$Pb-CA biodistribution (s.c. MKN45 model) 24 h p.i.
%ID ± SD (n=3)
0
5
10
15
20
25
30
35
40
45
50
55
60
Dex70
Dex250
Dex500-TriGalNAc 3:1
Dex500-TriGalNAc 9:1
Blood
Bladder
Small intestine
Colon
Spleen
Kidney R
Kidney L
Liver
Tumor
Urine

Figure 10

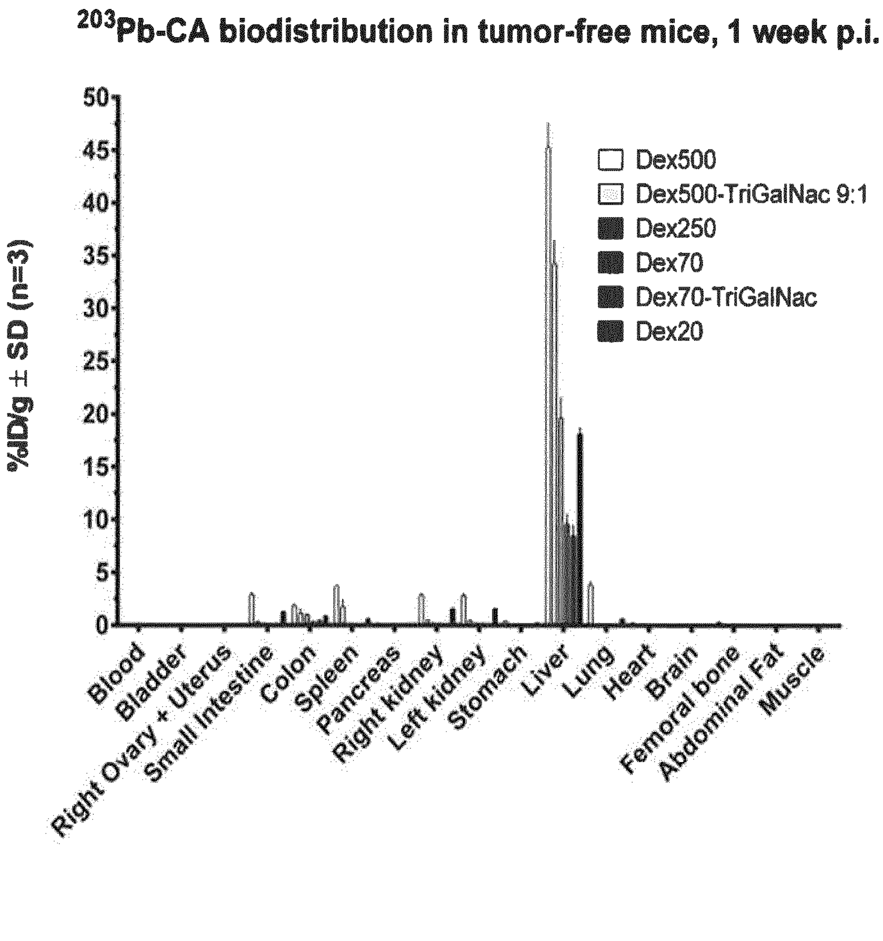
203Pb-CA biodistribution in tumor-free mice, 1 week p.i.
%ID/g ± SD (n=3)
50
45
40
35
30
25
20
15
10
5
0
Dex500
Dex500-TriGalNac 9:1
Dex250
Dex70
Dex70-TriGalNac
Dex20
Blood
Bladder
Right Ovary + Uterus
Small Intestine
Colon
Spleen
Pancreas
Right kidney
Left kidney
Stomach
Liver
Lung
Heart
Brain
Femoral bone
Abdominal Fat
Muscle

**Figure 11**

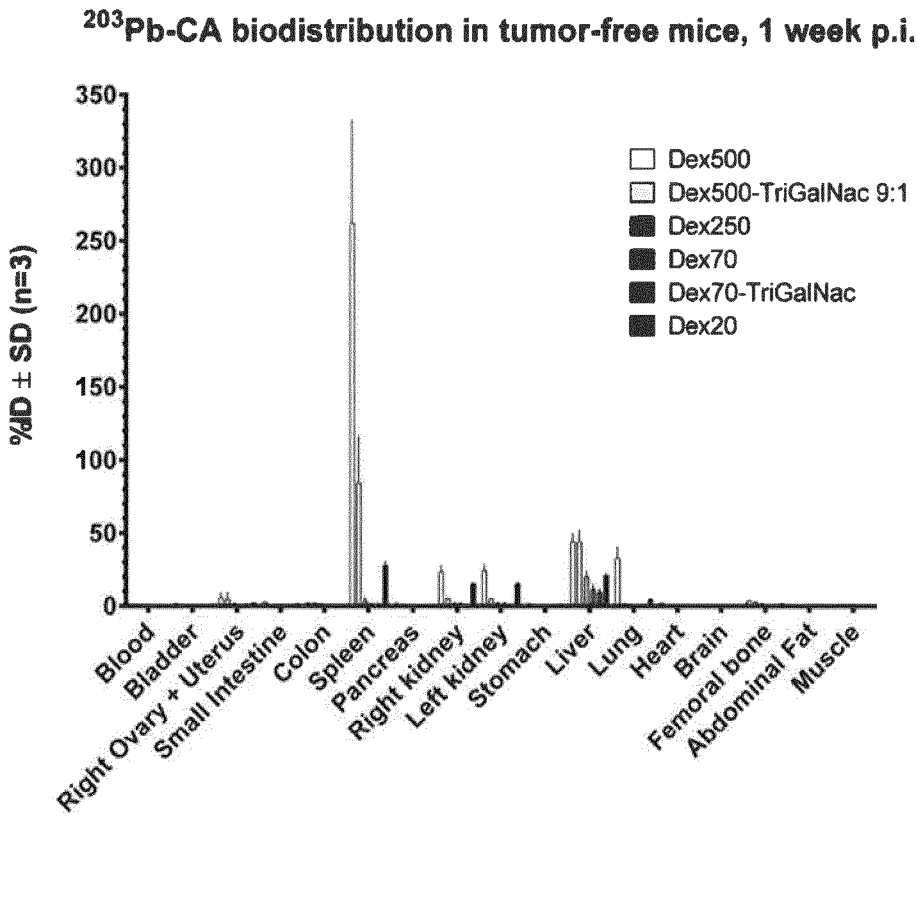

$^{203}$Pb-CA biodistribution in tumor-free mice, 1 week p.i.
%ID ± SD (n=3)
350
300
250
200
150
100
50
0
Dex500
Dex500-TriGalNac 9:1
Dex250
Dex70
Dex70-TriGalNac
Dex20
Blood
Bladder
Right Ovary + Uterus
Small Intestine
Colon
Spleen
Pancreas
Right kidney
Left kidney
Stomach
Liver
Lung
Heart
Brain
Femoral bone
Abdominal Fat
Muscle

Figure 12

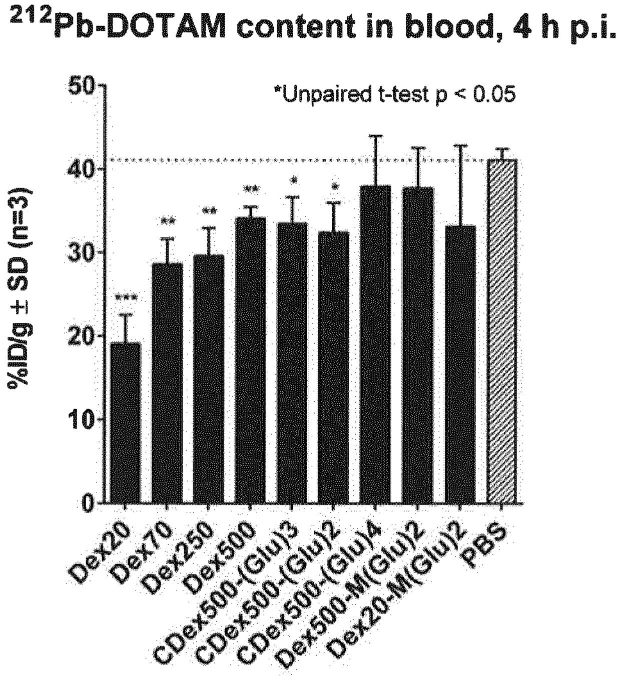
212Pb-DOTAM content in blood, 4 h p.i.
*Unpaired t-test p < 0.05
%ID/g ± SD (n=3)
0
10
20
30
40
50
***
**
**
**
*
*
Dex20
Dex70
Dex250
Dex500
CDex500-(Glu)3
CDex500-(Glu)2
CDex500-(Glu)4
Dex500-M(Glu)2
Dex20-M(Glu)2
PBS

Figure 13

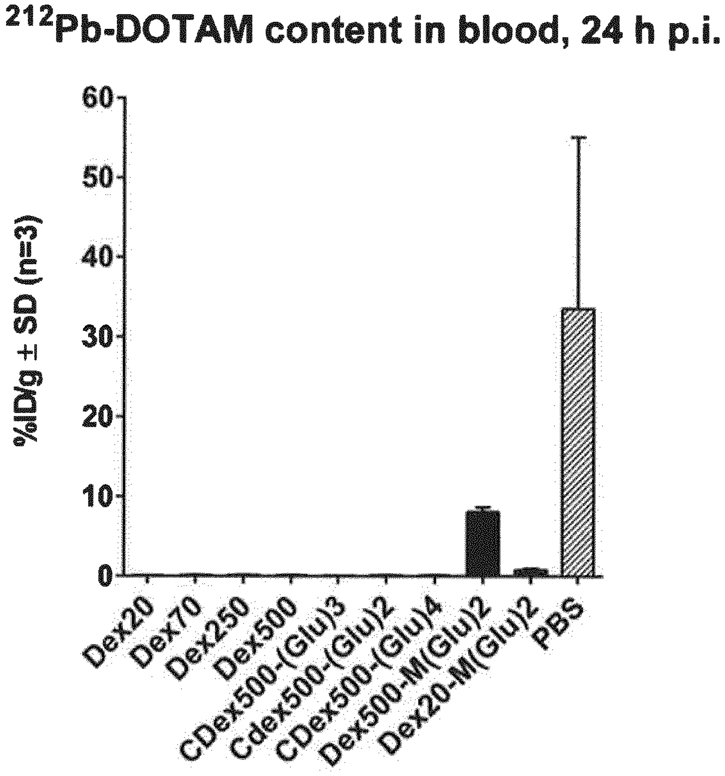
$^{212}$Pb-DOTAM content in blood, 24 h p.i.
%ID/g ± SD (n=3)
60
50
40
30
20
10
0
Dex20
Dex70
Dex250
Dex500
CDex500-(Glu)3
Cdex500-(Glu)2
CDex500-(Glu)4
Dex500-M(Glu)2
Dex20-M(Glu)2
PBS

Figure 14

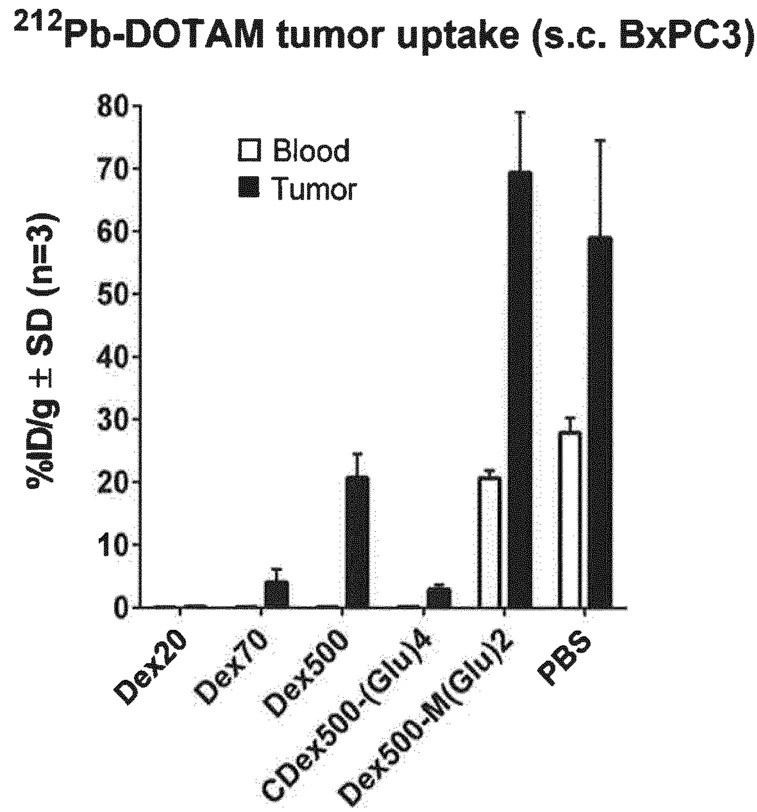

$^{212}$Pb-DOTAM tumor uptake (s.c. BxPC3)
%ID/g ± SD (n=3)
80
70
60
50
40
30
20
10
0
Blood
Tumor
Dex20
Dex70
Dex500
CDex500-(Glu)4
Dex500-M(Glu)2
PBS

**Figure 15**

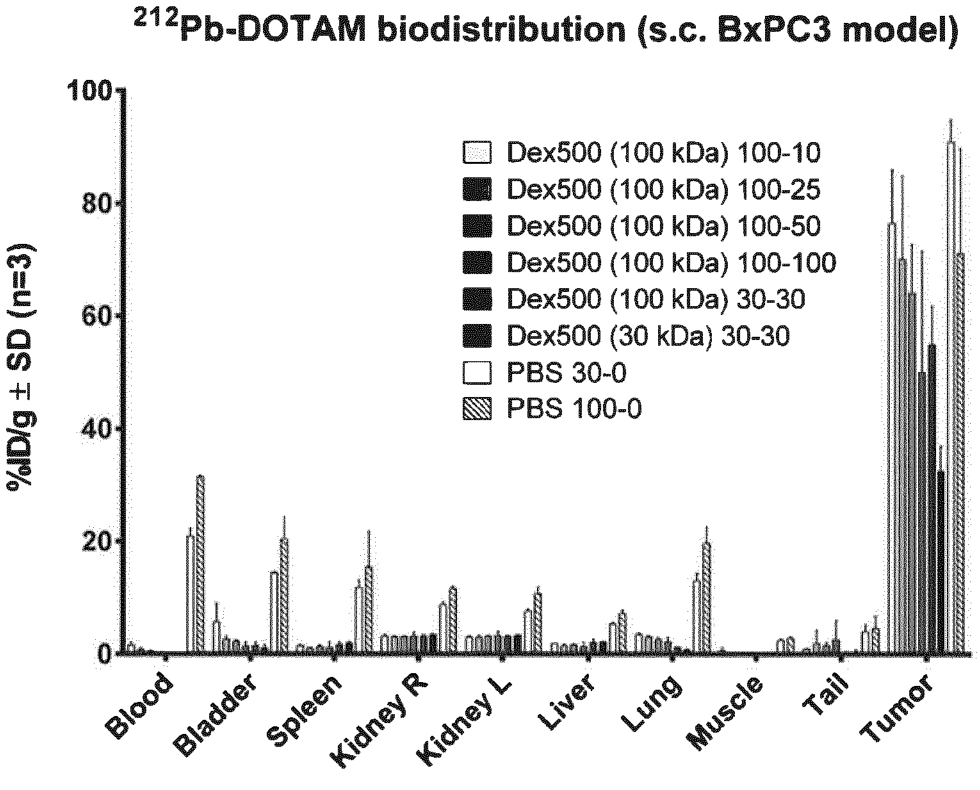
212Pb-DOTAM biodistribution (s.c. BxPC3 model)
%ID/g ± SD (n=3)
100
80
60
40
20
0
Dex500 (100 kDa) 100-10
Dex500 (100 kDa) 100-25
Dex500 (100 kDa) 100-50
Dex500 (100 kDa) 100-100
Dex500 (100 kDa) 30-30
Dex500 (30 kDa) 30-30
PBS 30-0
PBS 100-0
Blood
Bladder
Spleen
Kidney R
Kidney L
Liver
Lung
Muscle
Tail
Tumor

Figure 16

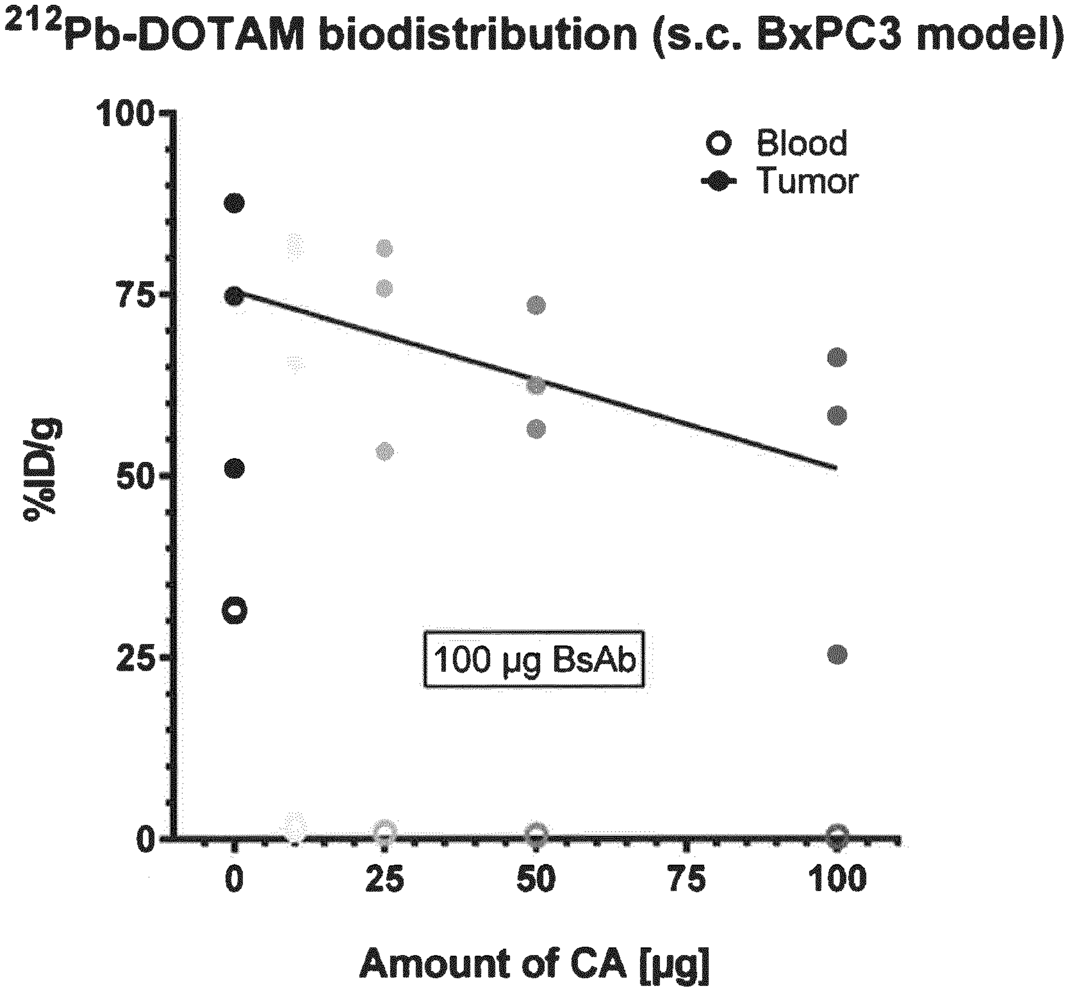

$^{212}$Pb-DOTAM biodistribution (s.c. BxPC3 model)
100
75
50
25
0
%ID/g
Blood
Tumor
100 µg BsAb
0
25
50
75
100
Amount of CA [µg]

# Figure 17

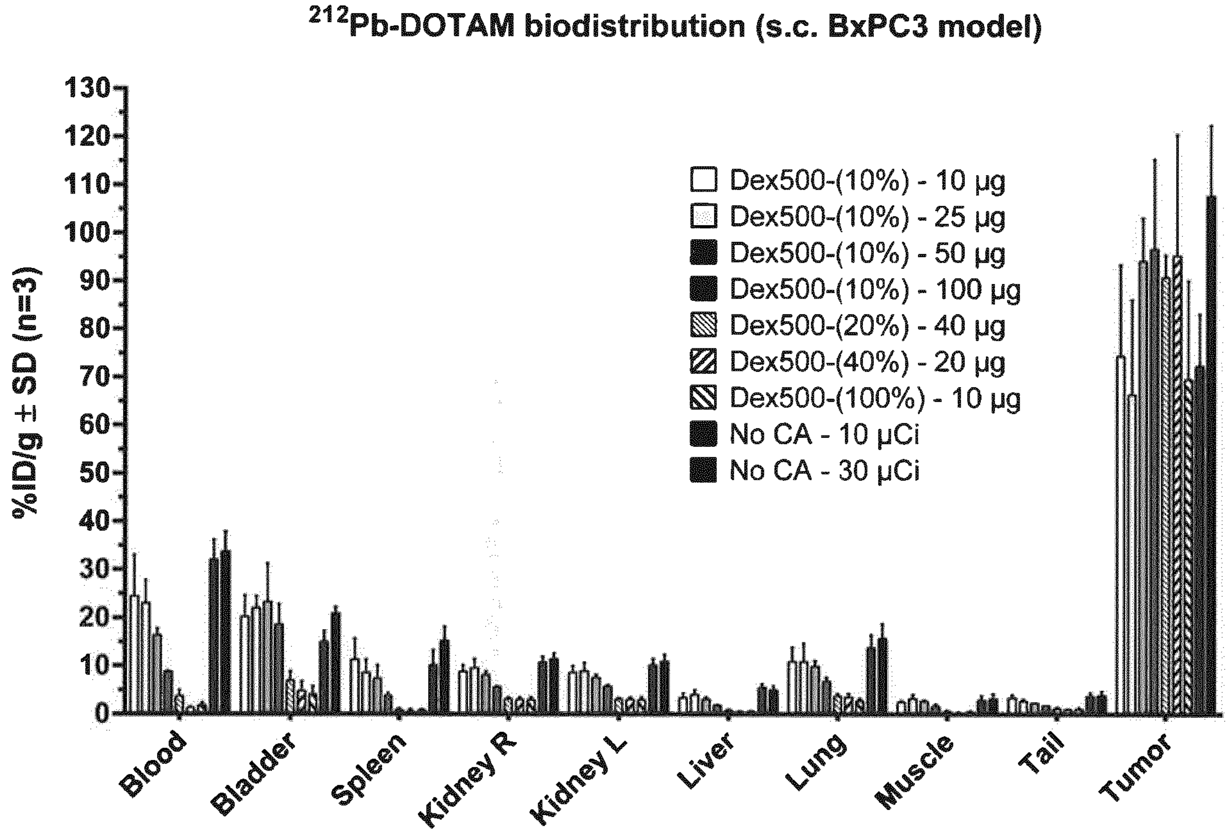

212Pb-DOTAM biodistribution (s.c. BxPC3 model)
%ID/g ± SD (n=3)
130
120
110
100
90
80
70
60
50
40
30
20
10
0
Dex500-(10%) - 10 µg
Dex500-(10%) - 25 µg
Dex500-(10%) - 50 µg
Dex500-(10%) - 100 µg
Dex500-(20%) - 40 µg
Dex500-(40%) - 20 µg
Dex500-(100%) - 10 µg
No CA - 10 µCi
No CA - 30 µCi
Blood
Bladder
Spleen
Kidney R
Kidney L
Liver
Lung
Muscle
Tail
Tumor

# Figure 18

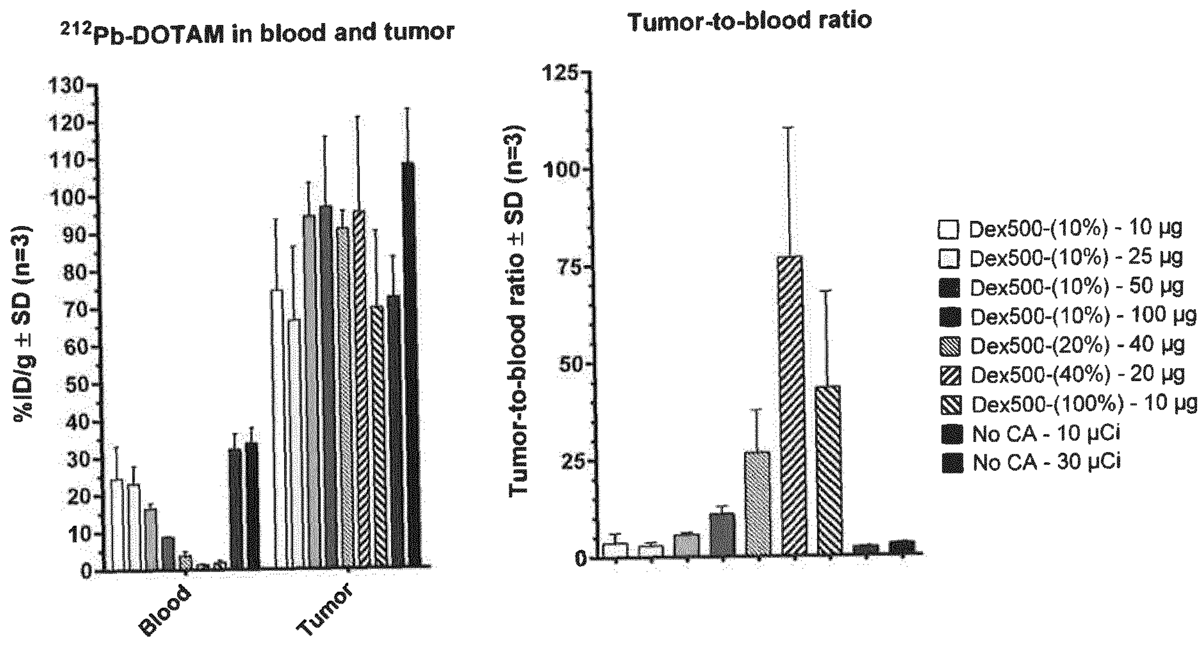

$^{212}$Pb-DOTAM in blood and tumor
%ID/g ± SD (n=3)
130
120
110
100
90
80
70
60
50
40
30
20
10
0
Blood
Tumor
Tumor-to-blood ratio
Tumor-to-blood ratio ± SD (n=3)
125
100
75
50
25
0
Dex500-(10%) - 10 µg
Dex500-(10%) - 25 µg
Dex500-(10%) - 50 µg
Dex500-(10%) - 100 µg
Dex500-(20%) - 40 µg
Dex500-(40%) - 20 µg
Dex500-(100%) - 10 µg
No CA - 10 µCi
No CA - 30 µCi

# Figure 19

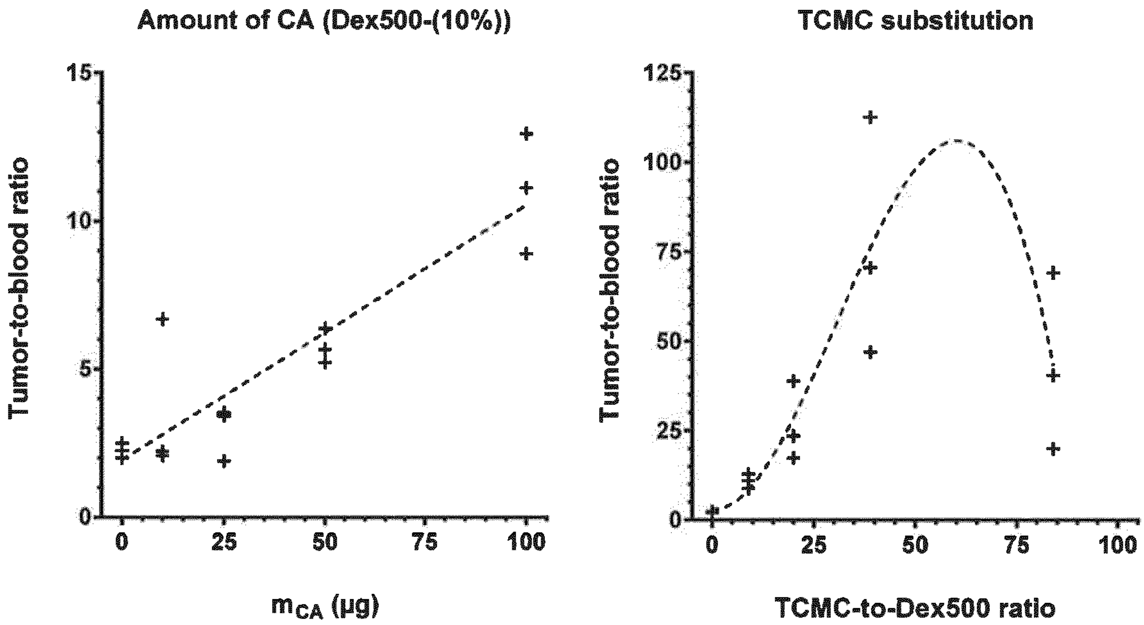
Amount of CA (Dex500-(10%))
Tumor-to-blood ratio
15
10
5
0
0
25
50
75
100
mCA (µg)
TCMC substitution
Tumor-to-blood ratio
125
100
75
50
25
0
0
25
50
75
100
TCMC-to-Dex500 ratio

Figure 20

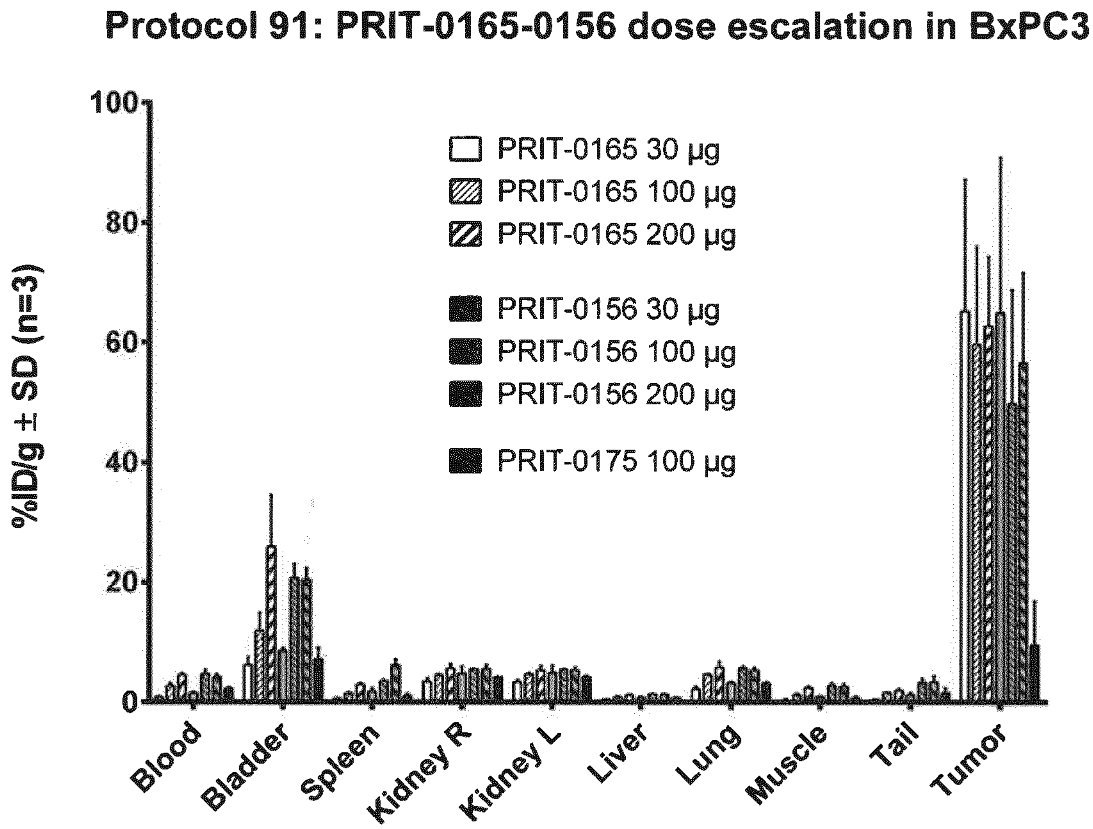
Protocol 91: PRIT-0165-0156 dose escalation in BxPC3
%ID/g ± SD (n=3)
100
80
60
40
20
0
PRIT-0165 30 µg
PRIT-0165 100 µg
PRIT-0165 200 µg
PRIT-0156 30 µg
PRIT-0156 100 µg
PRIT-0156 200 µg
PRIT-0175 100 µg
Blood
Bladder
Spleen
Kidney R
Kidney L
Liver
Lung
Muscle
Tail
Tumor

# Figure 21

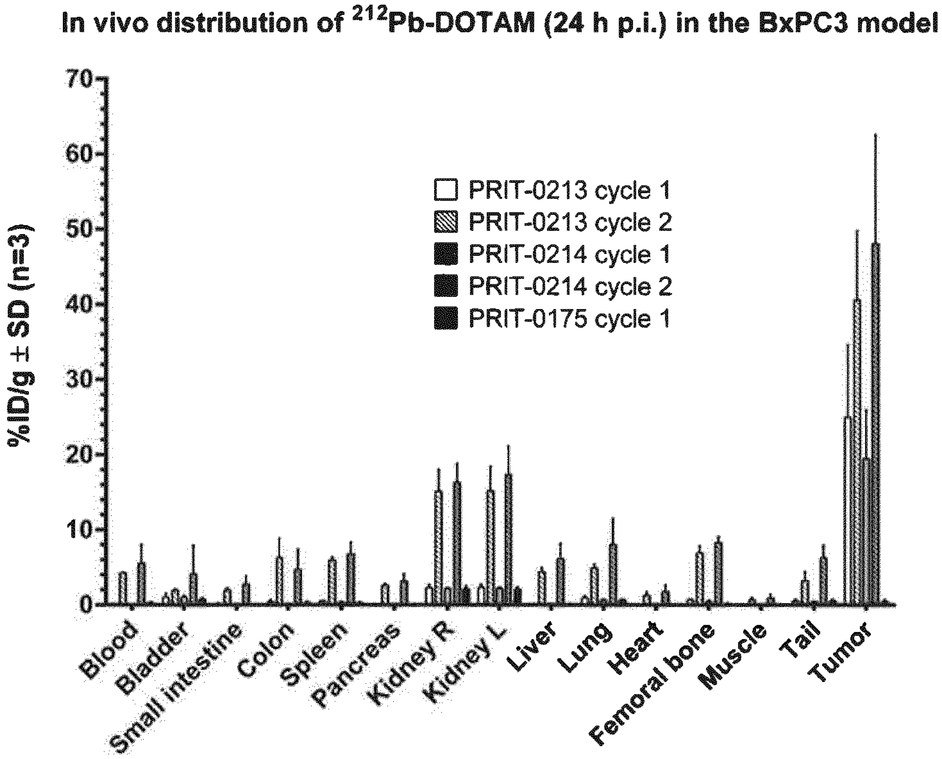
In vivo distribution of 212Pb-DOTAM (24 h p.i.) in the BxPC3 model
%ID/g ± SD (n=3)
70
60
50
40
30
20
10
0
PRIT-0213 cycle 1
PRIT-0213 cycle 2
PRIT-0214 cycle 1
PRIT-0214 cycle 2
PRIT-0175 cycle 1
Blood
Bladder
Small intestine
Colon
Spleen
Pancreas
Kidney R
Kidney L
Liver
Lung
Heart
Femoral bone
Muscle
Tail
Tumor

Figure 22

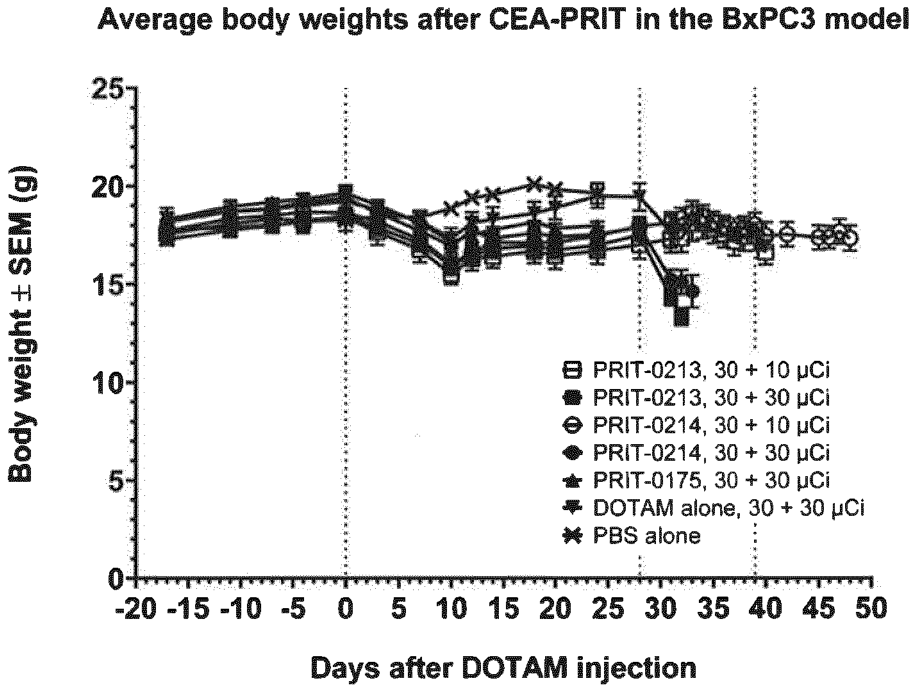


Figure 23

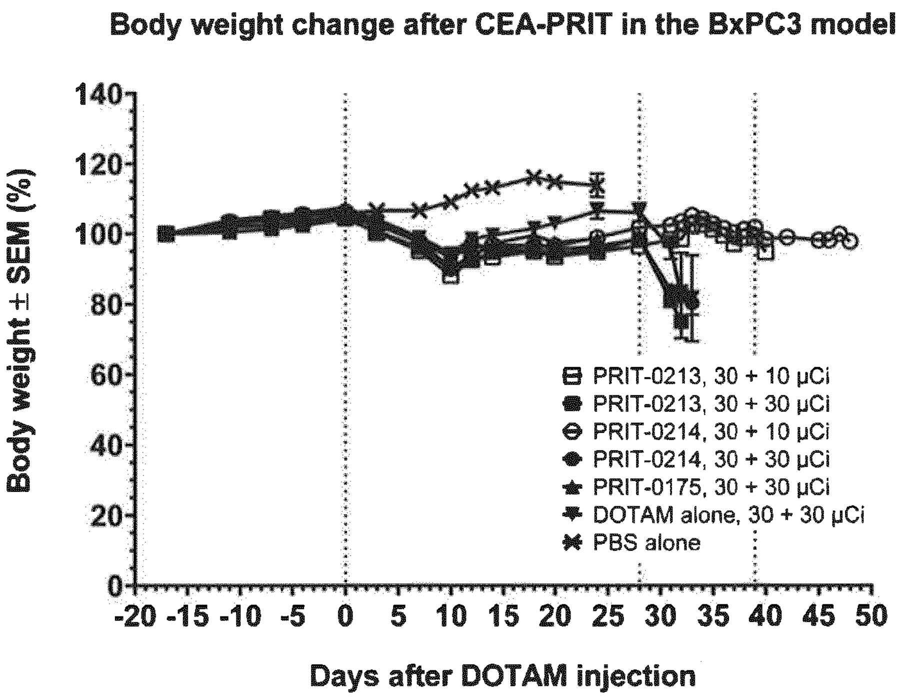

Figure 24

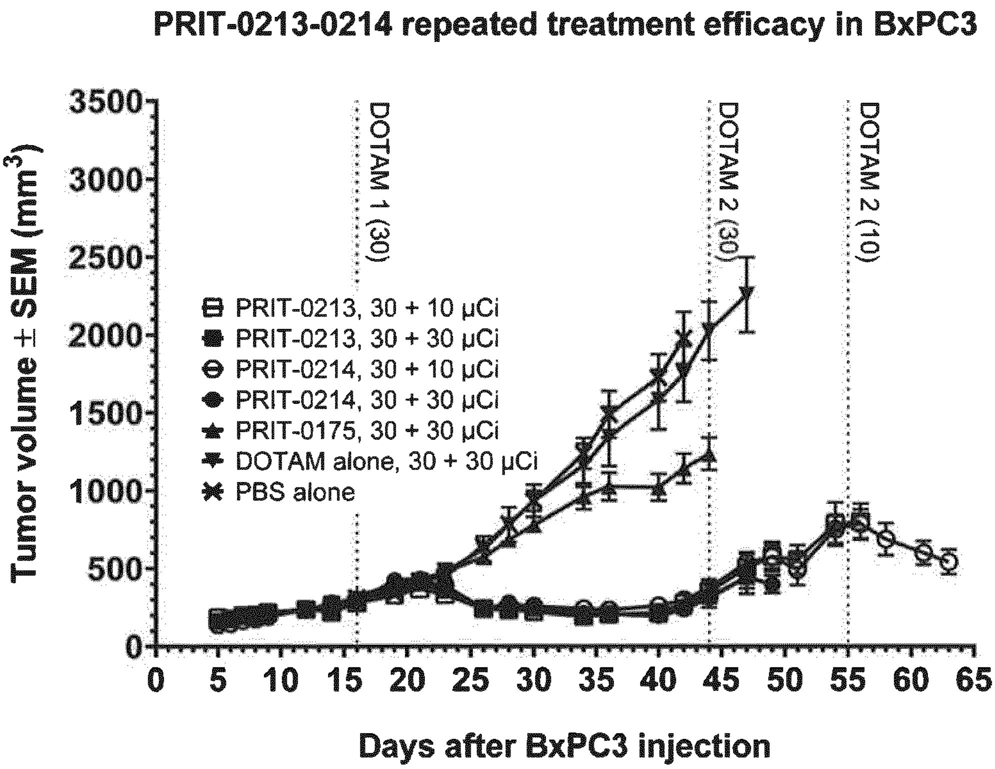
PRIT-0213-0214 repeated treatment efficacy in BxPC3
Tumor volume ± SEM (mm3)
3500
3000
2500
2000
1500
1000
500
0
DOTAM 1 (30)
DOTAM 2 (30)
DOTAM 2 (10)
PRIT-0213, 30 + 10 µCi
PRIT-0213, 30 + 30 µCi
PRIT-0214, 30 + 10 µCi
PRIT-0214, 30 + 30 µCi
PRIT-0175, 30 + 30 µCi
DOTAM alone, 30 + 30 µCi
PBS alone
0 5 10 15 20 25 30 35 40 45 50 55 60 65
Days after BxPC3 injection

# Figure 25

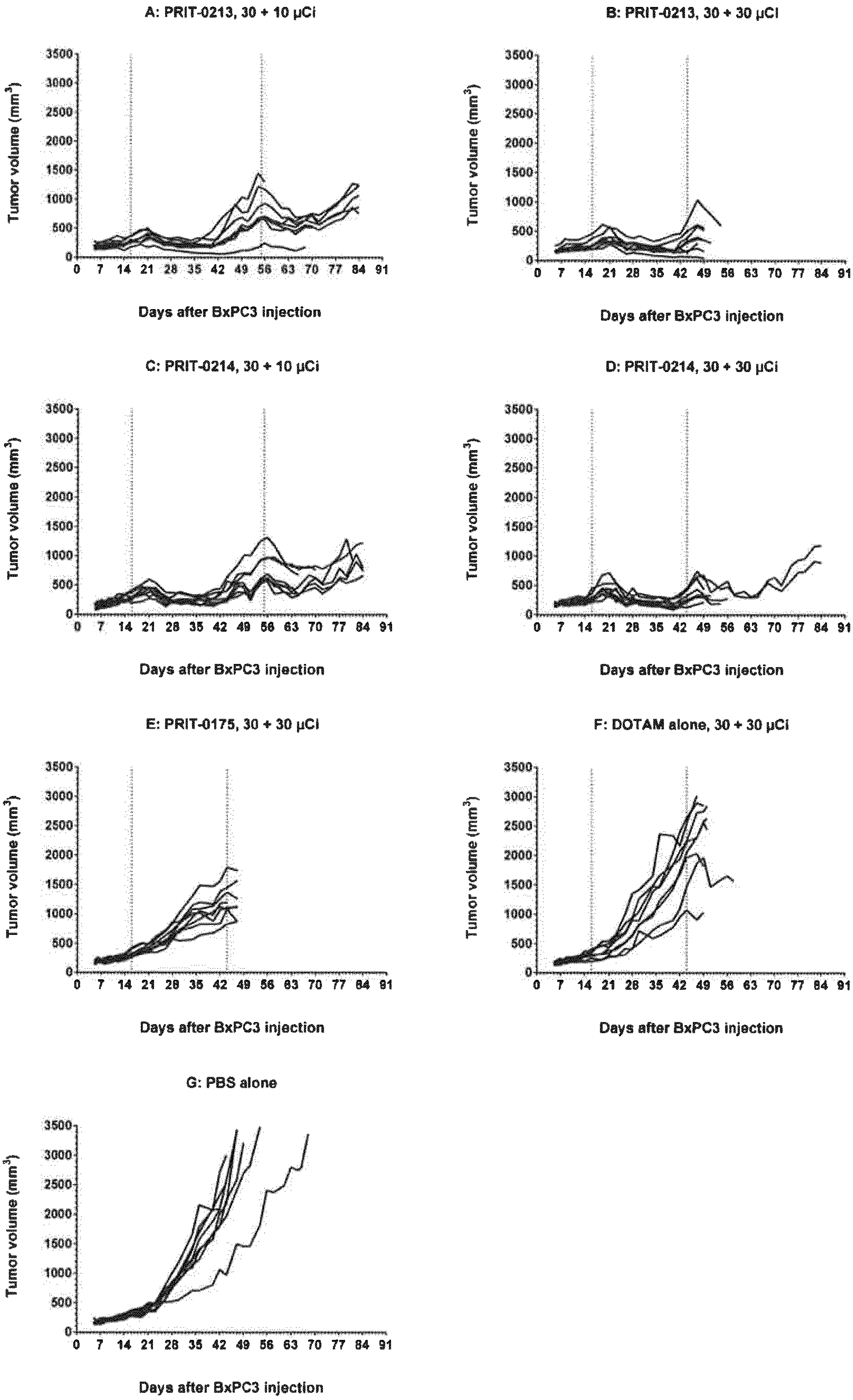
A: PRIT-0213, 30 + 10 µCi
B: PRIT-0213, 30 + 30 µCi
C: PRIT-0214, 30 + 10 µCi
D: PRIT-0214, 30 + 30 µCi
E: PRIT-0175, 30 + 30 µCi
F: DOTAM alone, 30 + 30 µCi
G: PBS alone
Tumor volume (mm³)
0
500
1000
1500
2000
2500
3000
3500
0 7 14 21 28 35 42 49 56 63 70 77 84 91
Days after BxPC3 injection

Figure 26

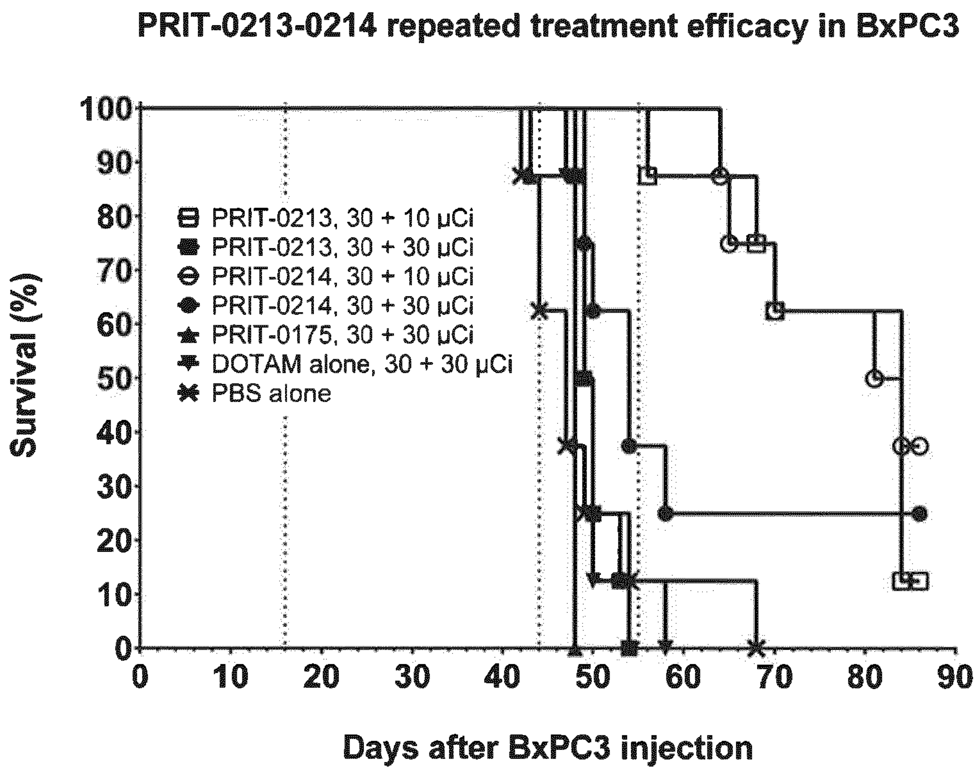
PRIT-0213-0214 repeated treatment efficacy in BxPC3
Survival (%)
100
90
80
70
60
50
40
30
20
10
0
0
10
20
30
40
50
60
70
80
90
Days after BxPC3 injection
PRIT-0213, 30 + 10 µCi
PRIT-0213, 30 + 30 µCi
PRIT-0214, 30 + 10 µCi
PRIT-0214, 30 + 30 µCi
PRIT-0175, 30 + 30 µCi
DOTAM alone, 30 + 30 µCi
PBS alone

# Figure 27

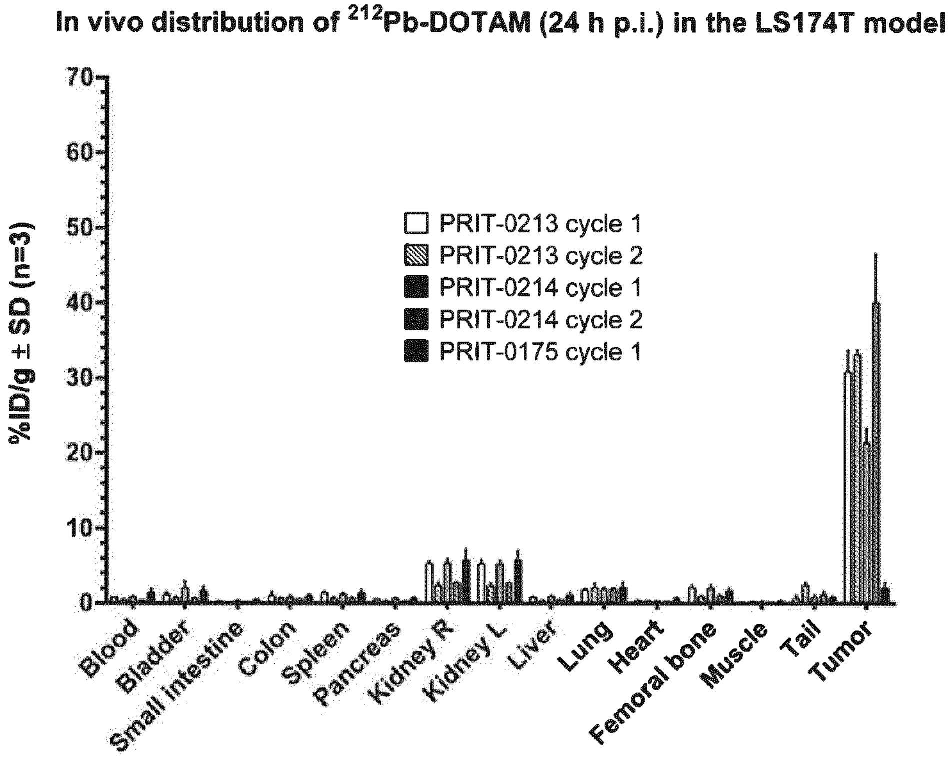
In vivo distribution of 212Pb-DOTAM (24 h p.i.) in the LS174T model
%ID/g ± SD (n=3)
70
60
50
40
30
20
10
0
PRIT-0213 cycle 1
PRIT-0213 cycle 2
PRIT-0214 cycle 1
PRIT-0214 cycle 2
PRIT-0175 cycle 1
Blood
Bladder
Small intestine
Colon
Spleen
Pancreas
Kidney R
Kidney L
Liver
Lung
Heart
Femoral bone
Muscle
Tail
Tumor

Figure 28

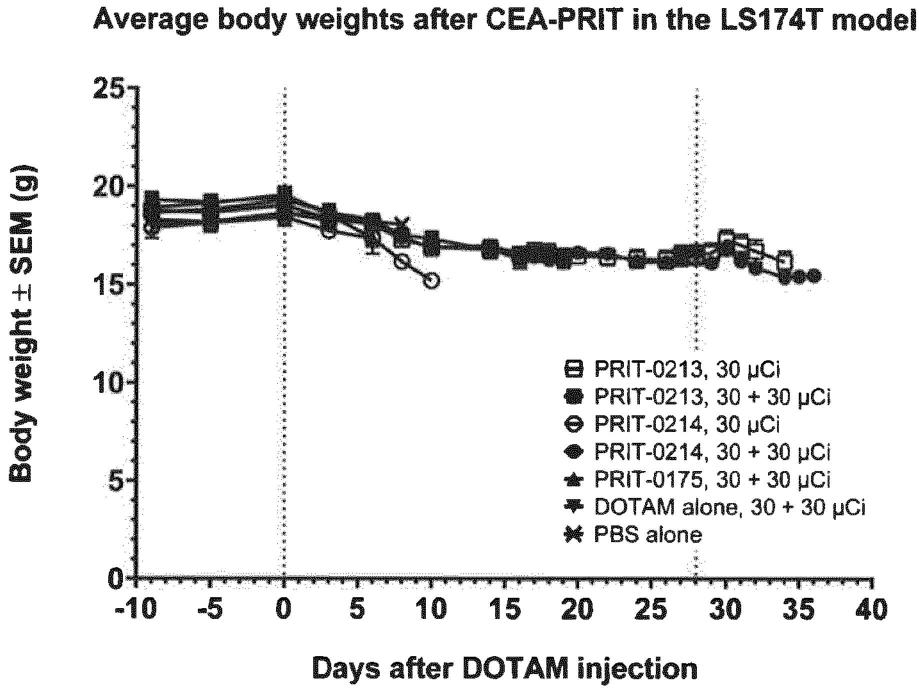


Figure 29

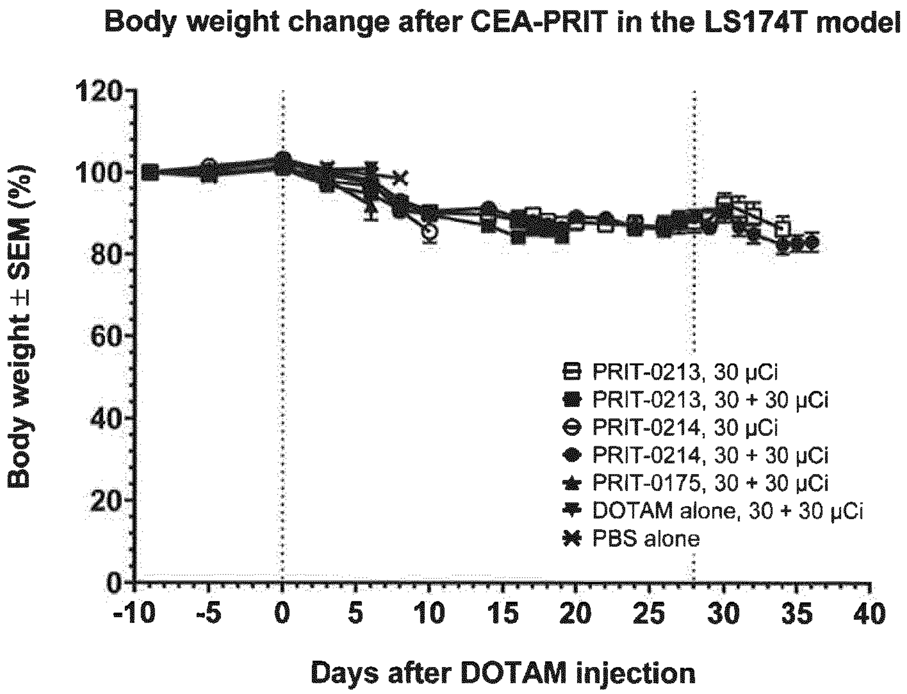

Figure 30

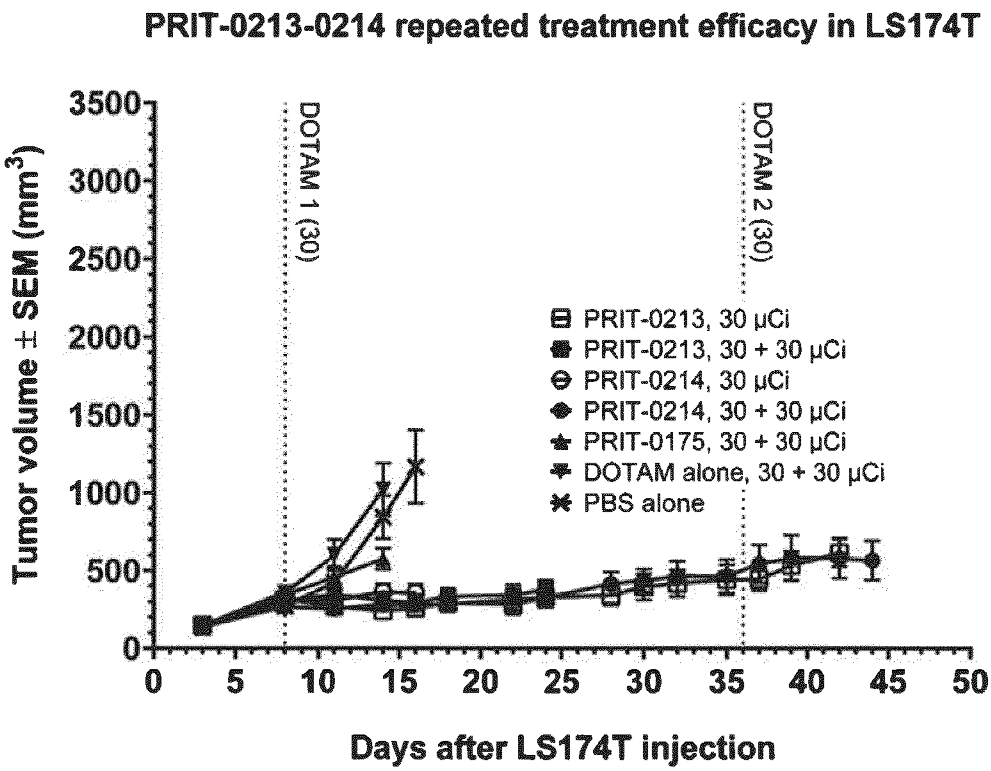
PRIT-0213-0214 repeated treatment efficacy in LS174T
Tumor volume ± SEM (mm3)
3500
3000
2500
2000
1500
1000
500
0
DOTAM 1 (30)
DOTAM 2 (30)
PRIT-0213, 30 µCi
PRIT-0213, 30 + 30 µCi
PRIT-0214, 30 µCi
PRIT-0214, 30 + 30 µCi
PRIT-0175, 30 + 30 µCi
DOTAM alone, 30 + 30 µCi
PBS alone
0
5
10
15
20
25
30
35
40
45
50
Days after LS174T injection

# Figure 31

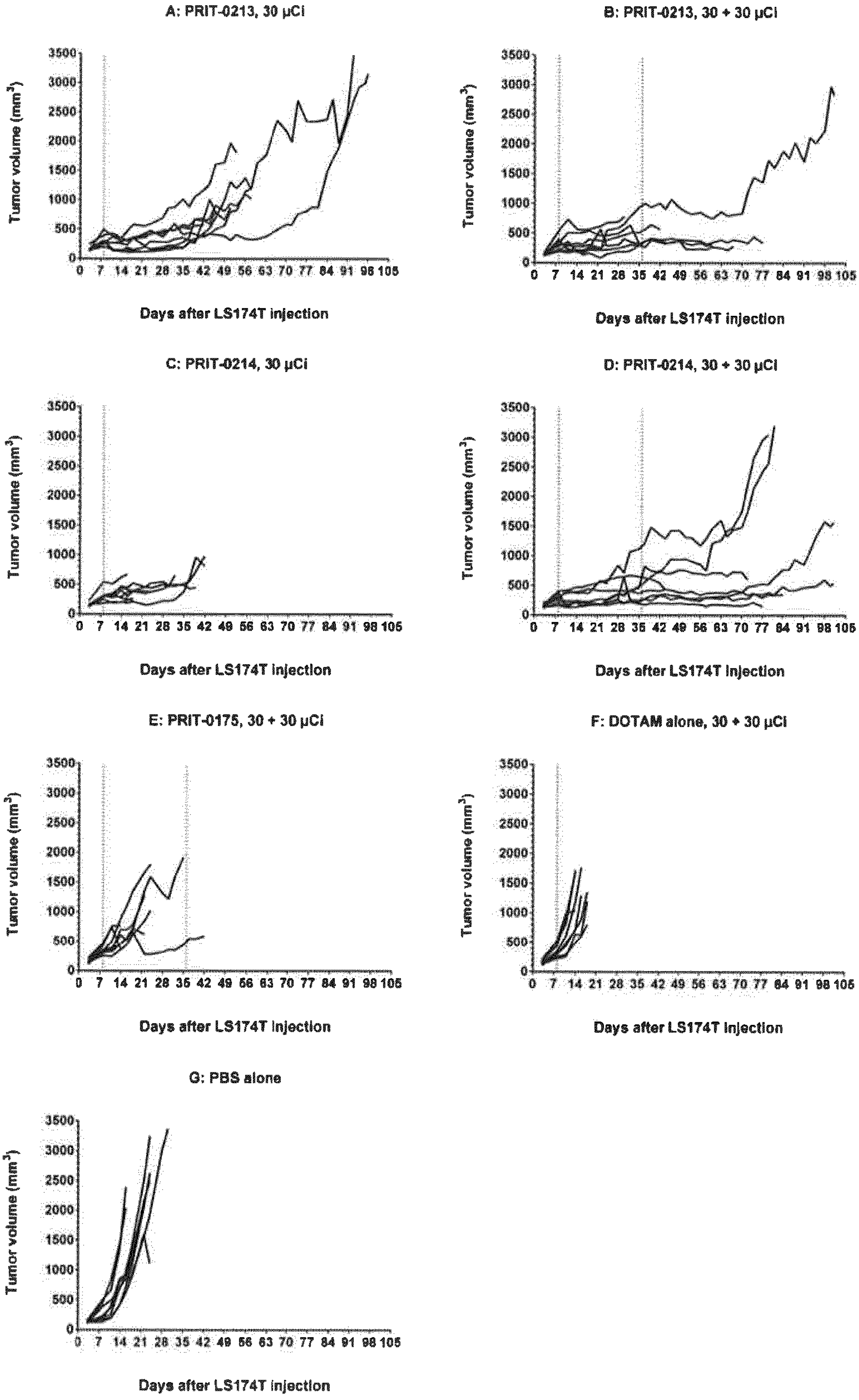
A: PRIT-0213, 30 µCi
B: PRIT-0213, 30 + 30 µCi
C: PRIT-0214, 30 µCi
D: PRIT-0214, 30 + 30 µCi
E: PRIT-0175, 30 + 30 µCi
F: DOTAM alone, 30 + 30 µCi
G: PBS alone
Tumor volume (mm³)
0
500
1000
1500
2000
2500
3000
3500
0 7 14 21 28 35 42 49 56 63 70 77 84 91 98 105
Days after LS174T injection

Figure 32

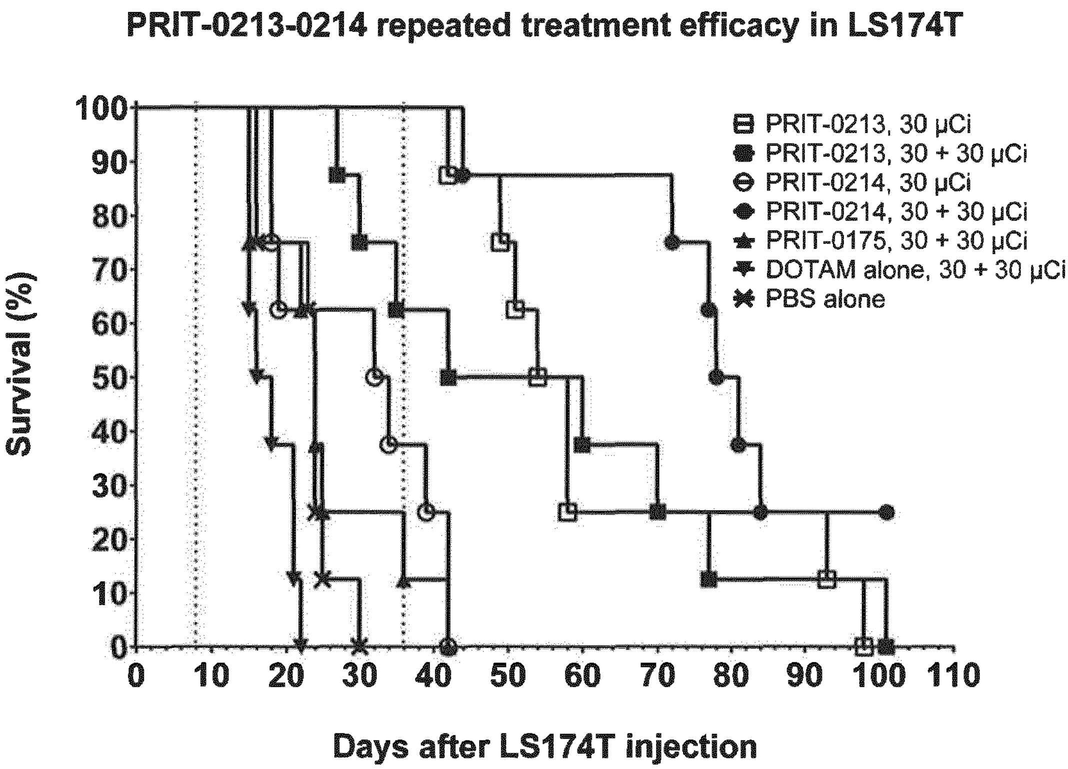
PRIT-0213-0214 repeated treatment efficacy in LS174T
Survival (%)
Days after LS174T injection
PRIT-0213, 30 μCi
PRIT-0213, 30 + 30 μCi
PRIT-0214, 30 μCi
PRIT-0214, 30 + 30 μCi
PRIT-0175, 30 + 30 μCi
DOTAM alone, 30 + 30 μCi
PBS alone

Figure 33

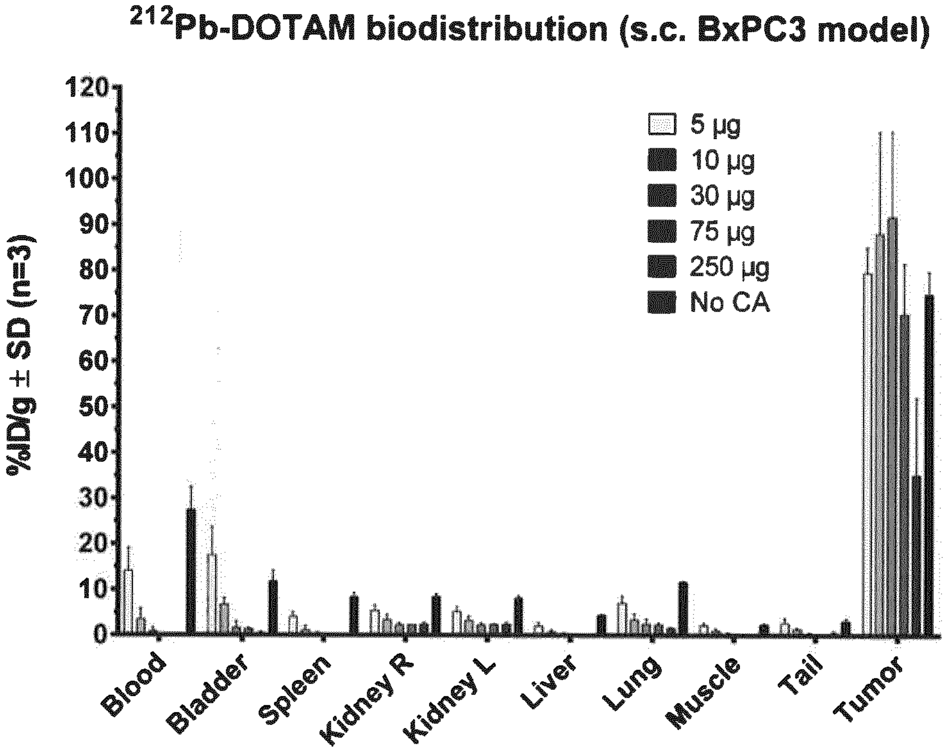


Figure 34

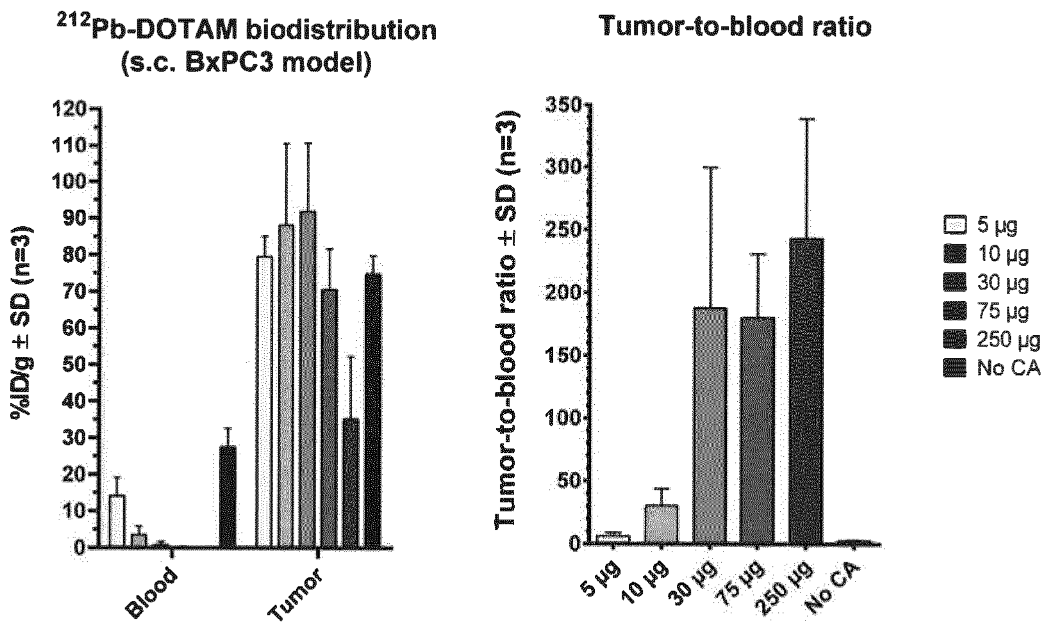

Figure 35

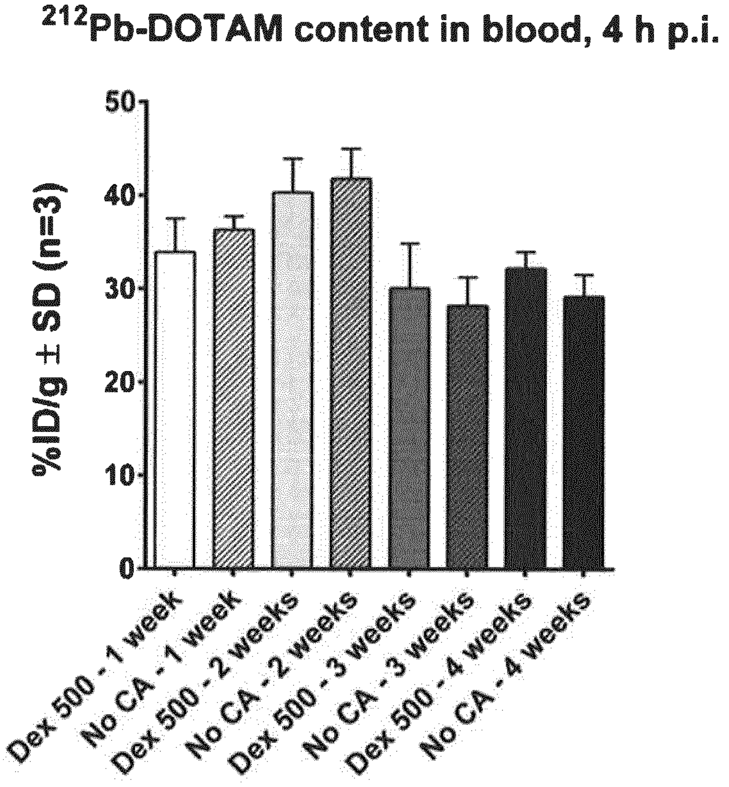
212Pb-DOTAM content in blood, 4 h p.i.
%ID/g ± SD (n=3)
50
40
30
20
10
0
Dex 500 - 1 week
No CA - 1 week
Dex 500 - 2 weeks
No CA - 2 weeks
Dex 500 - 3 weeks
No CA - 3 weeks
Dex 500 - 4 weeks
No CA - 4 weeks

Figure 36

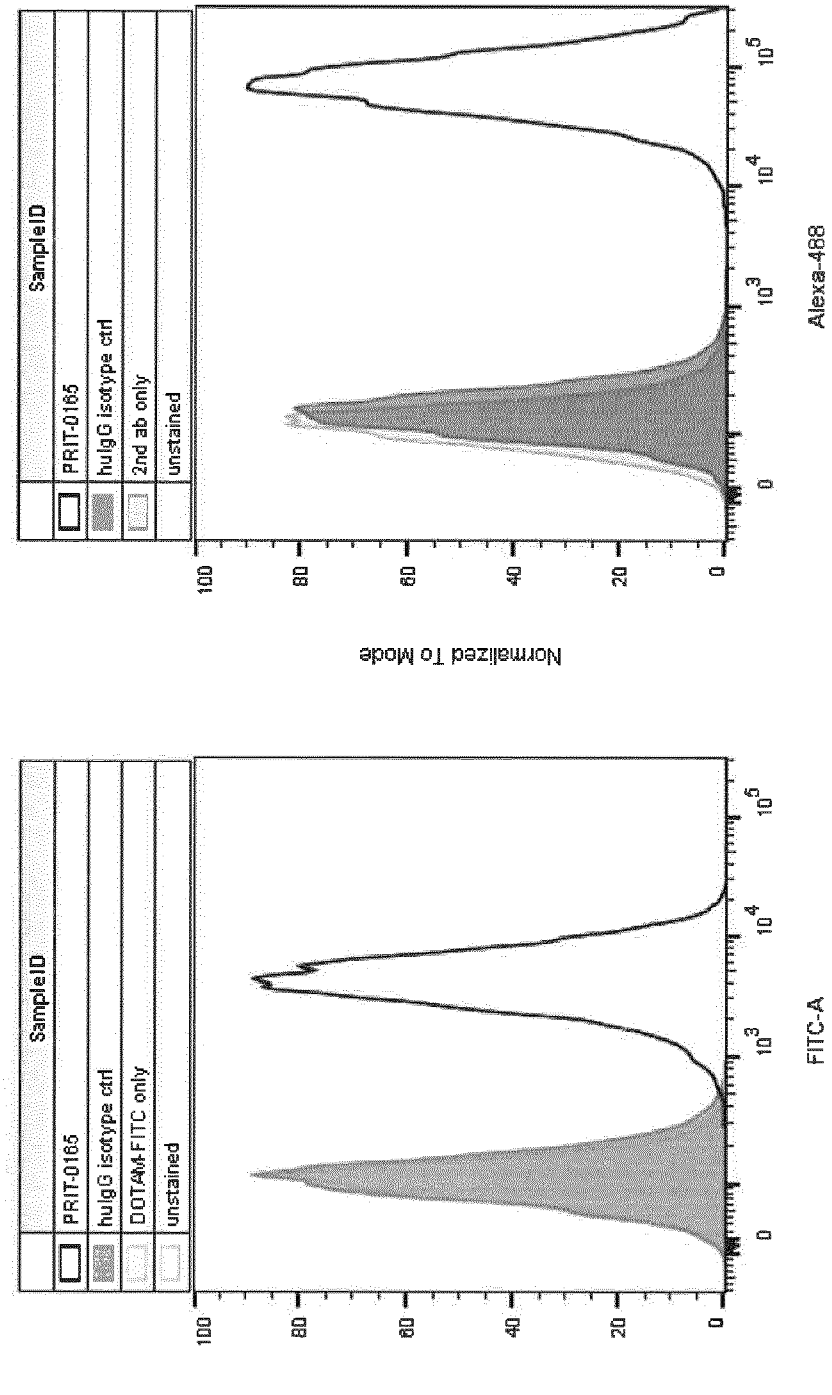
SampleID
PRIT-0165
huIgG isotype ctrl
DOTAM-FITC only
unstained
Normalized To Mode
FITC-A
SampleID
PRIT-0165
huIgG isotype ctrl
2nd ab only
unstained
Normalized To Mode
Alexa-488

Figure 37: Possible antibody Formats

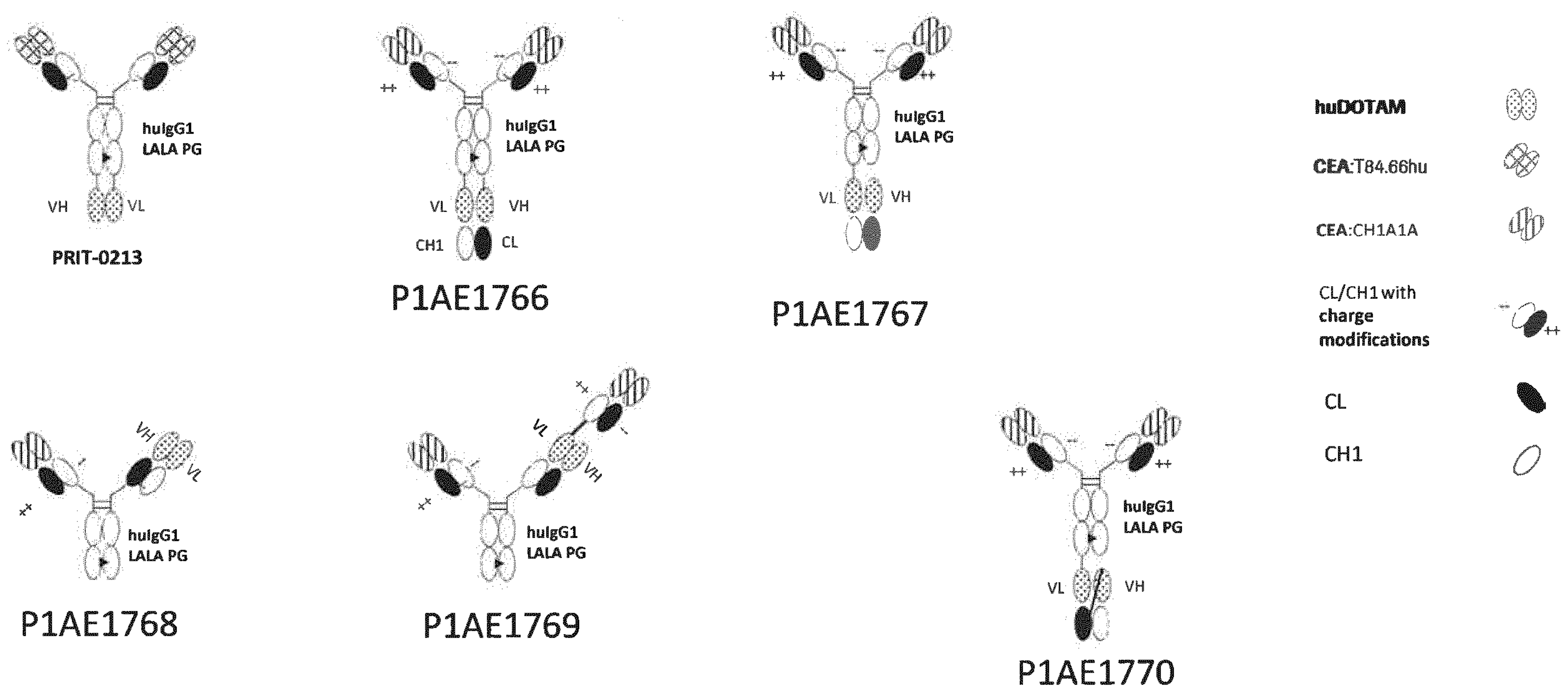

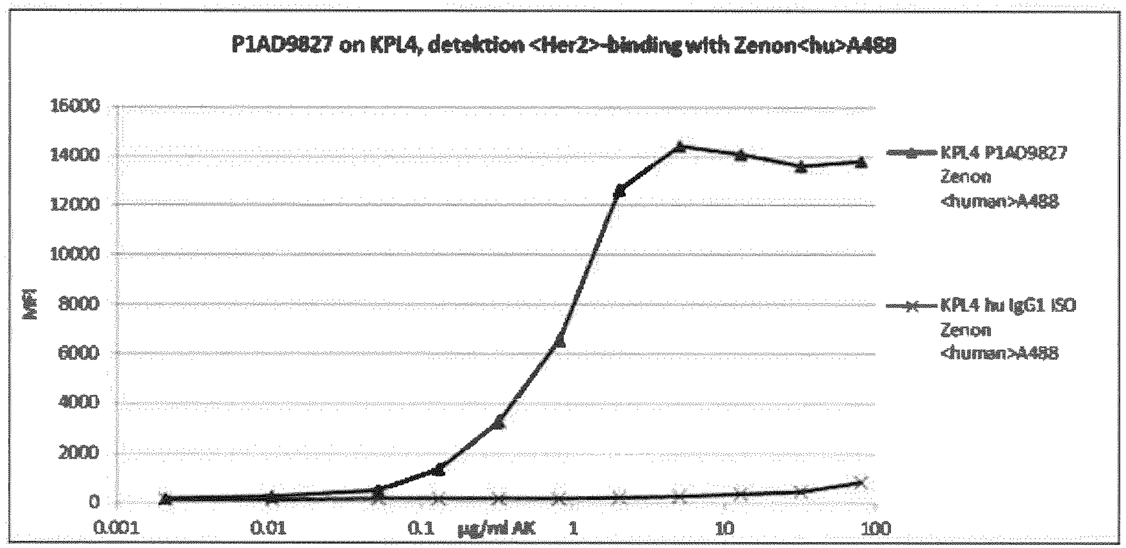


Figure 38: Binding of P1AD8927 to KPL-4 cells to demonstrate Her2 binding competence: Detection of antibodies using human IgG specific secondary antibodies

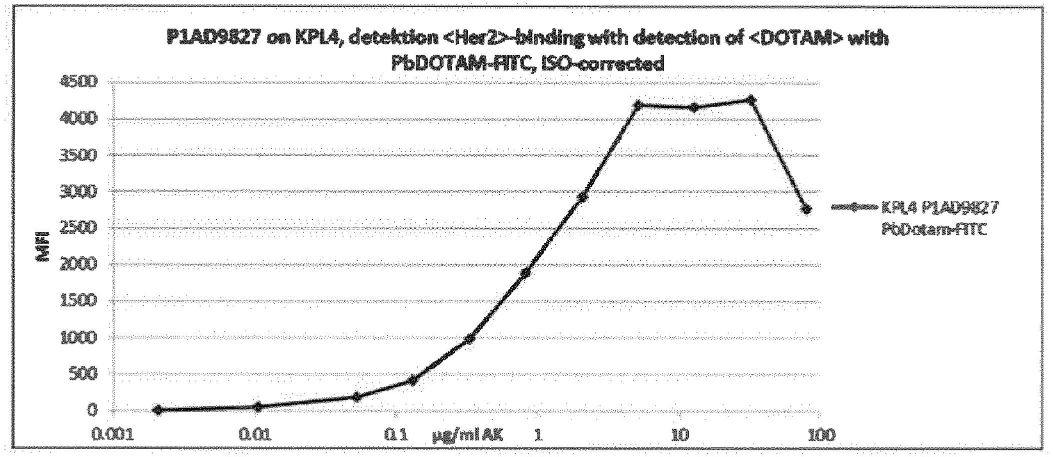


Figure 39: Binding of P1AD8927 to KPL-4 cells to demonstrate DOTAM binding competence: Isotypecorrected detection using Pb-DOTAM-FITC

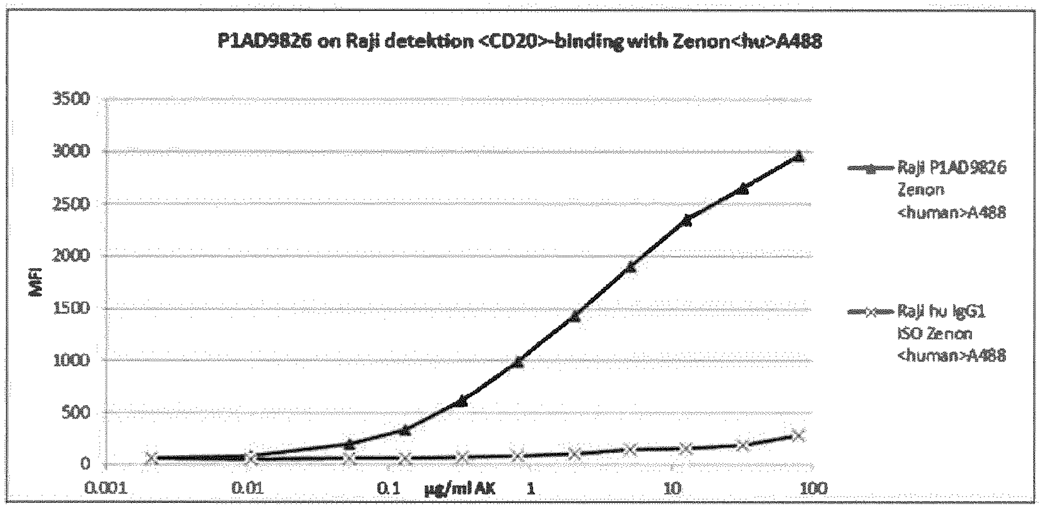


Figure 40: Binding of P1AD8926 to Raji cells to demonstrate CD20 binding competence: Detection of antibodies using human IgG specific secondary antibodies

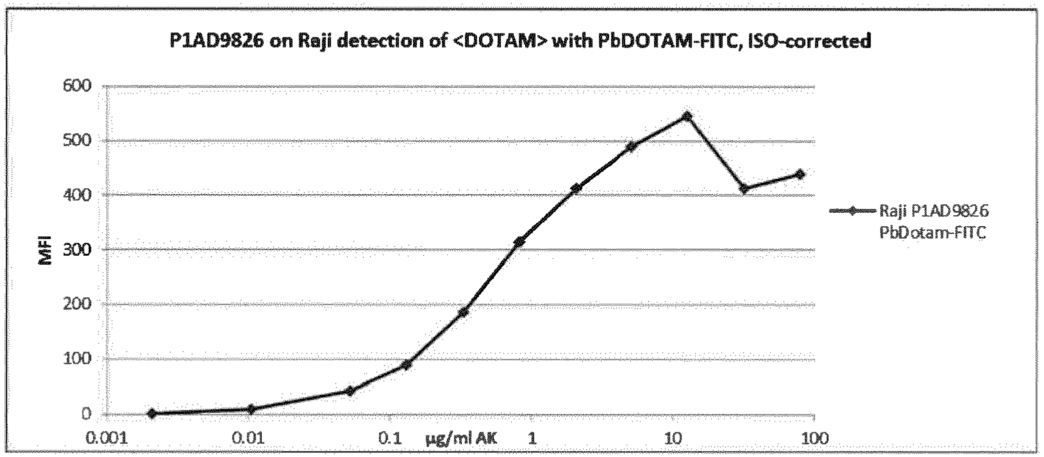


Figure 41: Binding of P1AD8926 to Raji cells to demonstrate DOTAM binding competence: Isotypecorrected detection using Pb-DOTAM-FITC

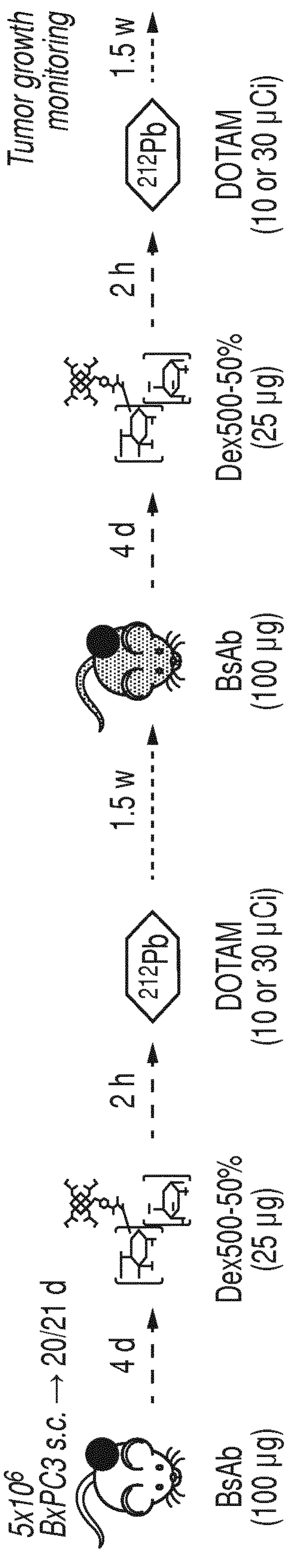


Design of Protocol 103

FIG. 42

Figure 43: In Vivo Radioactivity Distribution 24 Hours after 212Pb-DOTAM Injection (Treatment Cycle 1 and 2)

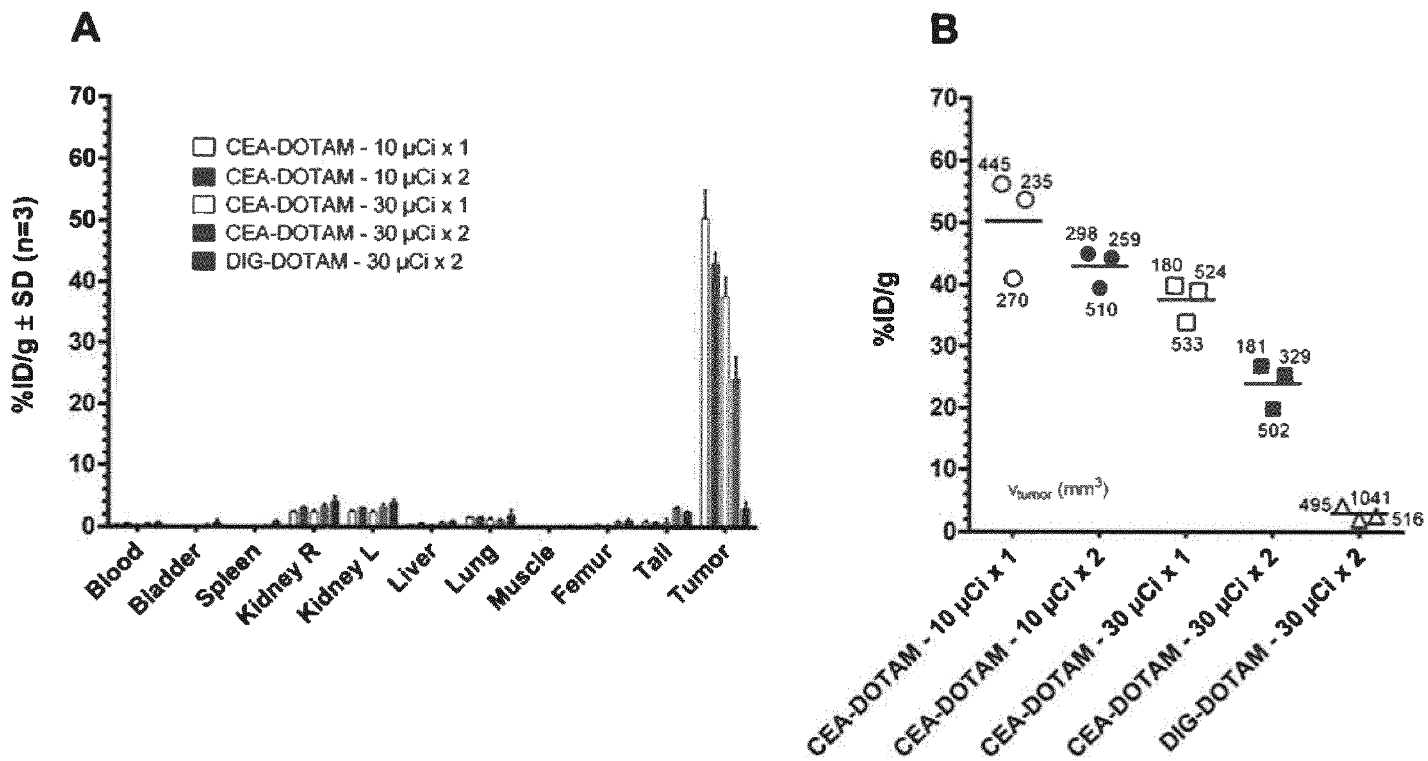

Figure 44: Tumor Development after CEA PRIT in the BxPC3 Model

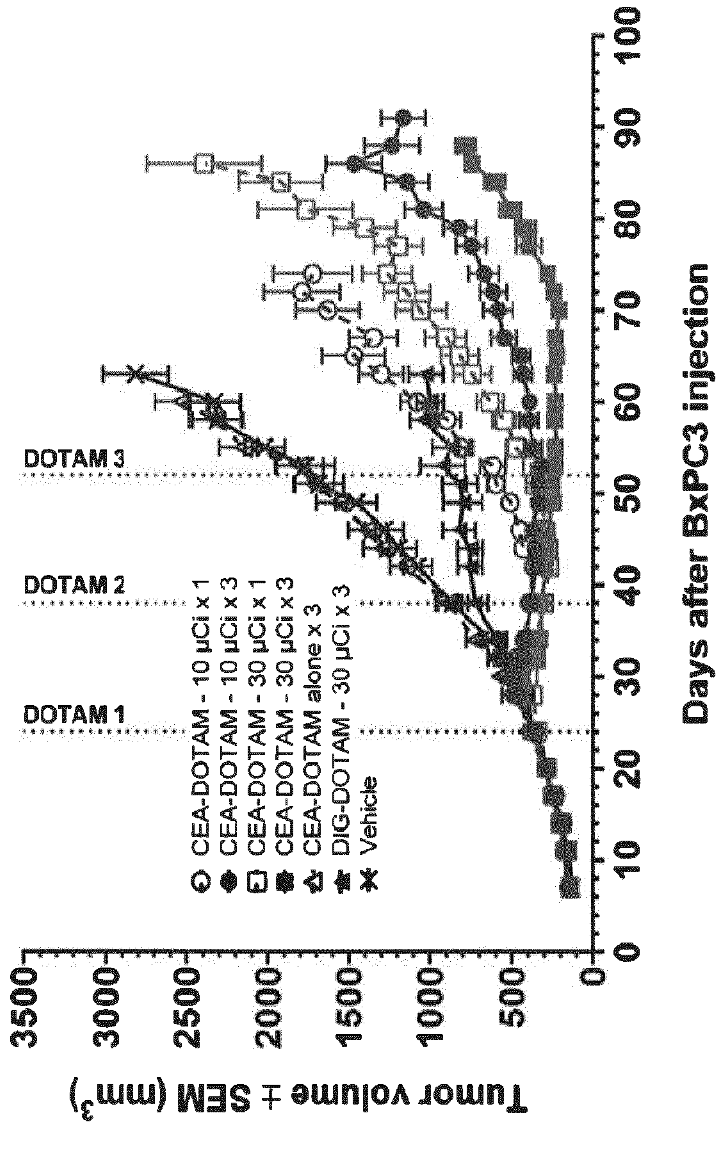

Figure 45: Individual Tumor Growth Curves after CEA PRIT in the BxPC3 Model

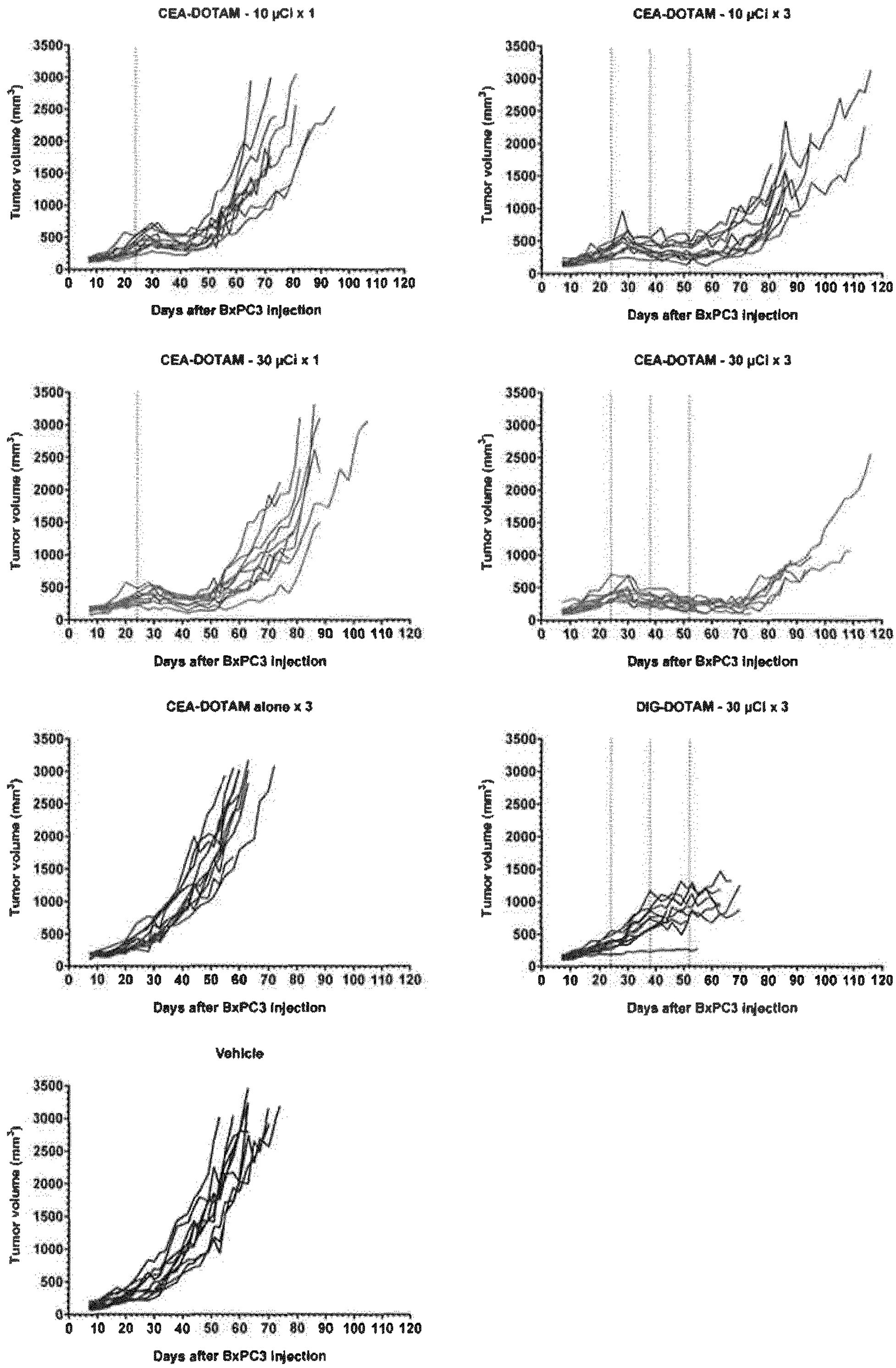

Figure 46: Survival after CEA PRIT in the BxPC3 Model

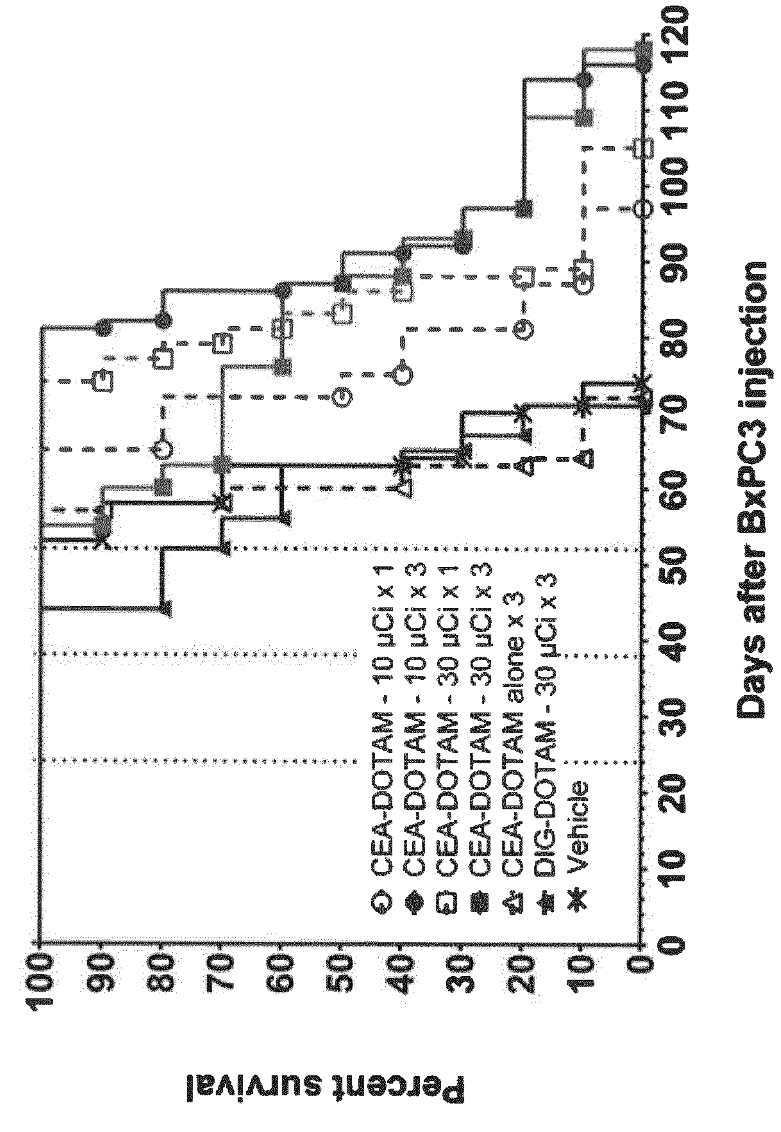

Figure 47: Average Body Weight Change after CEA PRIT in the BxPC3 Model

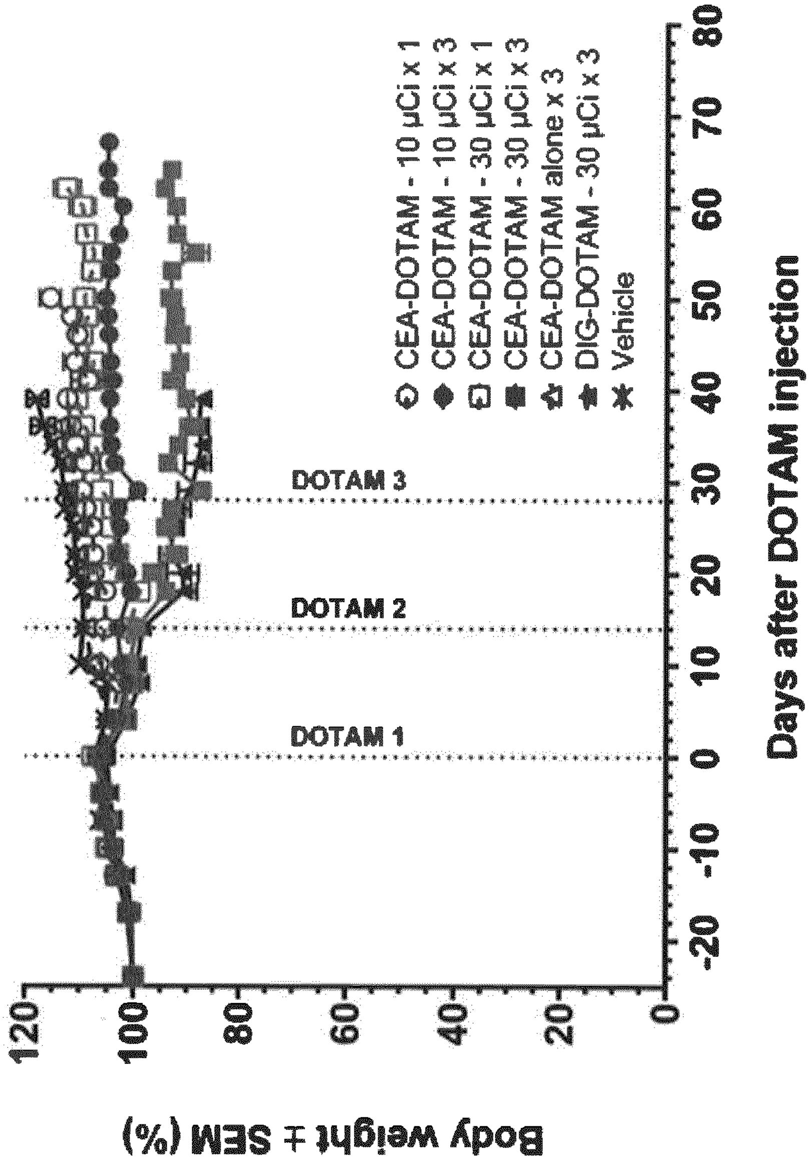

Figure 48: 203Pb-BsAb Distribution in SCID Mice Bearing SC BxPC3 Tumors

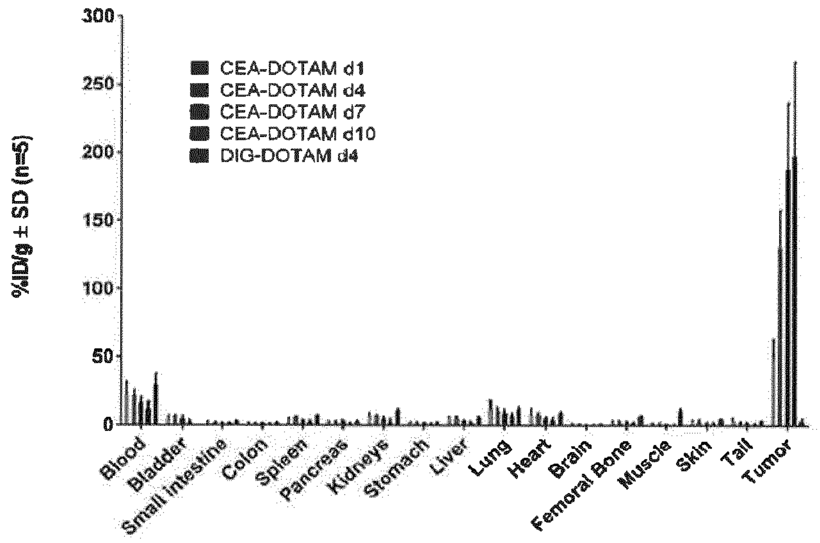


Figure 49: 203Pb-BsAb BxPC3 Tumor Accumulation in SCID Mice

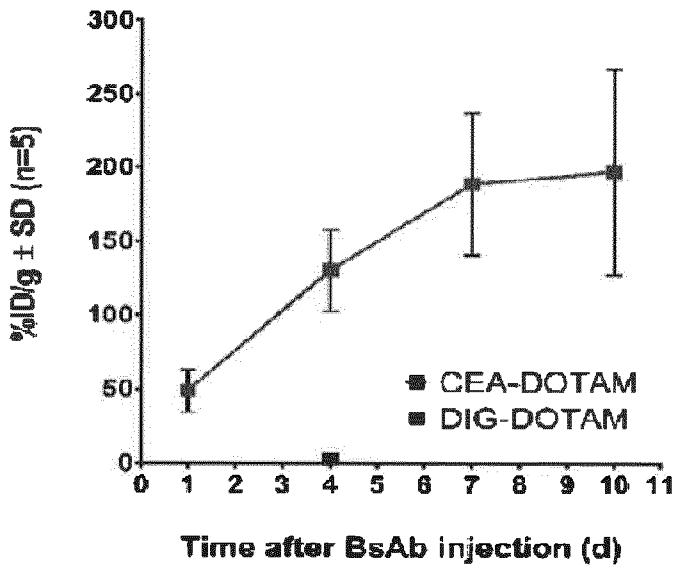

Figure 50: Biodistribution of Pretargeted 212Pb-DOTAM in SCID Mice Carrying SC BxPC3 Tumors

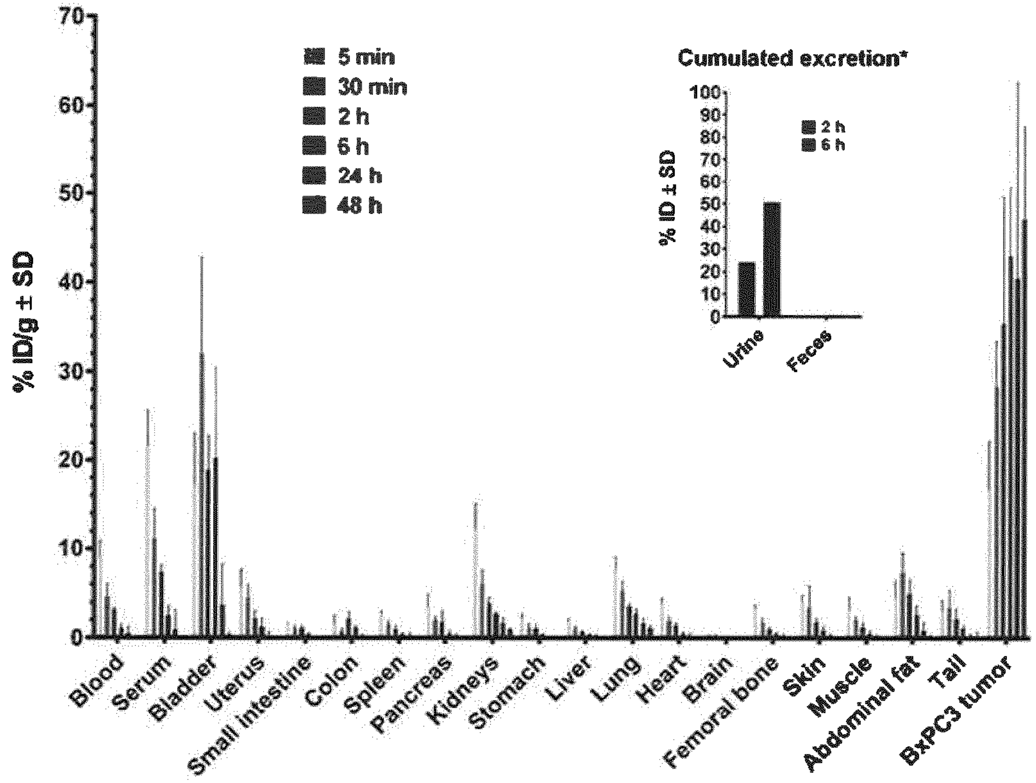

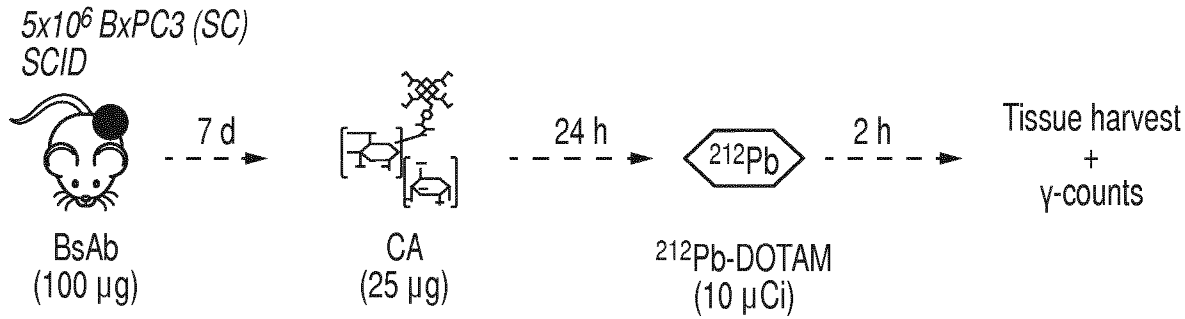


Design of Protocol 131

FIG. 51

Figure 52: Biodistribution of Pretargeted 212Pb-DOTAM in SCID Mice Carrying SC BxPC3 Tumors (2 h p.i.)

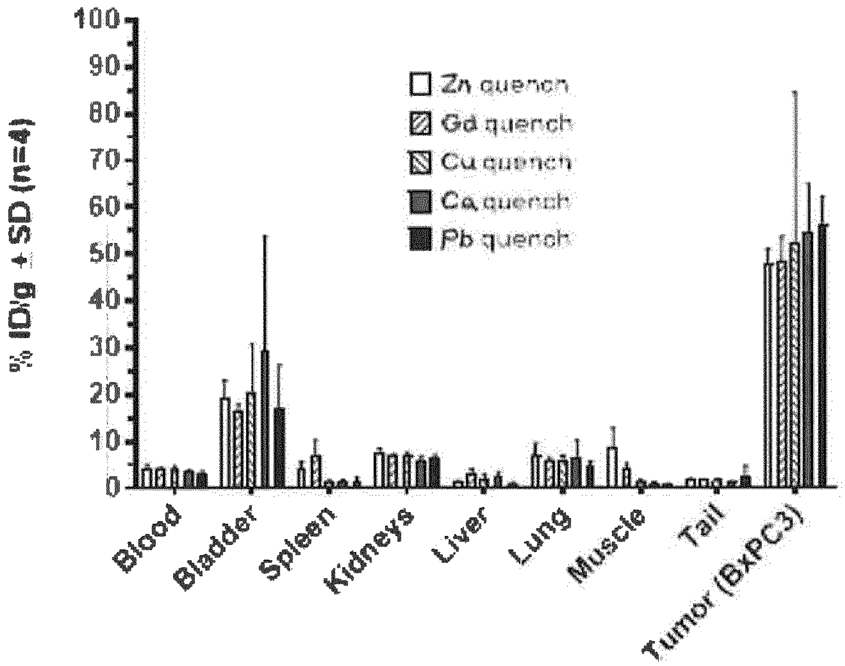


Figure 53:Distribution of 212Pb-DOTAM in Selected Normal Tissues (2 h p.i.)

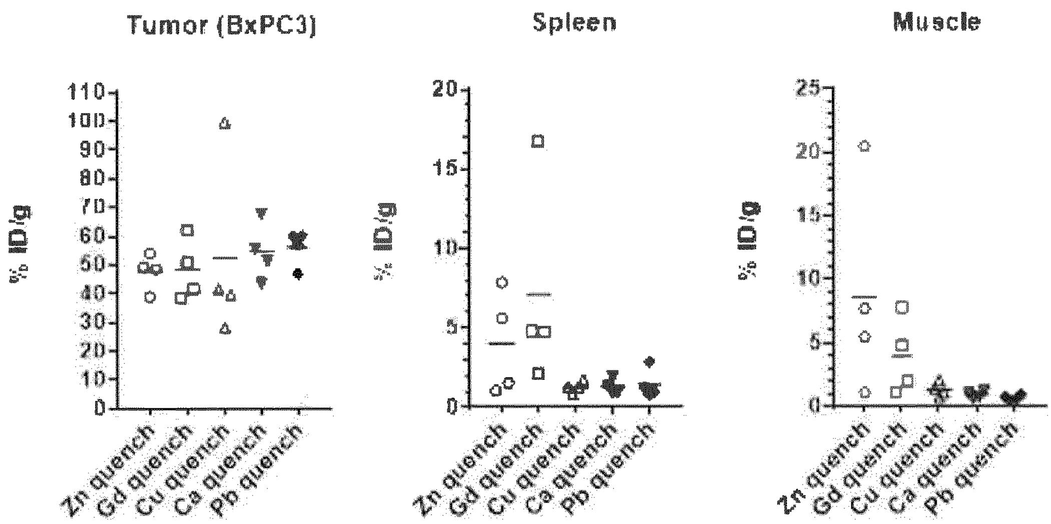

Figure 54: Biodistribution of Pretargeted 212Pb-DOTAM in SCID Mice Carrying SC WSU-DLCL2 Tumors (24 h p.i.)

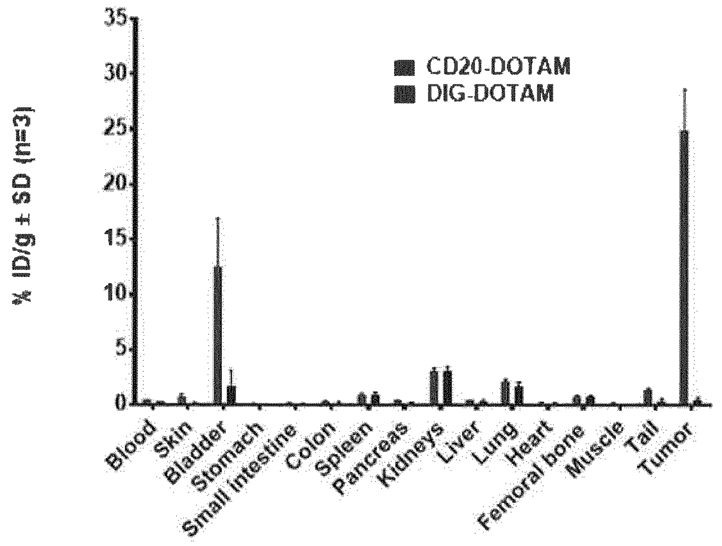


Figure 55: Biodistribution of Pretargeted 212Pb-DOTAM in SCID Mice Carrying SC NCI-N87 Tumors (24 h p.i.)

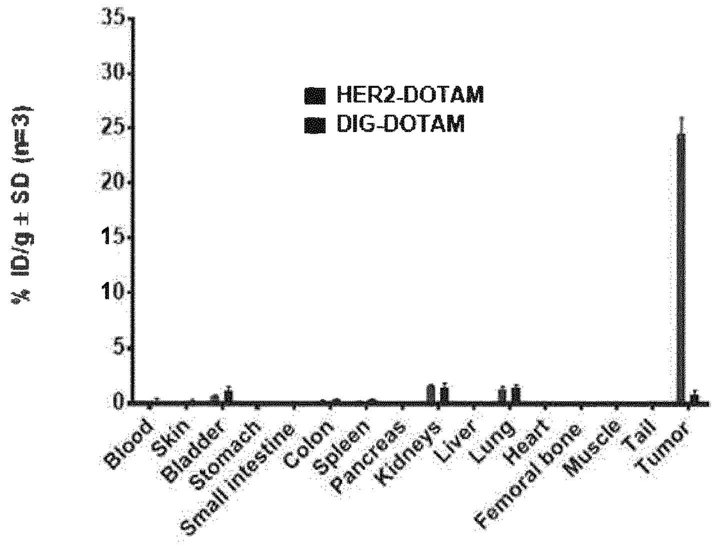

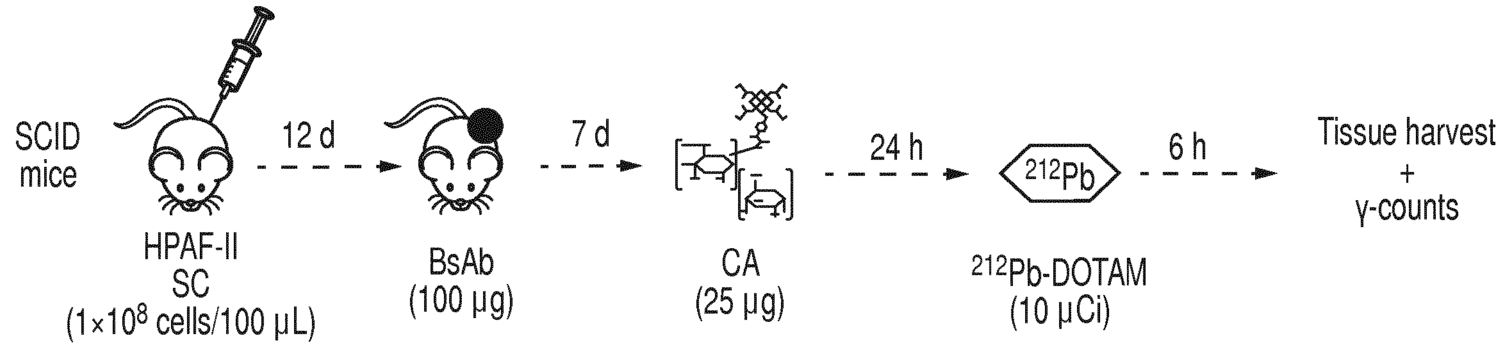


Design of Protocol 154

FIG. 56

Figure 57: Biodistribution of Pretargeted 212Pb-DOTAM in SCID Mice Carrying SC HPAF-II Tumors (6 h p.i.)

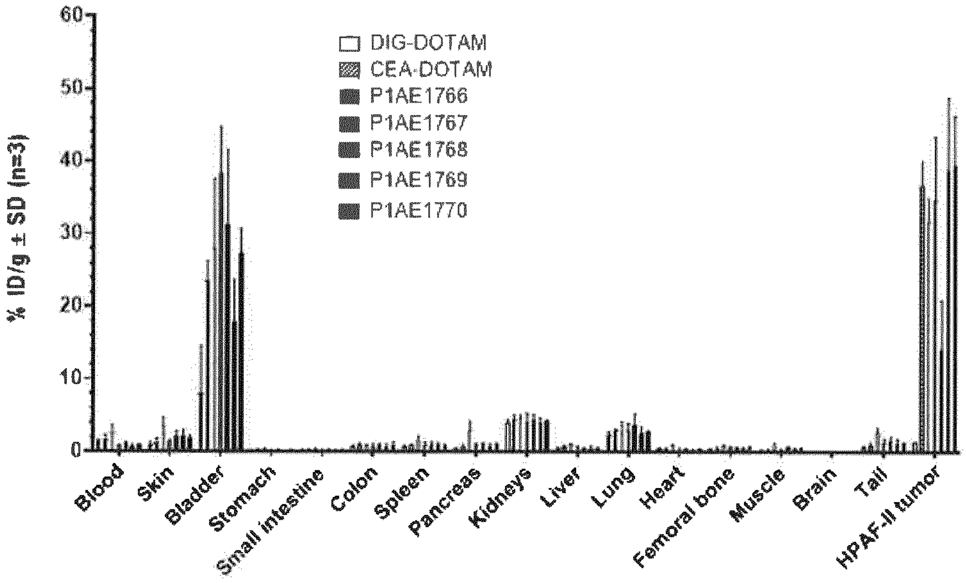


Figure 58: Accumulation of Pretargeted 212Pb-DOTAM in HPAF-II Tumors

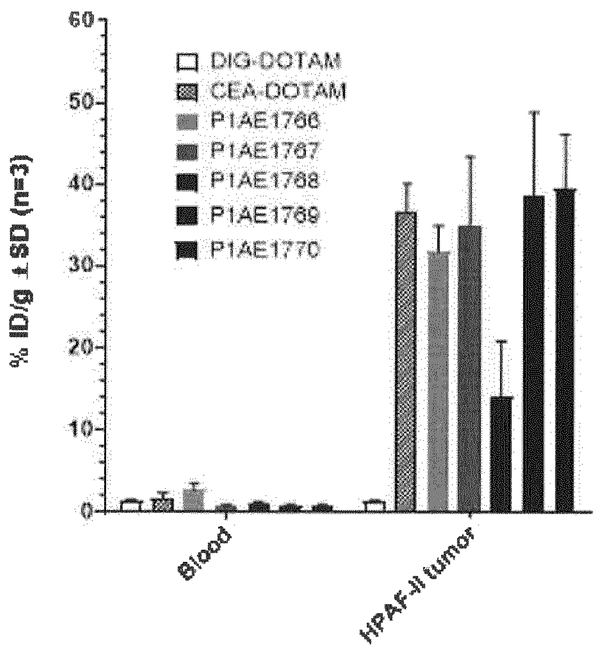

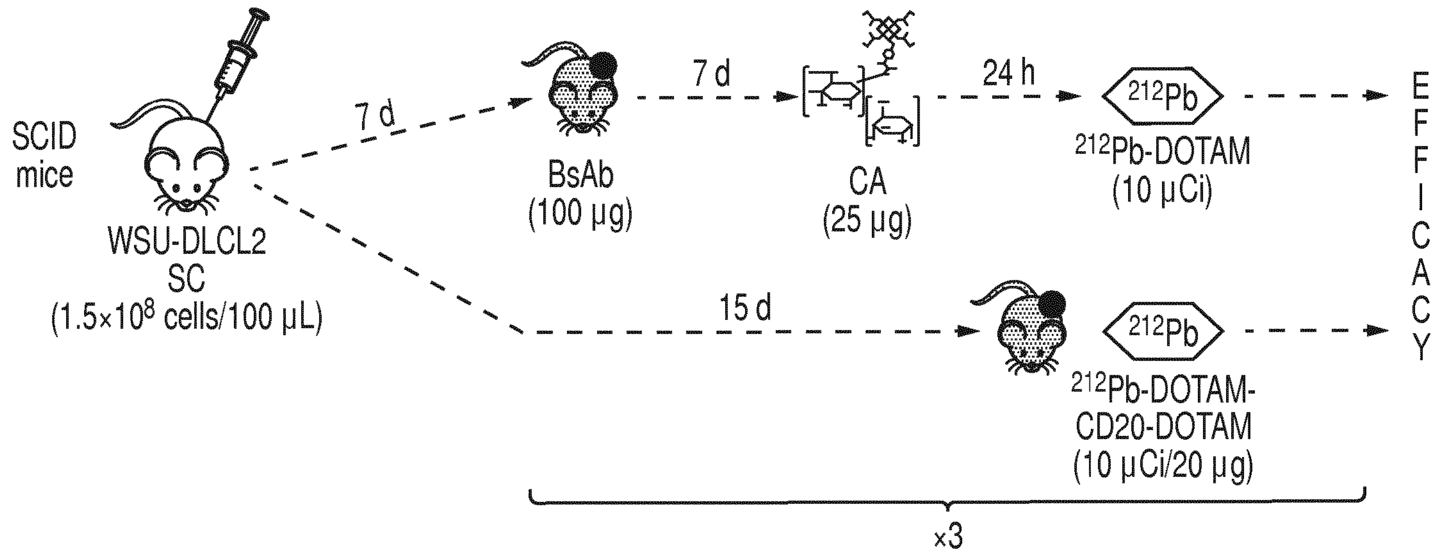


Experimental schedule of protocol 162

FIG. 59

Figure 60:Biodistribution of pretargeted 212Pb-DOTAM and 212Pb-DOTAM-CD20-DOTAM in SCID mice carrying SC WSU-DLCL2 tumors (24 h p.i.)

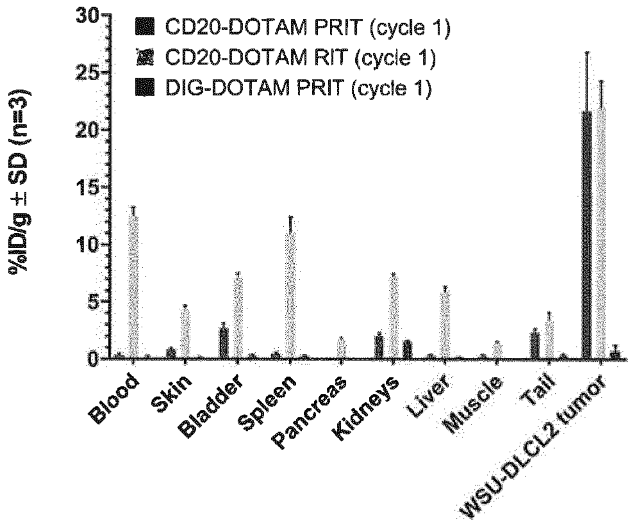


Figure 61:Average SC WSU-DLCL2 tumor volumes in SCID mice treated with CD20-targeted therapies or negative controls

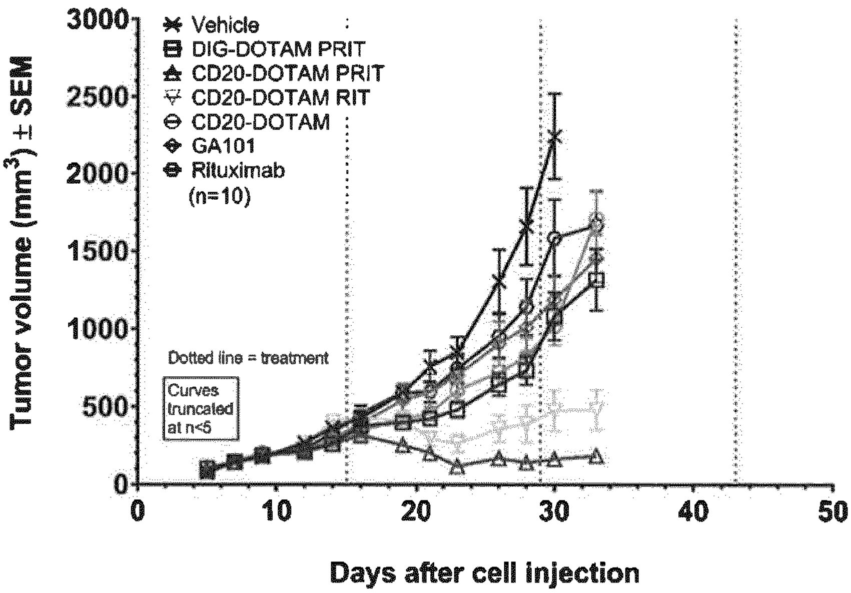

Figure 62: Average body weight development in SCID mice treated with CD20-targeted therapies or negative controls

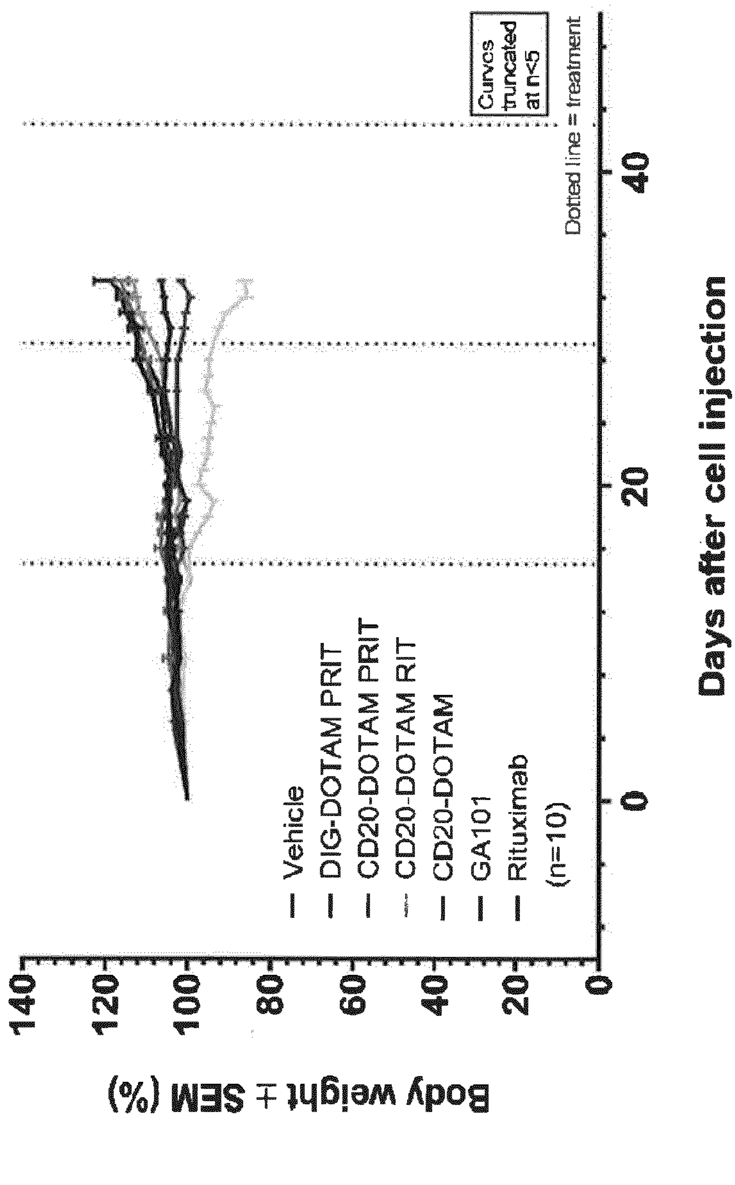

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010099536 A
- WO 2010115589 A1
- EP 2101823 B1
- WO 9316185 A
- US 5571894 A
- US 5587458 A
- US 5869046 A
- EP 404097 A
- WO 199301161 A
- US 6248516 B1
- US 5242824 A
- US 5846535 A
- US 20070189962
- WO 2016075278 A1
- WO 2017055389 A
- WO 2011034660 A
- US 6676924 B
- US 5874540 A
- WO 2001077342 A
- WO 2007024715 A
- US 6897044 B
- US 7129330 B
- US 20050079170 A
- US 6511663 B
- WO 2006020258 A
- US 20050163782 A
- US 5959083 A
- WO 1997001580 A
- WO 1992004053 A
- WO 199106305 A
- US 6350860 B
- US 20050100543 A
- WO 1995009917 A
- WO 2007109254 A
- WO 2009080252 A
- WO 2015150447 A
- WO 2009080253 A
- WO 2009080251 A
- WO 2016016299 A
- WO 2016172485 A
- WO 214180754 A
- WO 2010115589 A
- WO 96027011 A
- WO 2008077546 A
- US 20030157108 A, Presta, L
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S
- WO 199922764 A, Raju, S
- US 5500362 A
- WO 5821337 A
- WO 2006029879 A
- WO 2005100402 A
- WO 2013120929 A1
- US 6737056 B
- US 7332581 B
- WO 2012130831 A
- WO 2004056312 A
- US 6194551 B
- WO 9951642 A
- WO 2014177460 A
- US 5648260 A
- US 5624821 A
- WO 9429351 A
- US 4816567 A
- US 5648237 A
- US 5789199 A
- US 5840523 A
- US 5959177 A
- US 6040498 A
- US 6420548 B
- US 7125978 B
- US 6417429 B
- US 20050260186 A
- US 20060104968 A
- US 6267958 B
- US 6171586 B
- WO 2006044908 A

- WO 2013174949 A
- WO 200046251 A
- WO 200212437 A
- WO 2005007696 A
- WO 2006047367 A
- US 20070033661 A
- WO 2008027986 A
- WO 2016062734 A

**Non-patent literature cited in the description**


- **GOLDENBERG et al.** *Theranostics,* 2012, vol. 2 (5), 523-540
- **SU et al.** *Nucl Med Biol,* 2005, vol. 32, 741-747
- **YONG ; BRECHBIEL.** *Dalton Trans.,* 21 June 2001, vol. 40 (23), 6068-6076
- **CARTER, P et al.** *Immunotechnol.,* 1996, vol. 2, 73
- **CARTER, P. et al.** *Immunotechnol.,* 1996, vol. 2, 73
- **KABAT et al.** Sequences of Proteins of Immunological Interest. Public Health Service, National Institutes of Health, 1991
- **KABAT et al.** Sequences of Proteins of Immunological Interest,. NIH Publication, 1991, vol. 1-3, 91-3242
- **CHOTHIA ; LESK.** *J. Mol. Biol.,* 1987, vol. 196, 901-917
- **MACCALLUM et al.** J. Mol. Biol. 1996, vol. 262, 732-745
- **FLATMAN et al.** *J. Chromatogr. B,* 2007, vol. 848, 79-87
- **STADLER et al.** *Nature Medicine,* 12 June 2017
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHAPPELL et al.** *Nuclear Medicine and Biology,* 2000, vol. 27, 93-100
- **KRISSINEL ; HENRICK.** *J Mol Biol,* 2007, vol. 372 (3), 774-97
- **HUDSON et al.** *Nat. Med.,* 2003, vol. 9, 129-134
- The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **CAI ; BERGER.** *Antiviral Research,* 2011, vol. 90 (3), 143-50
- **MUFSON et al.** *Front. Biosci.,* 2006, vol. 11, 337-43
- **FRANKEL et al.** *Clin. Cancer Res.,* 2000, vol. 6, 326-334
- **KREITMAN et al.** *AAPS Journal,* 2006, vol. 8 (3), E532-E551
- **GOLDENBERG et al.** *Theranostics,* 2012, vol. 2, 5
- **SHARKEY et al.** *Cancer Res.,* 2003, vol. 63, 354-63
- **GOLD et al.** *Cancer Res.,* 2008, vol. 68, 4819-26
- **SHARKEY et al.** *Cancer Res.,* 2008, vol. 68, 5282-90
- **CUBAS et al.** *Biochim Biophys Acta,* 2009, vol. 1796, 309-14
- **COLOMA, M.J. et al.** *Nature Biotech,* 1997, vol. 15, 159-163
- **MORRISON, S.L.** *Nature Biotech,* 2007, vol. 25, 1233-1234
- **HOLLIGER, P. et al.** *Nature Biotech,* 2005, vol. 23, 1126-1136
- **FISCHER, N ; LEGER, 0.** *Pathobiology,* 2007, vol. 74, 3-14
- **SHEN, J. et al.** *Journal of Immunological Methods,* 2007, vol. 318, 65-74
- **WU, C. et al.** *Nature Biotech.,* 2007, vol. 25, 1290-1297
- **FISCHER, N ; LEGER, 0.** *Pathobiology,* 2007, vol. 74, 3-14
- **SCHAEFER et al.** *PNAS,* 2011, vol. 108, 1187-1191
- **KLEIN.** *MAbs,* 2016, vol. 8, 1010-20
- **RIDGWAY, J.B. et al.** *Protein Eng,* 1996, vol. 9, 617-621
- **MERCHANT, A.M. et al.** *Nat Biotechnol,* 1998, vol. 16, 677-681
- **MERCHANT, A.M. et al.** *Nature Biotech,* 1998, vol. 16, 677-681
- **ATWELL, S. et al.** *J. Mol. Biol.,* 1997, vol. 270, 26-35
- **CHOWDHURY.** *Methods Mol. Biol.,* 2008, vol. 207, 179-196
- **HOOGENBOOM et al.** Methods in Molecular Biology. Human Press, 2001, vol. 178, 1-37
- **CUNNINGHAM ; WELLS.** *Science,* 1989, vol. 244, 1081-1085
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492

- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GREVYS, A. et al.** *J. Immunol.,* 2015, vol. 194, 5497-5508
- **DUNCAN ; WINTER.** *Nature,* 1988, vol. 322, 738-40
- **KAM et al.** *Proc. Natl. Acad. Sci. USA,* 2005, vol. 102, 11600-11605
- **CHARLTON.** Methods in Molecular Biology. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** Methods in Molecular Biology. Humana Press, 2003, vol. 248, 255-268
- Epitope Mapping Protocols. **MORRIS.** Methods in Molecular Biology. Humana Press, 1996, vol. 66
- **HARLOW ; LANE.** Antibodies: A Laboratory Manual. Cold Spring Harbor Laboratory, 1988
- **CHEN et al.** *J. Mol. Biol.,* 1999, vol. 293, 865-881
- **PRESTA et al.** *Cancer Res.,* 1997, vol. 57, 4593-4599
- **HAENEL et al.** *Analytical Biochemistry,* 2005, vol. 339, 182-184
- **BEE et al.** *PloS One,* 2012, vol. 7 (4), e36261
- Remington's Pharmaceutical Sciences. 1980
- **LIGHTWOOD et al.** *J Immunol Methods,* 2006, vol. 316, 133-143
- **RS HAUN et al.** *Biotechniques,* 1992, vol. 13, 515-518
- **MZ LI et al.** *Nature Methods,* 2007, vol. 4, 251-256
- **SAMBROOK, J. et al.** Molecular cloning: A laboratory manual. Cold Spring Harbor Laboratory Press, 1989
- **MEISSNER, P. et al.** *Biotechnol. Bioeng.,* 2001, vol. 75, 197-203
- **MCCOY, A.J ; GROSSE-KUNSTLEVE, R.W. ; ADAMS, P.D. ; STORONI, L.C. ; READ, R.J.** *J. Appl. Cryst.,* 2007, vol. 40, 658-674
- **EMSLEY, P. ; LOHKAMP, B ; SCOTT, W.G. ; COWTAN, K.** *Acta Cryst D66,* 2010, 486-501
- **BOLCH WE ; ECKERMAN KF ; SGOUROS G ; THOMAS SR.** *J Nucl Med,* 2009, vol. 50, 477-484
- **SGOUROS G ; ROESKE JC ; MCDEVITT MR et al.** *J Nucl Med,* 2010, vol. 51, 311-328